# EUROPEAN PATENT APPLICATION

(11) **EP 2 338 888 A1**
(43) Date of publication of application: **29.06.2011**
(21) Application number: 09382303.7
(22) Date of filing: 24.12.2009
(51) Int. Cl.: C07D 471/04, A61K 31/437

(54) **Imidazopyridine derivatives as JAK inhibitors**

(71) Applicant: Almirall, S.A., 08022 Barcelona (ES)
(72) Inventor: Eastwood, Paul Robert, 08980, Sant Feliu de Llobregat (ES); Gonzalez Rodriguez, Jacob, 08980, Sant Feliu de Llobregat (ES); Bach Taña, Jordi, 08980, Sant Feliu de Llobregat (ES); Pages Santacana, Lluis Miquel, 08980, Sant Feliu de Llobregat (ES); Taltavull Moll, Joan, 08980, Sant Feliu de Llobregat (ES); Caturla Javaloyes, Juan Francisco, 08980, Sant Feliu de Llobrregat (ES)
(74) Representative: Elzaburu Marquez, Alberto

(57) **Abstract**

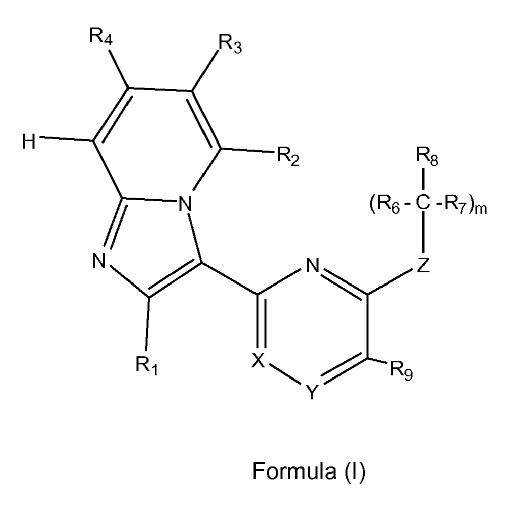

New imidazopyridine derivatives having the chemical structure of formula (I) are disclosed; as well as process for their preparation, pharmaceutical compositions comprising them and their use in therapy as inhibitors of Janus Kinases (JAK).

## Description

Cytokines have critical functions in regulating many aspects of immunity and inflammation, ranging from the development and differentiation of immune cells to the suppression of immune responses. Type I and type II cytokine receptors lack intrinsic enzymatic activity capable of mediating signal transduction, and thus require association with tyrosine kinases for this purpose. The JAK family of kinases comprises four different members, namely JAK1, JAK2, JAK3 and TYK2, which bind to type I and type II cytokine receptors for controlling signal transduction (Murray PJ, (2007). The JAK-STAT signalling pathway: input and output integration. J Immunol, 178: 2623). Each of the JAK kinases is selective for the receptors of certain cytokines. In this regard, JAK-deficient cell lines and mice have validated the essential role of each JAK protein in receptor signalling: JAK1 in class II cytokine receptors (IFN and IL-10 family), those sharing the gp130 chain (IL-6 family) and the common gamma chain (IL-2, IL-4, IL-7, IL-9, IL-15 and IL-21) (Rodig et at. (1998). Disruption of the JAK1 gene demonstrates obligatory and non-redundant roles of the JAKs in cytokine-induced biological response. Cell, 93:373; Guschin et at. (1995). A major role for the protein tyrosine kinase JAK1 in the JAK/STAT signal transduction pathway in response to interleukin-6. EMBO J. 14: 1421; Briscoe et at. (1996). Kinase-negative mutants of JAK1 can sustain interferon-gamma-inducible gene expression but not an antiviral state. EMBO J. 15:799); JAK2 in hematopoietic factors (Epo, Tpo, GM-CSF, IL-3, IL-5) and type II IFNs (Parganas et al., (1998). JAK2 is essential for signalling through a variety of cytokine receptors. Cell, 93:385); JAK3 in receptors sharing the common gamma chain (IL-2 family) (Park et al., (1995) Developmental defects of lymphoid cells in JAK3 kinase-deficient mice. Immunity, 3:771; Thomis et al., (1995) Defects in B lymphocyte maturation and T lymphocyte activation in mice lacking JAK3. Science, 270:794; Russell et al, (1995). Mutation of JAK3 in a patient with SCID: Essential role of JAK3 in lymphoid development. Science, 270:797); and Tyk2 in the receptors of IL-12, IL-23, IL-13 and type I IFNs (Karaghiosoff et al., (2000) Partial impairment of cytokine responses in Tyk2-deficient mice. Immunity, 13:549; Shimoda et al., (2000). Tyk2 plays a restricted role in IFNg signalling, although it is required for IL-12-mediated T cell function. Immunity, 13:561; Minegishi et al., (2006) Human Tyrosine kinase 2 deficiency reveals its requisite roles in multiple cytokine signals involved in innate and acquired immunity. Immunity, 25:745).

Receptor stimulation leads sequentially to JAK activation by phosphorylation, receptor phosphorylation, STAT protein recruitment and STAT activation and dimerization. The STAT dimer then functions as a transcription factor, translocating to the nucleus and activating the transcription of multiple response genes. There are seven STAT proteins identified: STAT1, STAT2, STAT3, STAT4, STAT5a, STAT5b and STAT6. Each particular cytokine receptor associates preferentially with a particular STAT protein. Some associations are independent of cell type (ex: IFNg- STAT1) while others may be cell type dependent (Murray PJ, (2007). The JAK-STAT signalling pathway: input and output integration. J Immunol, 178: 2623).

The phenotype of deficient mice has provided insights on the function of each JAK and the cytokine receptors signalling through them. JAK3 associates exclusively with the common gamma chain of the receptors for IL-2, IL-4, IL-7, IL-9, IL-15 and IL-21 cytokines. By virtue of this exclusive association, JAK3 knock out mice and common gamma chain deficient mice have an identical phenotype (Thomis et al., (1995) Defects in B lymphocyte maturation and T lymphocyte activation in mice lacking JAK3. Science, 270:794; DiSanto et at., (1995). Lymphoid development in mice with a targeted deletion of the interleukin 2 receptor gamma chain. PNAS, 92:377). Moreover, this phenotype is shared to a great extent with SCID patients that hold mutations/defects in the common gamma chain or JAK3 genes (O'Shea et al, (2004). JAK3 and the pathogenesis of severe combined immunodeficiency. Mol Immunol, 41: 727). JAK3 deficient mice are viable but display abnormal lymphopoiesis which leads to a reduced thymus size (10-100 fold smaller than wild type). JAK3-deficient peripheral T cells are unresponsive and have an activated/memory cell phenotype (Baird et al, (1998) T cell development and activation in JAK3-deficient mice. J. Leuk. Biol. 63: 669). The thymic defect in these mice strongly resembles that seen in IL-7 and IL-7 receptor knockout mice, suggesting that the absence of IL-7 signalling accounts for this defect in JAK3 -/-mice (von Freeden-Jeffry et al, (1995) Lymphopenia in Interleukin (IL)-7 Gene-deleted Mice Identifies IL-7 as a Nonredundant Cytokine. J Exp Med, 181:1519; Peschon et al, (1994). Early lymphocyte expansion is severely impaired in interleukin 7 receptor-deficient mice. J Exp Med, 180: 1955). These mice, like SCID humans, have no NK cells, probably due to the absence of IL-15 signalling, a survival factor for these cells. JAK3 knockout mice, unlike SCID patients, show deficient B cell lymphopoiesis while in human patients, B cells are present in circulation but are not responsive leading to hypoglobulinemia (O'Shea et al, (2004). JAK3 and the pathogenesis of severe combined immunodeficiency. Mol Immunol, 41: 727). This is explained by species-specific differences in IL-7 function in B and T cell development in mice and humans. On the other hand, Grossman et al. (1999. Dysregulated myelopoiesis in mice lacking JAK3. Blood, 94:932:939) have shown that the loss of JAK3 in the T-cell compartment drives the expansion of the myeloid lineages leading to dysregulated myelopoiesis. JAK2 deficient mice are embrionically lethal, due to the absence of definitive erythropoiesis. Myeloid progenitors fail to respond to Epo, Tpo, IL3 or GM-CSF, while G-CSF and IL-6 signallings are not affected. JAK2 is not required for the generation, amplification or functional differentiation of lymphoid progenitors (Parganas et al., (1998). JAK2 is essential for signalling through a variety of cytokine receptors. Cell, 93:385).

JAK1 deficient mice die perinatally due to a nursing defect. JAK1 binds exclusively to the gp130 chain shared by the IL6 cytokine family (i.e. LIF, CNTF, OSM, CT-1) and along with JAK3, is an essential component of the receptors sharing the common gamma chain, by binding to the non-shared receptor subunit. In this regard, JAK1 deficient mice show similar hematopoiesis defects as JAK3 deficient mice. In addition, they show defective responses to neurotrophic factors and to all interferons (class II cytokine receptors) (Rodig et al, (1998). Disruption of the JAK1 gene demonstrates obligatory and non-redundant roles of the Jaks in cytokine-induced biological response. Cell, 93:373).

Finally, Tyk2-deficient mice show an impaired response to IL-12 and IL-23 and only partially impaired to IFN-alpha (Karaghiosoff et al., (2000) Partial impairment of cytokine responses in Tyk2-deficient mice. Immunity, 13:549; Shimoda et al., (2000). Tyk2 plays a restricted role in IFNg signalling, although it is required for IL-12-mediated T cell function. Immunity, 13:561). However, human Tyk2 deficiency demonstrates that Tyk2 is involved in the signalling from IFN-α, IL6, IL10, IL12 and IL23 (Minegishi et al., (2006) Human Tyrosine kinase 2 deficiency reveals its requisite roles in multiple cytokine signals involved in innate and acquired immunity. Immunity, 25:745).

The role of JAK kinases in transducing the signal from a myriad of cytokines makes them potential targets for the treatment of diseases in which cytokines have a pathogenic role, such as inflammatory diseases, including but not limited to allergies and asthma, chronic obstructive pulmonary disease (COPD), psoriasis, autoimmune diseases such as rheumatoid arthritis, amyotrophic lateral sclerosis and multiple sclerosis, uveitis, transplant rejection, as well as in solid and hematologic malignancies such as myeloproliferative disorders, leukemia and lymphomas.

Inhibition of JAK kinases, especially JAK1 and JAK3, could give rise to potent immunosuppression which could be used therapeutically to prevent transplant rejection. In this regard, the JAK inhibitor CP-690,550 has shown efficacy in several animal models of transplantation (heretotopic heart transplantation in mice, cardiac allografts implanted in the ear of mice, renal allotransplantation in cynomolgous monkeys, aorta and tracheal transplantation in rats) by prolonging the mean survival time of grafts (West K (2009). CP-690550, a JAK3 inhibitor as an immunosuppressant for the treatment of rheumatoid arthritis, transplant rejection, psoriasis and other immune-mediated disorders. Curr. Op. Invest. Drugs 10: 491).

In rheumatoid joints, an imbalance between pro and anti-inflammatory cytokine activities favours the induction of autoimmunity, followed by chronic inflammation and tissue destruction. In this regard, the pathogenic role of IL-6 in rheumatoid arthritis (RA) has been validated clinically by the use of the anti-IL6R antibody tocilizumab. IL6 activates the transcription factor STAT3, through the use of JAK1 binding to the gp130 receptor chain (Heinrich et al., (2003) Principles of interleukin (IL)-6-type cytokine signalling and its regulation. Biochem J. 374: 1). Constitutive STAT3 mediates the abnormal growth and survival properties of RA synoviocytes (Ivashkiv and Hu (2003). The JAK/STAT pathway in rheumatoid arthritis: pathogenic or protective? Arth & Rheum. 48:2092). Other cytokines that have been implicated in the pathogenesis of arthritis include IL-12 and IL-23, implicated in Th1 and Th17 cell proliferation, respectively; IL-15, and GM-CSF (Mclnnes and Schett, (2007) Cytokines in the pathogenesis of rheumatoid arthritis. Nature Rew Immunol. 7:429). The receptors for these cytokines also utilize JAK proteins for signal transduction, making JAK inhibitors potential pleiotropic drugs in this pathology. Consequently, administration of several JAK inhibitors in animal models of murine collagen-induced arthritis and rat adjuvant-induced arthritis has been shown to reduce inflammation, and tissue destruction (Milici et al., (2008) Cartilage preservation by inhibition of Janus kinase 3 in two rodent models of rheumatoid arthritis. Arth. Res. 10:R14).

Inflammatory bowel disease (IBD) encloses two major forms of intestinal inflammation: ulcerative colitis and Crohn's disease. Growing evidence has shown that multiple cytokines, including interleukins and interferons, are involved in the pathogenesis of IBD (Strober et al, (2002). The immunology of mucosal models of inflammation. Annu Rev Immunol. 20: 495). Activation of the IL6/STAT3 cascade in lamina propia T cells has been shown to induce prolonged survival of pathogenic T cells (Atreya et al, (2000) Blockade of interleukin 6 trans signalling suppresses T-cell resistance against apoptosis in chronic intestinal inflammation: Evidence in Crohn disease and experimental colitis in vivo. Nature Med. 6:583). Specifically, STAT3 has been shown to be constitutively active in intestinal T cells of Crohn's disease patients and a JAK inhibitor has been shown to block the constitutive activation of STAT3 in these cells (Lovato et al, (2003) Constitutive STAT3 activation in intestinal T cells from patients with Crohn's disease. J Biol Chem. 278:16777). These observations indicate that the JAK-STAT pathway plays a pathogenic role in IBD and that a JAK inhibitor could be therapeutic in this setting.

Multiple sclerosis is an autoimmune demyelinating disease characterized by the formation of plaques in the white matter. The role of cytokines in the generation of multiple sclerosis has been long known. Potential therapies include blockade of IFN-g, IL-6, IL-12 and IL-23 (Steinman L. (2008) Nuanced roles of cytokines in three major human brain disorders. J Clin Invest. 118:3557), cytokines that signal through the JAK-STAT pathways. Use of tyrphostin, a JAK inhibitor, has been shown to inhibit IL-12 induced phosphorylation of STAT3, and to reduce the incidence and severity of active and passive experimental autoimmune encephalitis (EAE) (Bright et al., (1999) Tyrphostin B42 inhibits IL-12-induced tyrosine phosphorylation and activation of Janus kinase-2 and prevents experimental allergic encephalomyelitis. J Immunol. 162:6255). Another multikinase inhibitor, CEP701, has been shown to reduce secretion of TNF-alpha, IL6 and IL23 as well as the levels of phospho-Stat1, Stat3, and Stat5 in peripheral DCs of mice with EAE, significantly improving the clinical course of EAE in mice (Skarica et al, (2009). Signal transduction inhibition of APCs diminishes Th17 and Th1 responses in experimental autoimmune encephalomyelitis. J. Immunol. 182:4192.).

Psoriasis is an inflammatory skin disease which involves a process of immune cell infiltration and activation that culminates in epithelial remodelling. The current theory behind the cause of psoriasis states the existence of a cytokine network that governs the interaction between immune and epithelial cells (Nickoloff BJ. (2007). Cracking the cytokine code in psoriasis, Nat Med, 13:242). In this regard, IL-23 produced by dendritic cells is found elevated in psoriatic skin, along with IL-12. IL-23 induces the formation of Th17 cells which in turn produce IL-17 and IL-22, the latter being responsible for epidermis thickening. IL-23 and IL-22 induce the phosphorylation of STAT-3, which is found abundantly in psoriatic skin. JAK inhibitors may thus be therapeutic in this setting. In accordance, a JAK1/3 inhibitor, R348, has been found to attenuate psoriasiform skin inflammation in a spontaneous T cell dependent mouse model of psoriasis (Chang et al., (2009) JAK3 inhibition significantly attenuates psoriasiform skin inflammation on CD18 mutant PL/J mice. J Immunol. 183:2183). Th2 cytokine-driven diseases such as allergy and asthma could also be a target of JAK inhibitors. IL-4 promotes Th2 differentiation, regulates B-cell function and immunoglobulin class switching, regulates eotaxin production, induces expression of IgE receptor and MHC II on B cells, and stimulates mast cells. Other Th2 cytokines like IL5 and IL13 can also contribute to eosinophil recruitment in bronchoalveolar lavage by stimulating eotaxin production. Pharmacological inhibition of JAK has been shown to reduce the expression of IgE receptor and MHCII induced by IL-4 stimulation on B cells (Kudlacz et al., (2008). The JAK3 inhibitor CP-690,550 is a potent anti-inflammatory agent in a murine model of pulmonary eosinophilia. European J. Pharm. 582: 154). Furthermore, JAK3-deficient mice display poor eosinophil recruitment and mucus secretion to the airway lumen upon OVA challenge, as compared to wild type mice (Malaviya et al, (2000) Treatment of allergic asthma by targeting Janus kinase 3-dependent leukotriene synthesis in mast cells with 4-(3', 5'- dibromo-4'-hydroxyphenyl)amino-6,7-dimethoxyquinazoline (WHI-P97). JPET 295:912.). In this regard, systemic administration of the CP-690550 JAK inhibitor in mice has been shown to reduce the eosinophil count as well as the levels of eotaxin and IL13 in BAL in a murine model of pulmonary eosinophilia (Kudlacz et al., (2008). The JAK3 inhibitor CP-690,550 is a potent anti-inflammatory agent in a murine model of pulmonary eosinophilia. European J. Pharm. 582:154).

There is increasing evidence that cytokines play a pathogenetic role in ocular inflammatory disease such as uveitis or dry eye syndrome. Some cytokines implicated in experimental autoimmune uveitis, such as IL-2, IL-6, IL-12 and IFNg, would be amenable to JAK inhibition (Vallochi et al, (2007) The role of cytokines in the regulation of ocular autoimmune inflammation. Cytok Growth Factors Rev. 18:135). In this regard, drugs or biologicals that interfere with IL2 signalling such as cyclosporine or anti-IL2 receptor antibody (daclizumab) have shown efficacy in the treatment of keratoconjuctivitis sicca and refractory uveitis, respectively (Lim et al, (2006). Biologic therapies for inflammatory eye disease. Clin Exp Opht 34:365). Similarly, allergic conjunctivitis, a common allergic eye disease characterized by conjuctival congestion, mast cell activation and eosinophil infiltration, could benefit from JAK inhibition. STAT6-deficient mice, showing decreased TH2-mediated immune responses which are normally triggered by IL-4, do not develop the classical early and late phase responses, suggesting that IL-4 pathway abrogation through JAK inhibition may be therapeutic in this setting (Ozaki et al, (2005) The control of allergic conjuntivitis by suppressor of cytokine signalling (SOCS)3 and SOCS5 in a murine model. J Immunol, 175:5489). There is growing evidence of the critical role of STAT3 activity in processes involved in tumorigenesis like cell cycle dysregulation, promotion of uncontrolled growth, induction of survival factors and inhibition of apoptosis (Siddiquee et al., (2008) STAT3 as a target for inducing apoptosis in solid and haematological tumors. Cell Res. 18: 254). Antagonism of STAT3 by means of dominant-negative mutants or antisense oligonucleotides has shown to promote apoptosis of cancer cells, inhibition of angiogenesis and up-regulation of host immunocompetence. Inhibition of constitutively active STAT3 in human tumors by means of JAK inhibitors may provide a therapeutic option to the treatment of this disease. In this regard, the use of the JAK inhibitor tyrphostin has been shown to induce apoptosis of malignant cells and inhibit cell proliferation in vitro and in vivo (Meydan et al., (1996). Inhibition of acute lymphoblastic leukemia by a JAK-2 inhibitor. Nature, 379:645).

Hematological malignancies with dysregulated JAK-STAT pathways may benefit from JAK inhibition. Recent studies have implicated dysregulation of JAK2 kinase activity by chromosomal translocations and mutations within the pseudokinase domain (such as the JAK2V617F mutation) in a spectrum of myeloproliferative diseases (Ihle and Gililand, 2007), including polycythemia vera, mielofibrosis and essential thrombocythemia. In this regard, several JAK inhibitors that tackle JAK2 potently, such as TG-101209 (Pardanani et al., (2007). TG101209, a small molecular JAK2-selective inhibitor potently inhibits myeloproliferative disorder-associated JAK2V617F and MPLW515L/K mutations Leukemia. 21:1658-68), TG101348 (Wernig et al, (2008). Efficacy of TG101348, a selective JAK2 inhibitor, in treatment of a murine model of JAK2V617F-induced polycythemia vera. Cancer Cell, 13: 311), CEP701, (Hexner et al, (2008). Lestaurtinib (CEP701) is a JAK2 inhibitor that suppresses JAK2/STAT5 signalling and the proliferation of primary erythroid cells from patients with myeloproliferative disorders. Blood, 111: 5663), CP-690,550 (Manshouri et al, (2008) The JAk kinase inhibitor CP-690-550 suppresses the growth of human polycythemia vera cells carrying the JAK2V617F mutation. Cancer Sci, 99:1265), and CYT387 (Pardanani et al., (2009) CYT387, a selective JAK1/JAK2 inhibitor: in-vitro assessment of kinase selectivity and preclinical studies using cell lines and primary cells from polycythemia vera patients. Leukemia, 23:1441) have been proposed for treating myeloproliferative diseases on the basis of their antiproliferative activity on cells carrying the JAK2V617F mutation. Similarly, T-cell leukemia due to human T-cell leukemia virus (HTLV-1) transformation is associated with JAK3 and STAT5 constitutive activation (Migone et al, (1995) Constitutively activated Jak-STAT pathway in T cells transformed with HTLV-I. Science, 269: 79) and JAK inhibitors may be therapeutic in this setting (Tomita et al, (2006). Inhibition of constitutively active Jak-Stat pathway suppresses cell growth of human T-cell leukemia virus type I-infected T cell lines and primary adult T-cell leukemia cells. Retrovirology, 3:22). JAK1 activating mutations have also been identified in adult acute lymphoblastic leukemia of T cell origin (Flex et al, (2008) Somatically acquired JAK1 mutations in adult acute lymphoblastic leukemia. J. Exp. Med. 205:751-8) pointing to this kinase as a target for the development of novel antileukemic drugs.

Conditions in which targeting of the JAK pathway or modulation of the JAK kinases, particularly JAK1, JAK2 and JAK3 kinases are contemplated to be therapeutically useful for the treatment or prevention of diseases include: neoplastic diseases (e.g. leukemia, lymphomas, solid tumors); transplant rejection, bone marrow transplant applications (e.g., graft- versus-host disease); autoimmune diseases (e.g. diabetes, multiple sclerosis, rheumatoid arthritis, inflammatory bowel disease); respiratory inflammation diseases (e.g. asthma, chronic obstructive pulmonary disease), inflammation-linked ocular diseases or allergic eye diseases (e.g. dry eye, glaucoma, uveitis, diabetic retinopathy, allergic conjunctivitis or age-related macular degeneration) and skin inflammatory diseases (e.g., atopic dermatitis or psoriasis).

In view of the numerous conditions that are contemplated to benefit by treatment involving modulation of the JAK pathway or of the JAK kinases it is immediately apparent that new compounds that modulate JAK pathways and use of these compounds should provide substantial therapeutic benefits to a wide variety of patients.

Provided herein are novel imidazopyridine compounds for use in the treatment of conditions in which targeting of the JAK pathway or inhibition of JAK kinases can be therapeutically useful.

The compounds described in the present invention are simultaneously potent JAK1, JAK2 and JAK3 inhibitors, i.e. pan-JAK inhibitors. This property makes them useful for the treatment or prevention of pathological conditions or diseases such as myeloproliferative disorders (such as polycythemia vera, essential thrombocythemia or mielofibrosis), leukemia, lymphomas and solid tumors; bone marrow and organ transplant rejection; or immune-mediated diseases such as autoimmune and inflammation diseases, including rheumatoid arthritis, multiple sclerosis, inflammatory bowel disease (such as ulcerative colitis or Crohn's disease), inflammation-linked ocular diseases or allergic eye diseases (such as dry eye, uveitis, or allergic conjunctivitis), allergic rhinitis, asthma, chronic obstructive pulmonary disease (COPD), and skin inflammatory diseases (such as atopic dermatitis or psoriasis).

It has now been found that certain imidazopyridine derivatives are novel and potent JAK inhibitors and can therefore be used in the treatment or prevention of these diseases.

Thus the present invention is directed to new imidazopyridine derivatives of formula (I), or a pharmaceutically acceptable salt, or solvate, or N-oxide or stereoisomer thereof: 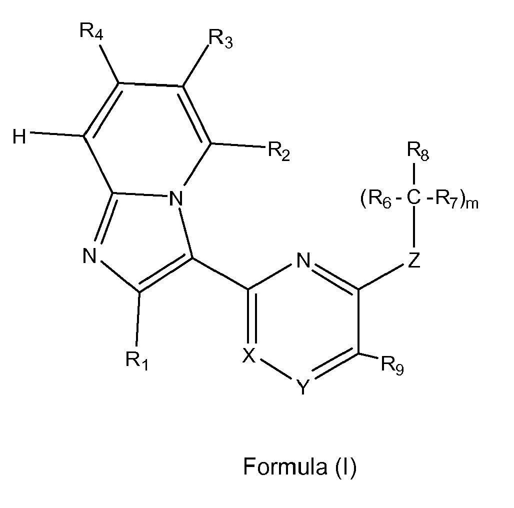 wherein,
m is 0 or an integer from 1 to 3;
Z is an oxygen atom or a group NR₅;
X and Y independently represent a nitrogen atom or a -CR₉ group, with wherein at least one of X and Y represents a nitrogen atom;
R₁, R₂, R₃, R₄ and R₉ each independently represent a hydrogen atom, a halogen atom, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group, a C₃-C₁₀ cycloalkyl group, a C₃-C₁₀ cycloalkenyl group, a monocyclic or polycyclic C₅-C₁₄ aryl group, a 5- to 14- membered heteroaryl group containing at least one heteroatom selected from O, S and N, a 5- to 14- membered heterocyclyl group containing at least one heteroatom selected from O, S and N, a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N, an aza-bicycloalkyl group having up to 12 carbon atoms or an aza-bicycloalkenyl group having up to 12 carbon atoms, wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from substituents Ra, and the alkyl groups are unsubstituted or substituted with one or more substituents selected from Rb;
or R₁, R₂, R₃, R₄ and R₉ independently represent a -SR₁₃ group, a -SOR₁₃ group, a -S(O)₂R₁₃ group, a -S(O)₂NR₁₃R₁₄ group, a -NR₁₃S(O)₂R₁₄ group, a -NR₁₃S(O)₂NR₁₄ group, a -OR₁₃ group, a -C(O)OR₁₃ group, a -O-C(O)R₁₃ group, a -C(O)-(CH₂)ₙ-R₁₃ group, a -NR₁₃R₁₄ group, a -C(O)-(CH₂)ₙ-NR₁₃R₁₄ group, a -NR₁₃C(O)-(CH₂)ₙ-R₁₄ group or a -NR₁₃C(O)-(CH₂)ₙ-NR₁₄R₁₅ group, wherein each n is 0, 1 or 2;
or in the case when two adjacent -CR₉ groups are present, two adjacent -CR₉ groups and the carbon atoms to which they are bonded optionally form a C₅-C₁₂ aryl group or a 4- to 12-membered heteroaryl, cycloalkyl or heterocyclyl group, said heteroaryl and heterocyclyl groups containing at least one heteroatom selected from O, S and N, the aryl, heteroaryl, cycloalkyl and heterocyclyl groups being unsubstituted or substituted by one or more substituents selected from a halogen atom, a linear or branched C₁-C₆ alkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N, wherein the alkyl, the aryl, the heteroaryl and the heterocyclyl substituents are unsubstituted or substituted by one or more substituents selected from a halogen atom, a hydroxy group, a cyano group, a linear or branched C₁-C₆ alkyl group or a C₁-C₄ haloalkyl group;
R₅ represents a hydrogen atom, a linear or branched C₁-C₆ alkyl group optionally substituted by one or more substituents selected from a hydroxy group, a cyano group, a C₁-C₄ haloalkyl group, a C₁-C₄ hydroxyalkyl group, a C₃-C₁₀ cycloalkyl group, a phenyl group or a 6 membered, saturated N-containing heterocyclyl ring, or R₅ represents a -S(O)₂R₁₀ group, a -S(O)₂NR₁₀R₁₁ group, a -C(O)OR₁₀ group, a -C(O)-(CH₂)ₙ-R₁₀ group, or a -C(O)-(CH₂)ₙ-NR₁₀R₁₁ group;
R₆ and R₇ each independently represents a hydrogen atom or a linear or branched C₁-C₆ alkyl group optionally substituted by one or more substituents selected from a hydroxy group, a cyano group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring;
R₈ represents a hydrogen atom, a halogen atom, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group, a C₃-C₁₀ cycloalkyl group, a C₃-C₁₀ cycloalkenyl group, a monocyclic or polycyclic C₅-C₁₄ aryl group, a 5- to 14- membered heteroaryl group containing at least one heteroatom selected from O, S and N, a 5- to 14-membered heterocyclyl group containing at least one heteroatom selected from O, S and N, a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N, an aza-bicycloalkyl group having up to 12 carbon atoms or a aza-bicycloalkenyl group having up to 12 carbon atoms, wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from Ra, -(C₁-C₄ alkyl)-CN groups, or -(C₁-C₄ alkyl)-C(O)NR'R" groups wherein R' and R" are the same or different and are selected from hydrogen atoms and linear or branched C₁-C₄ alkyl groups; and the alkyl groups are unsubstituted or substituted by one or more substituents selected from Rb;
or R₈ represents a -SR₁₃ group, a -SOR₁₃ group, a -S(O)₂R₁₃ group, a -S(O)₂NR₁₃R₁₄ group, a -NR₁₃S(O)₂R₁₄ group, a -NR₁₃S(O)₂NR₁₄ group, a -OR₁₃ group, a -C(O)OR₁₃ group, a -O-C(O)R₁₃ group, a -C(O)-(CH₂)ₙ-R₁₃ group, a -NR₁₃R₁₄ group,
a -C(O)-(CH₂)ₙ-NR₁₃R₁₄ group; a -NR₁₃C(0)-(CH₂)ₙ-R₁₄ group, or a -NR₁₃C(O)-(CH₂)ₙ-NR₁₄R₁₅ group, wherein n is 0, 1 or 2,
or R₈ together with R₅ and the nitrogen atom to which R₅ is bonded form a 4- to 10-membered, saturated heterocyclyl group, which contains, as heteroatoms, one or two nitrogen atoms and which is substituted by a linear or branched C₁-C₆ alkyl group, a monocyclic or polycyclic C₅-C₁₄ aryl group, a 5- to 14- membered heteroaryl group containing at least one heteroatom selected from O, S and N, a 5- to 14- membered heterocyclyl group containing at least one heteroatom selected from O, S and N,
a -SOR₁₀ group, a -C(O)-(CH₂)ₙ-R₁₀ group, or a -C(O)-(CH₂)ₙ-NR₁₀R₁₁ group, wherein each n is 0, 1 or 2, wherein the alkyl, aryl, heteroaryl and heterocyclyl groups are unsubstituted or substituted by one or more substituents selected from a halogen atom, a hydroxy group, a cyano group, a linear or branched C₁-C₆ alkyl group or a C₁-C₄ haloalkyl group, and wherein the alkyl groups are unsubstituted or substituted by one or more substituents selected from a halogen atom, a hydroxy group, a cyano group or a C₁-C₄ haloalkyl group;
provided that when m is zero, R₈ is other than a -SR₁₃ group, a -SOR₁₃ group, a -S(O)₂R₁₃ group, a -S(O)₂NR₁₃R₁₄ group, a -NR₁₃S(O)₂R₁₄ group, a -NR₁₃S(O)₂NR₁₄ group, a -OR₁₃ group, a -O-C(O)R₁₃ group, a -NR₁₃R₁₄ group, a -NR₁₃C(O)-(CH₂)ₙ-R₁₄ group, or a -NR₁₃C(O)-(CH₂)ₙ-NR₁₄R₁₅ group,
wherein
Ra is a halogen atom, a cyano group, a hydroxy group, a linear or branched C₁-C₆ alkyl group, a C₁-C₄ haloalkyl group, a C₁-C₄ hydroxyalkyl group, a C₃-C₇ cycloalkyl or a C₃-C₇ cycloalkenyl group, a monocyclic or polycyclic C₅-C₁₄ aryl group, which aryl group is unsubstituted or substituted by one or more halogen atoms, a 5- to 14- membered heteroaryl group containing at least one heteroatom selected from O, S and N, a 5- to 14- membered heterocyclyl group containing at least one heteroatom selected from O, S and N, a -SR₁₀ group, a -SOR₁₀ group, a -S(O)₂R₁₀ group, a -S(O)NR₁₀R₁₁ group, a -NR₁₀S(O)₂R₁₁ group, a -NR₁₀S(O)₂NR₁₁ group, a -OR₁₀ group, a -C(O)OR₁₀ group, a -O-C(O)R₁₀ group, a -C(O)-(CH₂)ₙ-R₁₀ group, a -NR₁₀R₁₁ group, a -C(O)-(CH₂)ₙ-NR₁₀R₁₁ group, a -NR₁₀C(O)-(CH₂)ₙ-R₁₁ group or a -NR₁₀C(O)-(CH₂)ₙ-NR₁₁R₁₂ group, wherein each n is 0, 1 or 2;
Rb is a cyano group, a C₁-C₄ haloalkyl group, a C₁-C₄ hydroxyalkyl group, a C₃-C₇ cycloalkyl or a C₃-C₇ cycloalkenyl group, a monocyclic or polycyclic C₅-C₁₄ aryl group, which aryl group is unsubstituted or substituted by one or more halogen atoms, a 5- to 14- membered heteroaryl group containing at least one heteroatom selected from O, S and N, a 5- to 14- membered heterocyclyl group containing at least one heteroatom selected from O, S and N, a -SR₁₀ group, a -SOR₁₀ group, a -S(O)₂R₁₀ group,
a -S(O)NR₁₀R₁₁ group, a -NR₁₀S(O)₂R₁₁ group, a -NR₁₀S(O)₂NR₁₁ group, a -OR₁₀ group, a -C(O)OR₁₀ group, a -O-C(O)R₁₀ group, a -C(O)-(CH₂)ₙ-R₁₀ group, a -NR₁₀R₁₁ group, a -C(O)-(CH₂)ₙ-NR₁₀R₁₁ group, a -NR₁₀C(O)-(CH₂)ₙ-R₁₁ group or a -NR₁₀C(O)-(CH₂)ₙ-NR₁₁R₁₂ group, wherein n is 0, 1 or 2;
R₁₀, R₁₁ and R₁₂ each independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, a bicyclyl group containing a monocyclic C₅-C₆ aryl or heteroaryl group bonded directly to a 5- to 6- membered cycloalkyl or heterocyclyl group which heteroaryl or heterocyclyl group contains 1, 2 or 3 nitrogen atoms, the haloalkyl, hydroxyalkyl, alkoxycarbonyl, cycloalkyl, phenyl, heteroaryl, heterocyclyl and bicyclyl group being unsubstituted or substituted by one or more substituents selected from substituents Rc, and the alkyl groups being unsubstituted or substituted with one or more substituents selected from substituents Rd;
Rc is a halogen atom, a hydroxy group, a cyano group, a linear or branched C₁-C₆ alkyl group, a C₁-C₄ haloalkyl group, a C₁-C₄ alkoxy group, a C₁-C₄ hydroxyalkyl group, a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃-C₇ heterocycloalkyl ketone group containing 1, 2 or 3 nitrogen atoms, said phenyl group being unsubstituted or substituted with one or more halogen atoms, and said heteroaryl, heterocyclyl and heterocycloalkyl ketone groups being unsubstituted or substituted by one or more linear or branched C₁-C₃ alkyl groups;
Rd is a cyano group, a C₁-C₄ haloalkyl group, a C₁-C₄ alkoxy group, a C₁-C₄ hydroxyalkyl group, a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃-C₇ heterocycloalkyl ketone group containing 1, 2 or 3 nitrogen atoms, said phenyl group being unsubstituted or substituted with one or more halogen atoms, and said heteroaryl, heterocyclyl and heterocycloalkyl ketone groups being unsubstituted or substituted by one or more linear or branched C₁-C₃ alkyl groups;
R₁₃, R₁₄, and R₁₅ each independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N, wherein the haloalkyl, hydroxyalkyl, alkoxycarbonyl, cycloalkyl, aryl, heteroaryl and heterocyclyl groups are unsubstituted or substituted by one or more substituents selected from the substituents Ra, and the alkyl groups are optionally substituted by one or more substitutents selected from Rb.

The invention further provides synthetic processes and intermediates described herein, which are useful for preparing said compounds.

The invention also provides a pharmaceutical composition comprising the compounds of the invention and a pharmaceutically-acceptable diluent or carrier.

The invention is also directed to the compounds of the invention as described herein, for use in the treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention also provides a method of treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis; comprising administering a therapeutically effective amount of the compounds of the invention or a pharmaceutical composition of the invention to a subject in need of such treatment.

The invention also provides a combination product comprising (i) the compounds of the invention as described herein; and (ii) one or more additional active substances which are known to be useful in the treatment of myeloproliferative disorders (such as polycythemia vera, essential thrombocythemia or mielofibrosis), leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease (such as ulcerative colitis or Crohn's disease), dry eye, uveitis, allergic conjunctivitis, allergic rhinitis, asthma, chronic obstructive pulmonary disease (COPD), atopic dermatitis and psoriasis.

As used herein the term C₁-C₆ alkyl embraces optionally substituted, linear or branched radicals having 1 to 6 carbon atoms, preferably 1 to 4 carbon atoms. Examples include methyl, ethyl, n-propyl, i-propyl, n-butyl, sec-butyl, t-butyl, n-pentyl, 1-methylbutyl, 2-methylbutyl, isopentyl, 1-ethylpropyl, 1,1-dimethylpropyl, 1,2-dimethylpropyl, n-hexyl, 1-ethylbutyl, 2-ethylbutyl-1,1-dimethylbutyl, 1,2-dimethylbutyl, 1,3-dimethylbutyl, 2,2-dimethylbutyl, 2,3-dimethylbutyl, 2-methylpentyl, 3-methylpentyl and iso-hexyl radicals.

As used herein, the term C₂-C₄ alkenyl embraces optionally substituted, linear or branched, mono or polyunsaturated radicals having 2 to 4 carbon atoms. Examples include vinyl, allyl, 1-propenyl, isopropenyl, 1-butenyl, 2-butenyl and 3-butenyl radicals.

As used herein, the term C₂-C₄ alkynyl embraces optionally substituted, linear or branched, mono or polyunsaturated radicals having 2 to 4 carbon atoms. Examples include 1-propynyl, 2-propynyl, 1-butynyl, 2-butynyl and 3-butynyl radicals.

When it is mentioned that alkyl, alkenyl or alkynyl radicals may be optionally substituted it is meant to include linear or branched alkyl, alkenyl or alkynyl radicals as defined above, which may be unsubstituted or substituted in any position by one or more substituents, for example by 1, 2 or 3 substituents. When two or more substituents are present, each substituent may be the same or different.

A said optionally substituted alkenyl group is typically unsubstituted or substituted with 1, 2 or 3 substituents which may be the same or different. Typically, substituents on an alkenyl group are themselves unsubstituted. Preferred substituents on the alkenyl groups are halogen atoms and hydroxy groups, and are more preferably halogen atoms.

A said optionally substituted alkynyl group is typically unsubstituted or substituted with 1, 2 or 3 substituents which may be the same or different. Typically, substituents on an alkynyl group are themselves unsubstituted. Preferred substituents on the alkynyl groups are halogen atoms and hydroxy groups, and are more preferably halogen atoms

As used herein, the term C₁-C₄ haloalkyl group is an alkyl group, for example a C₁₋₄ or C₁₋₂ alkyl group, which is bonded to one or more, preferably 1, 2 or 3 halogen atoms. Preferably, said haloakyl group is chosen from -CCl₃ and -CF₃.

As used herein, the term C₁-C₄ hydroxyalkyl embraces linear or branched alkyl radicals having 1 to 4 carbon atoms, any one of which may be substituted with one or more, preferably 1 or 2, more preferably 1 hydroxyl radicals. Examples of such radicals include hydroxymethyl, hydroxyethyl, hydroxypropyl, and hydroxybutyl.

As used herein, the term C₁-C₄ alkoxy (or alkyloxy) embraces optionally substituted, linear or branched oxy-containing radicals each having alkyl portions of 1 to 4 carbon atoms. An alkoxy group is typically unsubstituted or substituted with 1, 2 or 3 substituents which may be the same or different. Typically, the substituents on an alkoxy group are themselves unsubstituted. Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, a C₁-C₄ alkoxycarbonyl group is typically a said C₁-C₄ alkoxy group bonded to a carbonyl group.

As used herein, the term C₃-C₁₀ cycloalkyl embraces saturated monocyclic or polycyclic carbocyclic radicals having from 3 to 10 carbon atoms, preferably from 3 to 7 carbon atoms. A C₃-C₁₀ cycloalkyl radical is typically unsubstituted or substituted with 1, 2 or 3 substituents which may be the same or different. When a C₃-C₁₀ cycloalkyl radical carries 2 or more substituents, the substituents may be the same or different. Typically the substituents on a C₃-C₁₀ cycloalkyl group are themselves unsubstituted. Polycyclic cycloalkyl radicals contains two or more fused cycloalkyl groups, preferably two cycloalkyl groups. Typically, polycyclic cycloalkyl radicals are selected from decahydronaphthyl (decalyl), bicyclo[2.2.2]octyl, adamantly, camphyl or bornyl groups. Examples of monocyclic cyclocalkyl groups include cyclopropyl, cyclobutyl, cyclopentyl, cyclohexyl, cycloheptyl, cyclooctyl, cyclononyl and cyclodecyl.

As used herein, the term C₃-C₁₀ cycloalkenyl embraces partially unsaturated carbocyclic radicals having from 3 to 10 carbon atoms, preferably from 3 to 7 carbon atoms. A C₃-C₁₀ cycloalkenyl radical is typically unsubstituted or substituted with 1, 2 or 3 substituents which may be the same or different. When a C₃-C₁₀ cycloalkenyl radical carries 2 or more substituents, the substituents may be the same or different. Typically, the substituents on a cycloalkenyl group are themselves unsubstituted.
Examples include cyclobutenyl, cyclopentenyl, cyclohexenyl, cycloheptenyl, cyclooctenyl, cyclononenyl and cyclodecenyl.

As used herein, the term C₅-C₁₄ aryl radical embraces typically a C₅-C₁₄, preferably C₆-C₁₄, more preferably C₆-C₁₀ monocyclic or polycyclic aryl radical such as phenyl, naphthyl, anthranyl and phenanthryl. Phenyl is preferred. A said optionally substituted C₅-C₁₄ aryl radical is typically unsubstituted or substituted with 1, 2 or 3 substituents which may be the same or different. When a C₅₋₁₀ aryl radical carries 2 or more substituents, the substituents may be the same or different. Unless otherwise specified, the substituents on a C₅-C₁₄ aryl group are typically themselves unsubstituted.

As used herein, the term 5- to 14- membered heteroaryl radical embraces typically a 5-to 14- membered ring system, preferably a 5- to 10- membered ring system, more preferably a 5- to 6- membered ring system, comprising at least one heteroaromatic ring and containing at least one heteroatom selected from O, S and N. A 5- to 14-membered heteroaryl radical may be a single ring or two or more fused rings wherein at least one ring contains a heteroatom.

A said optionally substituted 5- to 14- membered heteroaryl radical is typically unsubstituted or substituted with 1, 2 or 3 substituents which may be the same or different. When a 5- to 14- membered heteroaryl radical carries 2 or more substituents, the substituents may be the same or different. Unless otherwise specified, the substituents on a 5- to 14- membered heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1 H-pyrazolo[3,4-d]pyrimidinyl, thieno[2,3-d] pyrimidnyl and the various pyrrolopyridyl radicals.

As used herein, the term 5- to 14-membered heterocyclyl radical embraces typically a non-aromatic, saturated or unsaturated C₅-C₁₄ carbocyclic ring system, preferably C₅-C₁₀ carbocyclic ring system, more preferably C₅-C₆ carbocyclic ring system, in which one or more, for example 1, 2, 3 or 4 of the carbon atoms preferably 1 or 2 of the carbon atoms are replaced by a heteroatom selected from N, O and S. A heterocyclyl radical may be a single ring or two or more fused rings wherein at least one ring contains a heteroatom. When a 5 to 14-membered heterocyclyl radical carries 2 or more substituents, the substituents may be the same or different.

A said optionally substituted 5- to 14-membered heterocyclyl radical is typically unsubstituted or substituted with 1, 2 or 3 substituents which may be the same or different. Typically, the substituents on a 5 to 14-membered heterocyclyl radical are themselves unsubstituted.

Examples of 5- to 14-membered heterocyclyl radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, 4,5-dihydro-oxazolyl, 2-benzofuran-1 (3H)-one, 1,3-dioxol-2-one and 3-aza-tetrahydrofuranyl.

Where a 5- to 14-membered heterocyclyl radical carries 2 or more substituents, the substituents may be the same or different.

As used herein, the term 6-membered saturated N-containing heterocyclic group is a C₆ saturated carbocyclic ring system in which one of the carbon atoms is replaced by N and optionally in which 1, 2, or 3, preferably 1 or 2, further carbon atoms are replaced by heteroatoms selected from N and O.

A said 6-membered saturated N-containing heterocyclic group is typically unsubstituted or substituted with 1, 2 or 3 substituents which may be the same or different. Typically, the substituents on a 6-membered saturated N-containing heterocyclic group are themselves unsubstituted, unless otherwise specified.

Examples of 6-membered saturated N-containing heterocyclic group include piperidyl and piperazinyl.

As used herein, the term C₃-C₇ heterocycloalkyl ketone group embraces typically a non-aromatic, saturated or unsaturated C₃-C₇ carbocyclic ring system, in which one of the carbon atoms is replaced by a C=O group and 1, 2 or 3, preferably 1 or 2, more preferably 1, further carbon atoms preferably are replaced by N. Examples include pyridone groups.

As used herein, the term aza-bicycloalkyl group having up to 12 carbon atbms denotes a fused ring system consisting of a cycloalkyl group and a N-containing heterocyclyl group, as defined herein

As used herein, the term aza-bicycloalkenyl group having up to 12 carbon atoms embraces an aza-bicycloalkyl group, as defined herein, containing at least one unsaturated carbon-carbon bond.

As used herein, a bicyclyl group containing a monocyclic C₅-C₉ aryl or heteroaryl group bonded directly to a 5- to 9- membered cycloalkyl or heterocyclyl group typically refers to groups where a monocyclic C₅-C₉ aryl or heteroaryl group is bonded to a 5- to 9-membered cycloalkyl or heterocyclyl group by a single bond. Examples include biphenyl groups or chromanyl groups.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

Typically when a cyclic radical is bridged by an alkylene or alkylenedioxy radical, the bridging alkylene radical is bonded to the ring at non-adjacent atoms.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclyl amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

Typically, R₁, R₂ and R₄ are the same or different and each represent a hydrogen atom, a halogen atom, a hydroxyl group, a cyano group, a linear or branched C₁-C₆ alkyl group, a C₁-C₄ haloalkyl group or C₁-C₄ hydroxyalkyl group;
R₃ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group, a C₃-C₁₀ cycloalkyl group, a 5- to 10- membered heterocyclyl group, a C₆-C₁₀ aryl group, a 5- to 10- membered heteroaryl group, a -C(O)OR' group or a -C(O)NR"R"' group, which cycloalkyl, heterocyclyl, aryl and heteroaryl groups are unsubstituted or substituted with one or more halogen atoms or a linear or branched C₁-C₆ alkyl group, a hydroxy group, a cyano group, or a C₁-C₄ alkoxy group; wherein R', R" and R'" are the same or different and each represents a hydrogen atom, a linear or branched C₁-C₆ alkyl group, a C₁-C₄ haloalkyl group or C₁-C₄ hydroxyalkyl group;
R₅ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group or a -(C₁-C₄alkyl)-(C₃-C₇ cycloalkyl) group, or R₅ together with R₈ and the nitrogen atom to which R₅ is bonded form a 5- to 9- membered, saturated heterocyclyl group, which contains, as heteroatoms, one or two nitrogen atoms and which heterocyclyl ring is unsubstituted or substituted with a -C(O)-(CH₂)ₙ-R' group or a -C(O)-(CH₂)ₙ-NR"R"' group, wherein n is 0, 1 or 2, R' represents a hydrogen atom, or a linear or branched C₁-C₆ alkyl group, a C₁-C₄ haloalkyl group, a C₁-C₄ hydroxyalkyl group, or a cyano group and R" and R"' are the same or different and each represents a hydrogen atom, a linear or branched C₁-C₆ alkyl group, a C₁-C₄ haloalkyl group, or C₁-C₄ hydroxyalkyl group;
R₆ and R₇ are the same or different and each represent a hydrogen atom, a linear or branched C₁-C₆ alkyl group, or a C₁-C₄ haloalkyl group or a C₁-C₄ hydroxyalkyl group;
R₉ is a hydrogen atom, a halogen atom, a hydroxyl group, a cyano group, a linear or branched C₁-C₆ alkyl group, a C₁-C₄ haloalkyl group, a C₁-C₄ hydroxyalkyl group, a 5-to 10- membered heterocyclyl group, or a 5- to 10- membered heteroaryl group, which heterocyclyl and heteroaryl groups are unsubstituted or substituted with one or more halogen atoms or a linear or branched C₁-C₆ alkyl group, a cyano group, a hydroxy group or a C₁-C₄ alkoxy substituents, or in the case when two adjacent -CR₉ groups are present, the two adjacent -CR₉ groups and the carbon atoms to which they are bonded optionally form a C₆-C₁₀ aryl group which is unsubstituted or substituted by one or more substituents selected from a halogen atom, a linear or branched C₁-C₆ alkyl group, a hydroxy group or a C₁-C₄ alkoxy group;
R₈ together with R₅ and the nitrogen atom to which R₅ is bonded form a said 5- to 9-membered heterocyclyl ring, or R₈ is a hydrogen atom, a linear or branched C₁-C₆ alkyl group, a C₁-C₄ haloalkyl group, a C₁-C₄ hydroxyalkyl group, a C₃-C₁₀ cycloalkyl group, a 5- to 10- membered heterocyclyl group, a C₆-C₁₀ aryl group , a 5- to 10- membered heteroaryl group, -L-Het-R"', -L-A, -A-SO₂-R', -A-SO-R"', -A-A', -A-L-C(O)NR'R", -A-L-CN, -A-C(O)-Het'-L-CN, -A-C(O)-NR'R", -A-C(O)_{z}-A", -A-C(O)-R"', -A-CO₂-R', -A-C(O)_{z}-L-A"', -A-C(O)_{z}-L-CN, or -A-C(O)_{z}-L-Het-R' group, wherein z is 1 or 2, R' and R" are the same or different and each represents a hydrogen atom or linear or a branched C₁-C₆ alkyl group, a C₁-C₄ haloalkyl group or a C₁-C₄ hydroxyalkyl group group, and R'" represents a linear or branched C₁-C₆ alkyl group, a C₁-C₄ haloalkyl group or a C₁-C₄ hydroxyalkyl group, the heterocyclyl and heteroaryl groups being optionally fused to a phenyl group, and wherein the cycloalkyl, heterocyclyl, aryl and heteroaryl groups being unsubstituted or substituted with one or more halogen atoms, a hydroxyl group, a cyano, a linear or branched C₁-C₄ alkyl group, or C₁-C₄ alkoxy group, and wherein
L is a linear or branched C₁-C₆ alkylene group,
Het represents O or NR, and Het' represents NR, wherein R is a hydrogen atom, a straight or branced C₁-C₄ alkyl group, a C₁-C₄ haloalkyl group, or C₁-C₄ hydroxyalkyl group,
A, A', A" and A"' are the same or different and each represent a C₃-C₁₀ cycloalkyl group, a 5- to 10- membered heterocyclyl group, a C₆-C₁₀ aryl group, or a 5- to 10-membered heteroaryl group, the cycloalkyl, heterocyclyl, aryl and heteroaryl groups being unsubstituted or substituted with one or more halogen atoms, a hydroxyl group, a cyano group, a linear or branched C₁-C₄ alkyl group, or a C₁-C₄ alkoxy group.

Typically, R₁, R₂ and R₄ are the same or different and each represent a hydrogen atom, a halogen atom, or a hydroxy or linear or branched C₁-C₄ alkyl group. Preferably, R₁, R₂ and R₄ are the same and each represents a hydrogen atom.

Typically, R₃ represents a hydrogen atom, a halogen atom, or a hydroxy, cyano, linear or branched C₁-C₄ alkyl, C₃-C₆ cycloalkyl, phenyl, 5- to 6-membered heteroaryl, C(O)OR', or-C(O)NR"R"' group, which cycloalkyl, phenyl and heteroaryl groups are unsubstituted or substituted with 1, 2 or 3 halogen atoms or linear or branched C₁-C₂ alkyl, hydroxy, cyano or C₁-C₂ alkoxy substituents, wherein R', R" and R'" are the same or different and each represents a hydrogen atom, or a linear or branched C₁-C₂ alkyl group. Preferably, R₃ represents a hydrogen atom, a halogen atom, or a cyano, C₃-C₄ cycloalkyl, phenyl, pyridyl, pyrazolyl, or C(O)OR' group, which cycloalkyl, phenyl, pyridyl and pyrazolyl groups are unsubstituted or substituted with 1 or 2 halogen atoms, wherein R' represents a hydrogen atom, or a linear or branched C₁-C₂ alkyl group.

Typically, R₅ represents a hydrogen atom, a halogen atom, or a hydroxy, linear or branched C₁-C₄ alkyl, or-(C₁₋C4 alkyl)-(C₃-C₆ cycloalkyl) group, or R₅ together with R₈ and the nitrogen atom to which R₅ is bonded form a 5- to 7- membered, saturated heterocyclyl group, which contains, as heteroatoms, one or two nitrogen atoms and which heterocyclyl ring is unsubstituted or substituted with -C(O)-(CH₂)ₙ-R' or -C(O)-(CH₂)ₙ-NR"R"', wherein n is 0 or 1, R' represents a hydrogen atom, or a linear or branched C₁-C₄ alkyl, or a cyano group and R" and R'" are the same or different and each represents a hydrogen atom, or a linear or branched C₁-C₄ alkyl group. Preferably, R₅ represents a hydrogen atom, or a hydroxy, linear or branched C₁-C₂ alkyl, or -(C₁-C₂ alkyl)-(C₃-C₄ cycloalkyl) group, or R₅ together with R₈ and the nitrogen atom to which R₅ is bonded form a 7- membered heterocyclyl ring, which contains as heteroatoms one or two nitrogem atoms, which heterocyclyl ring is unsubstituted or substituted with a -C(O)-CH₂-R' group wherein R' represents a a linear or branched C₁ C₂ alkyl or cyano group.

Typically, R₆ and R₇ are the same or different and each represent a hydrogen atom, or a linear or branched C₁-C₄ alkyl group. Preferably, R₆ and R₇ are the same or different and each represent a hydrogen atom, or a C₁-C₂ alkyl group.

Typically, R₉ is a hydrogen atom, a halogen atom, or a hydroxy, linear or branched C₁-C₄ alkyl, 5- to 6- membered heterocyclyl, or 5- to 6- membered heteroaryl group, which heterocyclyl and heteroaryl groups are unsubstituted or substituted with 1, 2 or 3 halogen atoms or linear or branched C₁-C₄ alkyl, or C₁-C₄ alkoxy substituents, or in the case when two adjacent -CR₉ groups are present, the two adjacent -CR₉ groups and the carbon atoms to which they are bonded optionally form a benzene ring which is unsubstituted or substituted by 1, 2 or 3 substituents selected from a halogen atom, or a linear or branched C₁-C₂ alkyl, or C₁-C₂ alkoxy substituents. Preferably, R₉ is a hydrogen atom, a halogen atom, or a C₁-C₂ alkyl, piperazine, pyridone or pyridine group, which piperazine, pyridone and pyridine groups are unsubstituted or substituted with 1 or 2 halogen atoms or C₁-C₂ alkoxy substituents, or in the case when two adjacent -CR₉ groups are present, the two adjacent -CR₉ groups and the carbon atoms to which they are bonded optionally form a benzene ring which is unsubstituted.

Typically, L is a linear or branched C₁-C₆ alkylene group. Preferably, L is a linear or branched C₁-C₅ alkylene group.

Typically, Het represents O or NR and Het' represents NR, wherein R is a hydrogen atom or a straight or branched C₁-C₄ alkyl group, preferably a hydrogen atom or a straight or branched C₁-C₂ alkyl group. Preferably, Het represents O.

Typically, A, A', A" and A"' are the same or different and each represent a C₃-C₆ cycloalkyl, 5- to 6- membered heterocyclyl, phenyl, 5- to 6- membered heteroaryl group, the cycloalkyl, heterocyclyl, phenyl and heteroaryl groups being unsubstituted or substituted with 1, 2 or 3 halogen atoms, or hydroxy, cyano, linear or branched C₁-C₂ alkyl, or C₁-C₂ alkoxy groups.

Typically, A is a 5- to 6-membered heterocyclyl group, phenyl or C₃-C₆ cycloalkyl group, said heterocyclyl, phenyl and cycloalkyl groups being unsubstituted or substituted with 1, 2 or 3, preferably 1 or 2, halogen atoms or hydroxy or C₁-C₂ alkyl groups. Preferably, A is a piperidinyl, phenyl or cyclohexyl group, which piperidinyl, phenyl and cyclohexyl groups are unsubstituted or substituted with one C₁-C₂ alkyl group.

Typically, A' is phenyl group, which is unsubstituted or substituted with 1, 2 or 3, halogen atoms, or cyano, hydroxy or C₁-C₂ alkyl groups. Preferably, A' is a phenyl group, which is unsubstituted or substituted with 1 or 2 halogen atoms or cyano groups.

Typically, A" is a 5- to 6-membered heterocyclyl, C₃-C₆ cycloalkyl or 5- or 6-membered heteroaryl group, which heterocyclyl, cycloalkyl and heteroaryl groups are unsubstituted or substituted with 1, 2 or 3, halogen atoms, or cyano, hydroxy or C₁-C₂ alkyl groups. Preferably, A" is a pyrrolidinyl, cyclopropyl or pyridinyl group, which pyrrolidinyl, cyclopropyl and pyridinyl groups are unsubstituted or substituted with 1 or 2 halogen atoms or cyano groups.

Typically, A'" is a 5- to 6-membered heteroaryl group, which heteroaryl group is unsubstituted or substituted with 1, 2 or 3, preferably 1 or 2 halogen atoms or hydroxy or C₁-C₂ alkyl groups. Preferably, A"' is an imidazolyl group.

Typically, R₈ together with R₅ and the nitrogen atom to which R₅ is bonded form a said 5- to 7-membered heterocyclyl ring, or R₈ is a hydrogen atom, or a linear or branched C₁-C₆ alkyl, C₆-C₁₀ cycloalkyl, 5- to 6- membered heterocyclyl, phenyl, 5- to 6-membered heteroaryl group, -L-Het-R"', -L-A, -A-SO₂-R', -A-A', -A-L-C(O)NR'R", -A-L-CN, -A-C(O)-Het'-L-CN, -A-C(O)-NR'R"^{|}, -A-C(O)-A", -A-C(O)-R"', -A-C(O)-L-A"', -A-C(O)-L-CN, or -A-C(O)-L-Het-R' group, wherein R' and R" are the same or different and each represents a hydrogen atom or linear or branched C₁-C₆ alkyl, C₁-C₂ haloalkyl or C₁-C₄ hydroxyalkyl group, R'" represents a linear or branched C₁-C₆ alkyl, C₁-C₂ haloalkyl or C₁-C₄ hydroxyalkyl group, the 5- to 6-membered heterocyclyl group being optionally fused to a phenyl group, and wherein the cycloalkyl, heterocyclyl, phenyl and heteroaryl groups are unsubstituted or substituted with 1, 2 or 3 halogen atoms, or hydroxy, cyano, C₁-C₂ alkyl, or C₁-C₂ alkoxy groups, and L, Het, Het', A, A', A" and A"' are as defined above.

Preferably, R₈ together with R₅ and the nitrogen atom to which R₅ is bonded form a said 7-membered heterocyclyl ring , or R₈ is a linear or branched C₁-C₃ alkyl, cyclohexyl, adamantyl, piperidinyl, phenyl, pyranyl, -L-Het-R"', -L-A, -A-SO₂-R', -A-A', -A-L-C(O)NR'R", -A-L-CN, -A-C(O)-Het'-L-CN, -A-C(O)-NR'R"^{|}, -A-C(O)-A", -A-C(O)-R"', -A-C(O)-L-A"', -A-C(O)-L-CN, or -A-C(O)-L-Het-R' group, wherein R' and R" are the same or different and each represents a hydrogen atom or linear or branched C₁-C₃ alkyl group, and R'" represents a linear or branched C₁-C₅ alkyl, C₁-C₂ haloalkyl or C₁-C₄ hydroxyalkyl group, the pyranyl group being optionally fused to a phenyl group, and wherein the cyclohexyl, adamantyl, piperidinyl, phenyl and pyranyl groups are unsubstituted or substituted with 1 or 2 halogen atoms, or hydroxy, C₁-C₂ alkyl, or C₁ C₂ alkoxy groups, and L, Het, Het', A, A', A" and A"' are as defined above.

Preferably, in the compound of formula (I), Z is a group NR₅.

Preferably, X and Y independently represent a nitrogen atom or a -CR₉ group, with the proviso that at least X or Y represent a nitrogen atom, and with a further proviso that when either X or Y represents a nitrogen atom, the remaining group represents a -CR₉ group.

Typically, in the compound of formula (I), R₁ represents a hydrogen atom, a halogen atom, a hydroxy group, a cyano group, a linear or branched C₁-C₆ alkyl group, a C₁-C₄ haloalkyl group, a C₁-C₄ hydroxyalkyl group, a C₁-C₄ alkoxy group, a C₁-C₄ alkoxycarbonyl group, a C₃-C₇ cycloalkyl group, a phenyl group, a pyridyl group, a 6 membered, saturated N-containing heterocyclyl ring, a -C(O)OR₁₃ group, a -C(O)-(CH₂)ₙ-R₁₃ group, a -NR₁₃R₁₄ group, or a -C(O)-(CH₂)ₙ-NR₁₃R₁₄ group, wherein n, R₁₃ and R₁₄ have the meanings defined above. Preferably R₁ represents a hydrogen atom, a halogen atom, a cyano group, a linear or branched C₁-C₆ alkyl group or a C₃-C₇ cycloalkyl group or a -NR₁₃R₁₄ group wherein R₁₃ and R₁₄ independently represent a hydrogen atom or a linear or branched C₁-C₃ alkyl group. More preferably R₁ represents a hydrogen atom or a -NR₁₃R₁₄ group wherein R₁₃ and R₁₄ independently represent a hydrogen atom or a linear or branched C₁-C₃ alkyl group. Most preferably R₁ represents a hydrogen atom.

Typically, in the compound of formula (1), R₂ represents a hydrogen atom, a halogen atom, a hydroxy group, a cyano group, a linear or branched C₁-C₆ alkyl group, a C₁-C₄ haloalkyl group, a C₁-C₄ alkoxy group, a C₁-C₄ hydroxyalkyl group, a C₁-C₄ alkoxycarbonyl group, a C₃-C₇ cycloalkyl group, a phenyl group, a pyridyl group or a 6 membered, saturated N-containing heterocyclyl ring. Preferably R₂ represents a hydrogen atom, a halogen atom, a cyano group, a linear or branched C₁-C₆ alkyl group or a C₃-C₇ cycloalkyl group. More preferably R₂ represents a hydrogen atom or a halogen atom. Most preferably R₂ represents a hydrogen atom.

Typically, in the compound of formula (1), R₃ represents a hydrogen atom, a halogen atom, a cyano group, a linear or branched C₁-C₆ alkyl group, a C₁-C₄ haloalkyl group, a C₁-C₄ hydroxyalkyl group, a C₁-C₄ alkoxy group, a C₁-C₄ alkoxycarbonyl group, a C₃-C₇ cycloalkyl group, a phenyl group, a 5- to 6- membered heteroaryl group containing at least one heteroatom selected from O, S and N, or a 6 membered, saturated N-containing heterocyclyl ring, the haloalkyl, hydroxyalkyl, alkoxycarbonyl, cycloalkyl, phenyl, heteroaryl and heterocyclyl group being unsubstituted or substituted by one or more substituents selected from a halogen atom, a hydroxy group, a cyano group or a linear or branched C₁-C₆ alkyl group, and the alkyl groups being unsubstituted or substituted by one or more cyano groups; or
R₃ represents a -S(O)₂NR₁₃R₁₄ group, a -NR₁₃S(O)₂NR₁₄ group, a -OR₁₃ group, a -C(O)OR₁₃ group, a -NR₁₃R₁₄ group, a -NC(O)-(CH₂)ₙ-R₁₃ group, a -C(O)-(CH₂)ₙ-NR₁₃R₁₄ group, a -NR₁₃C(O)-(CH₂)ₙ-R₁₄ group or a -NR₁₃C(O)-(CH₂)ₙ-NR₁₄R₁₅ group, wherein n is 0 or 1, and R₁₃, R₁₄, and R₁₅ independently represent a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring.

Preferably, R₃ represents a hydrogen atom, a halogen atom, a cyano group, a C₁-C₄ haloalkyl group, a C₃-C₄ cycloalkyl group, a phenyl group, a 5- to 6- membered monocyclic heteroaryl group containing 1, 2 or 3 heteroatoms selected from N, O and S, being the phenyl or heteroaryl group unsubstituted or substituted by one or more substituents selected from a halogen atom, a hydroxy group, a cyano group or a linear or branched C₁-C₆ alkyl group; or R₃ represents a -C(O)OR₁₃ group or a -C(O)-(CH₂)n-NR₁₃R₁₄ group wherein R₁₃ and R₁₄ independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring. More preferably R₃ represents a hydrogen atom, a halogen atom or a cyano group or a -C(O)-(CH₂)ₙ-NR₁₃R₁₄ group wherein n is 0 or 1 and R₁₃ and R₁₄ independently represent a hydrogen atom or a linear or branched C₁-C₃ alkyl group. Most preferably R₃ represents a halogen atom or a cyano group.

Typically, in the compound of formula (1), R₄ represents a hydrogen atom, a halogen atom, a hydroxy group, a cyano group, a linear or branched C₁-C₆ alkyl group, a C₁-C₄ haloalkyl group, a C₁-C₄ hydroxyalkyl group, a C₁-C₄ alkoxy group, a C₁-C₄ alkoxycarbonyl group, a C₃-C₇ cycloalkyl group, a phenyl group, a pyridyl group or a 6 membered, saturated N-containing heterocyclyl ring. Preferably R₄ represents a hydrogen atom, a halogen atom, a cyano group, a linear or branched C₁-C₆ alkyl group or a C₃-C₄ cycloalkyl group. More preferably R₄ represents a hydrogen atom.

Typically, in the compound of formula (1), R₅ represents a hydrogen atom, a C₁-C₄ alkyl - C₃-C₇ cycloalkyl group, a linear or branched C₁-C₆ alkyl group optionally substituted by one or more substituents selected from a hydroxy group, a cyano group, a linear or branched C₁-C₆ alkyl group, a C₁-C₄ haloalkyl group, a C₁-C₄ hydroxyalkyl group, a C₃-C₄ cycloalkyl group, a phenyl group or a 6 membered, saturated N-containing heterocyclyl ring. Preferably R₅ represents a hydrogen atom, a linear or branched C₁-C₆ alkyl group optionally substituted with a C₃-C₇ cycloalkyl group. More preferably R₅ represents a hydrogen atom or a linear or branched C₁-C₃ alkyl group.

Typically, in the compound of formula (1), R₆ and R₇ independently represent a hydrogen atom or a linear or branched C₁-C₆ alkyl group. Preferably R₆ and R₇ independently represent a hydrogen atom or a linear or branched C₁-C₃ alkyl group. More preferably R₆ and R₇ independently represent a hydrogen atom, a methyl group or an ethyl group.

Typically, in the compound of formula (1), R₈ represents a linear or branched C₁-C₆ alkyl group, a C₁-C₄ haloalkyl group, a C₃-C₇ cycloalkyl group, a phenyl group, a naphthyl group, a 5- to 6-membered monocyclic heteroaryl group containing 1, 2 or 3 heteroatoms selected from N, O and S, a 5- to 7- membered heterocyclyl group containing containing 1, 2 or 3 heteroatoms selected from N, O and S, or a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N, the alkyl, haloalkyl, alkoxy, cycloalkyl, phenyl, naphthyl, heteroaryl, heterocyclyl and bicyclyl groups being unsubstituted or substituted by one or more substituents selected from
a halogen atom,
a hydroxy group,
a cyano group,
a linear or branched C₁-C₆ alkyl group,
a C₁-C₄ haloalkyl group,
a C₃-C₇ cycloalkyl group,
a phenyl group unsubstituted or substituted by one or more halogen atoms,
a 5- to 6-membered monocyclic heteroaryl group containing 1, 2 or 3 heteroatoms selected from N, O and S,
a 6 membered, saturated N-containing heterocyclyl ring,
a -S(O)₂R₁₀ group, -S(O)₂NR₁₀R₁₁ group, a -NR₁₀S(O)₂NR₁₁ group,
a -OR₁₀ group, a -C(O)OR₁₀group, a -C(O)-(CH₂)ₙ-R₁₀ group, a -NR₁₀R₁₁ group, a -C(0)-(CH₂)ₙ-NR₁₀R₁₁ group, a -(C₁-C₄alkyl)-CN group, or a -(C₁-C₄ alkyl)-C(O)NR'R" group wherein R' and R" are the same or different and are selected from hydrogen atoms and linear or branched C₁-C₄ alkyl groups; wherein n is 0 or 1, and
R₁₀ and R₁₁, independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms, being the alkyl, haloalkyl, cycloalkyl, phenyl, heteroaryl, heterocyclyl and bicyclyl group group unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms being said heteroaryl group unsubstituted or substituted by one or more C₁-C₃ alkyl groups, or a C₃-C₇ heterocycloalkyl ketone group containing 1, 2 or 3 nitrogen atoms being said heterocycloalkyl ketone group unsubstituted or substituted by one or more C₁-C₃ alkyl groups, or,
R₈ represents a -S(O)₂NR₁₃R₁₄ group, a -NR₁₃S(O)₂NR₁₄ group, a -OR₁₃ group, a -C(O)OR₁₃ group or a -C(O)-(CH₂)ₙ-NR₁₃R₁₄ group, wherein n is 0 or 1, and R₁₃ and R₁₄ independently represent a hydrogen atom, a cyano group, a linear or branched C₁-C₆ alkyl group unsubstituted or substituted by one or more phenyl groups, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring, or
R₈ together with the R₅ and the nitrogen atom to which they are bonded form a 4- to 9-membered, saturated heterocyclyl group, which contains, as heteroatoms, one or two nitrogen atoms and which is substituted by a -C(O)-(CH₂)ₙ-R₁₀ group or a -C(O)-(CH₂)ₙ-NR₁₀R₁₁ group, wherein wherein n is 0 or 1, and R₁₀ and R₁₁ independently represent a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring,
provided that when m is zero, R₈ is other than a -S(O)₂NR₁₃R₁₄ group, a -NR₁₃S(O)₂NR₁₄ group, or a -OR₁₃ group.

Preferably, R₈ represents a linear or branched C₁-C₆ alkyl group, a C₁-C₄ haloalkyl group, a C₁-C₄ alkoxy group, a C₃-C₇ cycloalkyl group, an adamantyl group, a phenyl group, a 5- to 6-membered monocyclic heteroaryl group containing 1, 2 or 3 heteroatoms selected from N, O and S, a 5- to 7- membered heterocyclyl group containing containing 1, 2 or 3 heteroatoms selected from N, O and S, or a bicyclyl group containing a phenyl group bonded directly to a 5- to 7- membered heterocyclyl group containing at least one heteroatom selected from O, S and N, being the alkyl, haloalkyl, alkoxy, cycloalkyl, adamantyl, phenyl, heteroaryl, heterocyclyl and bicyclyl group unsubstituted or substituted by one or more substituents selected from
a halogen atom,
a hydroxy group,
a linear or branched C₁-C₆ alkyl group,
a phenyl group,
a pyridyl group,
a -S(O)₂R₁₀ group, a -C(O)OR₁₀ group, a -C(O)-(CH₂)ₙ-R₁₀ group, a - NR₁₀R₁₁ group, a -C(O)-(CH₂)ₙ-NR₁₀R₁₁ group, a -(C₁-C₄ alkyl)-CN group, or a -(C₁C₄ alkyl)-C(O)NR'R" group wherein R' and R" are the same or different and are selected from hydrogen atoms and linear or branched C₁-C₄ alkyl groups; wherein n is 0 or 1, and R₁₀ and R₁₁, independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms, being the alkyl, haloalkyl, cycloalkyl, phenyl, heteroaryl, heterocyclyl and bicyclyl group group unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms being said heteroaryl group unsubstituted or substituted by one or more C₁-C₃ alkyl groups, or a C₃-C₇ heterocycloalkyl ketone group containing 1, 2 or 3 nitrogen atoms being said heterocycloalkyl ketone group unsubstituted or substituted by one or more linear or branched C₁-C₃ alkyl groups, or,
R₈ represents a -OR₁₃ group wherein R₁₃ represents a linear or branched C₁-C₃ alkyl group unsubstituted or substituted by one or more phenyl groups, or
R₈ together with the R₅ and the nitrogen atom to which they are bonded form a 5- to 7-membered, saturated heterocyclyl group, which contains, as heteroatoms, one or two nitrogen atoms and which is substituted by a
-C(O)-(CH₂)ₙ-R₁₀ group or a -C(O)-(CH₂)ₙ-NR₁₀R₁₁ group, wherein n is 0 or 1, and R₁₀ and R₁₁ independently represent a hydrogen atom, a cyano group, a linear or branched C₁-C₃ alkyl group or a C₁-C₄ haloalkyl group,
provided that when m is zero, R₈ is other than an -OR₁₃ group.

Typically, in the compound of formula (I), R₉ represents a hydrogen atom, a halogen atom, a hydroxy group, a cyano group, a linear or branched C₁-C₆ alkyl group, a C₁-C₄ haloalkyl group, a C₁-C₄ alkoxy group, a C₃-C₇ cycloalkyl group, a C₁-C₄ alkyl - C₃-C₇ cycloalkyl group, a phenyl group, a 5- to 6-membered monocyclic heteroaryl group containing 1, 2 or 3 heteroatoms selected from N, O and S, a 5- to 7- membered heterocyclyl group containing at least one heteroatom selected from O, S and N, the alkyl, haloalkyl, alkoxy, cycloalkyl, phenyl, heteroaryl and heterocyclyl groups being unsubstituted or substituted by one or more substituents selected from
a halogen atom,
a hydroxy group,
a cyano group,
a linear or branched C₁-C₆ alkyl group,
a -C(O)-(CH₂)-R₁₀ group or a -OR₁₀ group, wherein R₁₀ represents a hydrogen atom, a cyano group, C₁-C₄ alkyl group, a C₁-C₄ haloalkyl group or a C₃-C₇ cycloalkyl group,
the alkyl, haloalkyl and cycloalkyl groups being unsubstituted or substituted by one or more substituents selected from a halogen atom, a hydroxy group, a cyano group, a linear or branched C₁-C₆ alkyl group or a C₁-C₄ haloalkyl group, or
R₉ represents a -S(O)₂R₁₃ group, a -C(O)OR₁₃ group, a -NR₁₃R₁₄ group or a -C(O)-(CH₂)n-NR₁₃R₁₄ group wherein n is 0 or 1 and R₁₃ and R₁₄ independently represent a hydrogen atom or a linear or branched C₁-C₆ alkyl group,
or in the case that two -CR₉ adjacent groups are present, both -CR₉ groups and the carbon atoms to which they are bonded optionally form a 5- to 9-membered aryl or heteroaryl group, said heteroaryl group containing at least one heteroatom selected from O, S and N, being the aryl and heteroaryl group unsubstituted or substituted by one or more substituents selected from a halogen atom or a linear or branched C₁-C₄ alkyl group.

Preferably, R₉ represents a hydrogen atom, a halogen atom, a cyano group, a linear or branched C₁-C₄ alkyl group, a C₁-C₄ haloalkyl group, a C₃-C₇ cycloalkyl group, a C₁-C₃ alkyl - C₃-C₇ cycloalkyl group, a 5- to 6-membered monocyclic heteroaryl group containing 1, 2 or 3 nitrogen atoms, a 5- to 7- membered heterocyclyl group containing containing 1, 2 or 3 nitrogen atoms, being the alkyl, cycloalkyl, heteroaryl and heterocyclyl group unsubstituted or substituted by one or more substituents selected from a cyano group or a -OR₁₀ group wherein R₁₀ represents a hydrogen atom or a C₁-C₃ alkyl group, or R₉ represents a -S(O)₂R₁₃ group, a -C(O)OR₁₃ group, a -NR₁₃R₁₄ group or a -C(O)-(CH₂)ₙ-NR₁₃R₁₄ group wherein n is 0 or 1 and R₁₃ and R₁₄ independently represent a hydrogen atom or a linear or branched C₁-C₆ alkyl group

In a particularly preferred embodiment, in the compound of formula (I)
m is 0 or 1,
Z is NR₅,
X is a nitrogen atom and Y is a group CR₉, or Y is a nitrogen atom and X is a group CR₉,
R₁ represents a hydrogen atom, a halogen atom, a cyano group, a linear or branched C₁-C₆ alkyl group or a C₃-C₄ cycloalkyl group or a -NR₁₃N₁₄ group wherein R₁₃ and R₁₄ independently represent a hydrogen atom or a linear or branched C₁-C₃ alkyl group,
R₂ represents a hydrogen atom, a halogen atom, a cyano group, a linear or branched C₁-C₆ alkyl group or a C₃-C₄ cycloalkyl group,
R₃ represents a hydrogen atom, a halogen atom, a cyano group, a C₃-C₄ cycloalkyl group, a 5- to 6- membered monocyclic aryl or heteroaryl group, the heteroaryl group containing 1, 2 or 3 heteroatoms selected from N, O and S, the aryl and heteroaryl groups being unsubstituted or substituted by one or more substituents selected from a halogen atom, a hydroxy group, a cyano group or a linear or branched C₁-C₆ alkyl group, or
R₃ represents a -C(O)OR₁₃ group, a -C(O)-NR₁₃R₁₄ group or a -NR₁₃C(O)R₁₄ group wherein R₁₃ and R₁₄ independently represent a hydrogen atom, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring,
R₄ represents a hydrogen atom, a halogen atom, a cyano group, a linear or branched C₁-C₆ alkyl group or a C₃-C₄ cycloalkyl group,
R₅ represents a hydrogen atom, a linear or branched C₁-C₃ alkyl group or a -C₁-C₃ alkyl - C₃-C₇ cycloalkyl group,
R₆ and R₇ independently represent a hydrogen atom or or a linear or branched C₁-C₃ alkyl group,
R₈ represents a linear or branched C₁-C₆ alkyl group, a C₁-C₄ haloalkyl group, a C₁-C₄ alkoxy group, a C₃-C₇ cycloalkyl group, an adamantyl group, a phenyl group, a 5- to 6-membered monocyclic heteroaryl group containing 1, 2 or 3 heteroatoms selected from N, O and S, a 5- to 7- membered heterocyclyl group containing containing 1, 2 or 3 heteroatoms selected from N, O and S, or a bicyclyl group containing a phenyl group bonded directly to a 5- to 7- membered heterocyclyl group containing at least one heteroatom selected from O, S and N, the alkyl, haloalkyl, alkoxy, cycloalkyl, adamantyl, phenyl, heteroaryl, heterocyclyl and bicyclyl groups being unsubstituted or substituted by one or more substituents selected from
a halogen atom,
a hydroxy group,
a linear or branched C₁-C₆ alkyl group,
a phenyl group,
a pyridyl group,
a -S(O)₂R₁₀ group, a -C(O)OR₁₀ group, a -C(O)-(CH₂)ₙ-R₁₀ group,
a -NR₁₀R₁₁ group, a -C(O)-(CH₂)ₙ-NR₁₀R₁₁ group, a -(C₁-C₄ alkyl)-CN
group, or a -(C₁-C₄ alkyl)-C(O)NR'R" group wherein R' and R" are the same or different and are selected from hydrogen atoms and linear or branched C₁-C₄ alkyl groups; wherein n is 0 or 1, and
R₁₀ and R₁₁, independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms, being the alkyl, haloalkyl, cycloalkyl, phenyl, heteroaryl, heterocyclyl and bicyclyl group group unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms being said heteroaryl group unsubstituted or substituted by one or more C₁-C₃ alkyl groups, or a C₃-C₇ heterocycloalkyl ketone group containing 1, 2 or 3 nitrogen atoms being said heterocycloalkyl ketone group unsubstituted or substituted by one or more linear or branched C₁-C₃ alkyl groups, or,
R₈ represents a -OR₁₃ group wherein R₁₃ represents a linear or branched C₁-C₃ alkyl group unsubstituted or substituted by one or more phenyl groups, or
R₈ together with R₅ and the nitrogen atom to which R₅ is bonded form a 5- to 7-membered, saturated heterocyclyl group, which contains, as heteroatoms, one or two nitrogen atoms and which is substituted by a -C(O)-(CH₂)ₙ-R₁₀ group or a -C(0)-(CH₂)ₙ-NR₁₀R₁₁ group, wherein n is 0 or 1, and R₁₀ and R₁₁ independently represent a hydrogen atom, a cyano group, a linear or branched C₁-C₃ alkyl group or a C₁-C₄ haloalkyl group,
R₉ represents a hydrogen atom, a halogen atom, a cyano group, a linear or branched C₁-C₄ alkyl group, a C₁-C₄ haloalkyl group, a C₃-C₇ cycloalkyl group, a
-C₁-C₃ alkyl - C₃-C₇ cycloalkyl group, a 5- to 6-membered monocyclic heteroaryl group containing 1, 2 or 3 nitrogen atoms, a 5- to 7- membered heterocyclyl group containing containing 1, 2 or 3 nitrogen atoms, the alkyl, cycloalkyl, heteroaryl and heterocyclyl groups being unsubstituted or substituted by one or more substituents selected from a cyano group or a -OR₁₀ group wherein R₁₀ represents a linear or branched C₁-C₃ alkyl group, or
R₉ represents a -S(O)₂R₁₃ group, a -C(O)OR₁₃ group or a -NR₁₃R₁₄ group wherein R₁₃ and R₁₄ independently represent a hydrogen atom or a linear or branched C₁-C₄ alkyl group,
or in the case that two -CR₉ adjacent groups are present, both -CR₉ groups and the carbon atoms to which they are bonded optionally form a 5- to 9-membered aryl group, being the aryl group unsubstituted or substituted by one or more substituents selected from a halogen atom or a linear or branched C₁-C₄ alkyl group.

In a further particularly preferred embodiment, in the compound of formula (I)
m is 0 or 1,
Z is NR₅,
X is a nitrogen atom and Y is a group CR₉, or Y is a nitrogen atom and X is a group CR₉,
R₁ represents a hydrogen atom,
R₂ represents a hydrogen atom,
R₃ represents a hydrogen atom, a halogen atom, a cyano group, a C₁-C₄ haloalkyl group, a cyclopropyl group, a phenyl group unsubstituted or substituted by one or more halogen atoms, a 5- to 6- membered monocyclic heteroaryl group containing 1, 2 or 3 heteroatoms selected from N and O, or
R₃ represents a -C(O)OR₁₃ group, a -C(O)-NR₁₃R₁₄ group or a -NR₁₃C(O)R₁₄ group wherein R₁₃ and R₁₄ independently represent a hydrogen atom or a methyl group,
R₄ represents a hydrogen atom,
R₅ represents a hydrogen atom, a methyl group, an ethyl group or a -C₁-C₃ alkyl - C₃-C₄ cycloalkyl group,
R₆ and R₇ independently represent a hydrogen atom or a methyl group,
R₈ represents a linear or branched C₁-C₆ alkyl group, a C₁-C₄ haloalkyl group, a C₁-C₄ alkoxy group, a C₃-C₇ cycloalkyl group, an adamantyl group, a phenyl group, a 5- to 6-membered monocyclic heteroaryl group containing 1, 2 or 3 heteroatoms selected from N, O and S, a 5- to 7- membered heterocyclyl group containing containing 1, 2 or 3 heteroatoms selected from N, O and S, or a bicyclyl group containing a phenyl group bonded directly to a 5- to 7- membered heterocyclyl group containing at least one heteroatom selected from O, S and N, the alkyl, haloalkyl, alkoxy, cycloalkyl, adamantyl, phenyl, heteroaryl, heterocyclyl and bicyclyl groups being unsubstituted or substituted by one or more substituents selected from
a halogen atom,
a hydroxy group,
a linear or branched C₁-C₆ alkyl group,
a phenyl group,
a pyridyl group,
a -S(O)₂R₁₀ group, a -C(O)OR₁₀ group, a -C(O)-(CH₂)ₙ-R₁₀ group,
a -NR₁₀R₁₁ group, a -C(O)-(CH₂)ₙ-NR₁₀R₁₁ group or a -R₁₀C(O)-(CH₂)n-NR₁₁R₁₂ group; wherein n is 0 or 1, and R₁₀ and R₁₁, independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms, being the alkyl, haloalkyl, cycloalkyl, phenyl, heteroaryl, heterocyclyl and bicyclyl group group unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms being said heteroaryl group unsubstituted or substituted by one or more C₁-C₃ alkyl groups, or a C₃-C₇ heterocycloalkyl ketone group containing 1, 2 or 3 nitrogen atoms being said heterocycloalkyl ketone group unsubstituted or substituted by one or more linear or branched C₁-C₃ alkyl groups, or,
R₈ represents a -OR₁₃ group wherein R₁₃ represents a linear or branched C₁-C₃ alkyl group unsubstituted or substituted by one or more phenyl groups, or
R₈ together with R₅ and the nitrogen atom to which R₅ is bonded form a 5- to 7-membered, saturated heterocyclyl group, which contains, as heteroatoms, one or two nitrogen atoms and which is substituted by a -C(O)-(CH₂)ₙ-R₁₀ group or a -C(O)-(CH₂)ₙ-NR₁₀R₁₁ group, wherein n is 0 or 1, and R₁₀ and R₁₁ independently represent a hydrogen atom, a cyano group, a linear or branched C₁-C₃ alkyl group or a C₁-C₄ haloalkyl group,
R₉ represents a hydrogen atom, a halogen atom, a cyano group, a linear or branched C₁-C₄ alkyl group, a C₃-C₇ cycloalkyl group, a 5- to 6-membered monocyclic heteroaryl group containing 1, 2 or 3 nitrogen atoms, a 5- to 7- membered heterocyclyl group containing containing 1, 2 or 3 nitrogen atoms, being the alkyl, cycloalkyl, heteroaryl, and heterocyclyl, unsubstituted or substituted by one or more substituents selected from a cyano group or a -OR₁₀ group wherein R₁₀ represents a linear or branched C₁-C₃ alkyl group, or
R₉ represents a -S(O)₂R₁₃ group, a -C(O)OR₁₃ group or a -NR₁₃R₁₄ group wherein R₁₃ and R₁₄ independently represent a hydrogen atom or a linear or branched C₁-C₄ alkyl group,
or in the case that two -CR₉ adjacent groups are present, both -CR₉ groups and the carbon atoms to which they are bonded optionally form a 5- to 6-membered aryl group, being the aryl group unsubstituted or substituted by one or more substituents selected from a halogen atom or a linear or branched C₁-C₄ alkyl group.

In a particularly preferred embodiment, the compound of the invention is of formula (I'), 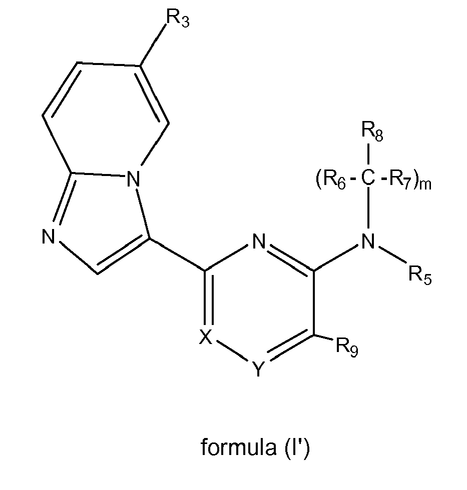 wherein X is a nitrogen atom and Y is a -CR₉ group, or Y is a nitrogen atom and X is a CR₉ group;
R₃ is a hydrogen atom, a halogen atom, a cyano group, a COOR' group, a C₃-C₄ cycloalkyl group, a pyridine group, a pyrazole group, or a phenyl group, which phenyl group is unsubstituted or substituted with a halogen atom, wherein R' is a hydrogen atom or a linear or branched C₁-C₄ alkyl group;
R₃ is a hydrogen atom, a halogen atom, a cyano group, a -C(O)OR' group, a C₃-C₄ cycloalkyl group, a pyridine group, a pyrazole group, or a phenyl group, which phenyl group is unsubstituted or substituted with a halogen atom, wherein R' is a hydrogen atom or a linear or branched C₁-C₄ alkyl group;
R₅ is a hydrogen atom, a methyl group, an ethyl group, a -CH₂-cyclopropyl group, or R₅ together with R₈ and the nitrogen atom to which R₅ is bonded form a 1,4-diazepane-1-yl group, which 1,4-diazepane-1-yl group is unsubstituted or substituted with a -C(O)CH₂CN group;
R₆ and R₇ each independently represent a hydrogen atom or a methyl group;
R₉ is a hydrogen atom, halogen atom, or a methyl, ethyl, piperazine, pyridone or pyridine group, which pyridine group is unsubstituted or substituted with a C₁-C₂ alkoxy group, or when Y is a group R₉, the two adjacent R₉ groups, together with the carbon atoms to which they are bonded, may form a benzene ring;
R₈ together with R₅ and the nitrogen atom to which R₅ is bonded form a said 1,4-diazepane-1-yl group, or R₈ is a hydrogen atom, or a linear or branched C₁-C₅ alkyl, cyclohexyl, adamantyl, pyranyl, piperidinyl, chromanyl, -(C₁-C₅ alkyl)-phenyl, -(C₁-C₅ alkyl)-cyclohexyl, -(C₁-C₅ alkyl)-(C₁-C₂ alkoxy), -A-SO₂-R', -A-A', -A-L-C(O)NR'R", -A-L-CN, -A-C(O)-N(C₁-C₂alkyl)-L-CN, -A-C(O)-NR'R", -A-C(O)-A", -A-C(O)-R"', -A-CO₂-R', -A-C(O)-L-A'", -A-C(O)-L-CN, or -A-C(O)-L-O-R' group, wherein R' and R" are the same or different and each represents a hydrogen atom or linear or a branched C₁-C₄ alkyl group, and R'" represents a linear or branched C₁-C₅ alkyl, C₁-C₂ haloalkyl or C₁₋C₄ hydroxyalkyl group, the cyclohexyl, adamantyl, pyranyl, piperidinyl and chromanyl groups being unsubstituted or substituted with a halogen atom, or a hydroxy, linear or branched C₁-C₂ alkyl, or C₁-C₂ alkoxy group, and wherein
L is a linear or branched C₁-C₅ alkylene group,
A is a piperidinyl or pyrrolidinyl group, which is unsubstituted or substituted with a C₁-C₂ alkyl group,
A' is a phenyl or pyridinyl group, which is unsubstituted or substituted with 1 or 2 halogen atoms or CN groups,
A" is a pyrrolidinyl, pyridinyl, pyrazolyl or cyclopropyl group, the pyrrolidinyl, pyridinyl, pyrazolyl and cyclopropyl groups being unsubstituted or substituted with 1 or 2 halogen atoms or cyano groups, and
A"' is an imidazolyl group.

Particular individual compounds of the invention include:
3-(4-{[(1S)-1-phenylethyl]amino}pyrimidin-2-yl)imidazo[1,2-a]pyridine-6-carbonitrile;
3-(4-{[(1R)-1-phenylethyl]amino}pyrimidin-2-yl)imidazo[1,2-a]pyridine-6-carbonitrile;
3-[4-(Benzylamino)pyrimidin-2-yl]imidazo[1,2-*a*]pyridine-6-carbonitrile;
3-(4-{[(1 S)-2-methoxy-1-methylethyl]amino}pyrimidin-2-yl)imidazo[1,2-a]pyridine-6-carbonitrile;
3-{4-[(Cyclohexylmethyl)amino]pyrimidin-2-yl}imidazo[1,2-a]pyridine-6-carbonitrile;
3-{4-[(2-Methoxyethyl)amino]pyrimidin-2-yl}imidazo[1,2-a]pyridine-6-carbonitrile;
3-{4-[(1-Adamantylmethyl)amino]pyrimidin-2-yl}imidazo[1,2-a]pyridine-6-carbonitrile;
3-{4-[(2,2-Dimethylpropyl)amino]pyrimidin-2-yl}imidazo[1,2-a]pyridine-6-carbonitrile;
3-{5-Bromo-4-[(2,2-dimethylpropyl)amino]pyrimidin-2-yl}midazo[1,2-a]pyridine-6-carbonitrile;
3-{4-[(2,2-Dimethylpropyl)amino]-5-piperazin-1-ylpyrimidin-2-yl}imidazo[1,2-a]pyridine-6-carbonitrile;
3-[4-[(2,2-Dimethylpropyl)amino]-5-(2-methoxypyridin-4-yl)pyrimidin-2-yl]imidazo[1,2-a]pyridine-6-carbonitrile;
3-[4-[(2,2-Dimethylpropyl)amino]-5-(2-oxo-1,2-dihydropyridin-4-yl)pyrimidin-2-yl]imidazo[1,2-a]pyridine-6-carbonitrile;
3-{4-[(2,2-Dimethylpropyl)amino]-5-pyridin-3-ylpyrimidin-2-yl}imidazo[1,2-a]pyridine-6-carbonitrile;
3-{4-[(3-Fluorobenzyl)amino]pyrimidin-2-yl}imidazo[1,2-a]pyridine-6-carbonitrile;
3-{4-[(4-Fluorobenzyl)amino]pyrimidin-2-yl}imidazo[1,2-a]pyridine-6-carbonitrile;
3-{4-[(2-Methylbenzyl)amino]pyrimidin-2-yl}imidazo[1,2-a]pyridine-6-carbonitrile; *T*ert-butyl-2-({[2-(6-cyanoimidazo[1,2-a]pyridin-3-yl)pyrimidin-4-yl]amino}methyl) piperidine-1-carboxylate;
3-(4-{[(1-Acetylpiperidin-2-yl)methyl]amino}pyrimidin-2-yl)imidazo[1,2-a]pyridine-6-carbonitrile;
3-[4-(Tetrahydro-2H-pyran-4-ylamino)pyrimidin-2-yl]imidazo[1,2-a]pyridine-6-carbonitrile;
3-(4-(8-Fluorochroman-4-ylamino)pyrimidin-2-yl)imidazo[1,2-a]pyridine-6-carbonitrile;
3-[4-(Cyclohexylamino)pyrimidin-2-yl]imidazo[1,2-a]pyridine-6-carbonitrile;
3-{4-[(*Trans*-4-hydroxycyclohexyl)amino]pyrimidin-2-yl}imidazo[1,2-a]pyridine-6-carbonitrile;
3-{4-[(5-Hydroxy-2-adamantyl)amino]pyrimidin-2-yl}imidazo[1,2-*a*]pyridine-6-carbonitrile;
3-{4-[(5-Hydroxy-2-adamantyl)amino]pyrimidin-2-yl}imidazo[1,2-a]pyridine-6-carbonitrile;
2-{4-[(2,2-Dimethylpropyl)amino]quinazolin-2-yl}imidazo[1,2-a]pyridine-6-carbonitrile;
3-{4-[Benzyl(methyl)amino]pyrimidin-2-yl}imidazo[1,2-a]pyridine-6-carbonitrile;
3-(4-{[(1 S)-1-phenylethyl]amino}pyrimidin-2-yl)imidazo[1,2-a]pyridine-6-carboxylic acid;
2-(6-Fluoroimidazo[1,2-a]pyridin-3-yl)-N-[(1 S)-1-phenylethyl]pyrimidin-4-amine; *Trans*-4-{[2-(6-fluoroimidazo[1,2-a]pyridin-3-yl)pyrimidin-4-yl]amino}cyclohexanol;
3-(4-{[(2S,7S)-5-hydroxy-2-adamantyl]amino}pyrimidin-2-yl)imidazo[1,2-a]pyridine-6-carbonitrile;
N-(2,2-dimethylpropyl)-2-imidazo[1,2-a]pyridin-3-ylpyrimidin-4-amine;
N-(2,2-dimethylpropyl)-2-imidazo[1,2-a]pyridin-3-yl-6-pyridin-3-ylpyrimidin-4-amine;
3-(6-{[(1 S)-1-phenylethyl]amino}pyrazin-2-yl)imidazo[1,2-a]pyridine-6-carbonitrile;
3-{6-[(Cyclohexylmethyl)amino]pyrazin-2-yl}imidazo[1,2-a]pyridine-6-carbonitrile;
6-(6-Fluoroimidazo[1,2-a]pyridin-3-yl)-N-[(1 S)-1-phenylethyl]pyrazin-2-amine;
6-(6-Chloroimidazo[1,2-a]pyridin-3-yl)-N-[(1 S)-1-phenylethyl]pyrazin-2-amine;
(S)-6-(imidazo[1,2-a]pyridin-3-yl)-N-(1-phenylethyl)pyrazin-2-amine;
6-(6-Cyclopropylimidazo[1,2-a]pyridin-3-yl)-N-[(1 S)-1-phenylethyl]pyrazin-2-amine;
*N*-[(1*S*)-1-phenylethyl]-6-(6-pyridin-3-ylimidazo[1,2-a ]pyridin-3-yl)pyrazin-2-amine;
*N*-[(1*S*)-l -phenylethyl]-6-(6-pyridin-4-ylimidazo[1,2-a]pyridin-3-yl)pyrazin-2-amine;
*N*-[(1*S*)-1-phenylethyl]-6-[6-(1 H-pyrazol-4-yl)imidazo[1,2-a]pyridin-3-yl]pyrazin-2-amine;
*N*-[(1*S)-*1-phenylethyl]-6-(6-phenylimidazo[1,2-a]pyridin-3-yl)pyrazin-2-amine;
6-[6-(4-Fluorophenyl)imidazo[1,2-a]pyridin-3-yl]-N-[(1*S*)-1-phenylethyl]pyrazin-2-amine;
3-(4-{[(3*R*)-1-(ethylsulfonyl)piperidin-3-yl]amino}pyrimidin-2-yl)imidazo[1,2-a]pyridine-6-carbonitrile;
3-(4-{[(3R)-1-(isopropylsulfonyl)piperidin-3-yl]amino}pyrimidin-2-yl)imidazo[1,2-a]pyridine-6-carbonitrile;
3-(4-{[(3R)-1-(cyanoacetyl)piperidin-3-yl]amino}pyrimidin-2-yl)imidazo[1,2-a]pyridine-6-carbonitrile;
3-(4-{[(3R)-1-propionylpiperidin-3-yl]amino}pyrimidin-2-yl)imidazo[1,2-a]pyridine-6-carbonitrile;
3-[4-({(3R)-1-[(1-cyanocyclopropyl)carbonyl]piperidin-3-yl}amino)pyrimidin-2-yl]imidazo[1,2-a]pyridine-6-carbonitrile;
3-(4-{[(3R)-1-(methoxyacetyl)piperidin-3-yl]amino}pyrimidin-2-yl)imidazo[1,2-a]pyridine-6-carbonitrile;
3-(4-{[(3R)-1-(3-hydroxy-3-methylbutanoyl)piperidin-3-yl]amino}pyrimidin-2-yl)imidazo[1,2-a]pyridine-6-carbonitrile;
3-(4-{[(3*R*)-1-(3,3-dimethylbutanoyl)piperidin-3-yl]amino}pyrimidin-2-yl)imidazo[1,2-*a*]pyridine-6-carbonitrile;
3-(4-([(3*R*)-1-(1*H*-imidazol-4-ylacetyl)piperidin-3-yl]aminolpyrimidin-2-yl)imidazo[1,2-*a*]pyridine-6-carbonitrile;
3-[4-({(3*R*)-1-[(5-cyanopyridin-2-yl)carbonyl]piperidin-3-yl}amino)pyrimidin-2-yl] imidazo[1,2-*a*]pyridine-6-carbonitrile;
3-(4-{[(3*R*)-1-(3,3,3-trifluoropropanoyl)piperidin-3-yl]amino}pyrimidin-2-yl)imidazo[1,2-*a*]pyridine-6-carbonitrile;
(3*R*)-3-{[2-(6-cyanoimidazo[1,2-a]pyridin-3-yl)pyrimidin-4-yl]amino}-*N*,*N*-dimethyl piperidine-1-carboxamide;
3-{4-[((3*R*)-1-{[(2*S*,4*S*)-2-cyano-4-fluoropyrrolidin-1-yl]carbonyl}piperidin-3-yl) amino]pyrimidin-2-yl}imidazo[1,2-*a*]pyridine-6-carbonitrile;
3-(4-{[(3*R*)-1-(5-cyanopyridin-2-yl)piperidin-3-yl]amino}pyrimidin-2-yl)imidazo[1,2-*a*]pyridine-6-carbonitrile;
3-(4-{[(3*R*)-1-(4-cyano-2-fluorophenyl)piperidin-3-yl]amino}pyrimidin-2-yl)imidazo [1,2-*a*]pyridine-6-carbonitrile;
3-{4-[[(3R)-1-(cyanoacetyl)piperidin-3-yl](methyl)amino]pyrimidin-2-yl}imidazo [1,2-*a*]pyridine-6-carbonitrile;
3-(4-{[(3*S*)-1-(cyanoacetyl)piperidin-3-yl]amino}pyrimidin-2-yl)imidazo[1,2-*a*]pyridine-6-carbonitrile;
*Cis*-3-(4-{1-(cyanoacetyl)-4-methylpiperidin-3-yl]amino}pyrimidin-2-yl)imidazo[1,2-*a*]pyridine-6-carbonitrile;
*Cis-3-(*4-{1-(ethylsulfonyl)-4-methylpiperidin-3-yl]amino}pyrimidin-2-yl)imidazo[1,2-*a*]pyridine-6-carbonitrile;
3-{4-[[1-(Ethylsulfonyl)-4-methylpiperidin-3-yl](methyl)amino]pyrimidin-2-yl} imidazo[1,2-*a*]pyridine-6-carbonitrile;
3-((3*R*)-3-{[2-(6-fluoroimidazo[1,2-*a*]pyridin-3-yl)pyrimidin-4-yl]amino}piperidin-1-yl)-3-oxopropanenitrile;
(*R*)-1-(3-(2-(6-fluoroimidazo[1,2-*a*]pyridin-3-yl)pyrimidin-4-ylamino)piperidine-1-carbonyl)cyclopropanecarbonitrile;
2-(6-Fluoroimidazo[1,2-*a*]pyridin-3-yl)-N-[(3*R*)-1-(3,3,3-trifluoropropanoyl)piperidin-3-yl]pyrimidin-4-amine;
2-(6-Fluoroimidazo[1,2-*a*]pyridin-3-yl)-N-[(3*R*)-1-(1*H*-pyrazol-4ylcarbonyl)piperidin-3-yl]pyrimidin-4-amine;
(3*R*)-N-(cyanomethyl)-3-{[2-(6-fluoroimidazo[1,2-*a*]pyridin-3-yl)pyrimidin-4-yl]amino}-*N-*methylpiperidine-1-carboxamide;
2-((*3R*)-3-{[2-(6-fluoroimidazo[1,2-a]pyridin-3-yl)pyrimidin-4-yl]amino}piperidin-1-yl)-2-methylpropanamide;
2-(6-Fluoroimidazo[1,2-a]pyridin-3-yl)-N-methyl-N-[(3*R*)-1-(3,3,3-trifluoro propanoyl)piperidin-3-yl]pyrimidin-4-amine;
(*R*)-3-(3-((2-(6-fluoroimidazo[1,2-a]pyridin-3-yl)pyrimidin-4-yl)(methyl)amino)piperidin-1-yl)-3-oxopropanenitrile;
(*R*)-1-(3-((2-(6-fluoroimidazo[1,2-a]pyridin-3-yl)pyrimidin-4-yl)(methyl)amino)piperidine-1-carbonyl)cyclopropanecarbonitrile;
3-((3*R*)-3-{ethyl[2-(6-fluoroimidazo[1,2-a]pyridin-3-yl)pyrimidin-4-yl]amino}piperidin-1-yl)-3-oxopropanenitrile;
*N*-ethyl-2-(6-fluoroimidazo[1,2-a]pyridin-3-yl)-*N*-[(3*R*)-1-(3,3,3-trifluoropropanoyl) piperidin-3-yl]pyrimidin-4-amine;
3-((3*R*)-3-{(cyclopropylmethyl)[2-(6-fluoroimidazo[1,2-a]pyridin-3-yl)pyrimidin-4-yl]amino}piperidin-1-yl)-3-oxopropanenitrile;
*N*-(cyclopropylmethyl)-2-(6-fluoroimidazo[1,2-a]pyridin-3-yl)-*N*-[(3*R*)-1-(3,3,3-trifluoropropanoyl)piperidin-3-yl]pyrimidin-4-amine;
3-((3*R*)-3-{[5-fluoro-2-(6-fluoroimidazo[1,2-*a*]pyridin-3-yl)pyrimidin-4-yl]amino}piperidin-1-yl)-3-oxopropanenitrile;
5-Fluoro-2-(6-fluoroimidazo[1,2-a]pyridin-3-yl)-*N*-[(3R)-1-(3,3,3-trifluoropropanoyl) piperidin-3-yl]pyrimidin-4-amine;
3-((3*R*)-3-{[2-(6-fluoroimidazo[1,2-a]pyridin-3-yl)-5-methylpyrimidin-4-yl]amino} piperidin-1-yl)-3-oxopropanenitrile;
2-(6-fluoroimidazo[1,2-*a*]pyridin-3-yl)-5-methyl-*N*-[(3*R*)-1-(3,3,3-trifluoropropanoyl) piperidin-3-yl]pyrimidin-4-amine;
3-((3*R*)-3-{[2-(6-fluoroimidazo[1,2-*a*]pyridin-3-yl)pyrimidin-4-yl]amino}pyrrolidin-1-yl)-3-oxopropanenitrile;
3-{4-[4-(cyanoacetyl)-1,4-diazepan-1-yl]pyhmidin-2-yl}imidazo[1,2-a]pyridine-6-carbonitrile;
3-((3*R*)-3-{[5-chloro-2-(6-fluoroimidazo[1,2-*a*]pyridin-3-yl)pyrimidin-4-yl]amino} piperidin-1-yl)-3-oxopropanenitrile;
3-((3*R*)-3-{[2-(6-fluoroimidazo[1,2-a]pyridin-3-yl)pyrimidin-4-yl]aminolpiperidin-1-yl)-2,2-dimethylpropanenitrile;
2-((3*R*)-3-{[2-(6-fluoroimidazo[1,2-a]pyridin-3-yl)pyrimidin-4-yl]amino}piperidin-1-yl)acetamide,
and pharmaceutically acceptable salts, solvates or N-oxides thereof.

Of outstanding interest are:
3-(4-{[(1*S*)-1-phenylethyl]amino}pyrimidin-2-yl)imidazo[1,2-*a*]pyridine-6-carbonitrile;
3-{4-[(4-Fluorobenzyl)amino]pyrimidin-2-yl}imidazo[1,2-*a*]pyridine-6-carbonitrile;
2-{4-[(2,2-Dimethyl)propyl)amino]quinazolin-2-yl}imidazo[1,2-*a*]pyridine-6-carbonitrile;
2-(6-Fluoroimidazo[1,2-a]pyridin-3-yl)-*N*-[(1*S*)-1-phenylethyl]pyrimidin-4-amine;
N-(2,2-dimethylpropyl)-2-imidazo[1,2-a]pyridin-3-yl-6-pyridin-3-ylpyrimidin-4-amine;
3-((3*R*)-3-{[2-(6-fluoroimidazo[1,2-*a*]pyridin-3-yl)pyrimidin-4-yl]amino}piperidin-1-yl)-3-oxopropanenitrile;
2-(6-Fluoroimidazo[1,2-a]pyridin-3-yl)-*N*-[(3*R*)-1-(3,3,3-trifluoropropanoyl)piperidin -3-yl]pyrimidin-4-amine;
(*R*)-3-(3-((2-(6-fluoroimidazo[1,2-a]pyridin-3-yl)pyrimidin-4-yl)(methyl)amino) piperidin-1-yl)-3-oxopropanenitrile;
3-((3*R*)-3-{[5-fluoro-2-(6-fluoroimidazo[1,2-*a*]pyridin-3-yl)pyrimidin-4-yl]amino} piperidin-1-yl)-3-oxopropanenitrile;
5-Fluoro-2-(6-fluoroimidazo[1,2-a]pyridin-3-yl)-*N*-[(3*R*)-1-(3,3,3-trifluoropropanoyl) piperidin-3-yl]pyrimidin-4-amine;
3-((3*R*)-3-{[2-(6-fluoroimidazo[1,2-*a*]pyridin-3-yl)-5-methylpyrimidin-4-yl]amino} piperidin-1-yl)-3-oxopropanenitrile;
2-(6-fluoroimidazo[1,2-*a*]pyridin-3-yl)-5-methyl-*N*-[(3*R*)-1-(3,3,3-trifluoropropanoyl) piperidin-3-yl]pyrimidin-4-amine;
and pharmaceutically aceeptable salts, solvates or N-oxides thereof.

According to one embodiment of the present invention, compounds of general formula (I) may be prepared by the following synthetic route as illustrated in Figure 1.

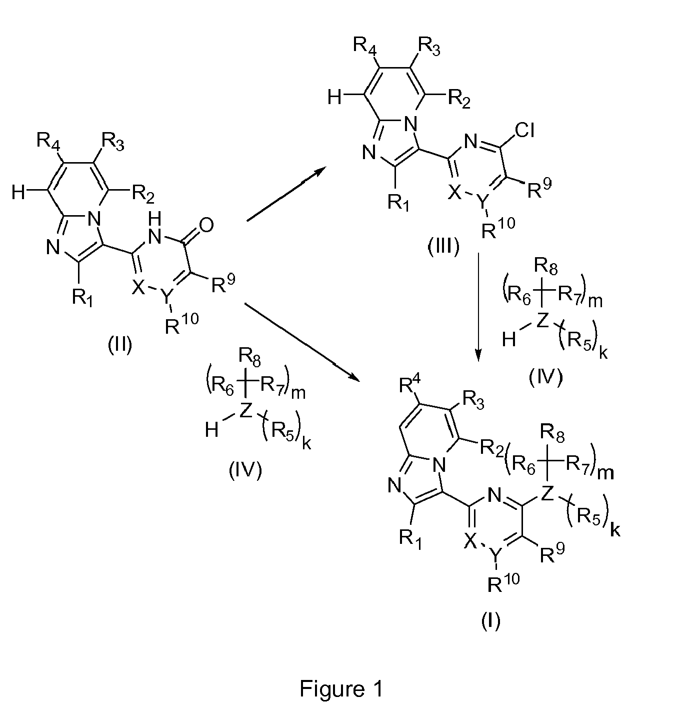

Compounds of formula (I) (where either X or Y is a nitrogen atom, the remaining atom being a carbon atom) may be obtained directly from compounds of formula (II) by treatment of (II) with an appropriate activating agent such as benzotriazolyloxy-tris-(dimethylamino)phosphonium hexafluorophosphate in the presence of a suitable base such as 1,8-diazabicyclo[5.4.0]undec-7-ene at temperatures ranging from 25 to 80 °C in a suitable solvent such as *N*,*N'*-dimethylformamide in the presence of a nucleophile of type (IV), following the protocol as described in the literature (J. Org. Chem., 2007, 72 (26),10194-10210). Alternatively, compounds of formula (II) may first be converted to chlorine-containing heteroaromatic compounds of formula (III), by treatment of (II) with a suitable chlorinating agent, for example phosphorous (V) oxychloride or phosphorous(V) chloride, at temperatures ranging from 25 °C to reflux. Compounds of formula (III) may then be converted to compounds of formula (I) by reaction with an appropriate nucleophile of formula (IV), such as an amine, in the presence of a base such as *N*,*N'*-diisopropylethylamine in a solvent such as *N*,*N'*-dimethylformamide at temperatures ranging from ambient temperature to reflux with or without the use of microwave irradiation.

Compounds of general formula (II) may be obtained as illustrated in Figure 2.

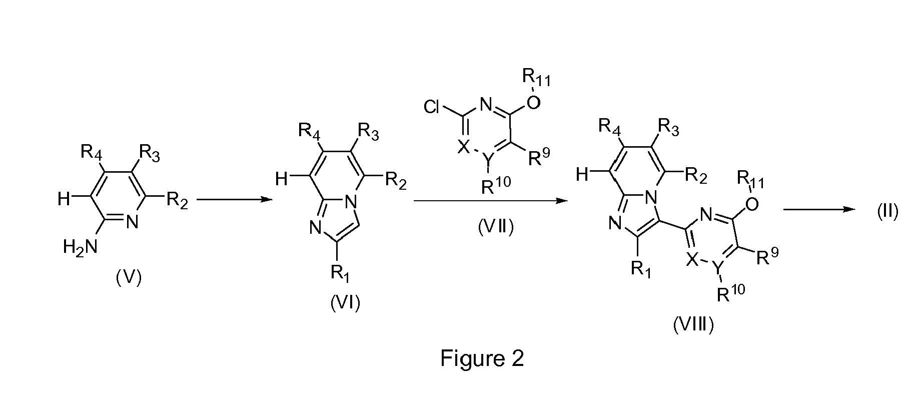

Treatment of 2-aminopyridines of formula (V) with a suitable alkylating agent such as 2-chloro- or 2-bromoacetaldehyde in the presence of a base, for example sodium hydrogen carbonate, in a suitable solvent such as acetonitrile or propan-2-ol at temperatures ranging from ambient temperature to reflux gives rise to imidazo[1,2-a]pyridines of formula (VI). Reaction of compounds of formula (VI) with compounds of formula (VII) under palladium-catalyzed coupling conditions with a suitable catalyst such as tetrakis(triphenylphosphine)palladium(0) or the catalytically active species generated from palladium(II)acetate/triphenylphosphine in the presence of a base, for example potassium acetate or potassium carbonate, in a solvent such as dioxane, ethanol or *N*,*N'*-dimethylacetamide or a mixture thereof at temperatures ranging from 100-160 °C with or without microwave irradiation, furnishes compounds of formula (VIII). In the particular case where R₁₁ = Me, treatment of compounds of formula (VIII) with a suitable reagent such as trimethylsilylchloride/sodium iodide in a suitable solvent such as acetonitrile at reflux or with potassium hydroxide in ethanol under microwave heating, gives rise to desired compounds of formula (II).

In the particular case where R₁ = H, X = N and Y = C, compounds of formula (II) may be prepared by an alternative synthetic approach as shown in Figure 3: 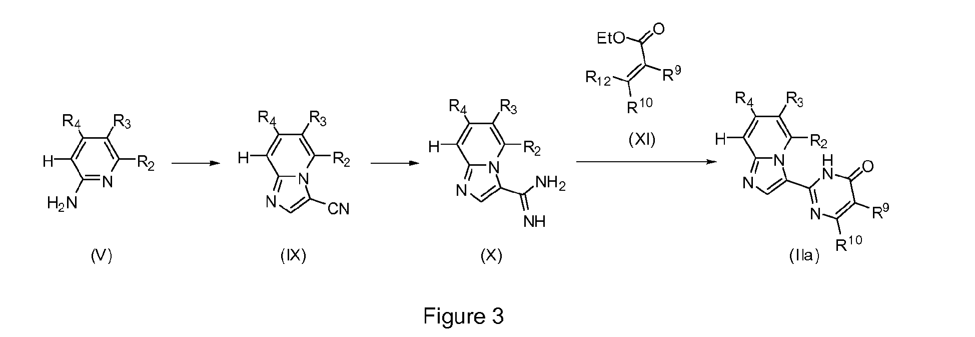 Imidazo[1,2-a]pyridine-3-carbonitriles of formula (IX) may be prepared by treatment of 2-aminopyridines of formula (V) with 3-methoxyacrylonitrile in the presence of N-bromosuccinimide in a suitable solvent such as dioxane or water at temperatures ranging from ambient temperature to reflux. Cyano derivatives of formula (IX) may be converted to the corresponding amidines of formula (X) by first formation of the corresponding imidate by reaction with alkoxy derivatives such as sodium methoxide or sodium ethoxide in a suitable alcoholic solvent at temperatures ranging from 0 °C to reflux followed by addition of ammonium chloride or ammonium hydroxide or by treatment with a suitable Lewis acid such as trimethylaluminium followed by addition of ammonium chloride in a suitable solvent such as toluene at temperatures ranging from 60 °C to reflux. Amidines of formula (X) may be reacted with unsaturated esters of formula (XI) (where R₁₂ is -OH, -OMe, -OEt or -NMe₂) to give pyrimidin-4-ones of formula (II). Such reactions may be carried out in the presence of a suitable base such as sodium ethoxide, triethylamine or sodium carbonate in a solvent such as ethanol, isopropanol, tetrahydrofuran, or water at temperatures ranging from ambient temperature to reflux.

Compounds of general formula (I) may be prepared via another synthetic pathway, as shown in figure 4.

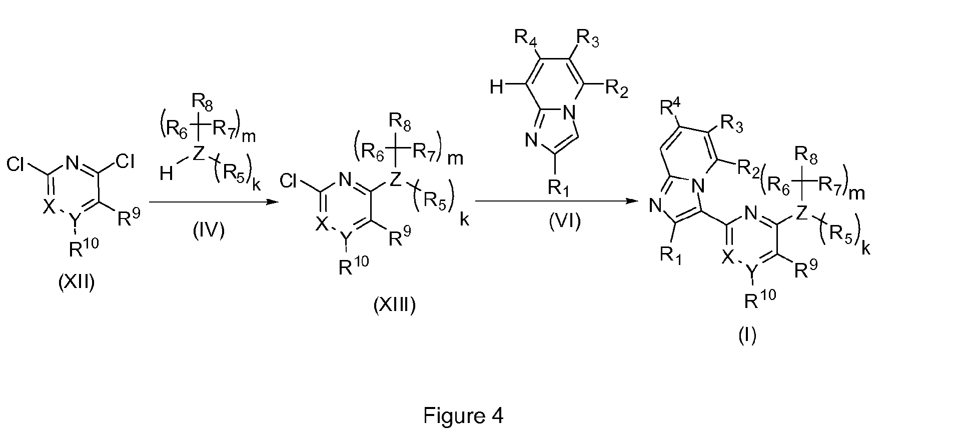

Treatment of bis-chloro heteroaromatic compounds of formula (XII) with nucleophiles (IV) in the presence of a base, such as *N*,*N'*-diisopropylethylamine, in an aprotic solvent such as *N*,*N'*-dimethylformamide at temperatures ranging from ambient temperature to reflux gives rises to compounds of formula (XIII). Reaction of compounds of formula (XIII) with aforementioned intermediates of formula (VI) using a suitable catalyst such as tetrakis(triphenylphosphine)palladium(0) or the catalytically active species generated from palladium(II)acetate/triphenylphosphine in the presence of a base, for example potassium acetate or potassium carbonate, in a solvent such as dioxane, ethanol or *N*,*N'*-dimethylacetamide or a solvent mixture thereof at temperatures ranging from 100-160 °C with or without microwave irradiation gives rise to desired compounds of formula (I).

In the particular cases of compounds of formula (I) bearing a halogen atom at R₃, R₉ or R₁₀, further reaction with a suitable boronic acid or boronate under Suzuki-Miyaura reaction conditions (Miyaura, N.; Suzuki, A. Chem. Rev. 1995, 95, 2457) gives rise to compounds or formula (I) where the corresponding halogen atom at R₃, R₉ or R₁₀ has been replaced with an aliphatic, aromatic or heteroaromatic residue. Such reactions may be catalyzed by a suitable palladium catalyst such as tetrakis(triphenylphospino)palladium (0), in a solvent such as 1,4-dioxane, in the presence of a base such as potassium carbonate, at a temperature ranging from 80-110 °C. Alternatively, compounds of formula (I) bearing a halogen atom at R₃, R₉ or R₁₀, may be further reacted with a suitable amine in the presence of a palladium catalyst to give compounds of formula (I) where the corresponding halogen atom at R₃, R₉ or R₁₀ has been replaced with an amine residue. Such reactions may be catalyzed by a suitable palladium catalyst generated from, for example, tris(dibenzylideneacetone)dipalladium(0) and biphenyl-2-yl-di-tert-butylphosphine, in a solvent such as toluene, in the presence of a base such as sodium tert-butoxide, at a temperature ranging from 80-150 °C with or without the use of microwave irradiation.

In another particular case, compounds of formula (I) bearing a hydrogen atom at R₉, may be further reacted by treatment with a halogenating agent such as N-bromosuccinimide or molecular bromine in a solvent such as acetic acid or N,N'-dimethylformamide to give compounds of formula (I), where R₉ is now a halogen residue.

In yet another particular case, compounds of formula (I), where R₅ is a hydrogen atom, may undergo further reaction with a suitable base such as sodium hydride in a solvent such as N,N'-dimethylformamide followed by the addition of an alkylating agent, such as methyl iodide at temperatures ranging from 0 °C to reflux, to furnish compounds of formula (I), where R₅ is now an alkyl group.

In yet another particular case, compounds of formula (I), in which the residue at R₆, R₇ or R₈ contains, in part, an amine moiety functionalized with an appropriate protecting group such as tert-butoxycarbonyl (BOC), may be deprotected at the amine moiety under standard conditions (Greene's Protective Groups in Organic Synthesis, ISBN: 0471697540). The corresponding free amine may then be further functionalized under standard conditions to give the corresponding amides, sulphonamides, ureas, and N-alkylated and arylated amines.

The syntheses of the compounds of the invention and of the intermediates for use therein are illustrated by the following Examples (1-85) (including Preparation Examples (Preparations 1-43)) are given in order to provide a person skilled in the art with a sufficiently clear and complete explanation of the present invention, but should not be considered as limiting of the essential aspects of its subject, as set out in the preceding portions of this description.

### PREPARATION 1

### Imidazo[1,2-a]pyridine-6-carbonitrile

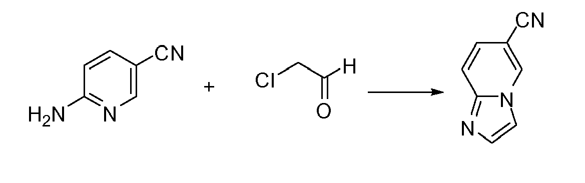

A solution of 6-aminonicotinonitrile (10 g, 80 mmol) in acetonitrile (300 mL) was treated with a 50% aqueous solution of 2-chloroacetaldehyde (26.4 mL, 210 mmol). The mixture was stirred and heated to reflux. After 6 hours, the mixture was cooled to room temperature. The mixture was concentrated to low bulk (approx. 100 mL), treated with saturated aqueous sodium hydrogencarbonate solution to neutral pH, and then extracted with dichloromethane (2 x 300 mL). The organic layer was dried (MgSO₄) and evaporated and the residue was stirred with diethyl ether (200 mL), filtered and dried in vacuum to give the title compound (22.54 g, 94%) as a pale brown solid.
LRMS (m/z): 144 (M+1)⁺.
¹H-NMR δ (CDCI₃): 7.29 (dd, 1 H), 7.71 (d, 1 H), 7.73 (d, 1 H), 7.80 (d, 1 H), 8.61-8.62 (m, 1 H).

### PREPARATION 2

### 6-Fluoroimidazo[1,2-a]pyridine

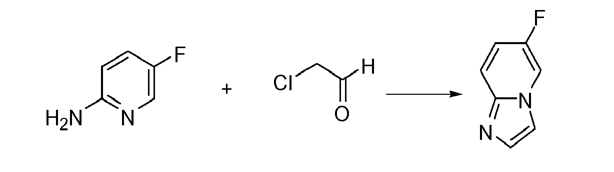

Obtained as a brown solid (93%) from 5-fluoropyridin-2-amine and 2-chloroacetaldehyde (50% in water) following the experimental procedure as described in preparation 1.
LRMS (m/z): 137 (M+1)⁺.
¹H-NMR δ (CDCI₃): 7.12 (m, 1 H), 7.57 - 7.67 (m, 2H), 7.70 (bs, 1 H), 8.04 - 8.13 (m, 1 H).

### PREPARATION 3

### 6-Chloroimidazo[1,2-a]pyridine

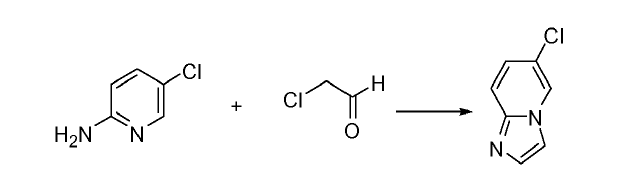

Obtained as a brown solid (71%) from 5-chloropyridin-2-amine and 2-chloroacetaldehyde (50% in water) following the experimental procedure as described in preparation 1 followed by purification of the crude product by flash chromatography (1:1 hexanes/ethyl acetate).
LRMS (m/z): 153 (M+1)⁺.
¹H-NMR δ (CDCI₃): 7.13 (d, 1 H), 7.53 - 7.60 (m, 2H), 7.66 (s, 1 H), 8.18 (s, 1 H).

### PREPARATION 4

### 3-(4-Hydroxypyrimidin-2-yl)imidazo[1,2-a]pyridine-6-carbonitrile

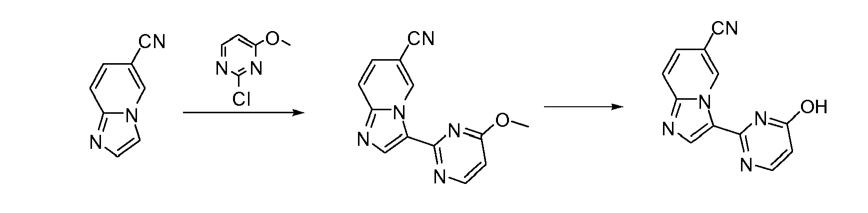

### a) 3-(4-Methoxypyrimidin-2-yl)imidazo[1,2-a]pyridine-6-carbonitrile

Nitrogen was bubbled for five minutes into a stirred mixture of 2-chloro-4-methoxypyrimidine (1.14 g, 7.9 mmol), imidazo[1,2-a]pyridine-6-carbonitrile (preparation 1, 0.75 g, 5.2 mmol), potassium carbonate (1.45 g, 10.5 mmol) and triphenylphosphine (0.55 g, 2.1 mmol) in 1,4-dioxane (10 mL) and ethanol (5 mL) in a microwave vessel. Then palladium (II) acetate (0.24 g, 1.1 mmol) was added and the vessel was sealed and subjected to microwave irradiation for 2 hours at 150 °C. The reaction was repeated 5 further times under the same conditions and the six experiments were combined and the solvent was evaporated. The residue was taken up in a mixture of ethyl acetate and water and filtered to remove an insoluble black solid. The organic layer was separated and extracted with 2M aqueous hydrochloric acid (3 x 80 mL). The combined aqueous layer was washed with diethyl ether and then treated with solid sodium hydrogencarbonate until a pH of approximately 6 was reached. The solid that formed was filtered and dried in vacuum to give a first crop of the title compound (2.65 g). The insoluble black solid was treated with 2M aqueous hydrochloric acid (2 x 60 mL) and filtered. The aqueous solution was treated with solid sodium hydrogencarbonate until a pH of approximately 6 was reached. The solid that formed was filtered and dried in vacuum to give a second crop of the title compound (0.77 g). Total yield = 3.42 g (43%).
LRMS (m/z): 252 (M+1)⁺.
¹H-NMR δ(DMSO-*d*₆): 4.08 (s, 3H), 6.88 (d, 1 H), 7.76 (dd, 1 H), 7.95 (dd, 1 H), 8.63 (s, 1 H), 8.67 (d, 1 H), 10.39 (bs, 1 H).

### b) 3-(4-Hydroxypyrimidin-2-yl)imidazo[1,2-a]pyridine-6-carbonitrile

Sodium iodide (11.8 g, 78.6 mmol) was added to a stirred suspension of 3-(4-methoxypyrimidin-2-yl)imidazo[1,2-a]pyridine-6-carbonitrile (preparation 4a, 3.3 g, 13.1 mmol) in acetonitrile (135 mL). Chlorotrimethylsilane (9.9 mL, 78.0 mmol) was added and the mixture was heated in a sealed tube at 80 °C for 24 hours and then cooled to ambient temperature. The solvent was removed under reduced pressure and the residue was suspended in water. Saturated aqueous sodium thiosulphate solution was added until the dark colour faded and a pale brown solid formed. The solid was filtered, washed with further saturated sodium thiosulphate solution and water and dried in vacuum to give the title compound (3.2 g, 99%) as a pale brown solid.
LRMS (m/z): 238 (M+1)⁺, 236 (M-1)⁻.
¹H-NMR δ(DMSO-*d*₆): 6.42 (d, 1 H), 7.82 (d, 1 H), 7.97 (d, 1 H), 8.27 (d, 1 H), 8.78 (s, 1 H), 10.43 (s, 1 H).

### PREPARATION 5

### 3-{4-[(3R)-Piperidin-3-ylamino]pyrimidin-2-yl)imidazo[1,2-a]pyridine-6-carbonitrile

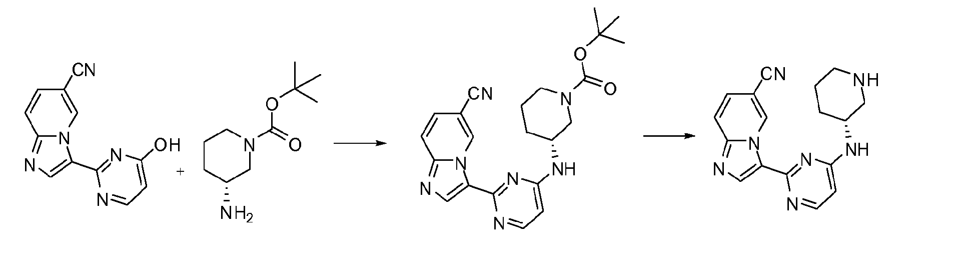

### a) Tert-butyl-(3R)-3-{[2-(6-cyanoimidazo[1,2-a]pyridin-3-yl)pyrimidin-4-yl]amino}piperidine-1-carboxylate

(Benzotriazol-1-yloxy)tris(dimethylamino)phosphonium hexafluorophosphate (0.480 g, 1.09 mmol) and 1,8-diazabicyclo[5.4.0]undec-7-ene (0.187 mL, 1.25 mmol) were added sequentially to a stirred solution of 3-(4-hydroxypyrimidin-2-yl)imidazo[1,2-a]pyridine-6-carbonitrile (preparation 4b, 0.2 g, 0.84 mmol) in *N*,*N'*-dimethylformamide (3 mL). After stirring for 15 minutes at ambient temperature, tert-butyl (3*R*)-3-aminopiperidine-1-carboxylate (0.418 g, 2.09 mmol) was added dropwise and the mixture was stirred for a further 16 hours at ambient temperature. After addition of water and extraction with ethyl acetate, the organic layer was washed with water, 4% aqueous sodium hydrogen carbonate solution, brine, dried (MgSO₄) and evaporated. The residue was purified by flash chromatography (99:1 to 98:2 dichloromethane/methanol) to obtain the title compound (70%) as a yellow solid.
LRMS (m/z): 420 (M+1)⁺.
¹H-NMR δ (DMSO-*d*₆):0.92 - 1.65 (m, 15H), 3.66 - 4.04 (m, 3H), 6.29 - 6.69 (m, 1 H), 7.91 (d, 2H), 8.12 - 8.33 (m, 1H), 8.40 - 8.68 (m, 1 H), 10.28 - 10.61 (m, 1 H).

### b) 3-{4-[(3R)-piperidin-3-ylamino]pyrimidin-2-yl)imidazo[1,2-a]pyridine-6-carbonitrile

Trifluoroacetic acid (1.0 mL, 12 mmol) was added dropwise to a solution of tert-butyl-(3*R*)-3-{[2-(6-cyanoimidazo[1,2-*a*]pyridin-3-yl)pyrimidin-4-yl]amino}piperidine-1-carboxylate (preparation 5a, 0.25 g, 0.6 mmol) in dichloromethane (5 mL) and the mixture was stirred at ambient temperature for one hour. After the reaction was complete, water was added and the mixture was neutralized with solid sodium hydrogen carbonate and then extracted with dichloromethane. The organic layer was dried (MgSO₄) and evaporated and the residue was purified by flash chromatography (96:4 dichloromethane/methanol) obtaining the title compound (99%) as a pale yellow solid.
LRMS (m/z): 320 (M+1)⁺.
¹H-NMR δ (DMSO-*d*₆): 1.08 - 1.87 (m, 4H), 2.01 (bs, 1 H), 2.55 - 3.35 (m, 4H), 4.13 (bs, 1 H), 6.45 (d, 1 H), 7.49 - 7.79 (m, 2H), 7.91 (d, 1 H), 8.21 (bs, 1 H), 8.55 (bs, 1 H), 10.44 (bs, 1 H).

### PREPARATION 6

### (R)-3-(4-(methyl(piperidin-3-yl)amino)pyrimidin-2-yl)imidazo[1,2-a]pyridine-6-

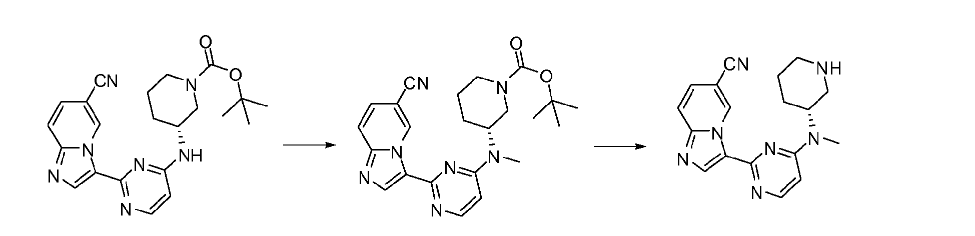

### a) Tert-butyl-3-[[2-(6-cyano-1,8a-dihydroimidazo[1,2-a]pyridin-3-yl)pyrimidin-4-yl](methyl)amino]piperidine-1-carboxylate

Sodium hydride (60% dispersion in mineral oil, 0.31 g, 7.75 mmol) was added portionwise to a stirred solution of *(R)-tert-butyl* 3-(2-(6-cyanoimidazo[1,2-a]pyridin-3-yl)pyrimidin-4-ylamino)piperidine-1-carboxylate (preparation 5a, 1.67 g, 3.9 mmol) in *N*,*N'*-dimethylformamide (15 mL). When hydrogen evolution had ceased, methyl iodide (0.48 mL, 7.7 mmol) was added and the reaction mixture was stirred overnight at ambient temperature. The solvent was removed and the residue was partitioned between ethyl acetate and water. The organic layer was dried (MgSO₄) and evaporated and the residue was purified by reverse phase chromatography (C-18 silica from Waters, water/acetonitrile/methanol as eluents [0.1% v/v formic acid buffered] 0% to 100%) to give the title compound (0.26 g, 16%) as an oil.
LRMS (m/z): 434 (M+1)⁺.
¹H-NMR δ (CDCI₃): 1.39 - 1.65 (m, 9H), 1.64 - 2.08 (m, 4H), 2.64 - 2.89 (m, 1 H), 3.09 (bs, 3H), 4.18 (bs, 2H), 6.41 - 6.48 (m, 1H), 7.40 (dd, 1H), 7.80 (d, 1H), 8.32 (d, 1 H), 8.65 (bs, 1 H), 10.55 (bs, 1 H).

**b) (*R*)-3-(4-(methyl(piperidin-3-yl)amino)pyrimidin-2-yl)imidazo[1,2-a]pyridine-6-**Obtained as a solid (60%) from *(R)-tert-butyl* 3-((2-(6-cyanoimidazo[1,2-a]pyridin-3-yl)pyrimidin-4-yl)(methyl)amino)piperidine-1-carboxylate (preparation 6a) following the experimental procedure as described in preparation 5b.
LRMS (m/z): 334 (M+1)⁺.

### PREPARATION 7

### 3-{4-[(3S)-piperidin-3-ylamino]pyrimidin-2-yl)imidazo[1,2-a]pyridine-6-carbonitrile

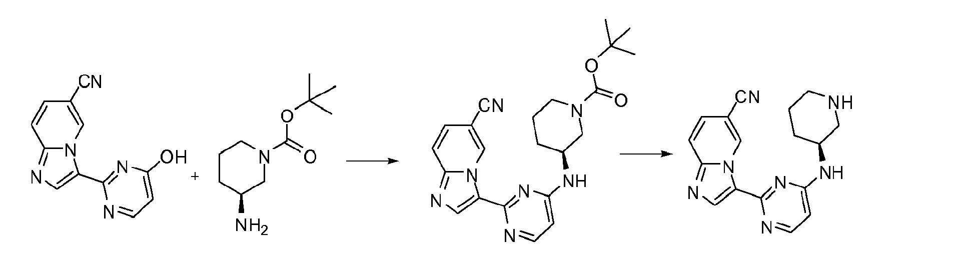

### a) Tert butyl-(3S)-3-{[2-(6-cyanoimidazo[1,2-a]pyridin-3-yl)pyrimidin-4-yl]amino)piperidine-1-carboxylate

Obtained as a brown solid (66%) from 3-(4-hydroxypyrimidin-2-yl)imidazo[1,2-a]pyridine-6-carbonitrile (preparation 4b) and tert-butyl (3*S*)-3-aminopiperidine-l-carboxylate following the experimental procedure as described in preparation 5a. After the reaction was complete the mixture was partitioned between water and ethyl acetate. The organic layer was washed with water, saturated aqueous sodium hydrogen carbonate solution, brine, dried (MgSO₄) and evaporated and the residue was purified by flash chromatography (98:2 to 96:4 dichloromethane/methanol).
LRMS (m/z): 420 (M+1)⁺.
¹H-NMR δ(CDCI₃): 1.60 (s, 9H), 6.30 (d, 1 H), 7.41 (d, 1 H), 7.78 (d, 1 H), 8.30 (d, 1H),8.61 (s,1H), 10.55 (s, 1H).

**b) 3-{4-[(3*S*)-piperidin-3-ylamino]pyrimidin-2-yl)imidazo[1,2-*a*]pyridine-6-carbonitrile**

Obtained as a solid (98%) from *tert-*butyl (3*S*)-3-{[2-(6-cyanoimidazo[1,2-a]pyridin-3-yl)pyrimidin-4-yl]amino}piperidine-1-carboxylate (preparation 7a) following the experimental procedure as described in preparation 5b
LRMS (m/z): 320 (M+1)⁺.
¹H-NMR δ (CDCI₃): 2.91 (s, 2H), 3.23 (dt, 1 H), 5.70 (s, 1 H), 6.40 - 6.12 (m, 1 H), 7.45 - 7.30 (m, 1 H), 7.88 - 7.67 (m, 1 H), 8.25 (s, 1 H), 8.59 (d, 1 H), 10.55 (s, 1 H).

### PREPARATION 8

### 2-(6-Fluoroimidazo[1,2-a]pyridin-3-yl)pyrimidin-4-ol

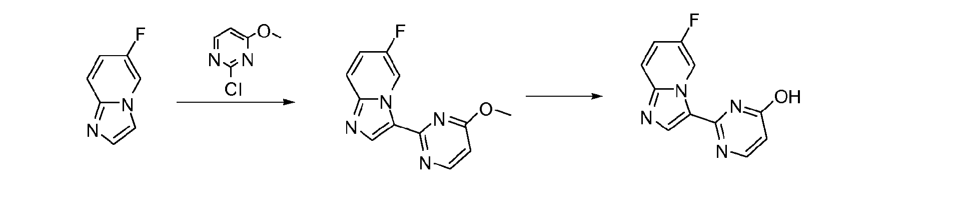

### a) 6-Fluoro-3-(4-methoxypyrimidin-2-yl)imidazo[1,2-a]pyridine

2-chloro-4-methoxypyrimidine (7.0 g, 48 mmol) and 6-fluoroimidazo[1,2-a]pyridine (preparation 2, 4.40 g, 32 mmol) were reacted according to the experimental procedure as described in preparation 4a. Ethyl acetate was added to the crude reaction mixture and the suspension was filtered through Celite®. The filtrate was washed with water and extracted several times with 2M aqueous hydrochloric acid. The combined aqueous layer was washed with diethyl ether and then neutralized with 8M aqueous sodium hydroxide solution. The product was extracted into chloroform and the organic phase was dried (MgSO₄ and evaporated to afford the title compound (90%) as a grey solid, which was used without further purification.
LRMS (m/z): 245 (M+1)⁺.
¹H-NMR δ (CDCI₃): 4.11 (s, 3H), 6.58 (d, 1 H), 7.60 (q, 1 H), 7.71 (dd, 1 H), 8.49 (d, 1H),8.61 (s, 1H),9.98 (dd, 1 H).

### b) 2-(6-Fluoroimidazo[1,2-a]pyridin-3-yl)pyrimidin-4-ol

To a solution of 6-fluoro-3-(4-methoxypyrimidin-2-yl)imidazo[1,2-a]pyridine (preparation 8a, 1.5 g, 6.1 mmol) in ethanol (10mL) was added a 35% aqueous solution of potassium hydroxide (9.8 mL, 61.3 mmol). The reaction mixture was subjected to microwave irradiation for 1 hour at 120 °C. The organic solvent was removed under reduced pressure and the aqueous phase was neutralized with 5M hydrochloric acid. The precipitate was filtered, washed with water and dried to afford the title compound (70%) as a white solid, which was used without further purification.
LRMS (m/z): 231 (M+1)⁺.
¹H-NMR δ (DMSO-*d*₆): 6.31 (s, 1 H), 7.77 (d, 1 H), 8.17 (s, 1 H), 8.73 (bs, 1 H), 9.94 (bs, 1 H), 12.75 (s, 1 H).

### PREPARATION 9

### (R)-2-(6-Fluoroimidazo[1,2-a]pyridin-3-yl)-N-(piperidin-3-yl)pyrimidin-4-amine

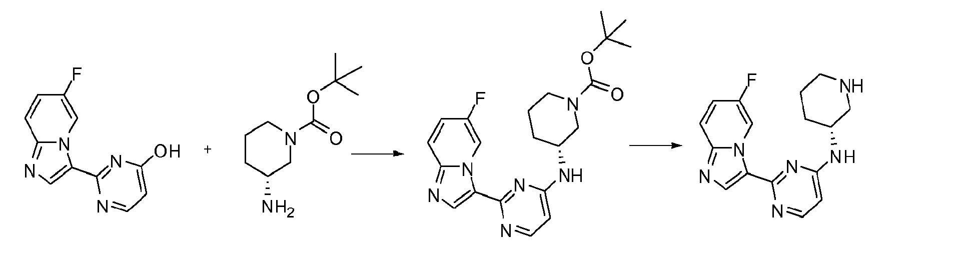

### a) Tert-butyl-(3R)-3-({[2-(6-fluoroimidazo[1,2-a]pyridin-3-yl)pyrimidin-4-yl]oxy}amino)piperidine-1-carboxylate

Obtained as a yellow solid (65%) from 2-(6-fluoroimidazo[1,2-a]pyridin-3-yl)pyrimidin-4-ol (preparation 8b) and (*R*)-*tert*-butyl 3-aminopiperidine-l-carboxylate following the experimental procedure as described in preparation 5a. The crude product was purified by reverse phase chromatography (C-18 silica from Waters, water/acetonitrile/methanol as eluents [0.1% v/v formic acid buffered] 0% to 100%).
LRMS (m/z): 413 (M+1)⁺.
¹H-NMR δ (CDCI₃): 1.44 (s, 9H), 2.13 - 1.53 (m, 4H), 3.65 - 3.23 (m, 4H), 5.14 (s, 1 H), 6.24 (d, 1 H), 7.23 (ddd, 1 H), 7.68 (dd, 1 H), 8.27 (d, 1 H), 8.54 (s, 1 H), 9.99 (s, 1H ).

### b) (R)-2-(6-Fluoroimidazo[1,2-a]pyridin-3-yl)-N-(piperidin-3-yl)pyrimidin-4-amine

Obtained as a yellow solid (70%) from (*R*)-*tert*-butyl 3-(2-(6-fluoroimidazo[1,2-a]pyridin-3-yl)pyrimidin-4-ylamino)piperidine-1-carboxylate (preparation 9a) following the experimental procedure as described in preparation 5b.
LRMS (m/z): 313 (M+1)⁺.
¹H-NMR δ (CDCl₃): 1.65 (dd, 2H), 1.90 - 1.71 (m, 3H), 2.00 - 1.89 (m, 2H), 2.88 (d, 3H), 5.57 (d, 1 H), 6.22 (d, 1 H), 7.22 (ddd, 1 H), 7.66 (dd, 1 H), 8.22 (d, 1 H), 8.53 (s, 1H),9.98(dd, 1 H).

### PREPARATION 10

### 1-{[2-((3R)-3-{[2-(6-fluoroimidazo[1,2-a]pyridin-3-yl)pyrimidin-4-yl]amino}piperidin-1-yl)-2-methylpropanoyl]oxy}pyrrolidine-2,5-dione

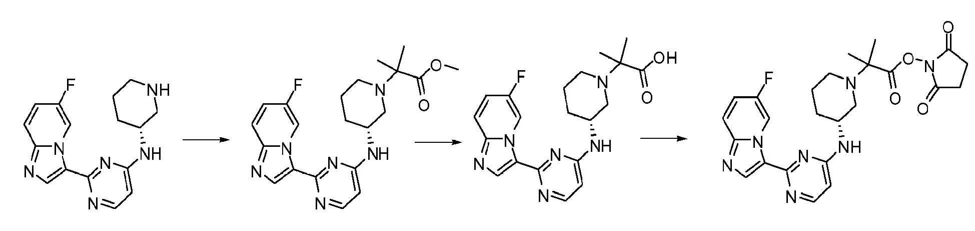

### a) Methyl 2-((3R)-3-{[2-(6-fluoroimidazo[1,2-a]pyridin-3-yl)pyrimidin-4-yl]amino}piperidin-1-yl)-2-methylpropanoate

A solution of (*R*)-2-(6-fluoroimidazo[1,2-a]pyridin-3-yl)-N-(piperidin-3-ylpyrimidin-4-amine (preparation 9b, 0.100 g, 0.32 mmol), ethyl 2-bromo-2-methylpropanoate (0.137 g, 0.70 mmol) and potassium carbonate (0.066 g, 0.48 mmol) in *N*,*N'-*dimethylformamide (1 mL) was stirred and heated to 80 °C. After 16 hours, water was added to the reaction mixture and the mixture was extracted with dichloromethane. The organic layer was dried (MgSO₄ and evaporated and the residue was purified by reverse phase chromatography (C-18 silica from Waters, water/acetonitrile/methanol as eluents [0.1% v/v formic acid buffered] 0% to 100%) to give the title compound (0.090 g, 66%) as a solid.
LRMS (m/z): 413 (M+1)⁺.

### b) 2-((3R)-3-{[2-(6-fluoroimidazo[1,2-a]pyridin-3-yl)pyrimidin-4-yl]amino}piperidin-1-yl)-2-methylpropanoic acid

Potassium hydroxide (0.028 g, 0.42 mmol) was added to a stirred solution of (R)-ethyl 2-(3-(2-(6-fluoroimidazo[1,2-a]pyridin-3-yl)pyrimidin-4-ylamino)piperidin-1-yl)-2-methylpropanoate (preparation 10a, 0.090 g, 0.21 mmol) in methanol (1 mL). The reaction mixture was stirred at room temperature overnight and then heated to 50 °C and stirred a further 24 hours. The mixture was neutralized with 2M aqueous hydrochloric acid and then extracted with chloroform. The organic layer was dried (MgSO₄) and evaporated to give the title compound (0.050 g, 60%) as an oil which was used without further purification.
LRMS (m/z): 399 (M+1)⁺.

### c)1-{[2-((3R)-3-{[2-(6-fluoroimidazo[1,2-a]pyridin-3-yl)pyrimidin-4-yl]amino}piperidin-1-yl)-2-methylpropanoyl]oxy}pyrrolidine-2,5-dione

N-Hydroxysuccinimide (0.016 g, 0.14 mmol) and 1,3-diisopropylcarbodiimide (0.022 mL, 0.14 mmol) were added sequentially to a cooled (ice-bath), stirred solution of (R)-2-(3-(2-(6-fluoroimidazo[1,2-a]pyridin-3-yl)pyrimidin-4-ylamino)piperidin-1-yl)-2-methylpropanoic acid (preparation 10b, 0.050 g, 0.13 mmol) in *N,N*'-dimethylformamide (1 mL). After stirring overnight at ambient temperature, water was added to the reaction mixture and the mixture was extracted with dichloromethane. The organic layer was dried (MgSO₄) and evaporated to give the crude title compound (0.050 g) as an oil which was used without further purification.
LRMS (m/z): 496 (M+1)⁺.

### PREPARATION 11

### (R)-2-(6-fluoroimidazo[1,2-a]pyridin-3-yl)-N-methyl-N-(piperidin-3-yl)pyrimidin-4-amine

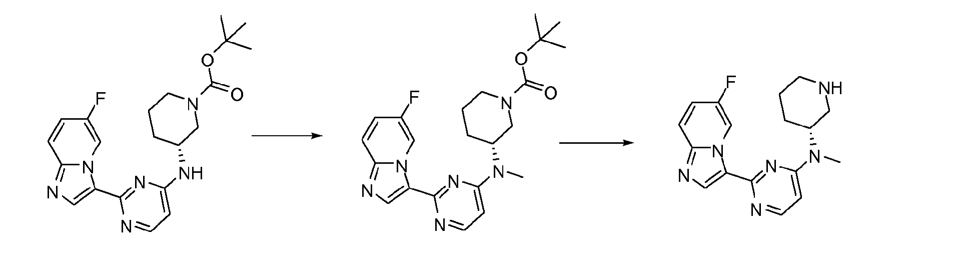

### a) (R)-tert-butyl 3-((2-(6-fluoroimidazo[1,2-a]pyridin-3-yl)pyrimidin-4-yl)(methyl)amino)piperidine-1-carboxylate

Obtained from tert-butyl (3*R*)-3-({[2-(6-fluoroimidazo[1,2-*a*]pyridin-3-yl)pyrimidin-4-yl]oxy}amino)piperidine-1-carboxylate (preparation 9a) and methyl iodide following the experimental procedure as described in preparation 6a. The crude product was purified by flash chromatography (0-100% ethyl acetate in hexane) to give the title compound (0.52 g, 55%) as an oil.
LRMS (m/z): 427 (M+1)⁺.
¹H-NMR δ (CDCl₃): 1.46 (bs, 9H), 1.67 (bs, 1 H), 1.72 - 1.93 (m, 2H), 1.95 - 2.05 (m, 2H), 2.68 (bs, 2H), 2.65 - 2.88 (m, 2H), 3.07 (s, 3H), 6.37 (d, 1H), 7.17 - 7.26 (m, 1 H), 7.68 (dd, 1 H), 8.31 (d, 1 H), 8.54 (s, 1 H), 9.96 (dd, 1 H)

### b) (R)-2-(6-fluoroimidazo[1,2-a]pyridin-3-yl)-N-methyl-N-(piperidin-3-yl)pyrimidin-4-amine

Obtained as a solid (51%) from (*R*)-*tert*-butyl 3-((2-(6-fluoroimidazo[1,2-a]pyridin-3-yl)pyrimidin-4-yl)(methyl)amino)piperidine-1-carboxylate (preparation 11a) following the experimental procedure as described in preparation 5b. The crude product was purified by reverse phase chromatography (C-18 silica from Waters, water/acetonitrile/methanol as eluents [0.1% v/v formic acid buffered] 0% to 100%).
LRMS (m/z): 327 (M+1)⁺.
¹H-NMR δ (CDCl₃): 1.60 - 2.02 (m, 6H), 2.58 (td, 1 H), 2.79 (t, 1 H), 3.04 (s, 3H), 3.13 (t, 1 H), 5.30 (s, 1 H), 6.33 (d, 1 H), 7.22 (ddd, 1 H), 7.67 (dd, 1 H), 8.27 (d, 1H), 8.54 (s, 1 H), 9.98 (ddd, 1 H)

### PREPARATION 12

### N-ethyl-2-(6-fluoroimidazo[1,2-a]pyridin-3-yl)-N-[(3R)-piperidin-3-yl]pyrimidin-4-amine

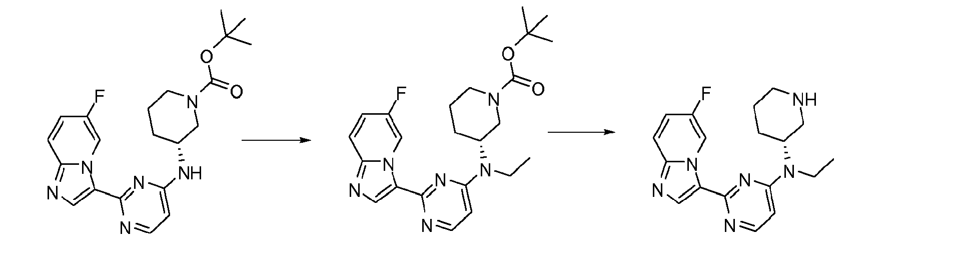

### a) (R)-tert-butyl-3-(ethyl(2-(6-fluoroimidazo[1,2-a]pyridin-3-yl)pyrimidin-4-yl)amino)piperidine-1-carboxylate

Obtained as a white solid (58%) from *tert*-butyl (3*R*)-3-({[2-(6-fluoroimidazo[1,2-a]pyridin-3-yl)pyrimidin-4-yl]oxy}amino)piperidine-1-carboxylate (preparation 9a) and ethyl iodide following the experimental procedure as described in preparation 6a. The crude product was purified by flash chromatography (9:1 dichloromethane/methanol).
LRMS (m/z): 441 (M+1)⁺.

### b) N-ethyl-2-(6-fluoroimidazo[1,2-a]pyridin-3-yl)-N-[(3R)-piperidin-3-yl]pyrimidin-4-amine

Obtained as a solid (100%) from (*R*)-*tert*-butyl 3-(ethyl(2-(6-fluoroimidazo[1,2-a]pyridin-3-yl)pyrimidin-4-yl)amino)piperidine-1-carboxylate (preparation 12a) following the experimental procedure as described in preparation 5b.
LRMS (m/z): 341 (M+1)⁺.

### PREPARATION 13

### N-(cyclopropylmethyl)-2-(6-fluoroimidazo[1,2-a]pyridin-3-yl)-N-[(3R)-piperidin-3-yl]pyrimidin-4-amine

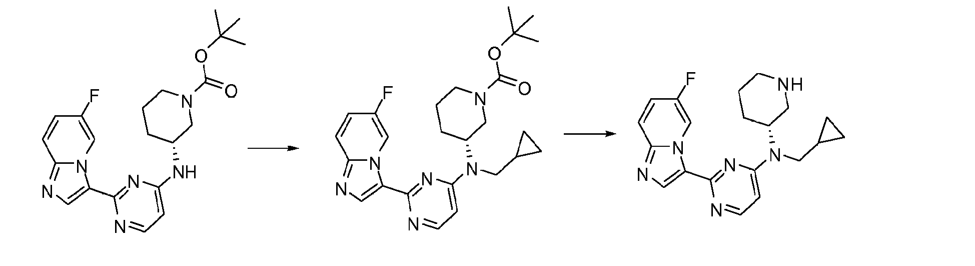

### a) Tert-butyl (3R)-3-{(cyclopropylmethyl)[2-(6-fluoroimidazo[1,2-a]pyridin-3-yl)pyrimidin-4-yl]amino}piperidine-1-carboxylate

Obtained as a white solid (47%) from *tert*-butyl (3*R*)-3-({[2-(6-fluoroimidazo[1,2-a]pyridin-3-yl)pyrimidin-4-yl]oxy}amino)piperidine-1-carboxylate (preparation 9a) and (bromomethyl)cyclopropane following the experimental procedure as described in preparation 6a. The crude product was purified by flash chromatography (92:8 dichloromethane/methanol).
LRMS (m/z): 467 (M+1)⁺.
¹H-NMR δ (CDCl₃): 0.39 (bs, 2H), 0.64 (bs, 2H), 1.12 (bs, 1H), 1.46 (bs, 3H), 1.60 (bs, 6H), 1.87 (bs, 2H), 2.06 (bs, 2H), 2.68 (bs, 1 H), 2.88 (bs, 1 H), 3.37 (bs, 1 H), 3.55 (bs, 2H), 4.02 - 4.43 (m, 2H), 6.48 (bs, 1 H), 7.22 (bs, 1 H), 7.68 (bs, 1 H), 8.30 (bs, 1 H), 8.53 (bs, 1 H), 10.01 (bs, 1 H)

### b) N-(cyclopropylmethyl)-2-(6-fluoroimidazo[1,2-a]pyridin-3-yl)-N-[(3R)-piperidin-3-yl]pyrimidin-4-amine

Obtained as a solid (85%) from *tert*-butyl (3*R*)-3-{(cydopropylmethyl)[2-(6-fluoroimidazo[1,2-*a*]pyridin-3-yl)pyrimidin-4-yl]amino}piperidine-1-carboxylate (preparation 13a) following the experimental procedure as described in preparation 5b.
LRMS (m/z): 367 (M+1)⁺.
¹H-NMR δ (CDCl₃): 0.39 (d, 2H), 0.63 (d, 2H), 1.12 (bs, 1 H), 1.74 - 1.94 (m, 2H), 2.03 (bs, 2H), 2.53 - 2.63 (m, 1 H), 2.81 (t, 2H), 3.06 - 3.25 (m, 3H), 3.39 (bs, 2H), 3.47 (bs, 2H), 6.44 (d, 1 H), 7.23 (ddd, 1 H), 7.68 (dd, 1 H), 8.27 (d, 1 H), 8.53 (s, 1 H), 10.02 (dd, 1 H)

### PREPARATION 14

### 2-Imidazo[1,2-a]pyridin-3-ylpyrimidin-4-ol

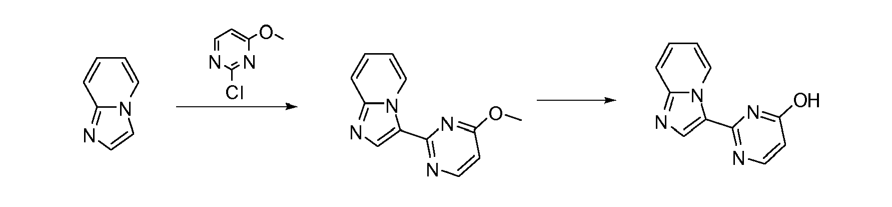

### a) 3-(4-Methoxypyrimidin-2-yl)imidazo[1,2-a]pyridine

Obtained as an off-white solid (39%) from 2-chloro-4-methoxypyrimidine and imidazo[1,2-*a*]pyridine following the experimental procedure as described in preparation 4a.
LRMS (m/z): 227 (M+1)⁺.
¹H-NMR δ (DMSO-*d₆*): 4.12 (s, 3H), 6.84 (d, 1H), 7.26 (t, 1H), 7.57-7.50 (m, 1 H), 7.83 (dd, 1 H), 8.56 (s, 1 H), 8.65 (dd, 1 H), 9.93 (dd, 1 H).

### b) 2-Imidazo[1,2-a]pyridin-3-ylpyrimidin-4-ol

A stirred mixture of 3-(4-methoxypyrimidin-2-yl)imidazo[1,2-a]pyridine (preparation 14a, 0.38 g, 1.7 mmol) and aqueous 48% hydrogen bromide (25 mL) was heated at 100 °C. After 3 hours, the solvent was evaporated and the residue was treated with isopropyl alcohol. The solid was collected by filtration and dried in vacuum to give the hydrobromide salt of the title compound (0.53 g, 99%) as an off-white solid.
LRMS (m/z): 213 (M+1)⁺.
¹H-NMR δ (DMSO-*d₆*): 6.63 (d, 1H), 7.66 (t, 1H), 8.13 - 8.00 (m, 2H), 8.42 (bs, 1 H), 8.96 (s, 1 H), 10.16 (d, 1 H).

### PREPARATION 15

### 2-Chloro-5-fluoro-4-methoxypyrimidine

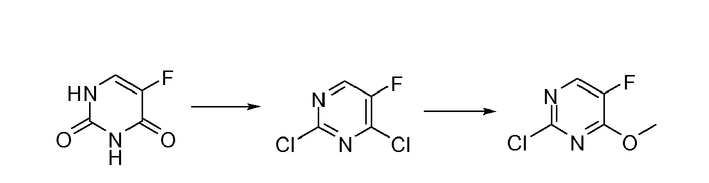

### a) 2,4-Dichloro-5-fluoropyrimidine

To a stirred mixture of 5-fluoropyrimidine-2,4(1H,3H)-dione (3.0 g, 23 mmol) and phosphorous pentachloride (14.41 g, 69 mmol) was added phosphorous oxychloride (12.6 mL, 130 mmol). The reaction mixture was heated to reflux, stirred 5 hours and then cooled to ambient temperature and stirred overnight. The mixture was carefully poured onto ice/water (600 mL) and stirred for 1 hour. Sodium chloride was added and the product was extracted into dichloromethane. The combined organic layer was dried (MgSO₄), filtered and evaporated to give the title compound (84%) as a yellow solid.
LRMS (m/z): 167 (M+1)⁺.
¹H-NMR δ (CDCl₃): 8.49 (s, 1 H)

### b) 2-Chloro-5-fluoro-4-methoxypyrimidine

Sodium (0.45 g, 19.6 mmol) was added in portions to methanol (20 mL). Once all the sodium had reacted, a solution of 2,4-dichloro-5-fluoropyrimidine (preparation 15a, 3.25 g, 19.5 mol) in methanol (10 mL) was added and the mixture was stirred at ambient temperature overnight. The mixture was diluted with water and extracted with diethyl ether. The organic layer was washed with water, brine, dried (MgSO₄), filtered and the solvent was removed under reduced pressure to give the title compound (81%) as an orange solid, which was used without further purification.
LRMS (m/z): 163 (M+1)⁺.
¹H-NMR δ (CDCl₃): 4.11 (s, 3H), 8.20 (d, 1 H)

### PREPARATION 16

### 5-Fluoro-2-(6-fluoroimidazo[1,2-a]pyridin-3-yl)pyrimidin-4-ol

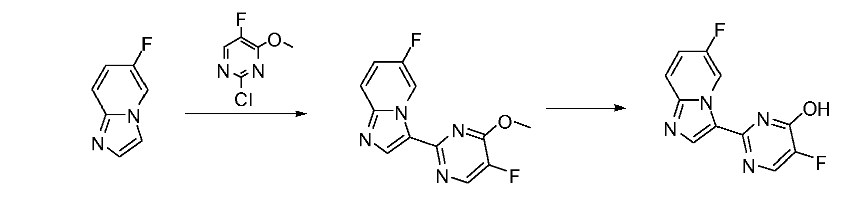

### a) 6-Fluoro-3-(5-fluoro-4-methoxypyrimidin-2-yl)imidazo[1,2-a]pyridine

2-Chloro-5-fluoro-4-methoxypyrimidine (preparation 15b, 1.10 g, 6.8 mmol) and 6-fluoroimidazo[1,2-a]pyridine (preparation 2, 1.3 g, 9.5 mmol) were reacted according to the experimental procedure as described in preparation 4a. The crude product obtained was purified by flash chromatography (10:1 to 1:4 hexanes/ethyl acetate) to give the title compound (10%) as a grey solid.
LRMS (m/z): 263 (M+1)⁺.
¹H-NMR δ (CDCl₃): 4.21 (s, 3H), 7.11 (ddd, 1 H), 7.28 (dd, 1 H), 8.38 (d, 1 H), 8.53 (s, 1 H), 9.87 (dd, 1 H)

### b) 5-Fluoro-2-(6-fluoroimidazo[1,2-a]pyridin-3-yl)pyrimidin-4-ol

To a solution of 6-fluoro-3-(5-fluoro-4-methoxypyrimidin-2-yl)imidazo[1,2-a]pyridine (preparation 16a, 200 mg, 0.76 mmol) in ethanol (4 mL) was added a 35% aqueous solution of potassium hydroxide (1.1 mL, 6.9 mmol). The reaction mixture was subjected to microwave irradiation for 1 hour at 130 °C. The organic solvent was removed under reduced pressure and the aqueous phase was washed with chloroform then neutralized with 5M hydrochloric acid. The precipitate was filtered and discarded and the filtrate was evaporated to dryness. The residue was triturated with several portions of a chloroform/methanol mixture and then dried in vacuum to give the title compound (59%) as a grey solid, which was used without further purification.
LRMS (m/z): 249 (M+1)⁺.

### PREPARATION 17

### (R)-5-fluoro-2-(6-fluoroimidazo[1,2-a]pyridin-3-yl)-N-(piperidin-3-yl)pyrimidin-4-amine

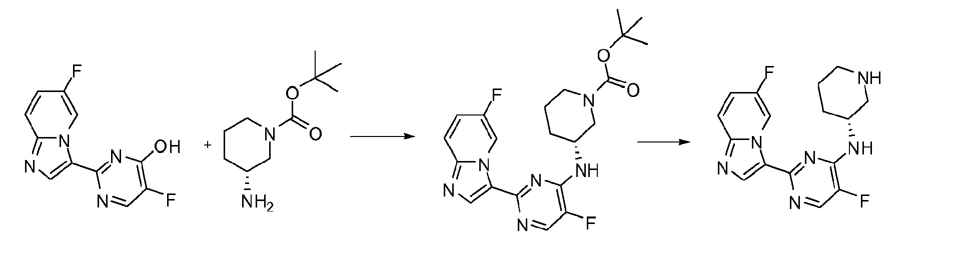

### a) (R)-tert-butyl 3-(5-fluoro-2-(6-fluoroimidazo[1,2-a]pyridin-3-yl)pyrimidin-4-ylamino)piperidine-1-carboxylate

Obtained as a solid (22%) from 5-fluoro-2-(6-fluoroimidazo[1,2-a]pyridin-3-yl)pyrimidin-4-ol (preparation 16b) and (R)-*tert*-butyl 3-aminopiperidine-1-carboxylate following the experimental procedure as described in preparation 5a. The crude product was purified by flash chromatography (9:1 dichloromethane/methanol).
LRMS (m/z): 431 (M+1)⁺.

### b) (R)-5-fluoro-2-(6-fluoroimidazo[1,2-a]pyridin-3-yl)-N-(piperidin-3-yl)pyrimidin-4-amine

Obtained as a solid (100%) from (*R*)-*tert*-butyl 3-(5-fluoro-2-(6-fluoroimidazo[1,2-a]pyridin-3-yl)pyrimidin-4-ylamino)piperidine-1-carboxylate (preparation 17a) following the experimental procedure as described in preparation 5b.
LRMS (m/z): 331 (M+1)⁺.

### PREPARATION 18

### 2-Chloro-4-methoxy-5-methylpyrimidine

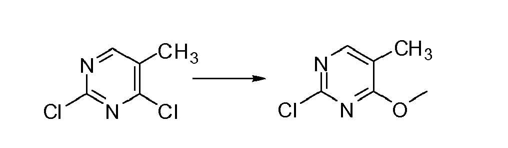

Obtained as a white solid (100%) from 2,4-dichloro-5-methylpyrimidine and sodium methoxide following the experimental procedure as described in preparation 15b.
LRMS (m/z): 159 (M+1)⁺.
¹H-NMR δ(CDCl₃): 2.12 (d, 3H), 4.03 (s, 3H), 8.10 (d, 1 H)

### PREPARATION 19

### 2-(6-Fluoroimidazo[1,2-a]pyridin-3-yl)-5-methylpyrimidin-4-ol

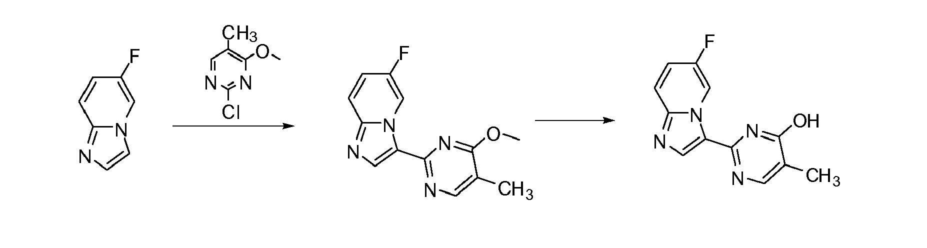

### a) 6-Fluoro-3-(4-methoxy-5-methylpyrimidin-2-yl)imidazo[1,2-a]pyridine

6-fluoroimidazo[1,2-a]pyridine (preparation 2, 1.0 g, 7.3 mmol) and 2-chloro-4-methoxy-5-methylpyrimidine (preparation 18, 1.70 g, 10.7 mmol) were reacted according to the experimental procedure as described in preparation 4a. The crude material obtained was purified by reverse phase chromatography (C-18 silica from Waters, water/acetonitrile/methanol as eluents [0.1% v/v formic acid buffered] 0% to 100%) to give the title compound (24%) as a white solid.
LRMS (m/z): 259 (M+1)⁺.
¹H-NMR δ (CDCl₃): 2.18 (s, 3H), 4.12 (s, 3H), 7.24 (dd, 1 H), 7.64 - 7.72 (m, 1 H), 8.29 (d, 1 H), 8.55 (s, 1 H), 9.95 (dt, 1 H)

### b) 2-(6-Fluoroimidazo[1,2-a]pyridin-3-yl)-5-methylpyrimidin-4-ol

6-Fluoro-3-(4-methoxy-5-methylpyrimidin-2-yl)imidazo[1,2-a]pyridine (preparation 19a, 0.45 g, 1.7 mmol) and a 35% aqueous solution of potassium hydroxide (2.79 mL, 17.4 mmol) were reacted according to the experimental procedure as described in preparation 16b. The reaction was concentrated in vacuum and water was added. The solution was neutralized with 2M aqueous hydrochloric acid and the resultant precipitate was filtered and dried to give the title compound (100%) as a white solid.
LRMS (m/z): 243 (M-1)⁺.
¹H NMR δ (DMSO-*d₆*): 3.33 (s, 3 H), 7.60 - 7.66 (m, 1 H), 7.87 (dd, , 1 H), 8.03 (brs, 1 H), 8.72 (s, 1 H), 9.91 (bs, 1 H).

### PREPARATION 20

### 2-(6-Fluoroimidazo[1,2-a]pyridin-3-yl)-5-methyl-N-[(3R)-piperidin-3-yl]pyrimidin-4-amine

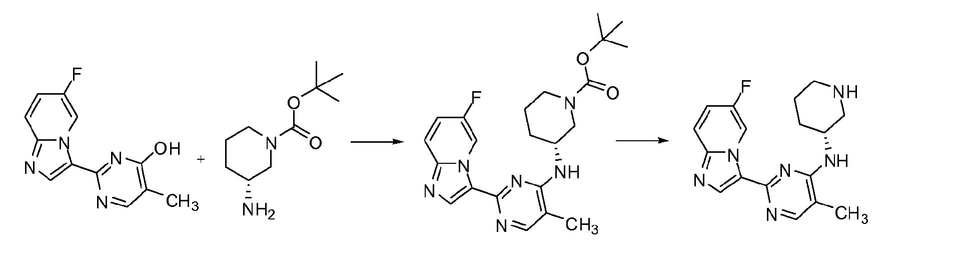

### a) Tert-butyl (3R)-3-{[2-(6-fluoroimidazo[1,2-a]pyridin-3-yl)-5-methylpyrimidin-4-yl]amino}piperidine-1-carboxylate

Obtained as a pale yellow solid (35%) from 2-(6-fluoroimidazo[1,2-a]pyridin-3-yl)-5-methylpyrimidin-4-ol (preparation 19b) and (R)-*tert*-butyl 3-aminopiperidine-1-carboxylate following the experimental procedure as described in preparation 5a. The crude product was purified by flash chromatography (9:1 dichloromethane/methanol).
LRMS (m/z): 427 (M+1)⁺.
¹H-NMR δ (CDCl₃): 1.51 (bs, 2H), 1.72 (bs, 9H), 2.01 - 2.29 (m, 5H), 3.42 (bs, 1 H), 3.56 - 3.83 (m, 3H), 4.37 (bs, 1 H), 7.27 (bs, 1 H), 7.73 (bs, 1 H), 8.13 (bs, 1H), 8.57 (bs, 1 H), 10.02 (bs, 1 H)

### b) 2-(6-Fluoroimidazo[1,2-a]pyridin-3-yl)-5-methyl-N-[(3R)-piperidin-3-yl]pyrimidin-4-amine

Obtained as a white solid (70%) from tert-butyl (3*R*)-3-{[2-(6-fluoroimidazo[1,2-a]pyridin-3-yl)-5-methylpyrimidin-4-yl]amino}piperidine-1-carboxylate (preparation 20a) following the experimental procedure as described in preparation 5b.
LRMS (m/z): 327 (M+1)⁺.
¹H-NMR δ (CDCl₃): 1.69 (bs, 3H), 1.74 - 2.04 (m, 4H), 2.87 (bs, 1 H), 3.21 (bs, 3H), 4.35 (bs, 1 H), 5.23 (bs, 1 H), 7.21 (bs, 1 H), 7.66 (bs, 1 H), 8.05 (bs, 1 H), 8.50 (s, 1H), 9.99 (bs, 1 H)

### PREPARATION 21

### 2-(6-Fluoroimidazo[1,2-a]pyridin-3-yl)-N-pyrrolidin-3-ylpyrimidin-4-amine

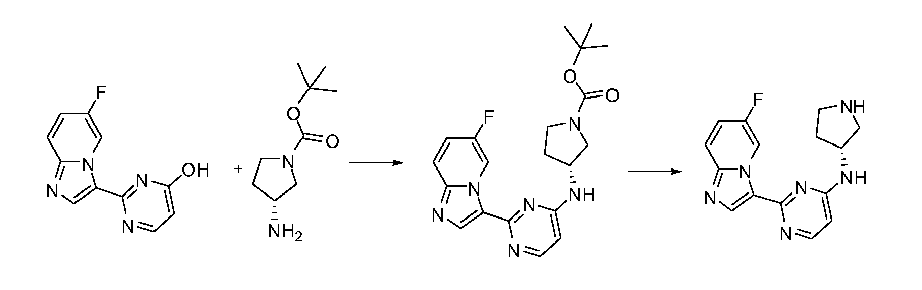

### a) Tert-butyl 3-{[2-(6-fluoroimidazo[1,2-a]pyridin-3-yl)pyrimidin-4-yl]amino}pyrrolidine-1-carboxylate

Obtained as an oil (44%) from 2-(6-fluoroimidazo[1,2-a]pyridin-3-yl)pyrimidin-4-ol (preparation 8b) and (R)-*tert*-butyl 3-aminopyrrolidine-1-carboxylate following the experimental procedure as described in preparation 5a. The crude product was purified by reverse phase chromatography (C-18 silica from Waters, water/acetonitrile/methanol as eluents [0.1 % v/v formic acid buffered] 0% to 100%).
LRMS (m/z): 399 (M+1)⁺.

### b) 2-(6-Fluoroimidazo[1,2-a]pyridin-3-yl)-N-pyrrolidin-3-ylpyrimidin-4-amine

Obtained as an oil (77%) from tert-butyl 3-{[2-(6-fluoroimidazo[1,2-*a*]pyridin-3-yl)pyrimidin-4-yl]amino}pyrrolidine-1-carboxylate (preparation 21a) following the experimental procedure as described in preparation 5b.
LRMS (m/z): 299 (M+1)⁺.
¹H-NMR δ (CDCl₃): 0.83 - 0.98 (m, 1 H), 1.82 (ddd, 1 H), 2.30 (dd, 1 H), 2.97 - 3.11 (m, 2H), 3.15 - 3.24 (m, 1H), 3.25 - 3.34 (m, 1 H), 5.31 (d, 1 H), 6.21 (d, 1 H), 7.24 (dd, 1 H), 7.68 (dd, 1 H), 8.26 (d, 1 H), 8.53 (s, 1 H), 10.00 (dd, 1 H)

### PREPARATION 22

### 3-[4-(1,4-Diazepan-1-yl)pyrimidin-2-yl]imidazo[1,2-a]pyridine-6-carbonitrile

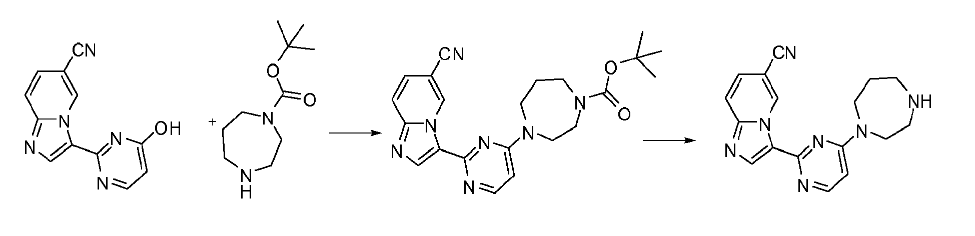

### a) Tert-butyl 4-[2-(6-cyanoimidazo[1,2-a]pyridin-3-yl)pyrimidin-4-yl]-1,4-diazepane-1-carboxylate

Obtained as a solid (48%) from 3-(4-hydroxypyrimidin-2-yl)imidazo[1,2-a]pyridine-6-carbonitrile (preparation 4b) and tert-butyl 1,4-diazepane-1-carboxylate following the experimental procedure as described in preparation 5a. The crude product was used without further purification.
LRMS (m/z): 420 (M+1)⁺.
¹H-NMR δ (DMSO-*d₆*): 1.03 (bs, 3H), 1.26 (bs, 4H), 1.56 - 1.98 (m, 4H), 3.20 - 3.33 (m, 2H), 3.51 - 3.78 (m, 4H), 3.81 - 4.05 (m, 2H), 6.71 (d, 1 H), 7.70 (d, 1 H), 7.91 (d, 1 H), 8.32 (bs, 1 H), 8.51 (s, 1 H), 10.42 (bs, 1 H)

### b) 3-[4-(1,4-Diazepan-1-yl)pyrimidin-2-yl]imidazo[1,2-a]pyridine-6-carbonitrile

Obtained as a solid (65%) from tert-butyl 4-(2-(6-cyanoimidazo[1,2-a]pyridin-3-yl)pyrimidin-4-yl)-1,4-diazepane-1-carboxylate (preparation 22a) following the experimental procedure as described in preparation 5b.
LRMS (m/z): 320 (M+1)⁺.

### PREPARATION 23

### 2-Chloro-4-methoxyquinazoline

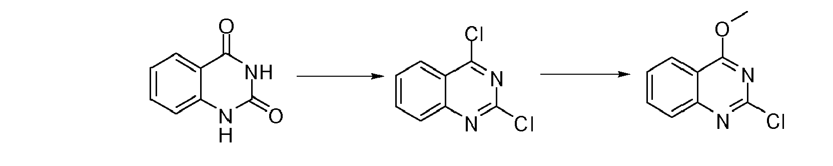

### a) 2,4-Dichloroquinazoline

Quinazoline-2,4(1H,3H)-dione (5.0 g, 30.8 mmol) was dissolved in phosphorous oxychloride (50 mL, 546 mmol) and the mixture was stirred and heated to 115 °C. After 16 hours, the mixture was evaporated and then co-evaporated with toluene. The residue was dissolved in ethyl acetate and triethylamine (20 mL) was added. After stirring for 5 minutes, water was added. The organic phase was washed with water, brine, dried (MgSO₄) and evaporated to give the title compound (6.45 g, 100%) as an off-white solid.
LRMS (m/z): 199 (M+1)⁺.
¹H-NMR δ (CDCl₃): 7.76 (ddd, 1 H), 8.03 - 7.99 (m, 2H), 8.28 (dt, 1 H).

### b) 2-Chloro-4-methoxyquinazoline

To a stirred solution of 2,4-dichloroquinazoline (preparation 23a, 2.0 g, 10.0 mmol) in methanol (100 mL) was added a 5M solution of sodium methoxide in methanol (2.1 mL, 10.5 mmol) and the mixture was stirred at ambient temperature for 48 hours. The solvent was evaporated and the mixture was partitioned between ethyl acetate and water. The organic phase was dried (MgSO₄) and evaporated to give the title compound (1.87 g, 96%) as a solid.
LRMS (m/z): 195 (M+1)⁺.
¹H-NMR δ (CDCl₃): 4.23 (s, 3H), 7.57 (ddd, 1 H), 7.88 - 7.84 (m, 2H), 8.17 - 8.11 (m, 1 H).

### PREPARATION 24

### (2-(4-Hydroxyquinazolin-2-yl)imidazo[1,2-a]pyridine-6-carbonitrile

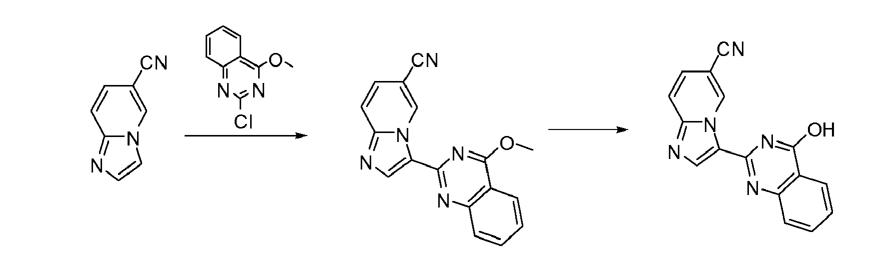

### a) 2-(4-Methoxyquinazolin-2-yl)imidazo[1,2-a]pyridine-6-carbonitrile Obtained as a white solid (74%) from imidazo[1,2-a]pyridine-6-carbonitrile (preparation

1, 0.15 g, 1.1 mmol) and 2-chloro-4-methoxyquinazoline (preparation 23b, 0.32 g, 1.6 mmol) following the experimental procedure as described in preparation 4a.
LRMS (m/z): 302 (M+1)⁺.
¹H-NMR δ (CDCl₃): 4.30 (s, 3H), 7.63 - 7.38 (m, 2H), 7.86 (dd, 1 H), 8.02 (d, 1 H), 8.18 (d, 1 H), 8.76 (s, 1 H), 10.83 (s, 1 H).

### b) (2-(4-Hydroxyquinazolin-2-yl)imidazo[1,2-a]pyridine-6-carbonitrile

Obtained as a brown solid (74%) from 3-(4-methoxyquinazolin-2-yl)imidazo[1,2-a]pyridine-6-carbonitrile (preparation 24a, 0.30 g, 1.0 mmol) following the experimental procedure as described in preparation 4b.
LRMS (m/z): 286 (M+1)⁺.

### PREPARATION 25

### 2-Chloro-4-methoxy-5-pyridin-3-ylpyrimidine

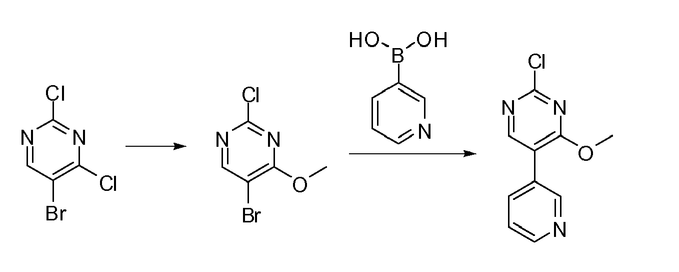

### a) 5-Bromo-2-chloro-4-methoxypyrimidine

A 30% solution of sodium methoxide in methanol (8.0 mL, 42.0 mmol) was added dropwise to a cooled (ice-bath) solution of 5-bromo-2,4-dichloropyrimidine (9.85 g, 43.2 mmol) in methanol (100 mL). The mixture was warmed to room temperature and stirred for 4 hours. The solvent was removed under reduced pressure and the residue was partitioned between water and dichloromethane. The organic layer was washed with water, brine, dried (MgSO₄) and evaporated to give the title compound (9.40 g, 93%) as a solid.
LRMS (m/z): 222/224 (M+1)⁺.
¹H-NMR δ (CDCl₃): 4.11 (s, 3H), 8.44 (s, 1 H).

### b) 2-Chloro-4-methoxy-5-pyridin-3-ylpyrimidine

Nitrogen was bubbled for 5 minutes through a mixture of 5-bromo-2-chloro-4-methoxypyrimidine (preparation 25a, 0.51 g, 2.3 mmol), pyridin-3-yl boronic acid (0.30 g, 2.5 mmol) and potassium carbonate (0.93 g, 6.7 mmol) in toluene (8.0 mL) and *N,N'-*dimethylformamide (1.0 mL) contained in a microwave vessel. Then [1,1'-bis(diphenylphosphino)ferrocene] dichloropalladium (II) complex with dichloromethane (1:1) (68 mg, 0.11 mmol) was added and the vessel was sealed and subjected to microwave irradiation for 10 minutes at 185 °C. The reaction mixture was filtered through Celite®, washing the filter cake with ethyl acetate. The combined filtrate and washings were evaporated and the residue was purified by flash chromatography (2:1 hexanes/ethyl acetate) to give the title compound (0.066 g, 14%) as a solid.
LRMS (m/z): 222 (M+1)⁺

### PREPARATION 26

### 3-(4-Hydroxy-5-pyridin-3-ylpyrimidin-2-yl)imidazo[1,2-a]pyridine-6-carbonitrile

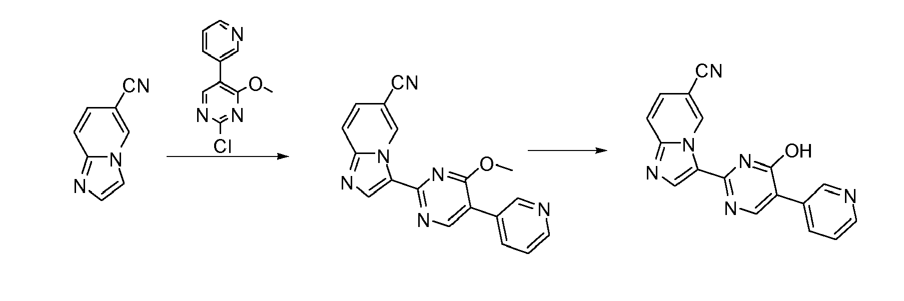

### a) 3-(4-Methoxy-5-pyridin-3-ylpyrimidin-2-yl)imidazo[1,2-a]pyridine-6-carbonitrile

2-Chloro-4-methoxy-5-pyridin-3-yl-pyrimidine (preparation 25b, 0.226 g, 1.0 mmol) and imidazo[1,2-a]pyridine-6-carbonitrile (preparation 1, 0.217 g, 1.5 mmol) were reacted according to the experimental procedure as described in preparation 4a. The crude product was purified by flash chromatography (9:1 ethyl acetate/methanol) to give the title compound (14%) as an off-white solid.
LRMS (m/z): 329 (M+1)⁺
¹H-NMR δ (CDCl₃): 4.19 (s, 3H), 6.50 (dd, 1 H), 7.44 (ddd, 1 H), 7.48 (dd, 1 H), 7.85 (dd, 1 H), 7.96 (ddd, 1 H), 8.62 (s, 1 H), 8.67 (dd, 1 H), 8.87 (dd, 1 H), 10.56 (dd, 1H).

### b) 3-(4-Hydroxy-5-pyridin-3-ylpyrimidin-2-yl)imidazo[1,2-a]pyridine-6-carbonitrile

Obtained as a brown solid (100%) from 3-(4-methoxy-5-pyridin-3-ylpyrimidin-2-yl)imidazo[1,2-a]pyridine-6-carbonitrile (preparation 26a) following the experimental procedure as described in preparation 4b.
LRMS (m/z): 315 (M+1)⁺.
¹H-NMR δ (CDCl₃): 6.86 (d, 1 H), 7.91 (d, 1 H), 8.04 (d, 1 H), 8.19 - 8.10 (m, 1 H), 8.81 (s, 1 H), 9.05 - 8.86 (m, 2H), 9.40 (s, 1 H), 10.43 (s, 1 H).

### PREPARATION 27

### 3-(4-Chloro-6-pyridin-3-ylpyrimidin-2-yl)imidazo[1,2-a]pyridine

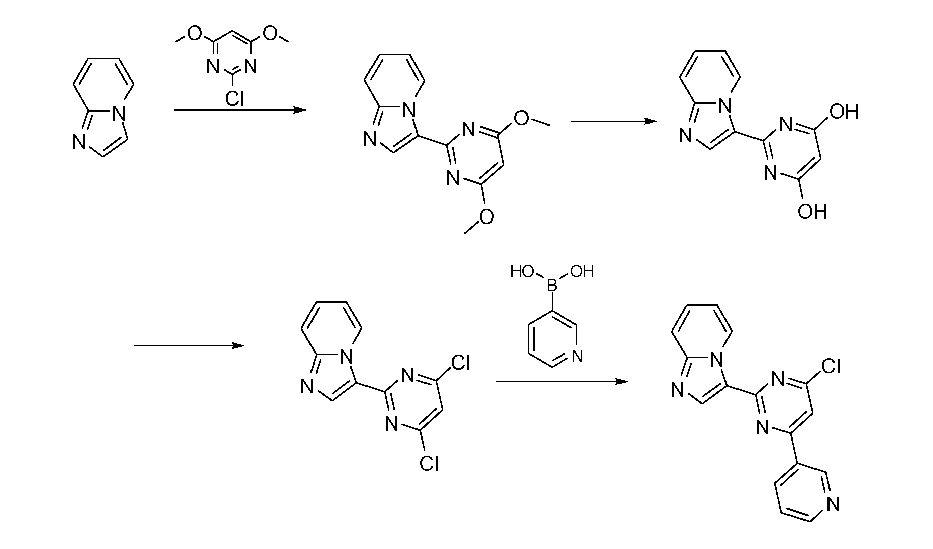

### a) 3-(4,6-Dimethoxypyrimidin-2-yl)imidazo[1,2-a]pyridine

Obtained as a brown solid (50%) from 2-chloro-4,6-dimethoxypyrimidine and imidazo[1,2-*a*]pyridine following the experimental procedure as described in preparation 4a.
LRMS (m/z): 282 (M+1)⁺.
¹H-NMR δ (CDCl₃): 4.03 (s, 6H), 6.12 (s, 1 H), 7.23 (td, 1 H), 7.50 (ddd, 1 H), 7.78 (dt, 1 H), 8.52 (s, 1 H), 9.83 (dt, 1 H).

### b) 2-Imidazo[1,2-a]pyridin-3-ylpyrimidine-4,6-diol

Obtained as a brown solid (100%) from 3-(4,6-dimethoxypyrimidin-2-yl)imidazo[1,2-a]pyridine (preparation 27a) following the experimental procedure as described in preparation 4b.
¹H-NMR δ (DMSO-*d₆*): 5.72 (s, 1 H), 7.69 (ddd, 1 H), 8.14 - 8.00 (m, 2H), 8.89 (s, 1 H), 10.30 (d, 1H).

### c) 3-(4,6-Dichloropyrimidin-2-yl)imidazo[1,2-a]pyridine

A solution of 2-imidazo[1,2-*a*]pyridin-3-ylpyrimidine-4,6-diol (preparation 27b, 330 mg, 1.45 mmol) in phosphorous(V) oxychloride (2.8 mL) was heated and stirred to 115 °C. After 18 hours, the mixture was evaporated and the residue was co-evaporated with toluene to give the title compound (154 mg, 40%).
LRMS (m/z): 291 (M+1)⁺.
¹H-NMR δ (CDCl₃): 7.48 (s, 1 H), 7.75 (t, 1 H), 8.20 (t, 1 H), 8.48 (d, 1 H), 8.90 (s, 1 H), 10.21 (d, 1 H).

### d) 3-(4-Chloro-6-pyridin-3-ylpyrimidin-2-yl)imidazo[1,2-a]pyridine

Nitrogen was bubbled for 5 minutes through a mixture of 3-(4,6-dichloropyrimidin-2-yl)imidazo[1,2-a]pyridine (preparation 27c, 0.14 g, 0.54 mmol), pyridin-3-yl boronic acid (0.044 g, 0.36 mmol) and 2M aqueous cesium carbonate solution (0.54 mL, 1.08 mmol) in 1,4-dioxane (6 mL) contained in a Schlenck tube. Then [1,1'-bis(diphenylphosphino) ferrocene] dichloropalladium (II) complex with dichloromethane (1:1) (0.029 g, 0.04 mmol) was added and the vessel was sealed and the contents were stirred and heated to 90 °C. After 18 hours, the reaction mixture was filtered through Celite®, washing the filter cake with ethyl acetate. The filtrate was evaporated and the residue was purified by flash chromatography (20:1 dichloromethane/ethanol) to give the title compound (0.038 g, 32%) as a solid.
LRMS (m/z): 333 (M+1)⁺.

### PREPARATION 28

### 2-Chloro-6-[(4-methoxybenzyl)oxy]pyrazine

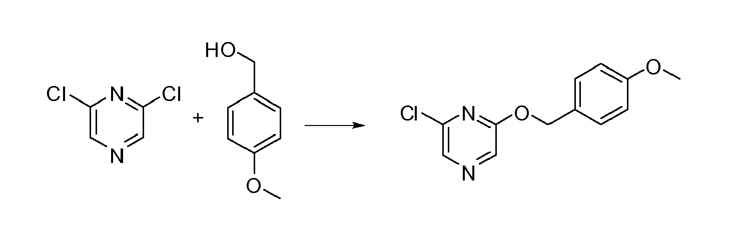

Sodium hydride (60% dispersion in mineral oil, 0.16 g, 4.0 mmol) was added portionwise to a stirred solution of 2,6-dichloropyrazine (0.50 g, 3.4 mmol) and (4-methoxyphenyl)methanol (0.51 g, 3.7 mmol) in *N,N'*-dimethylformamide. The reaction mixture was stirred at ambient temperature overnight then evaporated in vacuum. The residue was partitioned between ethyl acetate and water and the organic layer was washed with brine, dried (MgSO₄) and evaporated. The residue was purified by flash chromatography (9:1 to 4:1 hexanes/ethyl acetate) to give the title compound (0.687 g, 81%) as a solid.
LRMS (m/z): 251 (M+1)⁺.
¹H-NMR δ (CDCl₃): 3.83 (s, 3H), 5.32 (s, 2H), 6.93 (d, 2H), 7.40 (d, 2H), 8.14 (s, 1H), 8.15 (s, 1 H)

### PREPARATION 29

### 3-(6-Hydroxypyrazin-2-yl)imidazo[1,2-a]pyridine-6-carbonitrile

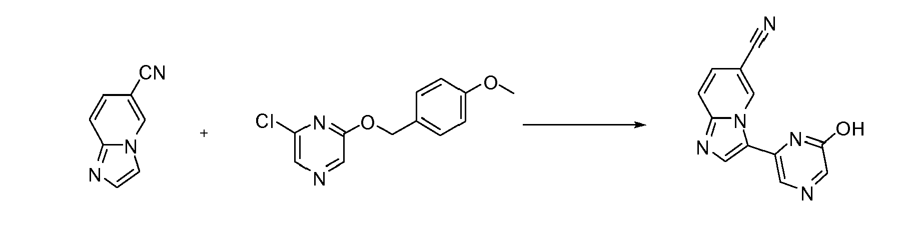

An oven dried resealable Schlenk tube was charged with 2-chloro-6-(4-methoxybenzyloxy)pyrazine (preparation 28, 0.68 g, 2.7 mmol), imidazo[1,2-a]pyridine-6-carbonitrile (preparation 1, 0.59 g, 4.1 mmol), potassium acetate (0.138 g, 1.41 mmol) and N,N'-dimethylacetamide (4 mL). The Schlenk tube was subjected to three cycles of evacuation-backfilling with argon then tetrakis(triphenylphospino)palladium(0) (0.34 g, 0.3 mol) was added. After three further cycles of evacuation-backfilling with argon, the Schlenk tube was capped and placed in an oil bath at 150 °C and the mixture was stirred for 4 hours. The mixture was evaporated in vacuum and the residue was partitioned between ethyl acetate and water. The organic layer was extracted with 2M aqueous hydrochloric acid and the aqueous layer was taken to pH 5-6 with solid sodium hydrogen carbonate and then extracted with ethyl acetate. The organic layer was dried (MgSO₄), evaporated and the residue was purified by column chromatography (98:2 to 90:10 dichloromethane/methanol) to give the title compound (0.102 g, 10%).
LRMS (m/z): 238 (M+1)⁺.

### PREPARATION 30

### 2-Chloro-N-(4-methoxybenzyl)-N-[(1S)-1-phenylethyl]pyrimidin-4-amine

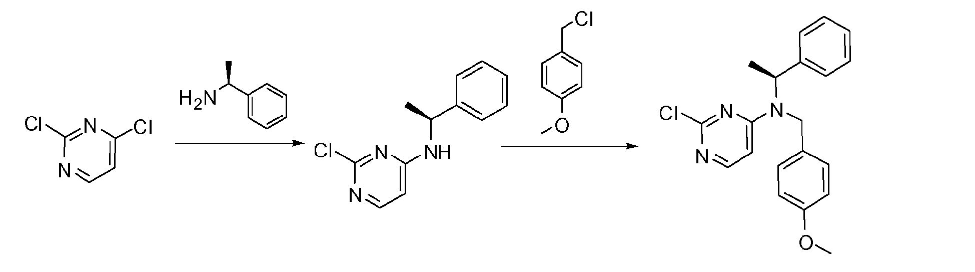

### a) 2-Chloro-N-[(1S)-1-phenylethyl]pyrimidin-4-amine

A stirred mixture of 2,4-dichloropyrimidine (1.27 g, 8.5 mmol), (*S*)-1-phenylethanamine (1.0 mL, 7.8 mmol) and diisopropylethylamine (1.6 mL, 8.6 mmol) in *N,N'-*dimethylformamide (12 mL) was heated to 90 °C in a sealed tube. After 16 hours, the mixture was evaporated in vacuum and then taken up in ethyl acetate. The organic layer was washed with water and brine, dried (MgSO₄) and evaporated. Purification by flash chromatography (300:1 dichloromethane/methanol) gave the title compound (0.65 g, 36%) as a pale yellow oil.
LRMS (m/z): 234 (M+1)⁺.
¹H-NMR δ (CDCl₃): 1.59 (d, 3H), 5.38 - 5.75 (m, 1H), 6.08 (d, 1H), 7.28 - 7.43 (m, 5H), 7.97 (d, 1 H)

### b) 2-Chloro-N-(4-methoxybenzyl)-N-[(1S)-1-phenylethyl]pyrimidin-4-amine

Sodium hydride (60% dispersion in mineral oil, 0.17 g, 4.3 mmol) was added portionwise to a solution of 2-chloro-*N*-[(1*S*)-1-phenylethyl]pyrimidin-4-amine (preparation 30a, 0.95 g, 4.1 mmol) in *N,N'*-dimethylformamide (10 mL). The mixture was stirred for 15 minutes, then 1-(chloromethyl)-4-methoxybenzene (0.58 mL, 4.3 mmol) was added and the reaction was stirred at room temperature for a further 5 hours. Water was added and the mixture was extracted with ethyl acetate. The organic layer was washed with water, brine, dried (MgSO₄ and evaporated in vacuum. The residue was triturated with several portions of hexane to give a thick oil. Purification by flash chromatography (5:1 hexanes/ethyl acetate) gave the title compound (0.94 g, 65%) as a white solid.
LRMS (m/z): 354 (M+1)⁺.
¹H-NMR δ (CDCl₃): 1.58 (d, 3H), 3.78 (bs, 3H), 4.21 - 4.44 (m, 3H), 6.11 (d, 1 H), 6.80 (d, 2H), 6.99 (d, 2H), 7.15 - 7.42 (m, 5H), 7.92 (d, 1 H)

### PREPARATION 31

### N-benzyl-2-chloro-N-methylpyrimidin-4-amine

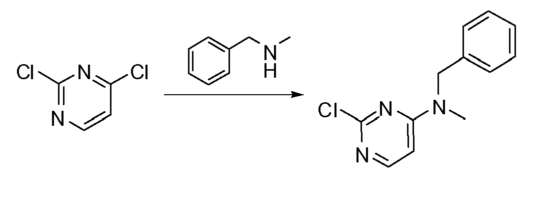

*N*-Methyl-1-phenylmethanamine (0.32 mL, 2.5 mmol) and diisopropylethylamine amine (0.58 mL, 3.3 mmol) were added sequentially to a solution of 2,4-dichloropyrimidine (0.50 g, 3.4 mmol) in *N,N'*-dimethylformamide (5 mL). The mixture was stirred in a sealed tube for 3 hours at 90 °C. The mixture was evaporated and the residue was dissolved in ethyl acetate and washed with water, dried (MgSO₄) filtered and evaporated. Purification by flash chromatography (3:1 hexanes/ethyl acetate) gave the title compound (0.51 g, 86%) as an oil.
LRMS (m/z): 234 (M+1)⁺.
¹H-NMR δ (CDCl₃): 3.08 (bs, 3H), 4.79 (bs, 2H), 6.35 (d, 1 H), 7.14 - 7.41 (m, 5H), 8.04 (d, 1 H)

### PREPARATION 32

### (S)-6-chloro-N-(1-phenylethyl)pyrazin-2-amine

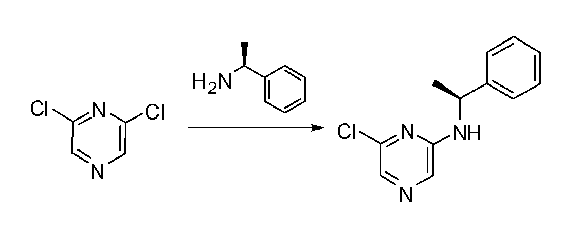

Obtained as a white solid (37%) from 2,6-dichloropyrazine (1.92 g, 12.9 mmol) and (S)-1-phenylethanamine (1.72 g, 14.2 mmol) following the experimental procedure as described in W0200399796.
LRMS (m/z): 234 (M+1)⁺.
¹H-NMR δ (CDCl₃): 1.58 (d, 3H), 4.86 (bs, 1 H), 5.08 (bs, 1 H), 7.24 - 7.32 (m, 5H), 7.60 (s, 1 H), 7.79 (s, 1 H)

### PREPARATION 33

### 1-(Ethylsulfonyl)-N,4-dimethylpiperidin-3-amine

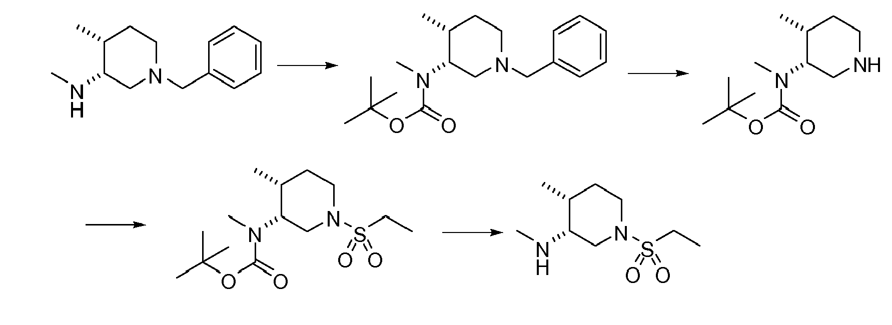

### a) Tert-butyl (1-benzyl-4-methylpiperidin-3-yl)methylcarbamate

Triethylamine (1.72 mL, 12.3 mmol) and di-tert-butyl dicarbonate (2.70 g, 12.4 mmol) were added sequentially to a stirred solution of *cis*-1-benzyl-*N*,4-dimethylpiperidin-3-amine (prepared as described in WO200560972; 2.70 g, 12.4 mmol) in dichloromethane (60 mL). The mixture was stirred at ambient temperature overnight. The mixture was washed with saturated aqueous sodium hydrogencarbonate solution, water, brine, dried (MgSO₄) and the solvent was removed under reduced pressure to give the title compound (3.40 g, 86%) as a solid.
LRMS (m/z): 319 (M+1)⁺.
¹H-NMR δ (CDCl₃): 1.00 (d, 3H), 1.51 (s, 9H), 1.64 (bs, 1 H), 1.98 (bs, 2H), 2.24 (bs, 2H), 2.49 (dd, 1 H), 2.77 (bs, 2H), 3.16 (s, 3H), 3.50 (s, 2H), 7.23 - 7.45 (m, 5H)

### b) Tert-butyl methyl(4-methylpiperidin-3-yl)carbamate

Palladium on charcoal (10%, 0.30 g) was added to a solution of tert-butyl (1-benzyl-4-methylpiperidin-3-yl)methylcarbamate (preparation 33a, 3.40 g, 10.7 mmol) in methanol (70 mL) and the mixture was stirred under an atmosphere of hydrogen for 20 hours. The catalyst was filtered off and the solvent was removed under reduced pressure to give the title compound (2.38 g, 97%) as an oil.
LRMS (m/z): 229 (M+1)⁺.
¹H-NMR δ (CDCl₃): 0.94 (d, 3H), 1.39 (s, 9H), 1.58 - 1.68 (m, 1 H), 2.11 - 2.20 (m, 2H), 2.68 (bs, 1 H), 2.73 - 2.81 (m, 2H), 2.83 (s, 3H), 2.99 (bs, 2H)

### c) Tert-butyl [1-(ethylsulfonyl)-4-methylpiperidin-3-yl]methylcarbamate

A solution of ethanesulfonyl chloride (0.86 mL, 9.1 mmol) in dichloromethane (3 mL) was added dropwise to a stirred, cooled (ice-bath) solution of tert-butyl methyl(4-methylpiperidin-3-yl)carbamate (preparation 33b, 1.89 g, 8.3 mmol) in dichloromethane (60 mL) and triethylamine (1.27 mL, 9.1 mmol). The mixture was warmed to room temperature and stirred overnight. The mixture was washed with saturated aqueous sodium hydrogencarbonate solution, water, brine, dried (MgSO₄) and the solvent was removed under reduced pressure to give the title compound (2.20 g, 83%) as a solid.
LRMS (m/z): 321 (M+1)⁺.
¹H-NMR δ (CDCl₃): 1.00 (d, 3H), 1.38 (t, 3H), 1.46 (s, 9H), 1.65 - 1.76 (m, 2H), 2.04 - 2.14 (m, 1H), 2.96 (d, 2H), 2.98 (s, 3H), 3.05 - 3.18 (m, 2H), 3.33 (bs, 1H), 3.51 (bs, 1 H), 3.64 (bs, 1 H)

### d) 1-(Ethylsulfonyl)-N,4-dimethylpiperidin-3-amine

A solution of trifluoroacetic acid (12.25 mL, 159 mmol) in dichloromethane (10 mL) was added to a stirred, cooled (ice-bath) solution of *tert*-butyl 1-(ethylsulfonyl)-4-methylpiperidin-3-yl(methyl)carbamate (preparation 33c, 3.0 g, 9.4 mmol) in dichloromethane (22 mL). The mixture was warmed to room temperature and stirred overnight. The solvent was evaporated and the residue was taken up in water and basified with 2M aqueous sodium hydroxide solution and then extracted with dichloromethane. The organic solvent was removed under reduced pressure to give the title compound (2.06 g, 97%) as an oil.
LRMS (m/z): 221 (M+1)⁺.
¹H-NMR δ (CDCl₃): 1.24 (d, 3H), 1.63 (t, 3H), 1.79 - 1.91 (m, 2H), 2.25 - 2.36 (m, 1 H), 2.81 (bs, 1 H), 2.90 (q, 2H), 3.06 (bs, 1 H), 3.21 - 3.37 (m, 1 H), 3.42 - 3.51 (m, 1 H), 3.68 (bs, 2H), 3.91 (s, 3H)

### PREPARATION 34

### Cis-3-(3-amino-4-methylpiperidin-1-yl)-3-oxopropanenitrile

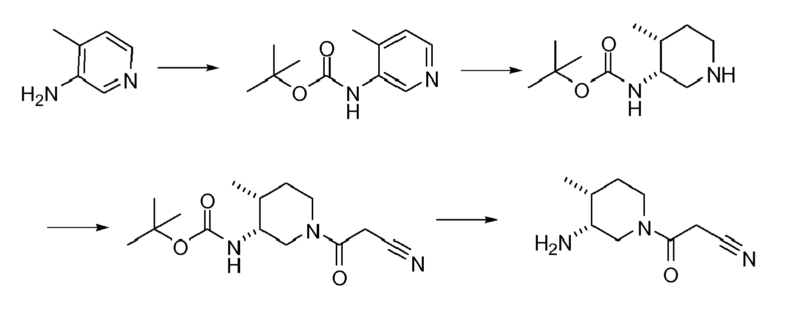

### a) Tert-butyl (4-methylpyridin-3-yl)carbamate

Sodium bis(trimethylsilyl)amide (1M in tetrahydrofuran, 32.0 mL, 32.0 mmol) was added dropwise to a cooled (ice-bath) solution of 4-methylpyridin-3-amine (1.72 g, 15.9 mmol) in tetrahydrofuran (30 mL). The mixture was warmed to ambient temperature and stirred for 5 minutes. The mixture was again cooled to 0 °C and a solution of di-tert-butyl dicarbonate (3.09 g, 14.2 mmol) in tetrahydrofuran was added. The mixture was warmed to ambient temperature and stirred for 18 hours. The pH of the mixture was adjusted to 5-6 using 0.1 M aqueous hydrochloric acid. The organic layer was dried (MgSO₄) and evaporated to give the title compound (2.16 g, 70%) as a dark oil.
LRMS (m/z): 209 (M+1)⁺.
¹H-NMR δ (CDCl₃): 1.43 (s, 9H), 2.27 (s, 3H), 6.25 (bs, 1 H), 7.10 (d, 1 H), 8.23 (d, 1H), 8.86 (s, 1 H).

### b) Cis-tert-butyl (4-methylpiperidin-3-yl)carbamate

To a solution of tert-butyl (4-methylpyridin-3-yl)carbamate (preparation 34a, 1.9 g, 9.2 mmol) in methanol (15 mL) was added 5% rhodium on carbon (1.0 g). The mixture was hydrogenated (pressure of 60 psi) at 50 °C for 48 hours. The catalyst was filtered through Celite® and the filter cake washed with methanol. The filtrate and washings were combined and concentrated under reduced pressure and the residue was purified by flash chromatography (98:2:0.2 to 90:10:1 dichloromethane/methanol/ammonia) to give the title compound (0.60 g, 31%) as a pale yellow solid.
LRMS (m/z): 215 (M+1)⁺.
¹H-NMR δ (CDCl₃): 0.91 (d, 3H), 1.28 (d, 2H), 1.45 (s, 9H), 1.71 (bs, 5H), 2.59 (t, 1 H), 2.72 (d, 1 H), 2.96 (d, 2H), 3.68 (d, 1 H), 5.30 (d, 1H).

### c) Cis-tert-butyl [1-(cyanoacetyl)-4-methylpiperidin-3-yl]carbamate

Triethylamine (0.26 mL, 1.8 mmol) and 3-[(2,5-dioxopyrrolidin-1-yl)oxy]-3-oxopropanenitrile (prepared as described in BE875054(A1), 0.364 g, 2.0 mmol) were added sequentially to a stirred solution of *cis-tert*-butyl 4-methylpiperidin-3-ylcarbamate (preparation 34b, 0.36 g, 1.7 mmol) in dichloromethane (6 mL). The mixture was stirred at ambient temperature for 24 hours and then evaporated. Water was added and the mixture was extracted with dichloromethane. The organic layer was dried (MgSO₄) and evaporated and the residue was purified by flash chromatography (98:2 dichloromethane/methanol) to give the title compound (0.39 g, 83%) as a pale yellow oil.
LRMS (m/z): 282 (M+1)⁺
¹H-NMR δ (CDCl₃): 0.99 (d, 3H), 1.29 (qd, 1H), 1.46 (s, 9H), 2.01-1.85 (m, 1H), 2.69 (td, 1 H), 3.17 (d, 1 H), 3.46 (s, 2H), 3.71-3.62 (m, 1 H), 3.87 (d, 1 H), 4.51 (t, 2H).

### d) Cis-3-(3-amino-4-methylpiperidin-1-yl)-3-oxopropanenitrile

Trifluoroacetic acid (2.0 mL, 26 mmol) was added dropwise to a solution of *cis-tert-*butyl [1-(cyanoacetyl)-4-methylpiperidin-3-yl]carbamate (preparation 34c, 0.39 g, 1.4 mmol) in dichloromethane (7 mL) and the mixture was stirred at room temperature for 1 hour. The mixture was then evaporated and taken up in water. The mixture was then taken to neutral pH with saturated aqueous sodium hydrogencarbonate solution and extracted with dichloromethane. The organic layer was dried (MgSO₄) and evaporated and the residue was purified by flash chromatography (98:2 to 90:10 dichloromethane/methanol) to give the title compound (0.180 g, 72%) as a pale yellow oil.
LRMS (m/z): 182 (M+1)⁺.
¹H-NMR δ (CDCl₃): 0.96 (d, 3H), 1.58 - 1.48 (m, 1H), 1.90 - 1.76 (m, 2H), 2.73 (ddd, 1 H), 3.03 - 2.88 (m, 1 H), 3.32 - 3.24 (m, 1 H), 3.64 (d, 2H), 3.88 - 3.76 (m, 1 H), 4.47 - 4.22 (m, 1 H).

### PREPARATION 35

### Cis-1-(ethylsulfonyl)-4-methylpiperidin-3-amine

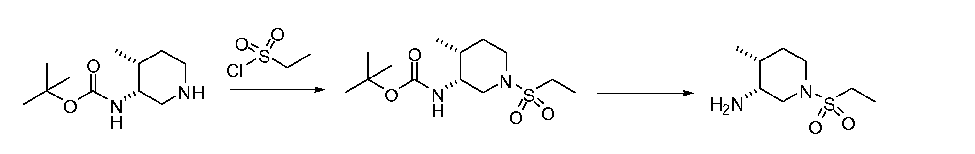

### a) Cis-tert-butyl [1-(ethylsulfonyl)-4-methylpiperidin-3-yl]carbamate

Triethylamine (0.33 mL, 2.3 mmol) and ethanesulfonyl chloride (0.17 mL, 1.7 mmol) were added sequentially to a cooled (ice-bath) solution of *cis-tert-butyl (4-*methylpiperidin-3-yl)carbamate (preparation 34b, 0.25 g, 1.2 mmol) in dichloromethane (3 mL). The mixture was warmed to ambient temperature and stirred for 18 hours. Water was added and the organic layer was washed with water and brine, dried (MgSO₄) and evaporated. The residue was purified by flash chromatography (98:2 dichloromethane/methanol) to give the title compound (0.31 g, 85%) as a brown solid.
LRMS (m/z): 307 (M+1)⁺.
¹H-NMR δ (CDCl₃): 0.96 (d, 3H), 1.44 (s, 12H), 1.62 (s, 1 H), 1.80 - 1.66 (m, 1 H), 2.74 (t, 1 H), 2.93 (dt, 3H), 3.49 (d, 1 H), 3.80 (dd, 3H), 4.95 (d, 1H).

### b) Cis-1-(ethylsulfonyl)-4-methylpiperidin-3-amine

*Cis-tert-*butyl [1-(ethylsulfonyl)-4-methylpiperidin-3-yl]carbamate (preparation 35a, 0.305 g, 1.0 mmol) was reacted according to the experimental procedure as described in preparation 34d. The crude mixture was evaporated, water was added and the mixture was then basified with saturated aqueous sodium hydrogencarbonate solution to neutral pH. The aqueous layer was extracted with dichloromethane and the organic layer was dried (MgSO₄) and evaporated to give the title compound (0.145 g, 70%) as an oil.
LRMS (m/z): 207 (M+1)⁺.
¹H-NMR δ (CDCl₃): 1.01 (d, 3H), 1.45 - 1.22 (m, 3H), 1.60 (d, 4H), 2.85 - 2.73 (m, 1 H), 3.13 - 2.85 (m, 3H), 3.86 - 3.47 (m, 2H).

### PREPARATION 36

### 1-((2S,4S)-2-cyano-4-fluoropyrrolidine-1-carbonyl)-3-methyl-1 H-imidazol-3-ium iodide

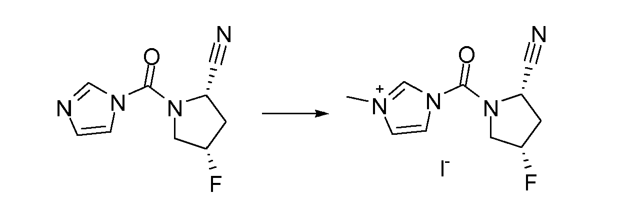

Methyl iodide (0.075 mL, 1.2 mmol) was added to a stirred solution of (2*S*,4*S*)-4-fluoro-1-(1H-imidazole-1-carbonyl)pyrrolidine-2-carbonitrile (0.29 mmol, prepared as described in US2005256310(A1)) in acetonitrile (1mL) and the reaction mixture was stirred at ambient temperature overnight. The solvent was removed under reduced pressure to give the title compound as a solid (99%), which was used without further purification.
LRMS (m/z): 223 (M-1)⁻.

### PREPARATION 37

### 1-((Cyanomethy))(methy))carbamoy))-3-methy)-1H-imidazo)-3-ium iodide

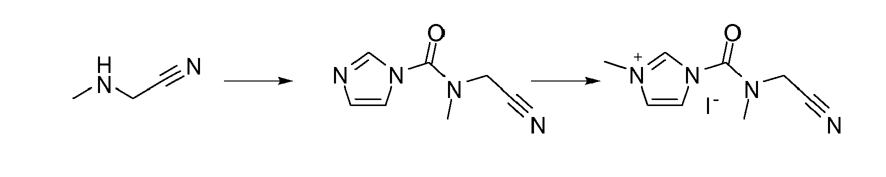

### a) N-(cyanomethyl)-N-methyl-1H-imidazole-1-carboxamide

2-(Methylamino)acetonitrile (1g, 14.3 mmol) was reacted with 1,1'-carbonyldiimidazole (2.5 g, 15.4 mmol) according to the experimental procedure as described in US2005256310(A1). The crude product was purified by flash chromatography (4:1, dichloromethane/methanol) to give the title compound (0.24 g, 10%) as an oil.
LRMS (m/z): 165 (M+1)⁺.
¹H-NMR δ (CDCl₃): 3.28 (s, 3H), 4.37 (s, 2H), 7.15 (s, 1 H), 7.30 (bs, 1 H), 7.97 (s, 1 H)

### b) 1-((Cyanomethyl)(methyl)carbamoyl)-3-methyl-1H-imidazol-3-ium iodide

Obtained as an oil (45%) from *N*-(cyanomethyl)-*N*-methyl-*1H*-imidazole-1-carboxamide (preparation 37a, 0.24g, 1.46 mmol) and methyl iodide (0.36mL, 5.9 mmol) following the experimental procedure as described in preparation 36. The isolated crude product was used without further purification.
LRMS (m/z): 179 (M-1)⁻.
¹H-NMR δ (DMSO-*d₆*): 3.34 (s, 3H), 3.91 (s, 2H), 4.64 (s, 3H), 7.06 (bs, 1H), 7.58 (bs, 1H), 8.09 (s, 1 H)

### PREPARATION 38

### 2-Methoxy-4-(4,4,5,5-tetramethyl-1,3,2-dioxaborolan-2-yl)pyridine

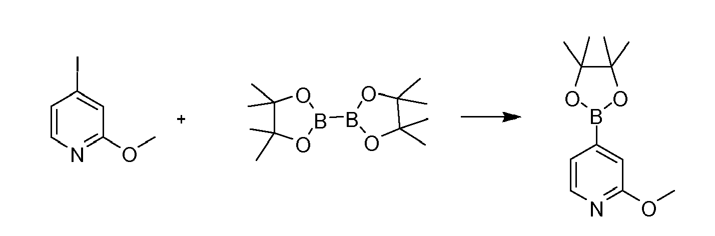

Nitrogen was bubbled for 5 minutes through a mixture of 4-iodo-2-methoxypyridine (0.099 g, 0.42 mmol), bis(pinacolato)diboron (0.12 g, 0.47 mmol) and potassium acetate (0.13 g, 1.31 mmol) in *N,N'*-dimethylformamide (1 mL) contained in a microwave vessel. Then [1,1'-bis(diphenylphosphino)ferrocene] dichloropalladium (II) complex with dichloromethane (1:1) (0.017 g, 0.02 mmol) was added and the vessel was sealed and subjected to microwave irradiation for 4 hours at 80 °C. The reaction mixture was diluted with ethyl acetate and filtered through Celite®. The filtrate was washed with saturated aqueous sodium hydrogencarbonate solution, water, brine, dried (MgSO₄) and evaporated to give the title compound (0.128 g, 82%) as a brown oil which was used without further purification.
LRMS (m/z): 236 (M+1)⁺.

### PREPARATION 39

### 1 H-pyrazole-3-carboxylic acid

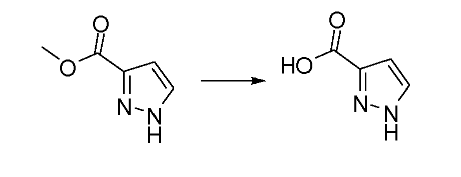

A 2M aqueous sodium hydroxide solution (1.8 mL, 3.6 mmol) was added to a solution of ethyl 1*H*-pyrazole-4-carboxylate (0.2g, 1.55 mmol) in ethanol (4 mL). The reaction mixture was stirred at room temperature overnight and at 80 °C for 2 further hours. The ethanol was evaporated and the mixture was neutralized to give a precipitate which was filtered and dried to give the title compound (0.089 g, 51 %) as a white solid.
LRMS (m/z): 113 (M+1)⁺.

### PREPARATION 40

### (E)-4-aminotricyclo[3.3.1.1^{3,7}]decan-1-ol

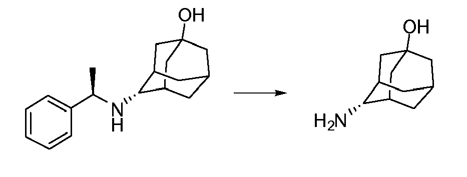

To a solution of 4-{[(1*R*)-1-phenylethyl]amino}adamantan-1-ol (0.400 g, 1.47 mmol; prepared as described in Tetrahedron. Lett. 2006, 47, 8063) in ethanol (58 mL) was added 10% palladium on charcoal (0.033 g) and ammonium formate (0.430 g, 6.82 mmol). The reaction mixture was stirred at 85 °C for one hour then filtered through Celite^{®} and the filtrate was evaporated to dryness to obtain the title compound as a solid (99%) which was used in the next step without further purification.
E-isomer: 1H-NMR δ (CDCl₃): 1.17-1.52 (m, 5H), 1.7-1.83 (m, 6H), 1.9 (m, 3H), 1.94 (m, 1 H), 2.09 (m, 1 H), 3.01 (bs, 1 H).

### PREPARATION 41

### (Z)-4-aminotricycio[3.3.1.1^{3,7}]decan-1-ol

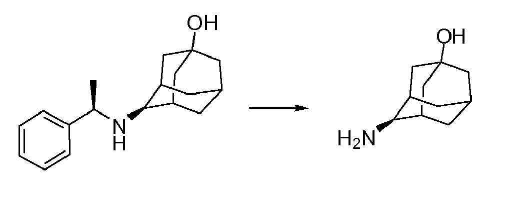

The title compound was obtained (99%) from the corresponding Z-Isomer (0.102 g, 0.38 mmol; prepared as described in Tetrahedron. Lett. 2006, 47, 8063;) following the experimental procedure as described in preparation 40. The crude product was used in the next step without further purification.
Z-isomer: 1H-NMR δ (CDCl₃): 1.51-1.4 (m, 2H), 1.7-1.57 (m, 6H), 1.9-1.8 (m, 4H), 2.09-2.00 (m, 1 H), 2.87 (bs, 1 H).

### PREPARATION 42

### 3-(4,5-Dichloropyrimidin-2-yl)-6-fluoroimidazo[1,2-a]pyridine

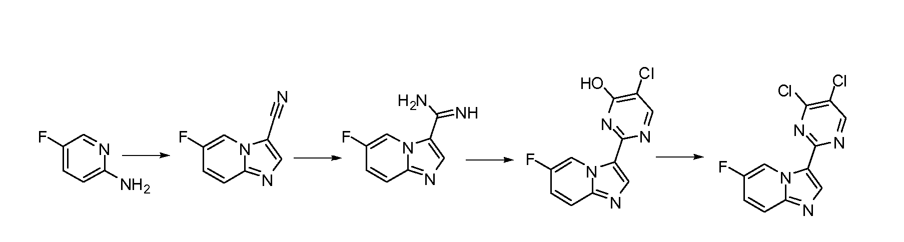

### a) 6-Fluoroimidazo[1 ,2-a]pyridine-3-carbonitrile

1-Bromopyrrolidine-2,5-dione (15.8 g, 89.2 mmol) was added portionwise to a cooled (ice-bath), stirred solution of (*E*)-3-methoxyacrylonitrile (7.49 mL, 89.2 mmol) in 1,4-dioxane/water (3:1, 600 mL). The solution was stirred for 30 minutes at 0 °C and then 5-fluoropyridin-2-amine (5.0 g, 44.6 mmol) was added. The mixture was warmed to ambient temperature over 2 hours and then was heated to 60 °C and stirred for 16 hours at 60 °C. The organic solvent was removed under reduced pressure and the residue was partitioned between ethyl acetate and aqueous sodium hydrogencarbonate solution. The organic phase was dried, filtered and evaporated to dryness. The crude product was purified by flash chromatography (15-50% ethyl acetate/hexane) to give the title compound (94%) as a brown solid.
LRMS (m/z): 162 (M+1)⁺.
¹H-NMR δ (DMSO-*d₆*): 7.71 (ddd, 1 H), 7.93 (dd, 1 H), 8.49 (s, 1 H), 8.98 (bs, 1 H).

### b) 6-Fuoroimidazo[1,2-a]pyridine-3-carboximidamide

To a solution of 6-fluoroimidazo[1,2-a]pyridine-3-carbonitrile (5.6g, 34.8 mmol, preparation 42a) in MeOH (80 mL) was added sodium methoxide (0.19 g, 3.4 mmol). The mixture was stirred at ambient temperature for 17 hours, then ammonium chloride (2.05 g, 38.3 mmol) was added and the mixture was refluxed for 4 hours. The solvent was removed under reduced pressure and the resulting solid was washed with ethyl acetate (8x30 mL). The title compound was obtained as a brown solid (58%) and was used in the next step without further purification.
LRMS (m/z): 179 (M+1)⁺. δ
¹H-NMR δ (DMSO-*d₆*): 7.70 (dd, 1 H), 7.89 (dd, 1 H), 8.35 (s, 1 H), 8.85 (brs, 1 H).

### c) 5-Chloro-2-(6-fluoroimidazo[1,2-a]pyridin-3-yl)pyrimidin-4-ol

To a solution of sodium (0.9 g, 39.1 mmol) in methanol (50 mL) was added 6-fluoroimidazo[1,2-a]pyridine-3-carboximidamide (3.5 g, 19.6 mmol, preparation 42b) and ethyl 2-chloro-3-oxopropanoate (5.8 g, 38.5 mmol). The mixture was refluxed under strong agitation for 18 hours. The reaction mixture was cooled to ambient temperature and filtered. The resulting solid was washed with methanol and ether to yield the title compound (70%), which was used in the next step without further purification.
LRMS (m/z): 265 (M+1)⁺.
¹H-NMR δ (DMSO-*d₆*): 7.43 (dd, 1 H), 7.73 (dd, 1 H), 7.95 (s, 1 H), 8.26 (s, 1 H), 10.18 (dd, 1H).

### d) 3-(4,5-Dichloropyrimidin-2-yl)-6-fluoroimidazo[1,2-a]pyridine

A solution of 5-chloro-2-(6-fluoroimidazo[1,2-a]pyridin-3-yl)pyrimidin-4-ol (2.6 g, 9.8 mmol, preparation 42c) in phosphoryl trichloride (26 mL) was stirred at 110 °C for 3 hours. The crude reaction was slowly added onto water, basified with 8N sodium hydroxide and extracted with ethyl acetate. The organic phase was washed with water and brine and the organic solvent was removed under reduced pressure to give the title compound (62%) as a solid.
LRMS (m/z): 284 (M+1)⁺.
¹H-NMR δ +(CDCl₃): 7.34 (ddd, 1 H), 7.75 (dd, 1 H), 8.66 (s, 1 H), 8.70 (s, 1 H), 9.75 (dd, 1H).

### PREPARATION 43

### 5-Chloro-2-(6-fluoroimidazo[1,2-a]pyridin-3-yl)-N-[(3R)-piperidin-3-yl]pyrimidin-4-amine

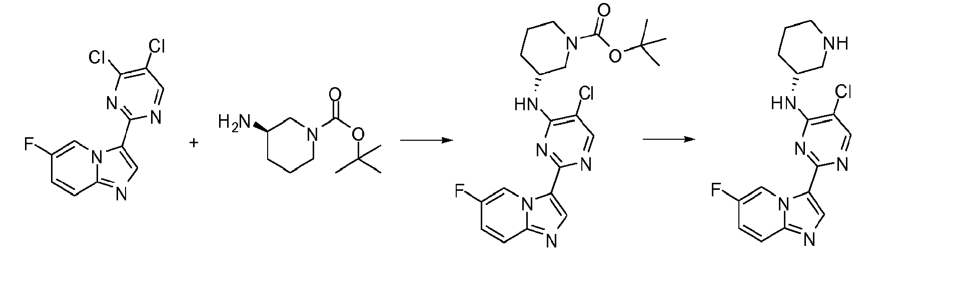

### a) Tert-butyl (3R)-3-{[5-chloro-2-(6-fluoroimidazo[1,2-a]pyridin-3-yl)pyrimidin-4-yl]amino}piperidine-1-carboxylate

A solution of 3-(4,5-dichloropyrimidin-2-yl)-6-fluoroimidazo[1,2-a]pyridine (0.52 g, 1.84 mmol, preparation 42d) and (*R*)-*tert*-butyl 3-aminopiperidine-1-carboxylate (1.12 g, 5.59 mmol) in ethanol (40 mL) was refluxed for 18 hours. The organic solvent was removed under reduced pressure. The residue obtained was triturated with ether and evaporated to dryness to obtain the title compound (99%) as an oil.
LRMS (m/z): 447 (M+1)⁺.

### b) 5-Chloro-2-(6-fluoroimidazo[1,2-a]pyridin-3-yl)-N-[(3R)-piperidin-3-yl]pyrimidin-4-amine

Obtained as a solid (40%) from *tert*-butyl (3*R*)-3-{[5-chloro-2-(6-fluoroimidazo[1,2-*a*] pyridin-3-yl)pyrimidin-4-yl]aminolpiperidine-1-carboxylate (preparation 43a) following the experimental procedure as described in preparation 5b.
LRMS (m/z): 347 (M+1)⁺.
¹H-NMR δ (CDCl₃): 1.64 (bs, 1 H), 1.80 (dd, 2 H), 1.92 - 2.00 (m, 1 H), 2.81 - 2.92 (m, 3 H), 3.24 (dd, 1 H), 4.26 - 4.35 (m, 2 H), 5.92 (bs, 1 H), 7.23 (dd, 1 H), 7.67 (dd, 1 H), 8.22 (s, 1 H), 8.51 (s, 1 H), 9.87 (dd, 1 H).

### EXAMPLE 1

### 3-(4-{[(1S)-1-phenylethyl]amino)pyrimidin-2-yl)imidazo[1,2-a]pyridine-6-carbonitrile

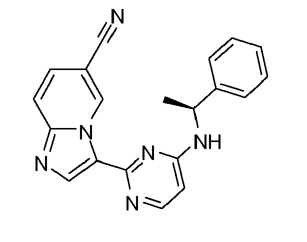

Obtained as a yellow solid (0.102 g, 40%) from 3-(4-hydroxypyrimidin-2-yl)imidazo[1,2-a]pyridine-6-carbonitrile (preparation 4b, 0.175 g, 0.74 mmol) and (*S*)-1-phenylethylamine (0.48 mL, 3.68 mmol) following the experimental procedure as described in preparation 5a. The solvent was removed in vacuum and the residue was purified by reverse phase chromatography (C-18 silica from Waters, water/acetonitrile/methanol as eluents [0.1% v/v formic acid buffered] 0% to 100%) to give the title compound (0.102 g, 40%) as a yellow solid.
LRMS (m/z): 341 (M+1)⁺.
¹H-NMR δ (DMSO-*d₆*): 1.53 (d, 3H), 5.28 (bs, 1 H), 6.52 (bs, 1 H), 7.22 (d, 1 H), 7.36 (d, 2H), 7.48 (d, 2H), 7.67 (d, 1 H), 7.87 (d, 1 H), 8.19 (d, 1 H), 8.26 (bs, 1 H), 8.42 (bs, 1 H), 10.27 (bs, 1 H).

### EXAMPLE 2

### 3-(4-{[(1R)-1-phenylethyl]amino}pyrimidin-2-yl)imidazo[1,2-a]pyridine-6-carbonitrile

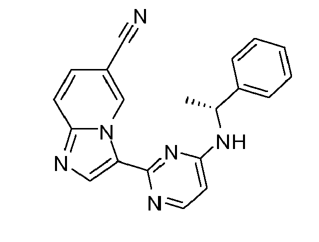

Obtained as a white solid (70%) from 3-(4-hydroxypyrimidin-2-yl)imidazo[1,2-a] pyridine-6-carbonitrile (preparation 4b) and (*R*)-1-phenyl-ethylamine following the experimental procedure as described in preparation 5a. After complete reaction, the mixture was partitioned between water and dichloromethane. The organic layer was washed with water, brine, dried (MgSO₄) and evaporated and the residue was purified by flash chromatography (98:2 to 96:4 dichloromethane/methanol).
LRMS (m/z): 341 (M+1)⁺.
¹H-NMR δ (DMSO-*d₆*): 1.52 (d, 3H), 5.30 (bs, 1 H), 6.52 (d, 1 H), 7.22 (d, 1 H), 7.34 (t, 2H), 7.48 (d, 2H), 7.68 (d, 1 H), 7.88 (d, 1 H), 8.19 (d, 1 H), 8.24 (d, 1 H), 8.43 (s, 1 H), 10.28 (bs, 1 H)

### EXAMPLE 3

### 3-[4-(Benzylamino)pyrimidin-2-yl]imidazo[1,2-a]pyridine-6-carbonitrile

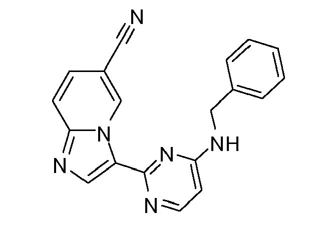

Obtained as a yellow solid (60%) from 3-(4-hydroxypyrimidin-2-yl)imidazo[1,2-a]pyridine-6-carbonitrile (preparation 4b) and phenylmethanamine following the experimental procedure as described in preparation 5a. The desired compound was obtained after purification of the reaction crude by reverse phase chromatography (C-18 silica from Waters, water/acetonitrile/methanol as eluents [0.1% v/v formic acid buffered] 0% to 100%).
LRMS (m/z): 327 (M+1)⁺.
¹H-NMR δ (DMSO-*d₆*): 5.76 (bs, 2H), 6.61 (bs, 1H), 7.25 - 7.4 (m, 5H), 7.68 (d, 1 H), 7.89 (d, 1 H), 8.17 - 8.36 (m, 2H), 8.48 (s, 1 H), 10.37 (bs, 1 H)

### EXAMPLE 4

### 3-(4-{[(1S)-2-methoxy-1-methylethyl]amino)pyrimidin-2-yl)imidazo[1,2-a]pyridine-6-carbonitrile

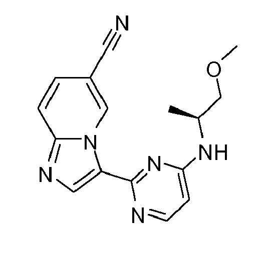

Obtained as a white solid (33%) from 3-(4-hydroxypyrimidin-2-yl)imidazo[1,2-a] pyridine-6-carbonitrile (preparation 4b) and (*S*)-1-methoxypropan-2-amine following the experimental procedure as described in preparation 5a. After complete reaction the mixture was partitioned between water and dichloromethane. The organic layer was washed with water, brine, dried (MgSO₄) and evaporated and the residue was purified by flash chromatography (98:2 to 96:4 dichloromethane/methanol).
LRMS (m/z): 307 (M-1)⁺.
¹H-NMR δ (CDCl₃): 1.36 (d, 3H), 3.43 (s, 3H), 4.27 (bs, 1H), 5.25 (bs, 1H), 6.26 (d, 1 H), 7.40 (d, 1 H), 7.77 (d, 1 H), 8.24 (d, 1 H), 8.58 (s, 1 H), 10.56 (s, 1 H)

### EXAMPLE 5

### 3-{4-[(Cyclohexylmethyl)amino]pyrimidin-2-yl}imidazo[1,2-a]pyridine-6-carbonitrile

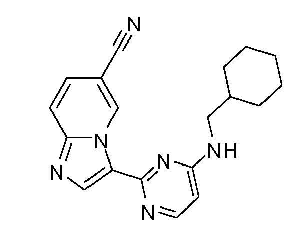

Obtained as a white solid (50%) from 3-(4-hydroxypyrimidin-2-yl)imidazo[1,2-a] pyridine-6-carbonitrile (preparation 4b) and cyclohexylmethanamine following the experimental procedure as described in preparation 5a. After complete reaction, the mixture was partitioned between water and dichloromethane. The organic layer was washed with water and then extracted with 2M aqueous hydrochloric acid. The aqueous phase was basified with 6M aqueous sodium hydroxide solution and then extracted with dichloromethane. The organic layer was dried (MgSO₄) and evaporated to give the title compound.
LRMS (m/z): 333 (M+1)⁺.
¹H-NMR δ (CDCl₃): 0.91 - 1.93 (m, 10H), 1.92 - 2.05 (m, 1 H), 3.13 - 3.33 (bs, 1 H), 3.40 (d, 2H), 6.24 (d, 1 H), 7.40 (d, 1 H), 7.78 (d, 1 H), 8.18 - 8.36 (m, 1 H), 8.59 (bs, 1H), 10.56 (bs, 1 H).

### EXAMPLE 6

### 3-{4-[(2-Methoxyethyl)amino]pyrimidin-2-yl}imidazo[1,2-a]pyridine-6-carbonitrile

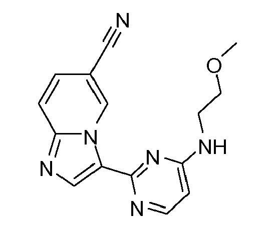

Obtained as a white solid (60%) from 3-(4-hydroxypyrimidin-2-yl)imidazo[1,2-a] pyridine-6-carbonitrile (preparation 4b) and 2-methoxyethanamine following the experimental procedure as described in preparation 5a.
LRMS (m/z): 295 (M+1)⁺.
¹H-NMR δ (DMSO-*d₆*): 3.08 - 3.76 (m, 4H), 3.36 (s, 3H), 6.50 (d, 1H), 7.73 (d, 1 H), 7.84 (bs, 1 H), 7.94 (d, 1 H), 8.23 (bs, 1 H), 8.52 (s, 1 H), 10.52 (bs, 1 H)

### EXAMPLE 7

### 3-{4-[(1-Adamantylmethyl)amino]pyrimidin-2-yl}imidazo[1,2-a]pyridine-6-carbonitrile

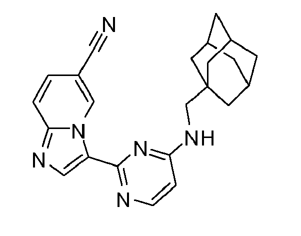

Obtained as a white solid (51%) from 3-(4-hydroxypyrimidin-2-yl)imidazo[1,2-a] pyridine-6-carbonitrile (preparation 4b) and 1-(1-adamantyl)methanamine following the experimental procedure as described in preparation 5a. The crude product was purified by flash chromatography (100:8:1 dichloromethane/methanol/aqueous ammonia solution).
LRMS (m/z): 385 (M+1)⁺.
¹H-NMR δ (DMSO-*d₆*): 1.60 - 1.66 (m, 12H), 1.86 - 2.03 (m, 3H), 4.67 (s, 2H), 6.48 (d, 1 H), 7.64 (bs, 1 H), 7.68 (d, 1 H), 7.89 (d, 1 H), 8.13 (d, 1 H), 8.48 (bs, H) 10.56 (bs, 1H).

### EXAMPLE 8

### 3-{4-[(2,2-Dimethylpropyl)amino]pyrimidin-2-yl}imidazo[1,2-a]pyridine-6-carbonitrile

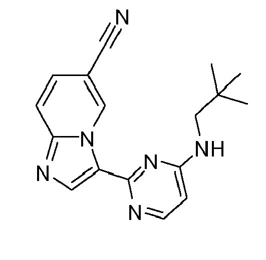

Obtained as a white solid (26%) from 3-(4-hydroxypyrimidin-2-yl)imidazo[1,2-a] pyridine-6-carbonitrile (preparation 4b) and 2,2-dimethylpropan-1-amine following the experimental procedure as described in preparation 5a. The crude product was purified by reverse phase chromatography (C-18 silica from Waters, water/acetonitrile/methanol as eluents [0.1% v/v formic acid buffered] 0% to 100%).
LRMS (m/z): 307 (M+1)⁺.
¹H-NMR δ(DMSO-*d₆*): 1.03 (s, 9H), 4.17 (d, 2H), 6.54 (d, 1 H), 7.64 - 7.73 (bs, 1 H), 7.72 (d, 1 H), 7.95 (d, 1 H), 8.19 (d, 1 H), 8.53 (s, 1 H), 10.59 (bs, 1 H).

### EXAMPLE 9

### 3-{5-Bromo-4-[(2,2-dimethylpropyl)amino]pyrimidin-2-yl}imidazo[1,2-a]pyridine-6-carbonitrile

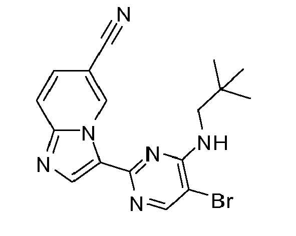

To a stirred solution of 3-{4-[(2,2-dimethylpropyl)amino]pyrimidin-2-yl}imidazo[1,2-*a*] pyridine-6-carbonitrile (example 8, 0.300 g, 1.0 mmol) in acetic acid (3.6 mL) was added potassium acetate (0.104 g, 1.1 mmol). The mixture was cooled (ice-bath) and a solution of bromine (0.050 mL, 1.0 mmol) in acetic acid (0.2 mL) was added dropwise. The reaction was then stirred at ambient temperature for 1 hour. Saturated aqueous sodium carbonate solution (30 mL) was added and the mixture was extracted with ethyl acetate. The organic layer was washed with brine, dried (MgSO₄) and evaporated to give the title compound (0.390 g, 98%) as an off-white solid.
LRMS (m/z): 385/387 (M+1)⁺.
¹H-NMR δ (CDCl₃): 1.06 (s, 9H), 3.49 (d, 2H), 5.62 (bt, 1 H), 7.42 (ddd, 1 H), 7.80 (ddd, 2H), 8.37 (d, 1 H), 8.60 (d, 1 H), 10.48 (m, 1 H)

### EXAMPLE 10

### 3-{4-[(2,2-Dimethylpropyl)amino]-5-piperazin-1-ylpyrimidin-2-yl}imidazo[1,2-a]pyridine-6-carbonitrile

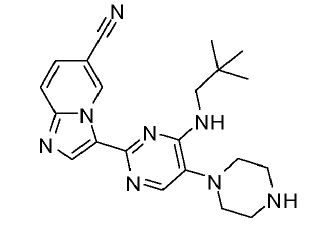

a) Nitrogen was bubbled for 5 minutes through a mixture 3-{5-bromo-4-[(2,2-dimethyl propyl)amino]pyrimidin-2-yl}imidazo[1,2-*a*]pyridine-6-carbonitrile (example 9, 0.105 g, 0.27 mmol), tert-butyl piperazine-1-carboxylate (0.152 g, 0.82 mmol), sodium *tert-*butoxide (0.079 g, 0.82 mmol) and biphenyl-2-yl-di-*tert*-butylphosphine (0.020 g, 0.07 mmol) in toluene (2.5 mL) contained in a microwave reaction vessel. Tris(dibenzylideneacetone)dipalladium (0) (0.125 g, 0.14 mmol) was then added and the vessel was sealed and subjected to microwave irradiation for 1 hour at 115 °C. Water and ethyl acetate were added to the mixture and the organic layer was dried (MgSO₄) and evaporated. Purification of the residue by reverse phase chromatography (C-18 silica from Waters, water/acetonitrile/methanol as eluents [0.1% v/v formic acid buffered] 0% to 100%) gave *tert*-butyl-4-{2-(6-cyanoimidazo[1,2-*a*]pyridin-3-yl)-4-[(2,2-dimethylpropyl)amino]pyrimidin-5-yl}piperazine-1-carboxylate (0.023 g, 16%) as a yellow solid.
   LRMS (m/z): 491 (M+1)⁺.
   ¹H-NMR δ (CDCl₃): 1.05 (s, 9H), 1.52 (d, 9H), 2.18 (d, 2H), 3.00 - 2.86 (m, 4H), 3.53 - 3.39 (m, 4H), 7.38 (d, 1 H), 7.77 (d, 1 H), 8.00 (s, 1 H), 8.55 (s, 1 H), 10.60 (s, 1 H).
b) To a solution of tert-butyl 4-{2-(6-cyanoimidazo[1,2-*a*]pyridin-3-yl)-4-[(2,2-dimethyl propyl)amino]pyrimidin-5-yl}piperazine-1-carboxylate (0.022 g, 0.04 mmol) in dioxane (1 mL) was added a 4M solution of hydrogen chloride in 1,4-dioxane (0.140 mL, 0.56 mmol). The mixture was stirred at ambient temperature for 20 hours and then solvent was evaporated and water was added. The aqueous layer was washed with dichloromethane and then basified with concentrated aqueous ammonia solution and extracted with dichloromethane. The organic layer was dried (MgSO₄) and evaporated to obtain (0.008 g, 41%) of the title compound as a pale yellow solid.
   LRMS (m/z): 391 (M+1)⁺.
   ¹H-NMR δ (CDCl₃): 1.06 (s, 9H), 3.13 - 2.90 (m, 8H), 3.46 - 3.40 (m, 2H), 5.91 (t, 1 H), 7.40 - 7.35 (m, 1 H), 7.77 (d, 1 H), 8.02 (s, 1 H), 8.54 (s, 1 H), 10.61 (s, 1 H).

### EXAMPLE 11

### 3-[4-[(2,2-Dimethylpropyl)amino]-5-(2-methoxypyridin-4-yl)pyrimidin-2-yl]imidazo[1,2-a]pyridine-6-carbonitrile

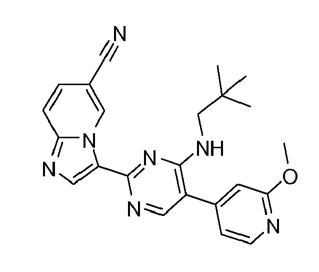

Obtained as a pale yellow solid (54%) from 3-[5-bromo-4-[(2,2-dimethylpropyl)amino] pyrimidin-2-yl}imidazo[1,2-*a*]pyridine-6-carbonitrile (example 9) and 2-methoxy-4-(4,4,5,5-tetramethyl-1,3,2-dioxaborolan-2-yl)pyridine (preparation 38) following the experimental procedure as described in preparation 27d. The crude product mixture was filtered through Celite^{®} washing the filter cake with ethyl acetate. The solvent was removed under reduced pressure and the residue was purified by reverse phase chromatography (C-18 silica from Waters, water/acetonitrile/methanol as eluents [0.1 % v/v formic acid buffered] 0% to 100%) to yield the desired compound.
LRMS (m/z): 414 (M+1)⁺.
¹H-NMR δ (CDCl₃): 1.03 (s, 9H), 3.47 (bs, 2H), 4.04 (s, 3H), 5.31 (d, 1H), 6.86 (s, 1 H), 6.99 (t, 1 H), 7.44 (d, 1 H), 7.82 (d, 1 H), 8.18 (s, 1 H), 8.34 (t, 1 H), 8.67 (s, 1 H), 10.62 (s, 1 H).

### EXAMPLE 12

### 3-[4-[(2,2-Dimethylpropyl)amino]-5-(2-oxo-1,2-dihydropyridin-4-yl)pyrimidin-2-yl]imidazo[1,2-a]pyridine-6-carbonitrile

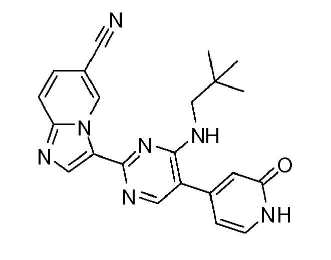

Obtained as solid (63%) from 3-[4-[(2,2-dimethylpropyl)amino]-5-(2-methoxypyridin-4-yl)pyrimidin-2-yl]imidazo[1,2-*a*]pyridine-6-carbonitrile (example 11) following the experimental procedure as described in preparation 4b.
LRMS (m/z): 400 (M+1)⁺.
¹H-NMR δ (DMSO-*d₆*): 1.05 (s, 9H), 3.47 (d, 2H), 6.34 (d, 1 H), 6.53 (s, 1 H), 7.45 (bs, 1 H), 7.60 (d, 1H), 7.87 (d, 1H), 8.05 (d, 1 H), 8.28 (s, 1H), 8.72 (s, 1 H), 10.54 (s, 1 H).

### EXAMPLE 13

### 3-{4-[(2,2-Dimethylpropyl)amino]-5-pyridin-3-ylpyrimidin-2-yl}imidazo[1,2-a]pyridine-6-carbonitrile

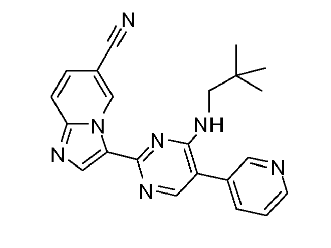

Obtained as a yellow solid (22%) from 3-(4-hydroxy-5-pyridin-3-ylpyrimidin-2-yl)imidazo [1,2-*a*]pyridine-6-carbonitrile (preparation 26b) and neopentylamine following the experimental procedure as described in preparation 5a followed by purification of the crude product by reverse phase chromatography (C-18 silica from Waters, water/acetonitrile/methanol as eluents [0.1% v/v formic acid buffered] 0% to 100%).
LRMS (m/z): 384 (M+1)⁺.
¹H-NMR δ (CDCl₃): 1.00 (s, 9H), 3.47 (d, 2H), 5.10 (t, 1 H), 7.43 (ddd, 1 H), 7.51 (dd, 2H), 7.81 (ddd, 2H), 8.15 (d, 1 H), 8.66 (s, 1 H), 8.78 - 8.71 (m, 2H), 10.63 (dd, 1 H)

### EXAMPLE 14

### 3-{4-[(3-Fluorobenzyl)amino]pyrimidin-2-yl}imidazo[1,2-a]pyridine-6-carbonitrile

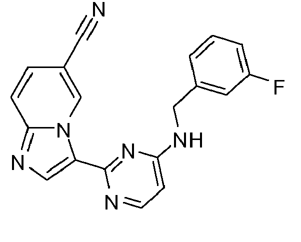

Obtained as a white solid (17%) from 3-(4-hydroxypyrimidin-2-yl)imidazo[1,2-a]pyridine -6-carbonitrile (preparation 4b) and (3-fluorophenyl)methanamine following the experimental procedure as described in preparation 5a, followed by purification of the crude product by reverse phase chromatography (C-18 silica from Waters, water/acetonitrile/methanol as eluents [0.1 % v/v formic acid buffered] 0% to 100%).
LRMS (m/z): 345 (M+1)⁺.
¹H-NMR δ (DMSO-*d₆*): 5.02 (bs, 2H), 6.64 - 6.97 (m, 1H), 7.29 - 7.46 (m, 1H), 7.59 (bs, 2H), 7.64 - 7.81 (m, 1 H), 7.87 - 8.07 (m, 1 H), 8.10 - 8.35 (m, 1 H), 8.57 (m, 2H), 8.79 (bs, 1 H), 10.66 (bs, 1 H)

### EXAMPLE 15

### 3-{4-[(4-Fluorobenzyl)amino]pyrimidin-2-yl}imidazo[1,2-a]pyridine-6-carbonitrile

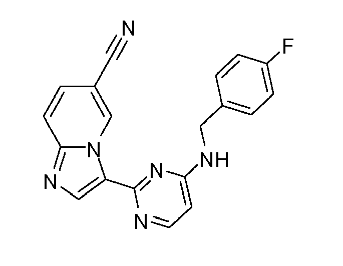

Obtained as a white solid (11%) from 3-(4-hydroxypyrimidin-2-yl)imidazo[1,2-a]pyridine -6-carbonitrile (preparation 4b) and (4-fluorophenyl)methanamine following the experimental procedure as described in preparation 5a, followed by purification of the crude product by reverse phase chromatography (C-18 silica from Waters, water/acetonitrile/methanol as eluents [0.1% v/v formic acid buffered] 0% to 100%).
LRMS (m/z): 345 (M+1)⁺.
¹H-NMR δ (CDCl₃): 4.65 (bs, 2H), 6.28 (d, 1 H), 6.95 - 7.13 (m, 2H), 7.26 (s, 1 H), 7.31 - 7.45 (m, 2H), 7.77 (d, 1H), 8.29 (d, 1H), 8.59 (s, 1 H), 10.49 (bs, 1 H)

### EXAMPLE 16

### 3-{4-[(2-Methylbenzyl)amino]pyrimidin-2-yl}imidazo[1,2-a]pyridine-6-carbonitrile

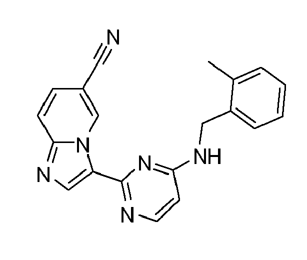

Obtained as a white solid (39%) from 3-(4-hydroxypyrimidin-2-yl)imidazo[1,2-a]pyridine -6-carbonitrile (preparation 4b) and o-tolylmethanamine following the experimental procedure as described in preparation 5a followed by addition of water and extraction with ethyl acetate. The organic layer was washed with water, brine, dried (MgSO₄) and evaporated in vacuum and the residue was purified by flash chromatography (99:1 to 98:2 dichloromethane/methanol) to yield the desired compound.
LRMS (m/z): 341 (M+1)⁺.
¹H-NMR δ (DMSO-*d₆*): 2.43 (m, 3H), 4.52 - 4.85 (m, 2H), 6.38 - 6.73 (m, 1 H), 7.10 - 7.30 (m, 3H), 7.30 - 7.44 (m, 1 H), 7.60 - 7.82 (m, 1 H), 7.91 - 7.94 (m, 1 H), 8.04 - 8.20 (m, 1 H), 8.20 - 8.36 (m, 1 H), 8.43 - 8.61 (m, 1 H), 10.40 (bs, 1 H).

### EXAMPLE 17

### Tert-butyl-2-({[2-(6-cyanoimidazo[1,2-a]pyridin-3-yl)pyrimidin-4-yl]amino}methyl) piperidine-1-carboxylate

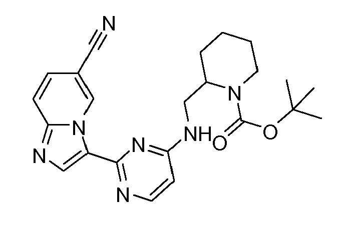

Obtained as a yellow solid (93%) from 3-(4-hydroxypyrimidin-2-yl)imidazo[1,2-a] pyridine-6-carbonitrile (preparation 4b) and tert-butyl 2-(aminomethyl)piperidine-1-carboxylate (prepared as described in Chem Pharm Bull, 1998, 787-796) following the experimental procedure as described in preparation 5a followed by addition of water and extraction with ethyl acetate. The organic layer was washed with water, brine, dried (MgSO₄) and evaporated in vacuum and the residue was purified by flash chromatography (98:2 dichloromethane/methanol) to give the title compound.
LRMS (m/z): 434 (M+1)⁺.
¹H-NMR δ (CDCl₃): 1.46 (s, 9H), 1.55 - 1.86 (m, 4H), 2.80 - 2.97 (m, 1 H), 3.10 - 3.25 (m, 1 H), 3.44 - 3.59 (m, 1 H), 3.76 - 4.15 (m, 2H), 4.30 - 4.51 (m, 1 H), 4.49
- 4.72 (m, 1 H), 6.24 (bs, 1 H), 7.42 (d, 1 H), 7.78 (d, 1 H), 8.22 (bs, 1 H), 8.59 (s, 1 H), 10.58 (bs, 1 H)

### EXAMPLE 18

### 3-(4-{[(1-Acetylpiperidin-2-yl)methyl]amino}pyrimidin-2-yl)imidazo[1,2-a]pyridine-6-carbonitrile

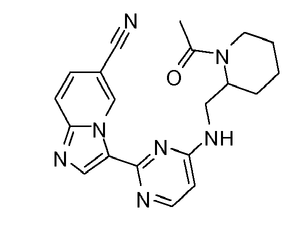

a) 3-{4-[(Piperidin-2-ylmethyl)amino]pyrimidin-2-yl}imidazo[1,2-*a*]pyridine-6-carbonitrile was obtained as a white solid (0.130 g, 80%) from tert-butyl 2-({[2-(6-cyanoimidazo [1,2-*a*]pyridin-3-yl)pyrimidin-4-yl]amino}methyl)piperidine-1-carboxylate (example 17, 0.213 g, 0.49 mmol) following the experimental procedure as described in preparation 5b. After the reaction was complete, water was added and the mixture was then neutralized with solid sodium hydrogen carbonate. The resultant precipitate was filtered, washed with water and dried in vacuum.
   LRMS (m/z): 334 (M+1)⁺.
   ¹H-NMR δ (DMSO-*d₆*): 1.37 - 1.62 (m, 4H), 1.65 - 1.85 (m, 2H), 1.88 - 2.03 (m, 1 H), 2.77 - 2.97 (m, 2H), 3.45 - 3.76 (m, 2H), 6.41 - 6.58 (m, 1 H), 7.64 - 7.77 (m, 1 H), 7.87 (bs, 1 H), 7.90 - 7.98 (m, 1 H), 8.18 - 8.40 (m, 1 H), 8.58 - 8.79 (m, 1 H), 10.46 (bs, 1 H).
b) Triethylamine (0.13 mL, 0.95 mol) and acetyl chloride (0.05 mL, 0.73 mmol) were added sequentially to a stirred solution of 3-{4-[(piperidin-2-ylmethyl)amino]pyrimidin-2-yl}imidazo[1,2-*a*]pyridine-6-carbonitrile (0.100 g, 0.3 mmol) in dichloromethane (4 mL) and the mixture was stirred at ambient temperature for 16 hours. The mixture was evaporated in vacuum and then taken up in dichloromethane and the organic layer was washed with 4% aqueous sodium hydrogen carbonate solution, water, brine, dried (MgSO₄) and evaporated. Purification of the residue by flash chromatography (98:2 to 96:4 dichloromethane/methanol) gave the title compound (0.040 g, 35%) as a white solid.
   LRMS (m/z): 376 (M+1)⁺.
   ¹H-NMR δ (DMSO-*d₆*): 1.11 - 1.88 (m, 6H), 2.56 (s, 3H), 3.46 - 3.95 (m, 2H) 4.14 - 4.31 (m, 1 H), 4.29 - 4.51 (m, 1 H), 4.95 (bs, 1 H), 6.26 - 6.60 (m, 1 H), 7.62 - 7.82 (m, 1 H), 7.85 - 8.03 (m, 1 H), 8.11 - 8.35 (m, 1 H), 8.49 - 8.73 (m, 1 H), 10.39 - 10.65 (m, 1 H)

### EXAMPLE 19

### 3-[4-(Tetrahydro-2H-pyran-4-ylamino)pyrimidin-2-yl]imidazo[1,2-a]pyridine-6-carbonitrile

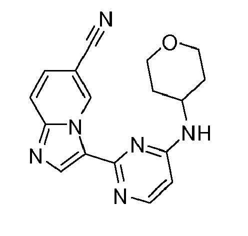

Obtained as a white solid (11%) from 3-(4-hydroxypyrimidin-2-yl)imidazo[1,2-a]pyridine -6-carbonitrile (preparation 4b) and tetrahydro-2*H*-pyran-4-amine hydrochloride following the experimental procedure as described in preparation 5a, followed by purification of the crude product by reverse phase chromatography (C-18 silica from Waters, water/acetonitrile/methanol as eluents [0.1% v/v formic acid buffered] 0% to 100%).
LRMS (m/z): 321 (M+1)⁺.
¹H-NMR δ (CDCl₃): 1.65 (d, 2H), 2.12 (d, 2H), 3.64 (d, 2H), 4.07 (d, 2H), 5.03 (bs, 1H), 6.26 (bs, 1H), 7.27 (d,1H), 7.41 (dd, 1H), 7.79 (d, 1H), 8.27 (bs, 1H), 8.58 (bs, 1 H), 10.54 (bs, 1 H)

### EXAMPLE 20

### 3-(4-(8-Fluorochroman-4-ylamino)pyrimidin-2-yl)imidazo[1,2-a]pyridine-6-carbonitrile

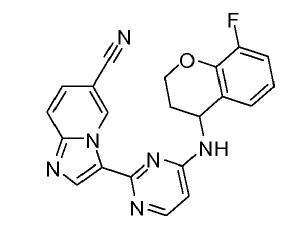

Obtained as a white solid (8%) from 3-(4-hydroxypyrimidin-2-yl)imidazo[1,2-a]pyridine-6-carbonitrile (preparation 4b) and (8-fluoro-3,4-dihydro-2H-chromen-4-yl) amine (prepared as described in WO2008043019) following the experimental procedure as described in preparation 5a, followed by purification of the crude product by reverse phase chromatography (C-18 silica from Waters, water/acetonitrile/methanol as eluents [0.1 % v/v formic acid buffered] 0% to 100%).
LRMS (m/z): 387 (M+1)⁺.
¹H-NMR δ (CDCl₃): 1.75 (bs, 1 H), 2.46 - 2.26 (m, 2H), 4.51 - 4.30 (m, 2H), 6.32 (d, 1 H), 6.87 (td, 1 H), 7.11 - 7.02 (m, 2H), 7.42 (dd, 1 H), 7.80 (d, 1 H), 8.34 (d, 1H), 8.61 (s, 1H), 10.57 (s, 1H).

### EXAMPLE 21

### 3-[4-(Cyclohexylamino)pyrimidin-2-yl]imidazo[1,2-a]pyridine-6-carbonitrile

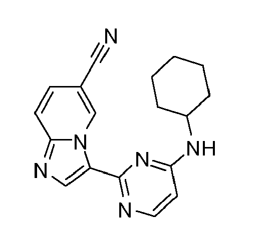

Obtained as an off-white solid (53%) from 3-(4-hydroxypyrimidin-2-yl)imidazo[1,2-a] pyridine-6-carbonitrile (preparation 4b) and cyclohexanamine following the experimental procedure as described in preparation 5a followed by addition of water and extraction with ethyl acetate. The organic layer was washed with water, brine, dried (MgSO₄) and evaporated. The residue was purified by flash chromatography (99:1 to 98:2 dichloromethane/methanol) to give the desired compound.
LRMS (m/z): 319 (M+1)⁺.
¹H-NMR δ (CDCb): 1.13 - 1.40 (m, 6H), 1.39 - 1.51 (m, 1 H), 1.64 - 1.77 (m, 1 H), 1.77 - 1.89 (m, 2H), 1.95 - 2.25 (m, 2H), 6.21 (d, 1 H), 7.41 (d, 1 H), 7.77 (d, 1 H), 8.15 - 8.37 (m, 1H), 8.58 (s, 1 H), 10.54 (s, 1 H)

### EXAMPLE 22

### 3-{4-[(Trans-4-hydroxycyclohexyl)amino]pyrimidin-2-yl}imidazo[1,2-a]pyridine-6-carbonitrile

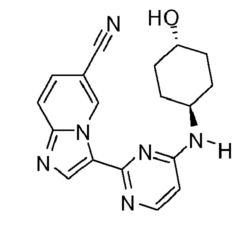

Obtained as a solid (17%) from 3-(4-hydroxypyrimidin-2-yl)imidazo[1,2-a]pyridine-6-carbonitrile (preparation 4b) and *trans*-4-aminocyclohexanol following the experimental procedure as described in preparation 5a, followed by purification of the crude product by reverse phase chromatography (C-18 silica from Waters, water/acetonitrile/ methanol as eluents [0.1 % v/v formic acid buffered] 0% to 100%).
LRMS (m/z): 335 (M+1)⁺.
¹H-NMR δ (CDCl₃): 1.61 - 1.32 (m, 4H), 2.17 (dd, 4H), 2.67 - 2.61 (m, 1 H), 3.73 (ddd, 1 H), 6.23 (d, 1 H), 7.40 (dd, 1 H), 7.79 (d, 1 H), 8.27 (d, 1 H), 8.58 (s, 1 H), 10.54 (s, 1 H).

### EXAMPLE 23

### 3-{4-[(5-Hydroxy-2-adamantyl)amino]pyrimidin-2-yl}imidazo[1,2-a]pyridine-6-carbonitrile

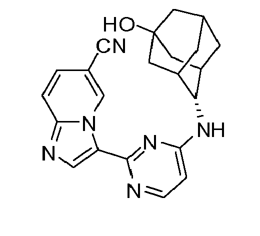

Obtained as a solid (1,6%) from 3-(4-hydroxypyrimidin-2-yl)imidazo[1,2-a]pyridine-6-carbonitrile (preparation 4b) and (*E*)-4-aminotricyclo[3.3.1.1^{3,7}]decan-1-ol (preparation 40) following the experimental procedure as described in preparation 5a, followed by purification by reverse phase chromatography (C-18 silica from Waters, water/acetonitrile/methanol as eluents [0.1 % v/v formic acid buffered] 0% to 100%).
LRMS (m/z): 387 (M+1)⁺.

### EXAMPLE 24

### 3-{4-[(5-Hydroxy-2-adamantyl)amino]pyrimidin-2-yl}imidazo[1,2-a]pyridine-6-carbonitrile

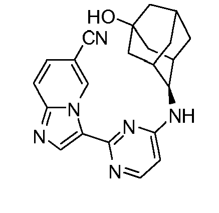

Obtained as a solid (6%) from 3-(4-hydroxypyrimidin-2-yl)imidazo[1,2-a]pyridine-6-carbonitrile (preparation 4b) and (*Z*)-4-aminotricyclo[3.3.1.1^{3,7}]decan-1-ol (preparation 41) following the experimental procedure as described in preparation 5a, followed by purification by reverse phase chromatography (C-18 silica from Waters, water/acetonitrile/methanol as eluents [0.1% v/v formic acid buffered] 0% to 100%).
LRMS (m/z): 387 (M+1)⁺.
¹H-NMR δ (CDCl₃): 2.00 - 1.64 (m, 13H), 6.26 (d, 1H), 7.42 (dd, 1H), 7.82 (d, 1 H), 8.27 (d, 1 H), 8.58 (s, 1 H), 10.51 (s, 1 H).

### EXAMPLE 25

### 2-{4-[(2,2-Dimethylpropyl)amino]quinazolin-2-yl}imidazo[1,2-a]pyridine-6-carbonitrile

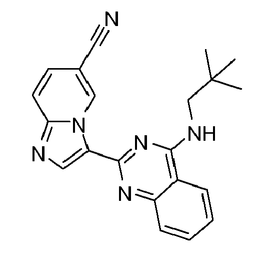

Obtained as a solid (14%) from 3-(4-hydroxyquinazolin-2-yl)imidazo[1,2-a]pyridine-6-carbonitrile (preparation 24b) and 2,2-dimethylpropan-1-amine following the experimental procedure as described in preparation 5a, followed by purification of the crude product by reverse phase chromatography (C-18 silica from Waters, water/acetonitrile/methanol as eluents [0.1% v/v formic acid buffered] 0% to 100%).
LRMS (m/z): 357 (M+1)⁺.
¹H-NMR δ (CDCl₃): 1.11 (s, 9H), 3.67 (d, 2H), 5.87 (t, 2H), 7.42 (dd, 1H), 7.49 (ddd, 1 H), 7.75 - 7.70 (m, 1 H), 7.83 - 7.76 (m, 2H), 7.97 - 7.91 (m, 1 H), 8.70 (s, 1 H), 10.88 (dd, 1 H)

### EXAMPLE 26

### 3-{4-[Benzyl(methyl)amino]pyrimidin-2-yl}imidazo[1,2-a]pyridine-6-carbonitrile

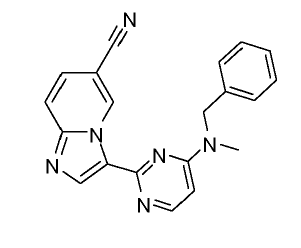

An oven dried resealable Schlenk tube was charged with imidazo[1,2-a]pyridine-6-carbonitrile (preparation 1, 0.30 g, 2.1 mmol), N-benzyl-2-chloro-N-methylpyrimidin-4-amine (preparation 31, 0.24 g, 1.0 mmol), potassium acetate (0.21 g, 2.1 mmol) and *N*,*N*'-dimethylacetamide (3 mL). The Schlenk tube was subjected to three cycles of evacuation-backfilling with argon then tetrakis(triphenylphosphine)palladium (0) (0.12 g, 0.10 mmol) was added. After three further cycles of evacuation-backfilling with argon, the Schlenk tube was capped and placed in an oil bath at 150 °C and the mixture was stirred overnight. The mixture was evaporated in vacuum and the residue was partitioned between ethyl acetate and water. The organic layer was washed with brine, dried (MgSO₄) and evaporated and the residue was purified by flash chromatography (99:1 to 98:2 dichloromethane/methanol) to give the title compound (0.105 g, 30%) as a pale brown solid.
LRMS (m/z): 341 (M+1)+.
¹H-NMR δ (CDCl₃): 3.22 (bs, 3H), 4.91 (bs, 2H), 6.40 (d, 1H), 7.27 - 7.41 (m, 6H), 7.76 (dd, 1 H), 8.31 (d, 1 H), 8.59 (s, 1 H), 10.49 (bs, 1 H)

### EXAMPLE 27

### 3-(4-{[(1S)-1-phenylethyl]amino}pyrimidin-2-yl)imidazo[1,2-a]pyridine-6-carboxylic acid

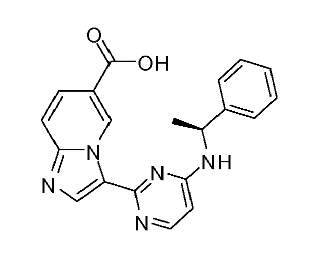

A mixture of 3-[4-((S)-1-Phenyl-ethylamino)-pyrimidin-2-yl]-imidazo[1,2-a]pyridine-6-carbonitrile (example 1, 0.037 g, 0.11 mmol) and potasium hydroxide (0.033 g, 0.59 mmol) in ethylene glycol (0.5 mL) was heated to 150 °C and stirred overnight. The mixture was then cooled to room temperature, water (5 mL) was added and the mixture was extracted with ethyl acetate. The aqueous layer was acidified to a pH of 5 with 5M aqueous hydrochloric acid and the suspension was extracted with chloroform. The organic layer was dried (MgSO₄) and evaporated to give the title compound (0.010 g, 25%) as an off-white solid.
LRMS (m/z): 360 (M+1)⁺, 358 (M-1)⁻.
¹H-NMR δ (DMSO-*d*₆): 1.51 (d, 3H), 5.43 (bs, 1 H), 6.46 (d, 1 H), 7.20 (d, 1 H), 7.31 (t, 2H), 7.50 (d, 2H), 7.73 (d, 1 H), 7.83 (d, 1 H), 8.13 (d, 1 H), 8.18 (d, 1 H), 8.36 (s, 1 H), 10.63 (bs, 1 H)

### EXAMPLE 28

### 2-(6-Fluoroimidazo[1,2-a]pyridin-3-yl)-N-[(1 S)-1-phenylethyl]pyrimidin-4-amine

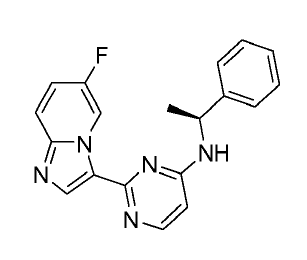

a) 2-(6-fluoroimidazo[1,2-*a*]pyridin-3-yl)-*N*-(4-methoxybenzyl)-*N*-[(1 S)-1-phenylethyl] pyrimidin-4-amine was obtained as a colourless oil (0.029 g, 8%) from 6-fluoroimidazo [1,2-a]pyridine (preparation 2, 0.127 g, 0.93 mmol), 2-chloro-N-(4-methoxybenzyl)-N-[(1S)-1-phenylethyl]pyrimidin-4-amine (preparation 30b, 0.300 g, 0.85 mmol), potassium acetate (0.138g, 1.41 mmol) and tetrakis(triphenylphosphine)palladium (0) (0.098 g, 0.08 mmol) following the experimental procedure as described in preparation 29. The crude product was purified by flash chromatography (1:1 hexanes/ethyl acetate).
   LRMS (m/z): 454 (M+1)⁺.
   ¹H-NMR δ (CDCl₃): 1.68 (d, 3H), 3.78 (s, 3H), 4.41 - 4.69 (m, 3H), 6.20 (d, 1H), 6.84 (d, 2H), 7.11 (d, 2H), 7.14 - 7.24 (m, 1 H), 7.26 - 7.39 (m, 5H), 7.64 - 7.69 (m, 1 H), 8.22 (d, 1 H), 8.52 (s, 1 H), 9.81 (bs, 1 H).
b) A mixture of 2-(6-fluoroimidazo[1,2-*a*]pyridin-3-yl)-*N*-(4-methoxybenzyl)-*N*-[(1*S*)-1-phenylethyl]pyrimidin-4-amine (0.029 g, 0.06 mmol) and trifluoroacetic acid (4 mL) was stirred and heated to 65 °C in a sealed tube. After 19 hours, the mixture was evaporated in vacuum and 2M aqueous hydrochloric acid and ethyl acetate were added to the residue. The aqueous layer was basified with solid sodium hydrogen carbonate and then extracted with chloroform. The organic layer was dried (MgSO₄) evaporated and the residue was purified by flash chromatography (100:1 to 50:1 dichloromethane/methanol), followed by purification by reverse phase chromatography (C-18 silica from Waters, water/acetonitrile/methanol as eluents [0.1% v/v formic acid buffered] 0% to 100%) to give the title compound (0.009 g, 42%) as a white solid.
   LRMS (m/z): 334 (M+1)⁺.
   ¹H-NMR δ (DMSO-*d*₆): 1.56 (d, 3H), 5.29 (bs, 1 H), 6.52 (bs, 1 H), 7.17 - 7.31 (m, 1 H), 7.30 - 7.43 (m, 2H), 7.49 (d, 2H), 7.52 - 7.61 (m, 1 H), 7.73 - 7.88 (m, 1 H), 8.21 (d, 2H), 8.37 (bs, 1 H), 9.78 (bs, 1 H).

### EXAMPLE 29

### Trans-4-{[2-(6-fluoroimidazo[1,2-a]pyridin-3-yl)pyrimidin-4-yl]amino}cyclohexanol

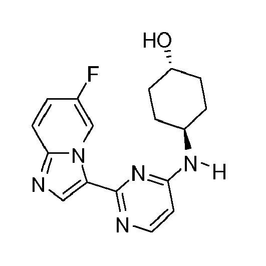

Obtained as a yellow solid (70%) from 2-(6-fluoroimidazo[1,2-*a*]pyridin-3-yl)pyrimidin-4-ol (preparation 8b) and *trans*-4-aminocyclohexanol following the experimental procedure as described in preparation 5a, followed by purification of the crude product by flash chromatography (dichloromethane/methanol 100:10).
LRMS (m/z): 328 (M+1)⁺.
¹H-NMR δ (CDCl₃): 1.38 (dd, 2H), 1.51 (dd, 2H), 2.08 (d, 2H), 2.20 (d, 2H), 3.67 (s, 1 H), 5.74 (d, 1 H), 6.22 (d, 1 H), 7.29 - 7.16 (m, 1 H), 7.73 - 7.59 (m, 1 H), 8.19 (s, 1H), 8.50 (s, 1H), 10.02 (s, 1H).

### EXAMPLE 30

### 3-(4-{[(2S,7S)-5-hydroxy-2-adamantyl]amino}pyrimidin-2-yl)imidazo[1,2-a]pyridine -6-carbonitrile

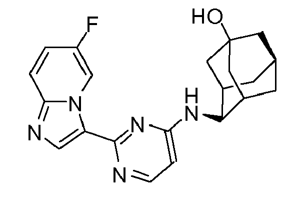

Obtained as a solid (30%) from 2-(6-fluoroimidazo[1,2-*a*]pyridin-3-yl)pyrimidin-4-ol (preparation 8b) and (4*S*,7*S*)-4-aminotricyclo[3.3.1.1^{3,7}]decan-1-ol following the same experimental procedure as described in preparation 5a with the exception that the reaction was carried out at 60 °C. The crude product was purified by flash chromatography (9:1 dichloromethane/methanol).
LRMS (m/z): 380 (M+1)⁺.
¹H-NMR δ (DMSO-*d*₆): 1.09 (bs, 1 H), 1.40 - 2.02 (m, 10 H), 1.63 (bs, 1 H), 2.10 (bs, 1 H), 2.29 (bs, 1 H), 4.07 (bs, 1 H), 4.46 (s, 1 H), 6.56 (d, 1 H), 7.53 (d, 1 H), 7.81 (dd, 1 H), 8.13 (d, 1 H), 8.37 (s, 1 H), 9.96 (bs, 1 H)

### EXAMPLE 31

### N-(2,2-dimethylpropyl)-2-imidazo[1,2-a]pyridin-3-ylpyrimidin-4-amine

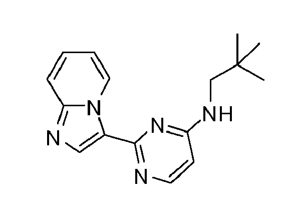

Obtained as a off-white solid (45%) from 2-imidazo[1,2-a]pyridin-3-ylpyrimidin-4-ol (preparation 14b) and neopentylamine following the experimental procedure as described in preparation 5a followed by addition of water and extraction with ethyl acetate. The organic layer was washed with water, brine, dried (MgSO₄) and evaporated. The residue was treated with isopropyl alcohol and the solid was collected by filtration and dried in vacuum to give the title compound.
LRMS (m/z): 282 (M+1)⁺.
¹H-NMR δ (DMSO-*d*₆): 1.01 (s, 9H), 6.48 (d, 1H), 7.18 (t, 1H), 7.49 - 7.39 (m, 1H), 7.55 (t, 1H), 7.77 (d, 1H), 8.17 (s, 1H), 8.39 (s, 1 H), 10.08 - 9.92 (m, 1 H).

### EXAMPLE 32

### N-(2,2-dimethylpropyl)-2-imidazo[1,2-a]pyridin-3-yl-6-pyridin-3-ylpyrimidin-4-amine

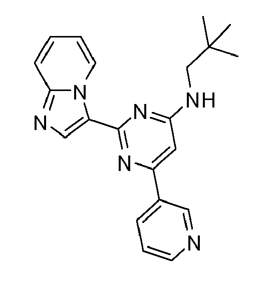

Diisopropylethylamine (0.07 mL, 0.40 mmol) and neopentylamine (0.05 mL, 0.42 mmol) were added to a stirred solution of 3-(4-chloro-6-pyridin-3-ylpyrimidin-2-yl)imidazo[1,2-a]pyridine (preparation 27d, 0.037 g, 0.11 mmol) in *N,N*'-dimethylformamide (1.0 mL) and the mixture was heated to 90 °C under an argon atmosphere. After 16 hours the mixture was evaporated in vacuum and then taken up in dichloromethane. The organic layer was washed with water and brine, dried (MgSO₄) and evaporated to give the title compound (0.027 g, 60%).
LRMS (m/z): 359 (M+1)⁺.
¹H-NMR δ (CDCl₃): 1.07 (s, 9H), 3.29 (s, 2H), 5.25 (s, 1 H), 6.61 (s, 1 H), 7.00 (t, 1 H), 7.38 - 7.29 (m, 1 H), 7.47 (dd, 1 H), 7.74 (d, 1 H), 8.40 - 8.33 (m, 1 H), 8.61 (s, 1 H), 8.73 (dd, 1 H), 9.28 (s, 1 H), 10.04 (d, 1 H).

### EXAMPLE 33

### 3-(6-{[(1 S)-1-phenylethyl]amino}pyrazin-2-yl)imidazo[1,2-a]pyridine-6-carbonitrile

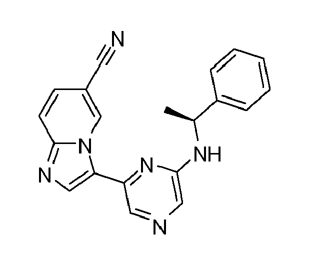

Obtained as a green solid (73%) from 6-chloro-*N*-(1-phenylethyl)pyrazin-2-amine (preparation 32) and imidazo[1,2-a]pyridine-6-carbonitrile (preparation 1) following the same experimental procedure as described in example 26 followed by purification of the crude product by flash chromatography (99:1 to 98:2 dichloromethane/methanol).
LRMS (m/z): 341 (M+1)⁺.
¹H-NMR δ (CDCl₃): 1.69 (d, 3H), 5.04 (t, 1 H), 5.21 (d, 1 H), 7.27 - 7.32 (m, 1 H), 7.34 - 7.51 (m, 5H), 7.75 (d, 1 H), 7.81 (s, 1 H), 8.25 (s, 1 H), 8.33 (s, 1 H), 10.06 (s, 1 H)

### EXAMPLE 34

### 3-{6-[(Cyclohexylmethyl)amino]pyrazin-2-yl}imidazo[1,2-a]pyridine-6-carbonitrile

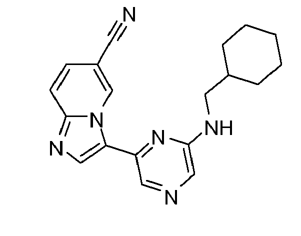

Obtained as a solid (26%) from 3-(6-hydroxypyrazin-2-yl)imidazo[1,2-a]pyridine-6-carbonitrile (preparation 29, 0.052 g, 0.22 mmol) and cyclohexylmethanamine (0.11 mL, 0.88 mmol) following the experimental procedure as described in preparation 5a.
LRMS (m/z): 333 (M+1)⁺.
¹H-NMR δ (DMSO-*d*₆): 0.97 - 1.13 (m, 2H), 1.25 (bs, 4H), 1.74 (bs, 4H), 1.91 (d, 1 H), 3.27 (t, 2H), 7.57 (t, 1 H), 7.69 (d, 1 H), 7.88 - 7.96 (m, 1 H), 8.40 (s, 1 H), 8.61 (s, 1 H), 10.41 (s, 1 H)

### EXAMPLE 35

### 6-(6-Fluoroimidazo[1,2-a]pyridin-3-yl)-N-[(1S)-1-phenylethyl]pyrazin-2-amine

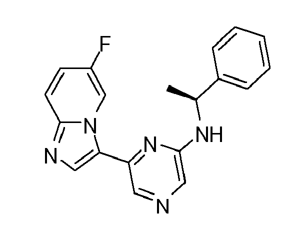

Obtained as a green solid (25%) from (*S*)-6-chloro-*N*-(1-phenylethyl)pyrazin-2-amine (preparation 32) and 6-fluoroimidazo[1,2-a]pyridine (preparation 2) following the experimental procedure as described in example 26 followed by purification of the crude product by reverse phase chromatography (C-18 silica from Waters, water/acetonitrile/methanol as eluents [0.1% v/v formic acid buffered] 0% to 100%).
LRMS (m/z): 334 (M+1)⁺.
¹H-NMR δ (CDCl₃): 1.66 (d, 3H), 5.01 - 5.12 (m, 2H), 7.15 - 7.23 (m, 1 H), 7.33 - 7.46 (m, 5H), 7.63 (dd, 1 H), 7.76 (s, 1 H), 8.15 (s, 1 H), 8.28 (s, 1 H), 9.39 (dd, 1 H)

### EXAMPLE 36

### 6-(6-Chloroimidazo[1,2-a]pyridin-3-yl)-N-[(1 S)-1-phenylethyl]pyrazin-2-amine

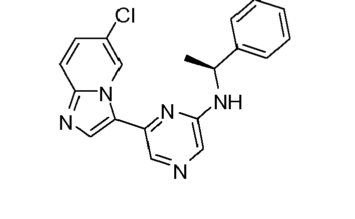

Obtained as a green solid (71%) from (*S*)-6-chloro-*N*-(1-phenylethyl)pyrazin-2-amine (preparation 32) and 6-chloroimidazo[1,2-a]pyridine (preparation 3) following the experimental procedure as described in example 26 followed by purification of the crude product by flash chromatography (99:1 to 97:3 dichloromethane/methanol).
LRMS (m/z): 350 (M+1)⁺.
¹H-NMR δ (CDCl₃): 1.60 (s, 3H), 5.08 (bs, 2H), 7.29 (bs, 2H), 7.37 (t, 2H), 7.41 - 7.50 (m, 1 H), 7.64 (d, 1 H), 7.75 (s, 1 H), 8.16 (s, 1 H), 8.30 (s, 1 H), 9.72 (s, 1 H)

### EXAMPLE 37

### (S)-6-(imidazo[1,2-a]pyridin-3-yl)-N-(1-phenylethyl)pyrazin-2-amine

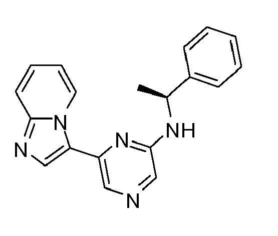

To a solution of 6-(6-chloroimidazo[1,2-*a*]pyridin-3-yl)-*N*-[(1*S*)-1-phenylethyl]pyrazin-2-amine (example 36, 0.100 g, 0.29 mmol) in ethanol (2 mL) was added 10% palladium on charcoal (0.006 g) and ammonium formate (0.180 g, 2.9 mmol). The mixture was stirred and heated to reflux for 24 hours. Further palladium (0.006 g) and ammonium formate (0.180 g) were added and stirring at reflux was continued for 24 hours. This process was repeated until little starting material remained. The reaction mixture was then filtered, evaporated and the residue was purified by reverse phase chromatography (C-18 silica from Waters, water/acetonitrile/methanol as eluents [0.1 % v/v formic acid buffered] 0% to 100%) to give the title compound (0.023 g, 25%) as a pale green solid.
LRMS (m/z): 316 (M+1)⁺.
¹H-NMR δ (CDCl₃): 1.64 (d, 3H), 5.00 (bs, 2H), 6.66 (t, 1 H), 7.28 - 7.34 (m, 0 H), 7.40 (bs, 4H), 7.65 (d, 1 H), 7.78 (s, 1 H), 8.10 (s, 1 H), 8.27 (s, 1 H), 8.95 (d, 1 H)

### EXAMPLE 38

### 6-(6-Cyclopropylimidazo[1,2-a]pyridin-3-yl)-N-[(1S)-1-phenylethyl]pyrazin-2-amine

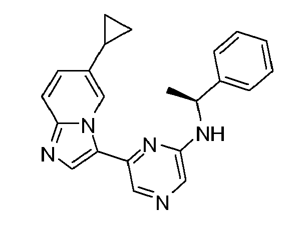

Nitrogen was bubbled for 5 minutes through a mixture of (*S*)-6-(6-chloroimidazo[1,2-a]pyridin-3-yl)-*N*-(1-phenylethyl)pyrazin-2-amine (example 36, 0.085 g, 0.24 mmol), cyclopropylboronic acid (0.081 g, 0.94 mmol) and potassium phosphate (0.180 g, 0.85 mmol) in toluene (1.0 mL) and water (0.1 mL) contained in a microwave vessel. Then palladium (II) acetate (0.004 g, 0.01 mmol) and tricyclohexylphosphine (0.014 mg, 0.05 mmol) were added and the vessel was sealed and subjected to microwave irradiation for 7 hours at 150 °C. The reaction was partitioned between dichloromethane and water and the organic phase was dried (MgSO₄) and the residue was purified by reverse phase chromatography (C-18 silica from Waters, water/acetonitrile/methanol as eluents [0.1% v/v formic acid buffered] 0% to 100%) to give the title compound (0.025 g, 29%) as a pale green solid.
LRMS (m/z): 356 (M+1)⁺.
¹H-NMR δ (CDCl₃): 0.73 (bs, 2H), 0.99 (d, 2H), 1.69 (d, 3H), 1.87 (bs, 1 H), 4.95 (d, 1 H), 5.05 - 5.25 (m, 1 H), 7.00 (d, 1 H), 7.28 - 7.48 (m, 5H), 7.59 (d, 1 H), 7.69 (d, 1 H), 8.10 (d, 1 H), 8.29 (d, 1 H), 9.42 (s, 1 H)

### EXAMPLE 39

### N-[(1S)-1-phenylethyl]-6-(6-pyridin-3-ylimidazo[1,2-a]pyridin-3-yl)pyrazin-2-amine

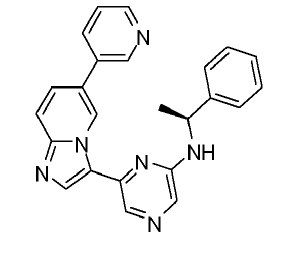

Nitrogen was bubbled for 5 minutes through a mixture of (*S*)-6-(6-chloroimidazo[1,2-a]pyridin-3-yl)-*N*-(1-phenylethyl)pyrazin-2-amine (example 36, 0.080 g, 0.23 mmol), pyridin-3-yl boronic acid (0.056 g, 0.46 mmol) and potassium carbonate (0.063 g, 0.46 mmol) in 1,4-dioxane (1.0 mL) and ethanol (0.5 mL) contained in a microwave vessel. Then tetrakis(triphenylphosphine)palladium (0) (0.026 g, 0.02 mmol) was added and the vessel was sealed and subjected to microwave irradiation for 3 hours at 150 °C. The solvent was removed under reduced pressure and the residue was partitioned between dichloromethane and water. The organic layer was dried (MgSO₄) and evaporated and the residue was purified by flash chromatography (98:2 to 96:4 dichloromethane/methanol) to give the title compound (0.040 g, 45%) as a pale green solid.
LRMS (m/z): 393 (M+1)⁺.
¹H-NMR δ (DMSO-*d*₆): 1.48 (d, 3H), 5.14 (quintet, 1 H), 7.08 - 7.18 (m, 4H), 7.58 (ddd, 1 H), 7.70 - 7.86 (m, 4H), 7.90 (s, 1 H), 8.16 (ddd, 1 H), 8.41 (d, 2H), 8.70 (dd, 1 H), 8.99 (dd, 1 H), 10.01 (dd, 1 H)

### EXAMPLE 40

### N-[(1S)-1-phenylethyl]-6-(6-pyridin-4-ylimidazo[1,2-a]pyridin-3-yl)pyrazin-2-amine

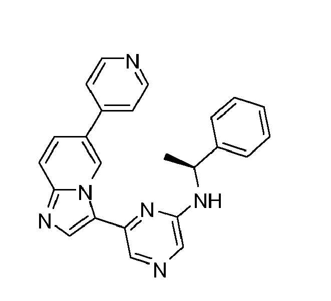

Obtained as a solid (36%) from (*S*)-6-(6-chloroimidazo[1,2-*a*]pyridin-3-yl)-N-(1-phenylethyl)pyrazin-2-amine (example 36) and 4-(4,4,5,5-tetramethyl-1,3,2-dioxaborolan-2-yl)pyridine following the experimental procedure as described in example 39 followed by purification of the crude product by flash chromatography (98:2 to 95:5 dichloromethane/methanol).
LRMS (m/z): 393 (M+1)⁺.
¹H-NMR δ (CDCl₃): 1.65 (d, 3H), 5.00 (d, 1 H), 5.16 (t, 1 H), 7.46 - 7.58 (m, 3H), 7.74 (s, 1 H), 7.82 (d, 1 H), 8.21 (s, 1 H), 8.35 (s, 1 H), 8.69 (d, 2H), 10.00 (s, 1 H)

### EXAMPLE 41

### N-[(1S)-1-phenylethyl]-6-[6-(1H-pyrazol-4-yl)imidazo[1,2-a]pyridin-3-yl]pyrazin-2-amine

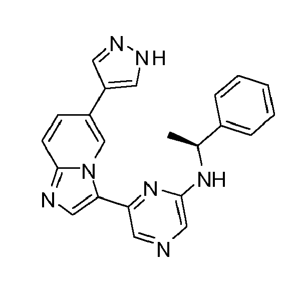

Obtained as a pale green solid (23%) from (S)-6-(6-chloroimidazo[1,2-a]pyridin-3-yl)-N-(1-phenylethyl)pyrazin-2-amine (example 36) and 4-(4,4,5,5-tetramethyl-1,3,2-dioxaborolan-2-yl)-1 H-pyrazole following the experimental procedure as described in example 39 followed by purification of the crude product by flash chromatography (98:2 to 96:4 dichloromethane/methanol).
LRMS (m/z): 382 (M+1)⁺.
¹H-NMR δ (DMSO-*d*₆): 1.61 (bs, 3H), 5.26 (bs, 1H), 7.14 - 7.46 (m, 5H), 7.64 - 8.48 (m, 7H), 9.94 (bs, 1 H)

### EXAMPLE 42

### N-[(1S)-1-phenylethyl]-6-(6-phenylimidazo[1,2-a]pyridin-3-yl)pyrazin-2-amine

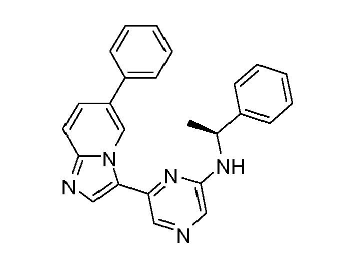

Obtained as a pale green solid (38%) from (S)-6-(6-chloroimidazo[1,2-a]pyridin-3-yl)-N-(1-phenylethyl)pyrazin-2-amine (example 36) and phenylboronic acid following the experimental procedure as described in example 39 followed by purification of the crude product by flash chromatography (98:2 to 97:3 dichloromethane/methanol).
LRMS (m/z): 392 (M+1)⁺.
¹H-NMR δ(CDCl₃): 1.25 (bs, 3H), 4.96 (bs, 1 H), 5.15 (bs, 1 H), 7.35 (bs, 7H), 7.40 - 7.86 (m, 5H), 8.19 (bs, 1 H), 8.34 (bs, 1 H), 8.76 (bs, 1 H), 9.93 (bs, 1 H)

### EXAMPLE 43

### 6-[6-(4-Fluorophenyl)imidazo[1,2-a]pyridin-3-yl]-N-[(1S)-1-phenylethyl]pyrazin-2-amine

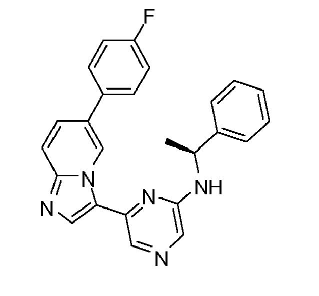

Obtained as a pale green solid (32%) from (S)-6-(6-chloroimidazo[1,2-a]pyridin-3-yl)-N-(1-phenylethyl)pyrazin-2-amine (example 36) and 4-fluorophenylboronic acid following the experimental procedure as described in example 39 followed by purification of the crude product by flash chromatography (98:2 to 97:3 dichloromethane/methanol).
LRMS (m/z): 410 (M+1)⁺.
¹H-NMR δ (CDCl₃): 1.58 (s, 3H), 2.18 (s, 1H), 7.17 (t, 1H), 7.22 - 7.30 (m, 9H), 7.46 - 7.60 (m, 1 H), 7.76 (dd, 1 H), 8.19 (s, 1 H), 8.33 (s, 1 H), 9.86 (dd, 1 H)

### EXAMPLE 44

### 3-(4-{[(3R)-1-(ethylsulfonyl)piperidin-3-yl]amino}pyrimidin-2-yl)imidazo[1,2-a]pyridine-6-carbonitrile

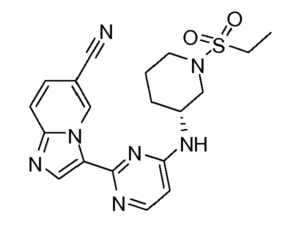

Triethylamine (0.064 mL, 0.46 mmol) and ethanesulfonyl chloride (0.032 mL, 0.34 mmol) were added sequentially to a cooled (ice-bath), stirred solution of 3-{4-[(3*R*)-piperidin-3-ylamino]pyrimidin-2-yl)imidazo[1,2-*a*]pyridine-6-carbonitrile (preparation 5b, 0.072 g, 0.23 mmol) in dichloromethane (3 mL). The mixture was warmed to ambient temperature and stirred for 3 hours. Water was then added and the organic layer was washed with water, brine, dried (MgSO₄) and evaporated. The residue was purified by flash chromatography (98:2 to 96:4 dichloromethane/methanol) to give the title compound (0.052 g, 56%) as a white solid.
LRMS (m/z): 412 (M+1)⁺.
¹H-NMR δ (CDCl₃): 1.47 - 1.27 (m, 4H), 1.94 (s, 4H), 3.08 - 2.87 (m, 3H), 3.49 (s, 3H), 5.33 (s, 1 H), 6.32 (s, 1 H), 7.77 (d, 1 H), 8.27 (s, 1 H), 8.60 (s, 1 H), 10.55 (t, 1H).

### EXAMPLE 45

### 3-(4-{[(3R)-1-(isopropylsulfonyl)piperidin-3-yl]amino}pyrimidin-2-yl)imidazo[1,2-a]pyridine-6-carbonitrile

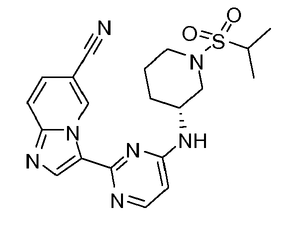

Obtained as a solid (18%) from 3-{4-[(3*R*)-piperidin-3-ylamino]pyrimidin-2-yl}imidazo[1,2-*a*]pyridine-6-carbonitrile (prepraration 5b) and propane-2-sulfonyl chloride following the same experimental procedure described in example 44. The crude product was purified by reverse phase chromatography (C-18 silica from Waters, water/acetonitrile/methanol as eluents [0.1% v/v formic acid buffered] 0% to 100%).
LRMS (m/z): 426 (M+1)⁺.
¹H-NMR δ (CDCl₃): 1.37 (dd, 6H), 1.67 - 2.09 (m, 4H), 2.88 - 2.98 (m, 1 H), 3.22 (dt, 1 H), 3.70 (bs, 4H), 5.41 (bs, 1 H), 6.35 (bs, 1 H), 7.40 (d, 1 H), 7.79 (d, 1 H), 8.28 (bs, 1 H), 8.61 (s, 1 H), 10.55 (s, 1 H)

### EXAMPLE 46

### 3-(4-{[(3R)-1-(cyanoacetyl)piperidin-3-yl]amino}pyrimidin-2-yl)imidazo[1,2-a]pyridine-6-carbonitrile

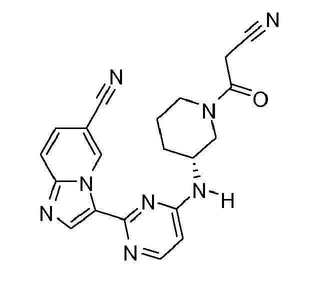

Triethylamine (0.10 mL, 0.72 mmol) and 3-[(2,5-dioxopyrrolidin-1-yl)oxy]-3-oxopropanenitrile (prepared as described in BE875054(A1), 0.148 g, 0.81 mmol) were added sequentially to a stirred solution of 3-{4-[(3*R*)-piperidin-3-ylamino]pyrimidin-2-yl}imidazo[1,2-*a*]pyridine-6-carbonitrile (preparation 5b, 0.200 g, 0.63 mmol) in dichloromethane (5 mL). The mixture was stirred at ambient temperature for 24 hours and then was evaporated. Water was added and the mixture was extracted with dichloromethane. The organic layer was dried (MgSO₄) evaporated and the residue was purified by flash chromatography (98:2 to 95:5 dichloromethane/methanol) to give the title compound (0.200 g, 83%) as an off-white solid.
LRMS (m/z): 387 (M+1)⁺.
¹H-NMR δ (DMSO-*d*₆): 1.67 - 1.49 (m, 2H), 1.89 - 1.70 (m, 2H), 2.11 - 1.98 (m, 2H), 2.51 (s, 2H), 3.66 (t, 1 H), 3.90 (t, 1 H), 4.66 (d, 1 H), 6.44 (d, 1 H), 7.69 (dt, 1 H), 7.90 (ddd, 1 H), 8.23 (s, 1 H), 8.61 (s, 1 H).

### EXAMPLE 47

### 3-(4-{[(3R)-1-propionylpiperidin-3-yl]amino}pyrimidin-2-yl)imidazo[1,2-a]pyridine-6-carbonitrile

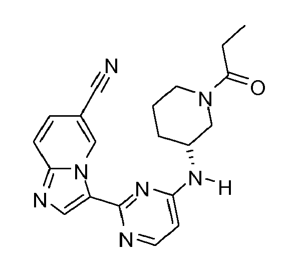

Triethylamine (0.024 mL, 0.17 mmol) and propionyl chloride (0.036 mL, 0.42 mmol) were added sequentially to a stirred solution of 3-{4-[(3*R*)-piperidin-3-ylamino]pyrimidin-2-yl}imidazo[1,2-*a*]pyridine-6-carbonitrile (preparation 5b, 0.050 g, 0.16 mmol) in dichloromethane (2 mL). The mixture was stirred at ambient temperature for 16 hours then evaporated in vacuum and the residue was taken up in dichloromethane. The organic layer was washed with water and brine, dried (MgSO₄) and evaporated. The residue was taken up in 2M aqueous hydrochloric acid and washed with ethyl acetate. The aqueous layer was neutralized with 2M aqueous sodium hydroxide solution and extracted with dichloromethane. The organic layer was dried (MgSO₄) and evaporated to give the title compound (0.032 g, 54%) as a white solid.
LRMS (m/z): 376 (M+1)⁺.
¹H-NMR δ (DMSO-*d*₆): 4.74 (d, 1 H), 6.47 (d, 1 H), 7.72 (s, 2H), 7.91 (d, 1 H), 8.24 (s, 1H), 8.57 (d, 1 H), 10.47 (s, 1 H).

### EXAMPLE 48

### 3-[4-({(3R)-1-[(1-cyanocyclopropyl)carbonyl]piperidin-3-yl}amino)pyrimidin-2-yl]imidazo[1,2-a]pyridine-6-carbonitrile

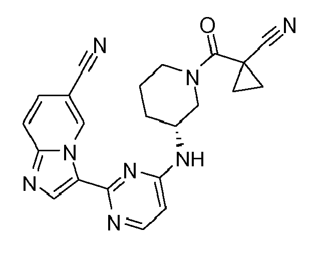

1,1'-carbonyldiimidazole (0.040 g, 0.25 mmol) was added to a stirred solution of 1-cyanocyclopropanecarboxylic acid (0.041 g, 0.37 mmol) in dichloroethane (1 mL). After stirring 15 minutes at ambient temperature, 3-{4-[(3*R*)-piperidin-3-ylamino]pyrimidin-2-yl}imidazo[1,2-*a*]pyridine-6-carbonitrile (preparation 5b, 0.040 g, 0.13 mmol) was added and the mixture was stirred overnight. The mixture was evaporated and the residue was purified by flash chromatography (98:2 to 95:5 dichloromethane/methanol) to give the title compound (0.035 g, 68%) as a white solid.
LRMS (m/z): 413 (M+1)⁺.
¹H-NMR δ (CDCl₃): 2.04 - 1.44 (m, 8H), 2.20 (s, 1H), 3.73 - 3.50 (m, 1H), 4.44 - 3.88 (m, 2H), 5.08 (bs, 1 H), 6.35 (bs, 1 H), 7.42 (dd, 1 H), 7.79 (dd, 1 H), 8.32 (d, 1 H), 8.64 (s, 1 H), 10.54 (s, 1 H).

### EXAMPLE 49

### 3-(4-{[(3R)-1-(methoxyacetyl)piperidin-3-yl]amino}pyrimidin-2-yl)imidazo[1,2-a]pyridine-6-carbonitrile

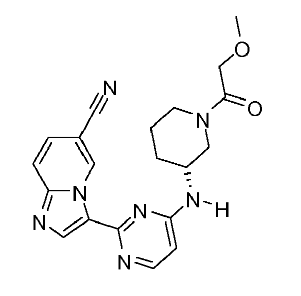

Obtained as an off white solid (57%) from 3-{4-[(3*R*)-piperidin-3-ylamino]pyrimidin-2-yl}imidazo[1,2-*a*]pyridine-6-carbonitrile (preparation 5b) and 2-methoxyacetic acid following the experimental procedure as described in example 48. The crude product was purified by reverse phase chromatography (C-18 silica from Waters, water/acetonitrile/methanol as eluents [0.1% v/v formic acid buffered] 0% to 100%).
LRMS (m/z): 392 (M+1)⁺.
¹H-NMR δ (CDCl₃): 1.57 (s, 3H), 2.19 - 1.60 (m, 4H), 2.92 (d, 1 H), 3.43 (s, 2H), 4.14 (d, 2H), 5.54 - 4.96 (m, 1 H), 6.30 (s, 1 H), 7.39 (d, 1 H), 7.77 (d, 1 H), 8.28 (d, 1 H), 8.59 (s, 1 H), 10.52 (s, 1 H).

### EXAMPLE 50

### 3-(4-{[(3R)-1-(3-hydroxy-3-methylbutanoyl)piperidin-3-yl]amino}pyrimidin-2-yl)imidazo[1,2-a]pyridine-6-carbonitrile

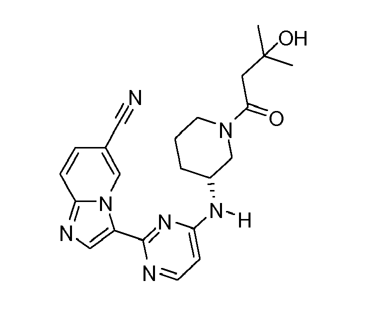

Obtained as an off white solid (23%) from 3-{4-[(3*R*)-piperidin-3-ylamino]pyrimidin-2-yl}imidazo[1,2-*a*]pyridine-6-carbonitrile (preparation 5b) and 3-hydroxy-3-methyl butanoic acid following the experimental procedure as described in example 48. The crude product was purified by reverse phase chromatography (C-18 silica from Waters, water/acetonitrile/methanol as eluents [0.1 % v/v formic acid buffered] 0% to 100%).
LRMS (m/z): 420 (M+1)⁺.
¹H-NMR δ (CDCl₃): 1.33 (d, 6H), 1.65 - 1.97 (m, 4H), 2.14 (bs, 1 H), 2.55 (s, 2H), 3.31 - 3.96 (m, 2H), 5.04 - 5.18 (m, 2H), 6.29 (d, 1 H), 7.40 (d, 1 H), 7.80 (t, 1 H), 8.30 (d, 1 H), 8.56 (s, 1 H), 8.62 (s, 1 H), 10.54 (s, 1 H)

### EXAMPLE 51

### 3-(4-{[(3R)-1-(3,3-dimethylbutanoyl)piperidin-3-yl]amino}pyrimidin-2-yl)imidazo [1,2-a]pyridine-6-carbonitrile

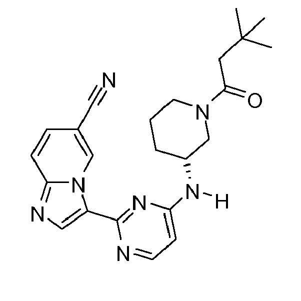

Obtained as an off white solid from 3-{4-[(3*R*)-piperidin-3-ylamino]pyrimidin-2-yl}imidazo[1,2-*a*]pyridine-6-carbonitrile (preparation 5b) and 3,3-dimethylbutanoic acid following the experimental procedure as described in example 48. The crude product was purified by reverse phase chromatography (C-18 silica from Waters, water/acetonitrile/methanol as eluents [0.1% v/v formic acid buffered] 0% to 100%).
LRMS (m/z): 418 (M+1)⁺.
¹H-NMR δ (CDCl₃): 0.99 (s, 9H), 1.71 (bs, 2H), 1.84 (bs, 2H), 2.13 (bs, 2H), 3.38 (bs, 1 H), 3.65 (bs, 2H), 3.96 (bs, 2H), 5.14 - 5.24 (m, 1 H), 6.26 (d, 1 H), 6.29 - 6.40 (m, 1 H), 7.41 (t, 1 H), 7.79 (s, 1 H), 8.23 - 8.35 (m, 1 H), 8.61 (s, 1 H)

### EXAMPLE 52

### 3-(4-{[(3R)-1-(1H-imidazol-4-ylacetyl)piperidin-3-yl]amino}pyrimidin-2-yl)imidazo [1,2-a]pyridine-6-carbonitrile

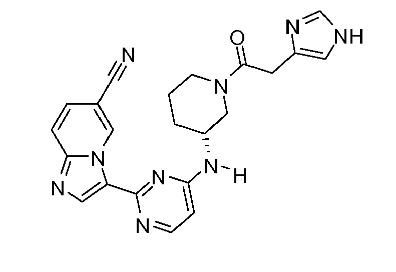

Triethylamine (0.15 mL, 1.1 mmol) and 1,1'-carbonyldiimidazole (0.228 g, 1.4 mmol) were added to a stirred solution of 2-(1 H-imidazol-4-yl)acetic acid (0.120 g, 0.95 mmol) in *N,N'*-dimethylformamide (2mL). After stirring for 20 minutes at ambient temperature a solution of (*R*)-3-(4-(piperidin-3-ylamino)pyrimidin-2-yl)imidazo[1,2-a]pyridine-6-carbonitrile (preparation 5b, 0.150 g, 0.47 mmol) in *N,N'*-dimethylformamide (2mL) was added and the reaction mixture was stirred overnight. The solvent was removed under reduced pressure and the residue was purified by reverse phase chromatography (C-18 silica from Waters, water/acetonitrile/methanol as eluents [0.1 % v/v formic acid buffered] 0% to 100%) to give the title compound (0.064 g, 32%) as a solid.
LRMS (m/z): 428 (M+1)⁺.
¹H-NMR δ (DMSO-*d*₆): 1.55 (bs, 1H), 1.73 (bs, 1H), 2.02 (bs, 2H), 2.52 - 2.66 (m, 1 H), 3.02 - 3.51 (m, 2H), 3.61 (d, 2H), 3.95 (d, 1 H), 4.67 (d, 1 H), 6.38 - 6.55 (m, 1 H), 6.87 (bs, 1H), 7.55 (s, 1 H), 7.69 (d, 1H), 7.90 (d, 1 H), 8.21 (s, 1H), 8.51 (s, 1 H), 8.63 (bs, 1 H), 10.45 (bs, 1 H)

### EXAMPLE 53

### 3-[4-({(3R)-1-[(5-cyanopyridin-2-yl)carbonyl]piperidin-3-yl}amino)pyrimidin-2-yl] imidazo[1,2-a]pyridine-6-carbonitrile

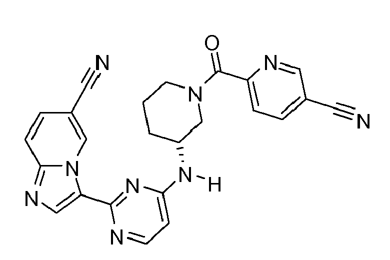

Obtained as an off white solid (23%) from 3-{4-[(3)-piperidin-3-ylamino]pyrimidin-2-yl}imidazo[1,2-*a*]pyridine-6-carbonitrile (preparation 5b) and 5-cyanopyridine-2-carboxylic acid following the experimental procedure as described in example 48 with the exception that *N,N'*-dimethylformamide was used as the reaction solvent. The crude product was purified by reverse phase chromatography (C-18 silica from Waters, water/acetonitrile/methanol as eluents [0.1% v/v formic acid buffered] 0% to 100%).
LRMS (m/z): 450 (M+1)⁺.
¹H-NMR δ (DMSO-*d*₆): 1.93 (m, 2H), 3.05 - 2.77 (m, 1H), 3.57 (d, 1H), 4.23 - 3.71 (m, 2H), 5.82 (s, 1 H), 6.51 (d, 1 H), 7.61 - 7.36 (m, 2H), 7.86 (dd, 2H), 8.60 - 8.07 (m, 2H), 10.51 (d, 1 H).

### EXAMPLE 54

### 3-(4-{[(3R)-1-(3,3,3-trifluoropropanoyl)piperidin-3-yl]amino}pyrimidin-2-yl)imidazo [1,2-a]pyridine-6-carbonitrile

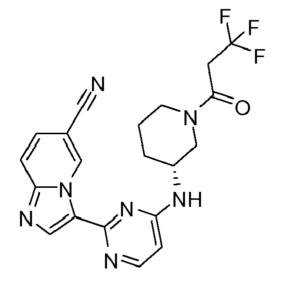

*N*-[(dimethylamino)(3*H*-[1,2,3]triazolo[4,5-*b*]pyridin-3-yloxy)methylene]-*N*-methyl-methanaminium hexafluorophosphate (0.060 g, 0.16 mmol) and diisopropylethylamine (0.028 mL, 0.16 mmol) were added to a stirred solution of 3,3,3-trifluoropropanoic acid (0.015 mL, 0.17 mmol) in *N,N'*-dimethylformamide (2 mL). The mixture was stirred for 10 minutes at ambient temperature and then 3-{4-[(3*R*)-piperidin-3-ylamino]pyrimidin-2-yl}imidazo[1,2-*a*]pyridine-6-carbonitrile (preparation 5b, 0.050 g, 0.16 mmol) was added. The mixture was stirred overnight and then was partitioned between water and dichloromethane. The organic layer was washed with water, brine, dried (MgSO₄) and evaporated and the residue was purified by flash chromatography (98:2 to 96:4 dichloromethane/methanol) to give the title compound (0.040 g, 60%) as a white solid.
LRMS (m/z): 430 (M+1)⁺.
¹H-NMR δ (CDCl₃): 1.47 - 1.07 (m, 2H), 1.82 (d, 2H), 2.16 (s, 1 H), 3.38 (dt, 2H), 3.93 - 3.54 (m, 1 H), 5.00 (d, 1 H), 6.30 (s, 1 H), 7.40 (d, 1 H), 7.77 (d, 1 H), 8.30 (s, 1 H), 8.58 (d, 1 H), 10.52 (s, 1 H).

### EXAMPLE 55

### (3R)-3-{[2-(6-cyanoimidazo[1,2-a]pyridin-3-yl)pyrimidin-4-yl]amino}-N,N-dimethyl piperidine-1-carboxamide

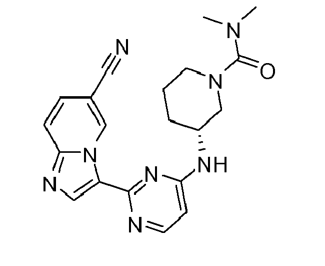

Triethylamine (0.019 mL, 0.14 mmol) and dimethylcarbamic chloride (0.017 mL, 0.18 mmol) were added to a stirred solution of 3-{4-[(3*R*)-piperidin-3-ylamino]pyrimidin-2-yl}imidazo[1,2-*a*]pyridine-6-carbonitrile (preparation 5b, 0.040 g, 0.13 mmol) in dichloromethane (1 mL). The mixture was stirred at ambient temperature for 2 hours then the mixture was diluted with dichloromethane and washed with water, brine, dried (MgSO₄) and evaporated. The solid was dissolved in 2M aqueous hydrochloric acid and washed with ethyl acetate. The aqueous layer was basified with 2M aqueous sodium hydroxide solution and the product was extracted into dichloromethane. The organic layer was dried (MgSO₄) and evaporated to obtain the title compound (0.032 g, 65%) as a pale yellow solid.
LRMS (m/z): 391 (M+1)⁺.
¹H-NMR δ (CDCl₃): 1.61 (bs, 1H), 1.85 (d, 1H), 2.89 (s, 6H), 3.31 (bs, 4H), 6.34 (bs, 1 H), 7.43 - 7.32 (m, 1 H), 7.77 (d, 1 H), 8.20 (s, 1 H), 8.58 (s, 1 H), 10.59 (s, 1 H).

### EXAMPLE 56

### 3-{4-[((3R)-1-{[(2S,4S)-2-cyano-4-fluoropyrrolidin-1-yl]carbonyl}piperidin-3-yl) amino]pyrimidin-2-yl}imidazo[1,2-a]pyridine-6-carbonitrile

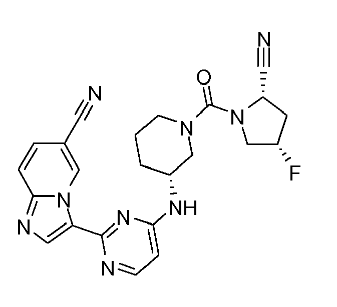

A solution of 1-((2*S*,4*S*)-2-cyano-4-fluoropyrrolidine-1-carbonyl)-3-methyl-1*H*-imidazol-3-ium iodide (preparation 36, 0.100 g, 0.29 mmol), (R)-3-(4-(piperidin-3-ylamino)pyrimidin-2-yl)imidazo[1,2-a]pyridine-6-carbonitrile (preparation 5b, 94 mg, 0.29 mmol) and triethylamine (0.075 mL, 0.54 mmol) in a mixture of dichloromethane (2 mL) and *N,N'*-dimethylformamide (1 mL) was stirred at ambient temperature overnight. The solvent was removed under reduced pressure and the residue was purified by reverse phase chromatography (C-18 silica from Waters, water/acetonitrile/methanol as eluents [0.1% v/v formic acid buffered] 0% to 100%) to obtain the title compound (0.022 g, 17%) as a solid.
LRMS (m/z): 460 (M+1)⁺.
¹H-NMR δ (CDCl₃): 1.62 - 2.13 (m, 4H), 2.24 - 2.44 (m, 1 H), 2.56 (bs, 1 H), 3.29 (bs, 1 H), 3.55 (bs, 1 H), 3.66 - 3.95 (m, 3H), 5.09 (bs, 1 H), 5.21 - 5.43 (m, 2H), 6.36 (bs, 1 H), 7.40 (d, 1 H), 7.76 (d, 1 H), 8.25 (bs, 1 H), 8.56 (s, 1 H), 10.53 (s, 1 H)

### EXAMPLE 57

### 3-(4-{[(3R)-1-(5-cyanopyridin-2-yl)piperidin-3-yl]amino}pyrimidin-2-yl)imidazo[1,2-a]pyridine-6-carbonitrile

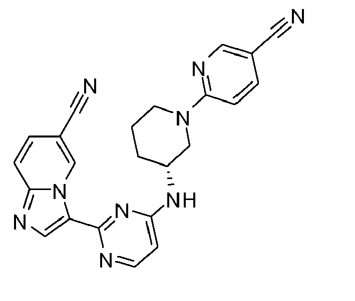

Triethylamine (0.020 mL, 0.14 mmol) and 6-chloronicotinonitrile (0.050 g, 0.37 mmol) were added to a stirred solution of 3-{4-[(3*R*)-piperidin-3-ylamino]pyrimidin-2-yl}imidazo[1,2-*a*]pyridine-6-carbonitrile (preparation 5b, 0.045 g, 0.14 mmol) in dichloromethane (2mL). The mixture was heated to 50 °C in a sealed tube and stirred overnight. The mixture was partitioned between dichloromethane and water and the organic layer was washed with water, brine, dried (MgSO₄) and evaporated. The residue was purified by flash chromatography (98:2 dichloromethane/methanol) to give a solid that was dissolved in 2M aqueous hydrochloric acid (2.0 mL) and washed with ethyl acetate. The aqueous layer was basified with 2M aqueous sodium hydroxide solution and the product was extracted with dichloromethane. The organic layer was dried (MgSO₄) and evaporated to obtain the title compound (0.018 g, 30%) as a white solid.
LRMS (m/z): 422 (M+1)⁺.
¹H-NMR δ (CDCl₃): 2.26 - 1.65 (m, 4H), 3.48 - 3.27 (m, 2H), 3.99 (dt, 2H), 4.51 (d, 1 H), 5.12 (s, 1 H), 6.31 (bs, 1 H), 6.69 (d, 1H), 7.41 (dd, 1 H), 7.63 (dd, 1 H), 7.81 (d, 1 H), 8.30 (d, 1 H), 8.41 (d, 1 H), 8.62 (s, 1 H), 10.54 (s, 1 H).

### EXAMPLE 58

### 3-(4-{[(3R)-1-(4-cyano-2-fluorophenyl)piperidin-3-yl]amino}pyrimidin-2-yl)imidazo [1,2-a]pyridine-6-carbonitrile

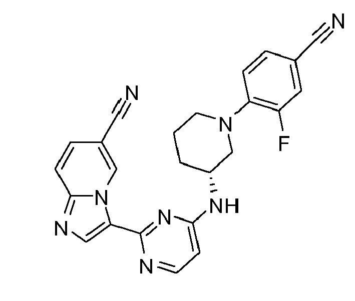

3,4-Difluorobenzonitrile (0.022 g, 0.16 mmol) and potassium carbonate (0.025 g, 0.18 mmol) were added to a stirred solution of 3-{4-[(3*R*)-piperidin-3-yiamino]pyrimidin-2-yl}imidazo[1,2-*a*]pyridine-6-carbonitrile (preparation 5b, 0.040 g, 0.13 mmol) in *N,N'-*dimethylformamide (1 mL). The mixture was heated to 80 °C in a sealed tube and stirred overnight. The mixture was evaporated and the residue was purified by flash chromatography (98:2 dichloromethane/methanol) and the solid obtained was triturated with diethyl ether to give the title compound (0.020 g, 36%) as a white solid.
LRMS (m/z): 439 (M+1)⁺.
¹H-NMR δ (CDCl₃) 2.06 - 1.79 (m, 4H), 3.21 (bs, 3H), 3.56 - 3.45 (m, 2H), 5.38 (s, 1 H), 6.31 (d, 1 H), 7.01 (t, 3H), 7.34 - 7.28 (m, 1 H), 7.44 - 7.36 (m, 2H), 7.79 (d, 1 H), 8.29 (d, 1 H), 8.60 (s, 1 H), 10.56 (s, 1 H).

### EXAMPLE 59

### 3-{4-[[(3R)-1-(cyanoacetyl)piperidin-3-yl](methyl)amino]pyrimidin-2-yl}imidazo [1,2-a]pyridine-6-carbonitrile

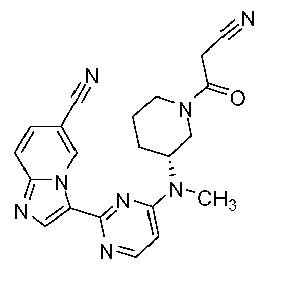

3-[(2,5-Dioxopyrrolidin-1-yl)oxy]-3-oxopropanenitrile (prepared as described in BE875054(A1), 0.020 g, 0.11 mmol) was added to a stirred solution of (R)-3-(4-(methyl(piperidin-3-yl)amino)pyrimidin-2-yl)imidazo[1,2-a]pyridine-6-carbonitrile (preparation 6b, 0.030 g, 0.09 mmol) in ethanol (2 mL). The mixture was stirred at ambient temperature for 48 hours and then was evaporated. Water was added and the mixture was extracted with dichloromethane. The organic layer was dried (MgSO₄) evaporated and the residue was purified by reverse phase chromatography (C-18 silica from Waters, water/acetonitrile/methanol as eluents [0.1% v/v formic acid buffered] 0% to 100%) to give the title compound (0.013 g, 36%) as a solid.
LRMS (m/z): 401 (M+1)⁺.
¹H-NMR δ (CDCl₃) 1.90 (bs, 2H), 2.07 (bs, 2H), 2.73 (bs, 1 H), 3.05 (bs, 3H), 3.11 - 3.24 (m, 1 H), 3.68 (bs, 2H), 3.78 (bs, 1 H), 4.74 (bs, 2H), 6.38 (bs, 1 H), 7.39 (bs, 1 H), 7.78 (bs, 1 H), 8.35 (bs, 1 H), 8.64 (bs, 1 H), 10.43 (bs, 1 H)

### EXAMPLE 60

### 3-(4-{[(3S)-1-(cyanoacetyl)piperidin-3-yl]amino}pyrimidin-2-yl)imidazo[1,2-a] pyridine-6-carbonitrile

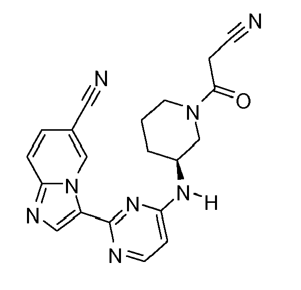

Obtained as a off white solid (62%) from 3-{4-[(3*S*)-piperidin-3-ylamino]pyrimidin-2-yl}imidazo[1,2-*a*]pyridine-6-carbonitrile (preparation 7b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (97:3 to 96:4 dichloromethane/methanol).
LRMS (m/z): 387 (M+1)⁺.
¹H-NMR δ (DMSO-*d*₆): 4.11 (s, 2H), 4.72 (d, 1 H), 6.49 (d, 1 H), 7.75 (d, 2H), 7.96 (d, 1 H), 8.29 (s, 1 H), 8.66 (s, 1 H), 10.50 (s, 1 H).

### EXAMPLE 61

### Cis-3-(4-{1-(cyanoacetyl)-4-methylpiperidin-3-yl]amino}pyrimidin-2-yl)imidazo [1,2-a]pyridine-6-carbonitrile

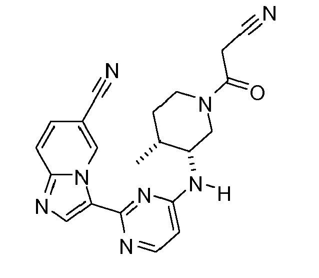

Obtained as a white solid (26%) from 3-(4-hydroxypyrimidin-2-yl)imidazo[1,2-a]pyridine-6-carbonitrile (preparation 4b) and *cis*-3-(3-amino-4-methylpiperidin-1-yl)-3-oxopropanenitrile (preparation 34d) following the experimental procedure as described in preparation 5a followed by heating the reaction to 40 °C for 48 hours. After the reaction was complete the mixture was partitioned between water and ethyl acetate. The organic layer was washed with water, saturated aqueous sodium hydrogen carbonate solution, brine, dried (MgSO₄) and evaporated and the residue was purified by reverse phase chromatography (C-18 silica from Waters, water/acetonitrile/ methanol as eluents [0.1 % v/v formic acid buffered] 0% to 100%).
LRMS (m/z): 401 (M+1)⁺.
¹H-NMR δ (CDCl₃):1.07 (t, 3H), 2.26 - 2.11 (m, 1 H), 3.31 (d, 2H), 3.58 (d, 1 H), 4.01 (d, 1 H), 4.43 (d, 1 H), 4.63 (d, 1 H), 5.06 - 4.93 (m, 1 H), 6.35 (dd, 1 H), 7.49 - 7.35 (m, 1 H), 7.80 (dd, 1 H), 8.29 (dd, 1 H), 8.57 (s, 1 H), 10.56 (d, 1 H).

### EXAMPLE 62

### Cis-3-(4-{1-(ethylsulfonyl)-4-methylpiperidin-3-yl]amino}pyrimidin-2-yl)imidazo [1,2-a]pyridine-6-carbonitrile

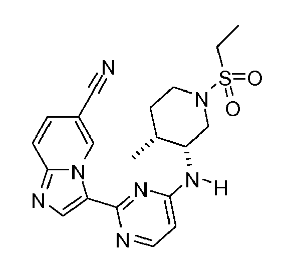

Obtained as a pale yellow solid (44%) from 3-(4-hydroxypyrimidin-2-yl)imidazo[1,2-a]pyridine-6-carbonitrile (preparation 4b) and *cis*-1-(ethylsulfonyl)-4-methylpiperidin-3-amine (preparation 35b) following the experimental procedure as described in preparation 5a followed by heating the reaction to 45 °C for 18 hours. After the reaction was complete the mixture was partitioned between water and ethyl acetate. The organic layer was washed with water, saturated aqueous sodium hydrogen carbonate solution, brine, dried (MgSO₄) and evaporated and the residue was purified by flash chromatography (98:2 dichloromethane/methanol).
LRMS (m/z): 426 (M+1)⁺.
¹H-NMR δ (CDCl₃): 1.04 (d, 3H), 1.38 (t, 3H), 1.75 - 1.52 (bs, 2H), 2.08 - 1.83 (m, 2H), 3.17 - 2.81 (m, 4H), 3.89 (d, 2H), 4.61 (s, 1 H), 5.48 (d, 1 H), 6.35 (d, 1 H), 7.40 (dd, 1 H), 7.77 (d, 1 H), 8.22 (d, 1 H), 8.55 (s, 1 H), 10.54 (s, 1 H).

### EXAMPLE 63

### 3-{4-[[1-(Ethylsulfonyl)-4-methylpiperidin-3-yl](methyl)amino]pyrimidin-2-yl} imidazo[1,2-a]pyridine-6-carbonitrile

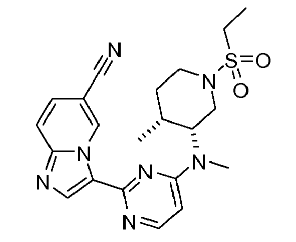

Obtained as a pale yellow solid (31%) from 3-(4-hydroxypyrimidin-2-yl)imidazo[1,2-a]pyridine-6-carbonitrile (preparation 4b) and 1-(ethylsulfonyl)-N,3-dimethylpiperidin-4-amine (preparation 33d) following the experimental procedure as described in preparation 5a followed by heating the reaction to 50 °C for 96 hours. The crude product was purified by flash chromatography (97:3 dichloromethane/methanol) and triturated with methanol to give the pure title compound.
LRMS (m/z): 440 (M+1)⁺.
¹H-NMR δ (DMSO-*d*₆): 1.03 (d, 3H), 1.24 (bs, 3H), 1.72 (bs, 2H), 1.91 - 2.05 (m,1 H), 2.51 (bs, 3H), 3.10 - 3.21 (m, 1 H), 3.23 - 3.36 (m, 4H), 3.40 - 3.40 (m, 2H), 7.86 - 7.93 (m, 1 H), 8.00 - 8.07 (m, 1 H), 8.41 (d, 1 H), 8.85 (bs, 1 H), 10.19 (bs, 1 H)

### EXAMPLE 64

### 3-((3R)-3-{[2-(6-fluoroimidazo[1,2-a]pyridin-3-yl)pyrimidin-4-yl]amino}piperidin-1-yl)-3-oxopropanenitrile

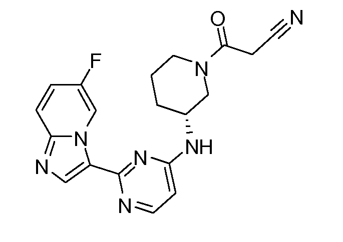

Obtained as an off white solid (98%) from 2-(6-fluoroimidazo[1,2-a]pyridin-3-yl)-N-(piperidin-3-yl)pyrimidin-4-amine (preparation 9b) and 3-[(2,5-dioxopyrrolidin-1-yl)oxy]-3-oxopropanenitrile (prepared as described in BE875054(A1)) following the experimental procedure as described in example 46. The crude product was purified by reverse phase chromatography (C-18 silica from Waters, water/acetonitrile/methanol as eluents [0.1 % v/v formic acid buffered] 0% to 100%).
LRMS (m/z): 380 (M+1)⁺.
¹H-NMR δ (CDCl₃): 1.80 (bs, 3H), 1.97 (bs, 1 H), 2.16 (bs, 2H), 3.57 (s, 2H), 3.67 (bs, 2H), 5.00 (s, 1 H), 6.28 (dd, 1 H), 7.30 - 7.19 (m, 1 H), 7.69 (td, 1 H), 8.29 (t, 1 H), 8.48 (s, 1 H), 8.55 (s, 1 H), 9.96 (ddd, 1 H).

### EXAMPLE 65

### (R)-1-(3-(2-(6-fluoroimidazo[1,2-a]pyridin-3-yl)pyrimidin-4-ylamino)piperidine-1-carbonyl)cyclopropanecarbonitrile

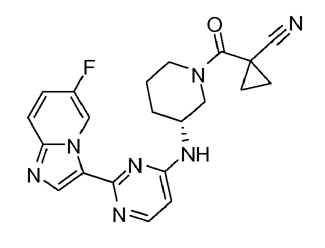

Obtained as a white solid (76%) from (R)-2-(6-fluoroimidazo[1,2-a]pyridin-3-yl)-N-(piperidin-3-yl)pyrimidin-4-amine (preparation 9b) and 1-cyanocyclopropanecarboxylic acid following the experimental procedure as described in example 48. The crude product was purified by flash chromatography (100:8 dichloromethane/methanol).
LRMS (m/z): 406 (M+1)⁺.
¹H-NMR δ (CDCl₃): 1.56 (s, 3H), 1.90 (dd, 2H), 2.19 (d, 2H), 4.34 - 4.16 (m, 2H), 4.96 (s, 1 H), 6.28 (s, 1 H), 7.23 (dd, 1 H), 7.68 (dd, 1 H), 8.29 (d, 1 H), 8.55 (s, 1H), 9.96 (d,1H).

### EXAMPLE 66

### 2-(6-Fluoroimidazo[1,2-a]pyridin-3-yl)-N-[(3R)-1-(3,3,3-trifluoropropanoyl)piperidin -3-yl]pyrimidin-4-amine

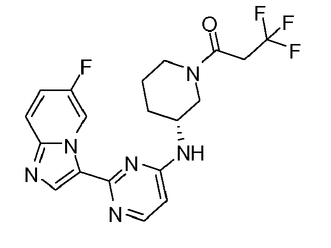

Obtained as a white solid (59%) from (R)-2-(6-fluoroimidazo[1,2-a]pyridin-3-yl)-N-(piperidin-3-yl)pyrimidin-4-amine (preparation 9b) and 3,3,3-trifluoropropanoic acid following the experimental procedure as described in example 54. The crude product was purified by reverse phase chromatography (C-18 silica from Waters, water/acetonitrile/methanol as eluents [0.1% v/v formic acid buffered] 0% to 100%).
LRMS (m/z): 423 (M+1)⁺.
¹H-NMR δ (CDCl₃): 1.25 (s, 2H), 1.73 (bs, 2H), 1.90 (bs, 2H), 2.15 (bs, 2H), 3.42 (bs, 2H), 6.22 (d, 1 H), 6.27 (d, 1 H), 7.25 - 7.19 (m, 1 H), 7.68 (td, 1 H), 8.29 (t, 1 H), 8.49 (s, 1 H), 8.53 (s, 1 H), 9.97 (s, 1 H).

### EXAMPLE 67

### 2-(6-Fluoroimidazo[1,2-a]pyridin-3-yl)-N-[(3R)-1-(1H-pyrazol-4ylcarbonyl)piperidin -3-yl]pyrimidin-4-amine

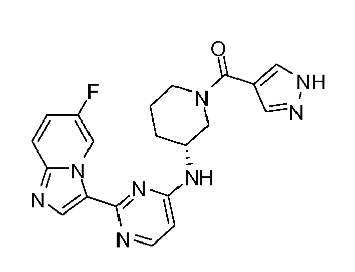

Obtained as a solid (33%) from (R)-2-(6-fluoroimidazo[1,2-a]pyridin-3-yl)-N-(piperidin-3-yl)pyrimidin-4-amine (preparation 9b) and 1*H*-pyrazole-4-carboxylic acid following the experimental procedure as described in example 52. The crude product was purified by reverse phase chromatography (C-18 silica from Waters, water/acetonitrile/methanol as eluents [0.1 % v/v formic acid buffered] 0% to 100%).
LRMS (m/z): 407 (M+1)⁺.
¹H-NMR δ (CDCl₃): 2.65 (d, 4H), 3.21 - 3.24 (m, 2H), 3.50 (d, 2H), 3.79 (d, 2H), 6.27 (d, 1 H), 7.00 (bs, 1 H), 7.23 - 7.29 (m, 1 H), 7.71 (dd, 1 H), 7.91 (s, 1 H), 8.28 (d, 1 H), 8.47 (s, 1 H), 9.97 (dd, 1 H)

### EXAMPLE 68

### (3R)-N-(cyanomethyl)-3-{[2-(6-fluoroimidazo[1,2-a]pyridin-3-yl)pyrimidin-4-yl] amino}-N-methylpiperidine-1-carboxamide

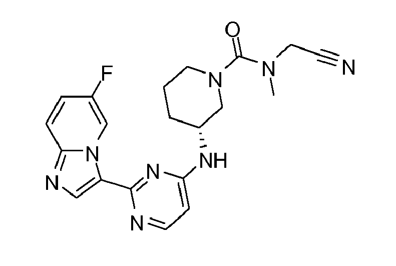

Obtained as a solid (33%) from (*R*)-2-(6-fluoroimidazo[1,2-*a*]pyridin-3-yl)-N-(piperidin-3-yl)pyrimidin-4-amine (preparation 9b) and 1-((cyanomethyl)(methyl)carbamoyl)-3-methyl-1H-imidazol-3-ium iodide (preparation 37b) following the experimental procedure as described in example 56. The crude product was purified by reverse phase chromatography (C-18 silica from Waters, water/acetonitrile/methanol as eluents [0.1 % v/v formic acid buffered] 0% to 100%).
LRMS (m/z): 409 (M+1)⁺.
¹H-NMR δ (CDCl₃): 1.67 (bs, 1 H), 1.83 (bs, 1 H), 2.01 (bs, 1 H), 2.26 (bs, 2H), 3.00 (s, 3H), 3.30 (bs, 1 H), 3.44 (bs, 2H), 3.66 (bs, 1 H), 3.96 - 4.17 (m, 2H),

5.75 (bs, 1 H), 6.28 (bs, 1 H), 7.18 - 7.32 (m, 1 H), 7.71 (dd, 1 H), 8.26 (bs, 1 H), 8.50 (s, 1 H), 10.00 (dd, 1 H)

### EXAMPLE 69

### 2-((3R)-3-{[2-(6-fluoroimidazo[1,2-a]pyridin-3-yl)pyrimidin-4-yl]amino}piperidin-1-yl)-2-methylpropanamide

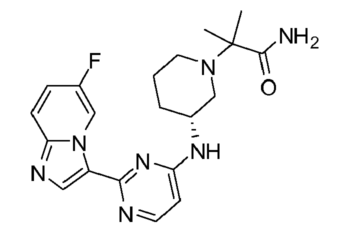

Concentrated aqueous ammonium hydroxide solution (0.01 mL) was added to a stirred solution of (*R*)-2,5-dioxopyrrolidin-1-yl 2-(3-(2-(6-fluoroimidazo[1,2-a]pyridin-3-yl) pyrimidin-4-ylamino)piperidin-1-yl)-2-methylpropanoate (preparation 10c) in 1,4-dioxane (1 mL) and the mixture was stirred at ambient temperature overnight. The reaction mixture was diluted with water and acidified with 2M aqueous hydrochloric acid and extracted with dichloromethane. The organic phase was washed with 4% aqueous sodium hydrogen carbonate solution and brine. The aqueous phase was extracted with further dichloromethane and the combined organic phase was dried (MgSO₄), evaporated and the residue was purified by reverse phase chromatography (C-18 silica from Waters, water/acetonitrile/methanol as eluents [0.1% v/v formic acid buffered] 0% to 100%) to obtain the title compound (0.009 g, 33%) as a solid.
LRMS (m/z): 398 (M+1)⁺.
¹H-NMR δ (CDCl₃): 1.15 (d, 6H), 1.90 (bs, 4H), 2.01 - 2.14 (m, 2H), 2.28 - 2.45 (m, 2H), 2.74 (bs, 1 H), 3.76 - 3.87 (m, 2H), 5.53 (bs, 1 H), 6.20 (d, 1 H), 7.27 (dd, 1 H), 7.72 (dd, 1 H), 8.26 (bs, 1 H), 8.52 (s, 1 H), 10.01 (dd, 1 H)

### EXAMPLE 70

### 2-(6-Fluoroimidazo[1,2-a]pyridin-3-yl)-N-methyl-N-[(3R)-1-(3,3,3-trifluoro propanoyl)piperidin-3-yl]pyrimidin-4-amine

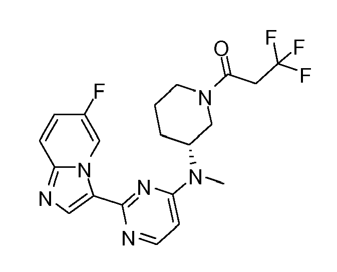

Obtained as a white solid (83%) from (R)-2-(6-fluoroimidazo[1,2-a]pyridin-3-yl)-N-methyl-N-(piperidin-3-yl)pyrimidin-4-amine (preparation 11b) and 3,3,3-trifluoro propanoic acid following the experimental procedure as described in example 54. The crude product was purified by flash chromatography (0-5% methanol in dichloromethane).
LRMS (m/z): 437 (M+1)⁺.
¹H-NMR δ (CDCl₃): 1.83 - 2.14 (m, 4H), 3.05 (s, 2H), 3.07 (s, 3H), 3.12 - 3.41 (m, 3H), 3.78 (bs, 2H), 6.36 (t, 1 H), 7.24 (dd, 1 H), 7.70 (dt, 1 H), 8.30 - 8.40 (m, 1 H), 8.53 (s, 1 H), 9.91 (dd, 1 H)

### EXAMPLE 71

### (R)-3-(3-((2-(6-fluoroimidazo[1,2-a]pyridin-3-yl)pyrimidin-4-yl)(methyl)amino) piperidin-1-yl)-3-oxopropanenitrile

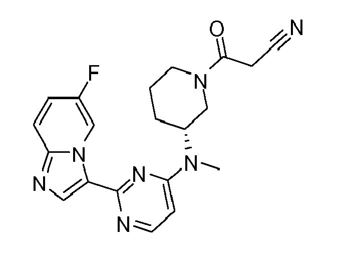

Obtained as a white solid (77%) from (R)-2-(6-fluoroimidazo[1,2-a]pyridin-3-yl)-N-methyl-N-(piperidin-3-yl)pyrimidin-4-amine (preparation 11 b) and 3-[(2,5-dioxopyrrolidin -1-yl)oxy]-3-oxopropanenitrile (prepared as described in BE875054(A1)) following the experimental procedure as described in example 46. The crude product was purified by flash chromatography (0-5% methanol in dichloromethane).
LRMS (m/z): 394 (M+1)⁺.
¹H-NMR δ (CDCl₃): 1.71 - 2.14 (m, 4H), 2.57 - 2.68 (m, 1 H), 2.80 (s, 3H), 3.06 (s, 2H), 3.13 - 3.35 (m, 2H), 3.71 - 3.86 (m, 2H), 4.62 - 4.75 (m, 1 H), 6.37 (dd, 1 H), 7.24 (dt, 1 H), 7.68 (dt, 1 H), 8.31 - 8.40 (m, 1 H), 8.54 (s, 1 H), 9.86 (dd, 1 H)

### EXAMPLE 72

### (R)-1-(3-((2-(6-fluoroimidazo[1,2-a]pyridin-3-yl)pyrimidin-4-yl)(methyl)amino) piperidine-1-carbonyl)cyclopropanecarbonitrile

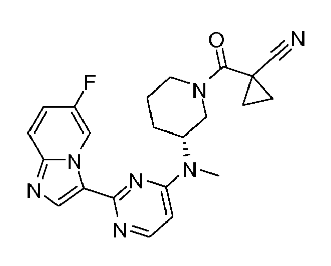

Obtained as a white solid (47%) from (R)-2-(6-fluoroimidazo[1,2-a]pyridin-3-yl)-N-methyl-N-(piperidin-3-yl)pyrimidin-4-amine (preparation 11b) and 1-cyanocyclopropane carboxylic acid following the experimental procedure as described in example 48. The crude product was purified by flash chromatography (0-8% methanol in dichloromethane) followed by trituration with diethyl ether.
LRMS (m/z): 420 (M+1)⁺.
¹H-NMR δ (CDCl₃): 1.55 (bs, 4H), 1.90 - 2.14 (m, 4H), 2.80 - 2.94 (m, 1 H), 3.08 (s, 3H), 3.15 - 3.31 (m, 2H), 4.53 (d, 2H), 6.39 (bs, 1 H), 7.23 (dd, 1 H), 7.68 (dd, 1 H), 8.33 (d, 1 H), 8.51 (bs, 1 H), 9.91 (bs, 1 H)

### EXAMPLE 73

### 3-((3R)-3-{ethyl[2-(6-fluoroimidazo[1,2-a]pyridin-3-yl)pyrimidin-4-yl]amino} piperidin-1-yl)-3-oxopropanenitrile

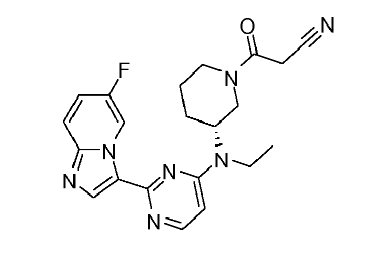

Obtained as a white solid (77%) from N-ethyl-2-(6-fluoroimidazo[1,2-a]pyridin-3-yl)-N-[(3R)-piperidin-3-yl]pyrimidin-4-amine (preparation 12b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (92:8 dichloromethane/methanol).
LRMS (m/z): 408 (M+1)⁺.
¹H-NMR δ (CDCl₃): 1.30 (bs, 3H), 1.77 (bs, 2H), 2.02 (bs, 2H), 2.84 (d, 1 H), 3.18 (bs, 2H), 3.39 - 3.59 (m, 4H), 3.79 (bs, 1 H), 4.72 (bs, 1 H), 6.33 (bs, 1 H), 7.23 (bs, 1 H), 7.68 (bs, 1 H), 8.32 (bs, 1 H), 8.37 - 8.55 (m, 1 H), 9.80 - 10.01 (m, 1 H)

### EXAMPLE 74

### N-ethyl-2-(6-fluoroimidazo[1,2-a]pyridin-3-yl)-N-[(3R)-1-(3,3,3-trifluoropropanoyl) piperidin-3-yl]pyrimidin-4-amine

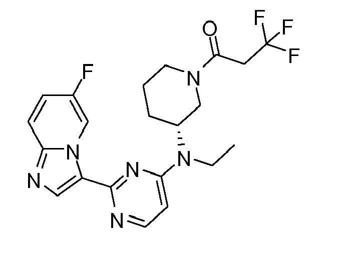

Obtained as a solid (52%) from N-ethyl-2-(6-fluoroimidazo[1,2-a]pyridin-3-yl)-N-[(3R)-piperidin-3-yl]pyrimidin-4-amine (preparation 12b) and 3,3,3-trifluoropropanoic acid following the experimental procedure as described in example 54. The crude product was purified by flash chromatography (92:8 dichloromethane/methanol).
LRMS (m/z): 451 (M+1)⁺.
¹H-NMR δ (CDCl₃): 1.30 (t, 3H), 1.92 - 2.18 (m, 2H), 2.51 - 2.66 (m, 1 H), 2.82 (bs, 1 H), 3.14 (s, 2H), 3.19 - 3.28 (m, 1 H), 3.32 (q, 2H), 3.51 (bs, 2H), 3.74 - 3.92 (m, 1 H), 4.79 (t, 1 H), 6.34 (d, 1 H), 7.23 (d, 1 H), 7.68 (dt, 1 H), 8.29 - 8.36 (m, 1 H), 8.42 - 8.54 (m, 1 H), 9.88 - 9.99 (m, 1 H)

### EXAMPLE 75

### 3-((3R)-3-{(cyclopropylmethyl)[2-(6-fluoroimidazo[1,2-a]pyridin-3-yl)pyrimidin-4-yl]amino}piperidin-1-yl)-3-oxopropanenitrile

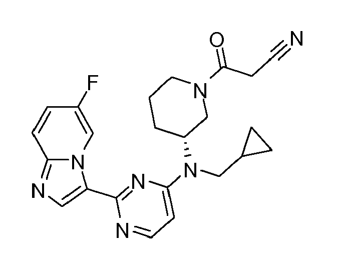

Obtained as a white solid (77%) from N-(cyclopropylmethyl)-2-(6-fluoroimidazo[1,2-a]pyridin-3-yl)-N-[(3R)-piperidin-3-yl]pyrimidin-4-amine (preparation 13b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (0-8% methanol in dichloromethane).
LRMS (m/z): 434 (M+1)⁺.
¹H-NMR δ (CDCl₃): 0.39 (bs, 2H), 0.67 (s, 2H), 1.08 (bs, 1 H), 1.95 - 2.18 (m, 4H), 2.97 (bs, 1 H), 3.14 - 3.36 (m, 2H), 3.21 (s, 2H), 3.57 (s, 2H), 3.79 (bs, 1 H), 4.66 - 4.85 (m, 1 H), 6.48 (dd, 1 H), 7.18 - 7.30 (m, 1 H), 7.68 (dd, 1 H), 8.27 - 8.38 (m, 1 H), 8.51 (s, 1 H), 9.92 (dd, 1 H)

### EXAMPLE 76

### N-(cyclopropylmethyl)-2-(6-fluoroimidazo[1,2-a]pyridin-3-yl)-N-[(3R)-1-(3,3,3-trifluoropropanoyl)piperidin-3-yl]pyrimidin-4-amine

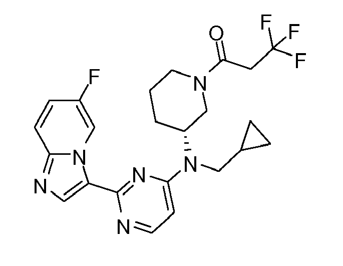

Obtained as a white solid (75%) from N-(cyclopropylmethyl)-2-(6-fluoroimidazo[1,2-a]pyridin-3-yl)-N-[(3R)-piperidin-3-yl]pyrimidin-4-amine (preparation 13b) and 3,3,3-trifluoropropanoic acid following the experimental procedure as described in example 54.
LRMS (m/z): 477 (M+1)⁺.
¹H-NMR δ (CDCl₃): 0.38 (d, 2H), 0.65 (d, 2H), 1.11 (bs, 1 H), 1.46 (d, 2H), 1.92 - 2.19 (m, 2H), 2.92 (bs, 1 H), 3.09 - 3.28 (m, 3H), 3.47 (bs, 2H), 3.86 (bs, 1 H), 4.72 - 4.91 (m, 2H), 6.47 (t, 1 H), 7.24 (dd, 1 H), 7.68 (dd, 1 H), 8.27 - 8.38 (m, 1H), 8.51 (s, 1 H), 9.97 (ddd, 1 H)

### EXAMPLE 77

### 3-((3R)-3-{[5-fluoro-2-(6-fluoroimidazo[1,2-a]pyridin-3-yl)pyrimidin-4-yl]amino} piperidin-1-yl)-3-oxopropanenitrile

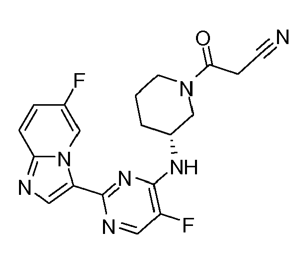

Obtained as a white solid (67%) from (R)-5-fluoro-2-(6-fluoroimidazo[1,2-a]pyridin-3-yl)-N-(piperidin-3-yl)pyrimidin-4-amine (preparation 17b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (0-8% methanol in dichloromethane).
LRMS (m/z): 398 (M+1)⁺.
¹H-NMR δ (CDCl₃): 1.73 - 2.00 (m, 2H), 2.21 (bs, 2H), 3.29 - 3.39 (m, 1 H), 3.45 (s, 2H), 3.48 - 3.68 (m, 2H), 4.24 - 4.41 (m, 2H), 5.18 (bs, 1 H), 7.68 (ddd, 1 H), 8.16 (d, 1 H), 8.40 (s, 1 H), 8.51 (s, 1 H), 9.80 - 9.91 (m, 1 H)

### EXAMPLE 78

### 5-Fluoro-2-(6-fluoroimidazo[1,2-a]pyridin-3-yl)-N-[(3R)-1-(3,3,3-trifluoropropanoyl) piperidin-3-yl]pyrimidin-4-amine

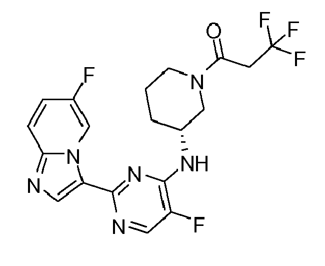

Obtained as a white solid (63%) from (R)-5-fluoro-2-(6-fluoroimidazo[1,2-a]pyridin-3-yl)-N-(piperidin-3-yl)pyrimidin-4-amine (preparation 17b) and 3,3,3-trifluoropropanoic acid following the experimental procedure as described in example 54. The crude product was purified by flash chromatography (0-8% methanol in dichloromethane).
LRMS (m/z): 441 (M+1)⁺.
¹H-NMR δ (CDCl₃): 1.69 - 2.01 (m, 3H), 2.18 (bs, 1 H), 3.16 - 3.27 (m, 1 H), 3.19 - 3.46 (m, 2H), 3.48 (d, 1 H), 3.54 - 3.65 (m, 1 H), 3.89 - 4.23 (m, 1 H), 4.26 - 4.37 (m, 1 H), 5.04 - 5.27 (m, 1 H), 7.25 (ddd, 1 H), 7.69 (td, 1 H), 8.16 (dd, 1 H), 8.37 - 8.54 (m, 1 H), 9.87 (ddd, 1 H)

### EXAMPLE 79

### 3-((3R)-3-{[2-(6-fluoroimidazo[1,2-a]pyridin-3-yl)-5-methylpyrimidin-4-yl]amino} piperidin-1-yl)-3-oxopropanenitrile

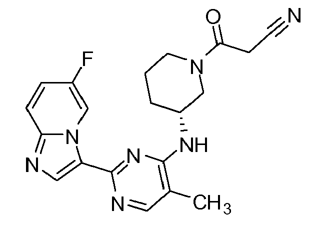

Obtained as a white solid (66%) from 2-(6-fluoroimidazo[1,2-a]pyridin-3-yl)-5-methyl-N-[(3R)-piperidin-3-yl]pyrimidin-4-amine (preparation 20b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (0-8% methanol in dichloromethane).
LRMS (m/z): 394 (M+1)⁺.
¹H-NMR δ (CDCl₃): 1.82 (bs, 2H), 2.09 (bs, 5H), 3.40 (bs, 2H), 3.72 - 3.88 (m, 1 H), 4.17 (d, 1 H), 4.34 (bs, 2H), 4.53 - 4.82 (m, 1 H), 5.30 (s, 1 H), 7.22 (bs, 1 H), 7.67 (bs, 1 H), 8.04 - 8.18 (m, 1 H), 8.39 - 8.56 (m, 1H), 9.95 (d, 1 H)

### EXAMPLE 80

### 2-(6-fluoroimidazo[1,2-a]pyridin-3-yl)-5-methyl-N-[(3R)-1-(3,3,3-trifluoropropanoyl) piperidin-3-yl]pyrimidin-4-amine

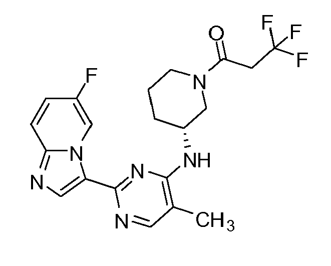

Obtained as a white solid (59%) from 2-(6-fluoroimidazo[1,2-a]pyridin-3-yl)-5-methyl-N-[(3R)-piperidin-3-yl]pyrimidin-4-amine (preparation 20b) and 3,3,3-trifluoropropanoic acid following the experimental procedure as described in example 54. The crude product was purified by flash chromatography (0-8% methanol in dichloromethane).
LRMS (m/z): 437 (M+1)⁺.
¹H-NMR δ (CDCl₃): 1.68 (s, 3H), 1.77 (bs, 1 H), 1.86 (bs, 2H), 2.07 (d, 2H), 2.19 (bs, 1 H), 3.12 - 3.23 (m, 1 H), 3.62 (bs, 1 H), 3.74 - 3.94 (m, 2H), 4.39 (bs, 1 H), 4.99 (d, 1 H), 7.16 - 7.26 (m, 1 H), 7.62 - 7.73 (m, 1 H), 8.11 (s, 1 H), 8.41 - 8.54 (m, 1 H), 8.48 (s, 1 H), 9.96 (bs, 1 H)

### EXAMPLE 81

### 3-((3R)-3-{[2-(6-fluoroimidazo[1,2-a]pyridin-3-yl)pyrimidin-4-yl]amino}pyrrolidin-1-yl)-3-oxopropanenitrile

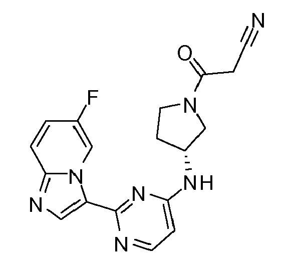

Obtained as a white solid (15%) from (R)-2-(6-fluoroimidazo[1,2-a]pyridin-3-yl)-N-(pyrrolidin-3-yl)pyrimidin-4-amine (preparation 21 b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (9:1 dichloromethane/methanol).
LRMS (m/z): 366 (M+1)⁺.
¹H-NMR δ (CDCl₃): 2.03 - 2.17 (m, 1 H), 2.28 - 2.52 (m, 2H), 3.50 (s, 2H), 3.63 - 3.79 (m, 2H), 3.81 - 3.89 (m, 1 H), 3.95 - 4.02 (m, 1 H), 6.26 (dd, 1 H), 7.20 - 7.31 (m, 1 H), 7.69 (ddd, 1 H), 8.28 (d, 1 H), 8.51 (d, 1 H), 9.98 (dd, 1 H)

### EXAMPLE 82

### 3-{4-[4-(cyanoacety))-1,4-diazepan-1-y)]pyrimidin-2-y)}imidazo[1,2-a]pyridine-6-carbonitrile

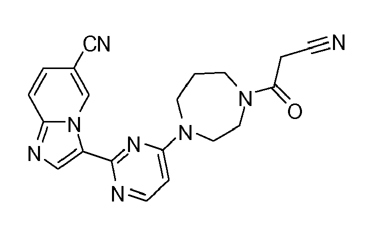

Obtained as a white solid (5%) from 3-(4-(1,4-diazepan-1-yl)pyrimidin-2-yl)imidazo[1,2-a]pyridine-6-carbonitrile (preparation 22b) and 3-[(2,5-dioxopyrrolidin-1-yl)oxy]-3-oxopropanenitrile (prepared as described in BE875054(A1)) following the experimental procedure as described in example 46. The crude product was purified by reverse phase chromatography (C-18 silica from Waters, water/acetonitrile/methanol as eluents [0.1 % v/v formic acid buffered] 0% to 100%).
LRMS (m/z): 387 (M+1)⁺.
¹H-NMR δ (CDCl₃): 1.22 - 1.40 (m, 2H), 3.41 (s, 2H), 3.46 - 3.58 (m, 2H), 3.60 (bs, 2H), 3.65 - 3.94 (m, 2H), 4.26 - 4.38 (m, 2H), 6.41 (dd, 1 H), 7.42 (ddd, 1 H), 7.79 (dd, 1 H), 8.35 (t, 1 H), 8.55 (d, 1 H), 10.55 (d, 1 H)

### EXAMPLE 83

### 3-((3R)-3-{[5-chloro-2-(6-fluoroimidazo[1,2-a]pyridin-3-yl)pyrimidin-4-yl]amino} piperidin-1-yl)-3-oxopropanenitrile

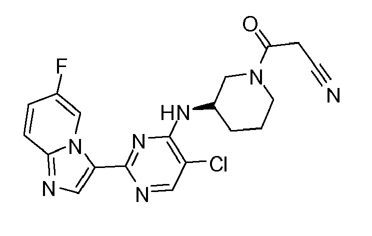

Obtained as a solid (68%) from 5-chloro-2-(6-fluoroimidazo[1,2-a]pyridin-3-yl)-N-[(3R)-piperidin-3-yl]pyrimidin-4-amine (preparation 43b) and 3-[(2,5-dioxopyrrolidin-1-yl)oxy]-3-oxopropanenitrile (prepared as described in BE875054(A1)) following the experimental procedure as described in example 46. The crude product was purified by flash chromatography (0-10% methanol in dichloromethane.
LRMS (m/z): 414 (M+1)⁺.
¹H NMR δ (CDCl₃): ppm 1.71 - 2.03 (m, 2H), 2.20 (dd, 1 H), 3.29 - 3.52 (m, 2H), 3.57 (s, 2H), 3.59 - 3.70 (m, 1 H), 3.80 - 3.94 (m, 1 H), 4.23 - 4.42 (m, 1 H), 5.33 - 5.49 (m, 1 H), 7.29 (dd, 1 H), 7.73 (dd, 1 H), 8.27 (s, 1 H), 8.56 (s, 1 H), 9.82 (dd, 1 H).

### EXAMPLE 84

### 3-((3R)-3-{[2-(6-fluoroimidazo[1,2-a]pyridin-3-yl)pyrimidin-4-yl]amino}piperidin-1-yl)-2,2-dimethylpropanenitrile

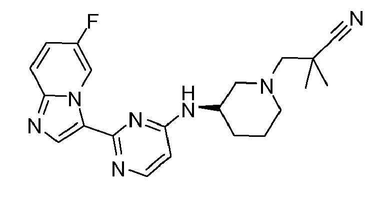

(*R*)-2-(6-fluoroimidazo[1,2-a]pyridin-3-yl)-N-(piperidin-3-yl)pyrimidin-4-amine (0.45 g, 1.44 mmol, preparation 9b) and 2-cyano-2-methylpropyl 4-methylbenzenesulfonate (0.18 g, 0.72 mmol, example 17b from US2007/299111A1) were mixed and stirred at 125 °C for 42 hours. The mixture was dissolved in ethyl acetate and washed with 1 N sodium hydroxide and brine. The organic layer was dried and evaporated and the residue obtained was purified by reverse phase chromatography (C-18 silica from Waters, water/acetonitrile/methanol as eluents [0.1% v/v formic acid buffered] 0% to 100%) to give the title compound (3%) as a solid.
LRMS (m/z): 394 (M+1)⁺.
¹H NMR δ (CDCl₃): 1.34 (s, 3H), 1.39 (s, 3H), 1.81 (bs, 4H), 2.39 - 2.53 (m, 2H), 2.60 (bs, 2H), 2.80 (br. s., 2H), 2.92 (bs, 1 H), 5.67 (bs, 1 H), 6.24 (d, 1H), 7.23 (ddd, 1 H), 7.69 (dd, 1 H), 8.21 (bs, 1 H), 8.51 (s, 1 H), 10.00 (dd, 1 H).

### EXAMPLE 85

### 2-((3R)-3-{[2-(6-fluoroimidazo[1,2-a]pyridin-3-yl)pyrimidin-4-yl]amino}piperidin-1-yl)acetamide

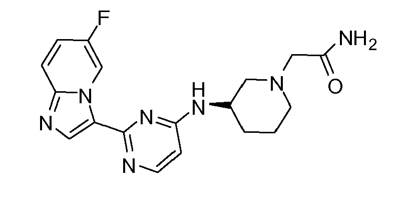

Triethylamine (0.09 mL) followed by 2-bromoacetamide (0.049 g, 0.36 mmol) were added dropwise to a solution of (*R*)-2-(6-fluoroimidazo[1,2-a]pyridin-3-yl)-N-(piperidin-3-yl)pyrimidin-4-amine (0.1 g, 0.32 mmol, preparation 9b) in dichloromethane (5 mL). The mixture was stirred at ambient temperature for 18 hours. The solvent was removed under reduced pressure and the residue was purified by reverse phase chromatography (C-18 silica from Waters, water/acetonitrile/methanol as eluents [0.1 % v/v formic acid buffered] 0% to 100%) to obtain the title compound (61 %) as a solid.
LRMS (m/z): 370 (M+1)⁺.
¹H NMR δ (DMSO-*d*₆): 1.46 (bs, 1 H), 1.62 (bs, 1 H), 1.71 - 1.89 (m, 2H), 2.36 (d, 2H), 2.83 (d, 1H), 3.17 (s, 2H), 4.28 (bs, 2H), 6.43 (d, 1H), 7.17 (bs, 1H), 7.37 (bs, 1 H), 7.52 (ddd, 1 H), 7.60 (d, 1 H), 7.81 (dd, 1 H), 8.29 (s, 1 H), 8.40 (s, 1 H), 9.97 (bs, 1 H).

### PHARMACOLOGICAL ACTIVITY

### In vitro JAK kinase Assays

Compounds were screened for their ability to inhibit JAK1, JAK2 and JAK3 ussing the assays as indicated below.

The catalytic domains of human JAK1 (aa 850-1154), JAK2 (aa 826-1132), JAK3 (aa 795-1124) and Tyk2 (aa 871-1187) were expressed as N-terminal GST-fusion proteins using a baculovirus expression system and were purchased from Carna Bioscences. The enzymatic activity was assayed using as substrate a biotinylated peptide, poly (GT)-Biotin (CisBio). The peptide concentration in the reactions was 60 nM for JAK1, 20 nM for JAK2, 140 nM for JAK3 and 50 nM for Tyk2. The degree of phosphorylation was detected by TR-FRET (time-resolved fluorescence energy transfer).

IC₅₀s of compounds were measured for each kinase in a reaction mixture containing the enzyme, ATP and the peptide in 8 mM MOPS (pH 7.0), 10 mM MgCl₂, 0.05% β-mercaptoethanol, 0.45 mg/mL BSA. The ATP concentration in the reactions was 3 µM for JAK1, 0.2 µM for JAK2, 0.6 µM for JAK3 and 1.8 µM for Tyk2. The enzymatic reactions took place for 30 minutes at room temperature. Then, the reactions were stopped with 20 µL of quench detection buffer (50 mM HEPES, 0.5 M KF, EDTA 0.25 M, 0.1% (w/v) BSA, pH 7.5) containing 0.115 µg/mL of anti-phosphoTyr (PT66)-Cryptate (CisBio) and a variable concentration of SA-XL665 (CisBio) to keep the SA-B ratio constant. Incubate for 3 h and read on Victor 2V spectrofluorometer (PerkinElmer) set to read fluorescence resonance energy transfer.

Some of the acronyms used above have the following meaning:
AA: aminoacids
GST: glutathione-S-transferase
MOPS: 3-(N-morpholino)propane sulfonic acid
BSA: bovine serum albumin
ATP: adenosine tri-phosphate
EDTA: ethylenediaminetetraacetic acid
HEPES: 4-(2-hydroxyethyl)-1-piperazineethanesulfonic acid

Table 1 depicts IC₅₀ values for certain exemplary compounds described in the invention. In Table 1, "A" represents an IC₅₀ value of less than 0.1 µM (100 nM), "B" represents an IC₅₀ value in the range of 0.1 µM (100 nM) to 1 µM, and C represents an IC₅₀ value higher than 1 µM.

**Table 1**

| Example No. | IC₅₀ JAK3 (µM) | IC₅₀ JAK2 (µM) | IC₅₀ JAK1 (µM) |
|---|---|---|---|
| 1 | A | A | B |
| 6 | B | B | c |
| 13 | B | B | C |
| 21 | A | A | B |
| 22 | A | A | C |
| 31 | B | A | B |
| 38 | B | A | C |
| 42 | C | B | C |
| 57 | A | A | C |
| 64 | A | A | A |
| 67 | B | A | B |
| 68 | A | A | B |
| 76 | B | A | C |
| 82 | C | B | C |

### Combinations

The imidazopyridine derivatives of the invention may also be combined with other active compounds in the treatment of a pathological condition or disease susceptible to amelioration by inhibition of Janus Kinases.

The combinations of the invention can optionally comprise one or more additional active substances which are known to be useful in the treatment of myeloproliferative disorders (such as polycythemia vera, essential thrombocythemia or mielofibrosis), leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis, such as (a) Dyhydrofolate reductase inhibitors, such as Methotrexate or CH-1504; (b) DHODH inhibitors such as leflunomide, teriflunomide, or the compounds described in the International Patent Application Nos. WO2008/077639 and WO2009021696; (c) Immunomodulators such as Glatiramer acetate (Copaxone), Laquinimod or Imiquimod; (d) Inhibitors of DNA synthesis and repair, such as Mitoxantrone or Cladribine; (e) Anti-alpha 4 integrin antibodies, such as Natalizumab (Tysabri); (f) Alpha 4 integrin antagonists such as R-1295, TBC-4746, CDP-323, ELND-002, Firategrast or TMC-2003; (g) Corticoids and glucocorticoids such as prednisone or methylprednisolone, fluticasone, mometasone, or beta-metasone; (h) Fumaric acid esters, such as *BG-12;* (i) Anti-TNF alpha antibodies, such as Infliximab, Adalimumab, or Certolizumab pegol; (j) Soluble TNF alpha receptors such as Ethanercept; (k) Anti-CD20 monoclonal antibodies such as Rituximab, Ocrelizumab Ofatumumab or TRU-015; (I) Anti-CD52 such as alemtuzumab; (m) Anti-CD25 such as daclizumab; (n) Anti-CD88, such as eculizumab or pexilizumab; (o) Anti-IL12R /IL23R, such as ustekinumab; (p) Calcineurin inhibitors such as cyclosporine A or tacrolimus; (q) IMPDH inhibitors, such as mycophenolate mophetyl; (r) Cannabinoid receptor agonists such as Sativex; (s) Chemokine CCR1 antagonists such as MLN-3897 or PS-031291; (t) Chemokine CCR2 antagonists such as INCB-8696; (u) NF-kappaB activation inhibitors such as MLN-0415; (v) S1 P receptor agonists such as fingolimod, BAF-312, or ACT128800; (w) S1P liase inhibitors such as LX2931; (x) Syk inhibitors, such as R-112; (y) PKC inhibitors, such as NVP-AEB071; (z) M3 antagonist such as tiotropium or aclidinium; (aa) Long-acting beta adrenergic agonists such as salmeterol, formoterol or indacaterol; (bb) Vitamin D derivatives like calcipotriol (Daivonex); (cc) Phosphosdiesterase IV inhibitors such as roflumilast or GRC-4039; (dd) p38 Inhibitors such as ARRY-797; (ee) MEK inhibitors, such as ARRY-142886 or ARRY-438162; (ff) PI3Kδγ inhibitors; (gg) Interferons comprising Interferon beta 1 a such as Avonex from Biogen Idec, CinnoVex from CinnaGen and Rebif from EMD Serono, and Interferon beta 1 b such as Betaferon from Schering and Betaseron from Berlex; and (hh) Interferon alpha such as Sumiferon MP.

Specific examples of suitable corticoids and glucocorticoids that can be combined with the JAK inhibitors of the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Specific examples of suitable Syk kinase inhibitors that can be combined with the JAK inhibitors of the present invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Specific examples of suitable M3 antagonists (anticholinergics) that can be combined with the JAK inhibitors of the present invention are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-1 04135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Specific examples of suitable long-acting beta adrenergic agonists (β2-agonists) that can be combined with the JAK inhibitors of the present invention are terbutaline sulphate, eformoterol fumarate, formoterol fumarate, bambuterol, procaterol hydrochloride, sibenadet hydrochloride, mabuterol hydrochloride, albuterol sulphate, salbutamol sulphate, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, Bedoradrine sulphate, Indacaterol, Trantinterol hydrochloride, AZD-3199, GSK-159802; GSK-597901, GSK-678007, GSK-642444;GSK-961081; AR-C98955AA, Milveterol hydrochloride, BI-1744-CL, and compounds described in the International Patent Applications Nos. WO2007/124898, W02006/122788A1, WO2008/046598 and W02008095720.

Specific examples of suitable Phosphosdiesterase IV inhibitors that can be combined with the JAK inhibitors of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1 H)-one (T-440), (-)-trans-2-[3'-[3-(N-Cydopropylcarbamoyl)-4-oxo-1,4-dihydro-1,8-naphthyridin-1-yl]-3-fluorobiphenyl-4-yl]cyclopropanecarboxylic acid (MK-0873), CDC-801, UK-500001, BLX-914, 2-carbomethoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, GRC-4039, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the International Patent Applications Nos. WO03/097613, WO2004/058729, WO 2005/049581, WO 2005/123693 and WO 2005/123692

Examples of suitable PI3Kδγ inhibitors that can be combined with the JAK inhibitors of the present invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

The compounds of formula (I) and the combinations of the invention may be used in the treatment of myeloproliferative disorders, leukemia, lymphoid malignancies and solid tumors; bone marrow and organ transplant rejection; and immune-mediated diseases, wherein the use of a JAK inhibitor is expected to have a beneficial effect, for example rheumatoid arthritis, multiple sclerosis, inflammatory bowel disease (such as ulcerative colitis or Crohn's disease), dry eye, uveitis, allergic conjunctivitis, allergic rhinitis, asthma, chronic obstructive pulmonary disease (COPD), atopic dermatitis and psoriasis.

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two active agents could be taken in the morning and the other (s) later in the day. Or in another scenario, one or two active agents could be taken twice daily and the other (s) once daily, either at the same time as one of the twice-a-day dosing occurred, or separately. Preferably at least two, and more preferably all, of the active agents would be taken together at the same time. Preferably, at least two, and more preferably all active agents would be administered as an admixture.

The invention is also directed to a combination product of the compounds of the invention together with one or more other therapeutic agents for use in the treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention also encompasses the use of a combination of the compounds of the invention together with one or more other therapeutic agents for the manufacture of a formulation or medicament for treating these diseases.

The invention also provides a method of treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis; comprising administering a therapeutically effective amount of a combination of the compounds of the invention together with one or more other therapeutic agents.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the imidazopyridine derivatives of the invention, and the other optional active compounds may be administered together in the same pharmaceutical composition or in different compositions intended for separate, simultaneous, concomitant or sequential administration by the same or a different route.

One execution of the present invention consists of a kit of parts comprising a imidazopyridine derivative of the invention together with instructions for simultaneous, concurrent, separate or sequential use in combination with another active compound useful in the treatment of myeloproliferative disorders, leukemia, lymphoid malignancies and solid tumors; bone marrow and organ transplant rejection; and immune-mediated diseases, more in particular useful in the treatment of rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

Another execution of the present invention consists of a package comprising a imidazopyridine derivative of the invention and another active compound useful in the treatment of myeloproliferative disorders, leukemia, lymphoid malignancies and solid tumors; bone marrow and organ transplant rejection; and immune-mediated diseases, more in particular useful in the treatment of rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

### Pharmaceutical Compositions

Pharmaceutical compositions according to the present invention comprise the compounds of the invention in association with a pharmaceutically acceptable diluent or carrier.

The invention further provides pharmaceutical compositions comprising the compounds of the invention in association with a pharmaceutically acceptable diluent or carrier together with one or more other therapeutic agents for use in the treatment of a pathological condition or disease susceptible to amelioration by inhibiton of Janus Kinases (JAK), such as the ones previously described.

The invention is also directed to pharmaceutical compositions of the invention for use in the treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis. The invention also encompasses the use of a pharmaceutical composition of the invention for the manufacture of a medicament for treating these diseases.

The invention also provides a method of treatment of a pathological condition or disease susceptible to amelioration by inhibiton of Janus Kinases (JAK), in particular wherein the pathological condition or disease is selected from myeloproliferative disorders, leukemia, lymphoid malignancies and solid tumors; bone marrow and organ transplant rejection; and immune-mediated diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis, comprising administering a therapeutically effective amount of a pharmaceutical composition of the invention.

The present invention also provides pharmaceutical compositions which comprise, as an active ingredient, at least an imidazopyridine of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

The pharmaceutically acceptable excipients which are admixed with the active compound or salts of such compound, to form the compositions of this invention are well-known per se and the actual excipients used depend inter alia on the intended method of administering the compositions.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 0.01-3000 mg, more preferably 0.5-1000 mg of active ingredient or the equivalent amount of a pharmaceutically acceptable salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical compositions may conveniently be presented in unit dosage form and may be prepared by any of the methods well known in the art of pharmacy.

The pharmaceutical compositions of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as Genuair® (formerly Novolizer® SD2FL) which is described in the following patent applications: WO 97/000703, WO 03/000325 and WO 03/061742.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

The following preparations forms are cited as formulation examples:

### COMPOSITION EXAMPLE 1

50,000 capsules, each containing 100 mg of 3-((3*R*)-3-{[2-(6-fluoroimidazo[1,2-a]pyridin-3-yl)pyrimidin-4-yl]amino}piperidin-1-yl)-3-oxopropanenitrile (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 3-((3*R*)-3-{[2-(6-fluoroimidazo[1,2-a]pyridin-3-yl)pyrimidin-4-yl]amino}piperidin-1-yl)-3-oxopropanenitrile (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, or solvate, or N-oxide or stereoisomer thereof: 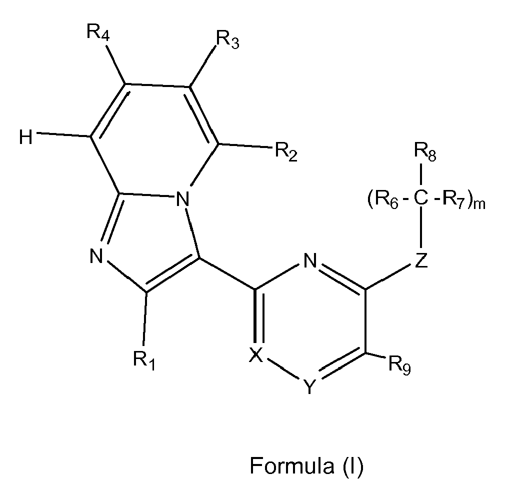 wherein,
m is 0 or an integer from 1 to 3;
Z is an oxygen atom or a group NR₅;
X and Y independently represent a nitrogen atom or a -CR₉ group, with wherein at least one of X and Y represents a nitrogen atom;
R₁, R₂, R₃, R₄ and R₉ each independently represent a hydrogen atom, a halogen atom, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group, a C₃-C₁₀ cycloalkyl group, a C₃-C₁₀ cycloalkenyl group, a monocyclic or polycyclic C₅-C₁₄ aryl group,
a 5- to 14- membered heteroaryl group containing at least one heteroatom selected from O, S and N, a 5- to 14- membered heterocyclyl group containing at least one heteroatom selected from O, S and N, a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N, an aza-bicycloalkyl group having up to 12 carbon atoms or an aza-bicycloalkenyl group having up to 12 carbon atoms,
wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from substituents Ra, and the alkyl groups are unsubstituted or substituted with one or more substituents selected from Rb;
or R₁, R₂, R₃, R₄ and R₉ independently represent a -SR₁₃ group, a -SOR₁₃ group,
a -S(O)₂R₁₃ group, a -S(O)₂NR₁₃R₁₄ group, a -NR₁₃S(O)₂R₁₄ group, a -NR₁₃S(O)₂NR₁₄ group, a -OR₁₃ group, a -C(O)OR₁₃ group, a -O-C(O)R₁₃ group, a -C(O)-(CH₂)ₙ-R,₃ group, a -NR₁₃R₁₄ group, a -C(O)-(CH₂)ₙ-NR₁₃R₁₄ group, a -NR₁₃C(O)-(CH₂)ₙ-R₁₄ group or a -NR₁₃C(O)-(CH₂)ₙ-NR₁₄R₁₅ group, wherein each n is 0, 1 or 2;
or in the case when two adjacent -CR₉ groups are present, two adjacent -CR₉ groups and the carbon atoms to which they are bonded optionally form a C₅-C₁₂ aryl group or a 4- to 12-membered heteroaryl, cycloalkyl or heterocyclyl group, said heteroaryl and heterocyclyl groups containing at least one heteroatom selected from O, S and N, the aryl, heteroaryl, cycloalkyl and heterocyclyl groups being unsubstituted or substituted by one or more substituents selected from a halogen atom, a linear or branched C₁-C₆ alkyl group, a monocyclic or polycyclic C₅-C₁₄ aryl group, a 5- to 14- membered heteroaryl group containing at least one heteroatom selected from O, S and N, or a 5-to 14- membered heterocyclyl group containing at least one heteroatom selected from O, S and N, wherein the alkyl, the aryl, the heteroaryl and the heterocyclyl substituents are unsubstituted or substituted by one or more substituents selected from a halogen atom, a hydroxy group, a cyano group, a linear or branched C₁-C₆ alkyl group or a C₁-C₄ haloalkyl group;
R₅ represents a hydrogen atom, a linear or branched C₁-C₆ alkyl group optionally substituted by one or more substituents selected from a hydroxy group, a cyano group, a C₁-C₄ haloalkyl group, a C₁-C₄ hydroxyalkyl group, a C₃-C₁₀ cycloalkyl group, a phenyl group or a 6 membered, saturated N-containing heterocyclyl ring, or R₅ represents a -S(O)₂R₁₀ group, a -S(O)₂NR₁₀R₁₁ group, a -C(O)OR₁₀ group,
a -C(O)-(CH₂)ₙ-R₁₀ group, or a -C(O)-(CH₂)ₙ-NR₁₀R₁₁ group;
R₆ and R₇ each independently represents a hydrogen atom or a linear or branched C₁-C₆ alkyl group optionally substituted by one or more substituents selected from a hydroxy group, a cyano group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring;
R₈ represents a hydrogen atom, a halogen atom, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group, a C₃-C₁₀ cycloalkyl group, a C₃-C₁₀ cycloalkenyl group, a monocyclic or polycyclic C₅-C₁₄ aryl group, a 5- to 14- membered heteroaryl group containing at least one heteroatom selected from O, S and N, a 5- to 14-membered heterocyclyl group containing at least one heteroatom selected from O, S and N, a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N, an aza-bicycloalkyl group having up to 12 carbon atoms or a aza-bicycloalkenyl group having up to 12 carbon atoms,
wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from Ra, -(C₁-C₄ alkyl)-CN groups, or -(C₁-C₄ alkyl)-C(O)NR'R" groups wherein R' and R" are the same or different and are selected from hydrogen atoms and linear or branched C₁-C₄ alkyl groups; and the alkyl groups are unsubstituted or substituted by one or more substituents selected from Rb;
or R₈ represents a -SR₁₃ group, a -SOR₁₃ group, a -S(O)₂R₁₃ group, a -S(O)₂NR₁₃R₁₄ group, a -NR₁₃S(O)₂R₁₄ group, a -NR₁₃S(O)₂NR₁₄ group, a -OR₁₃ group, a -C(O)OR₁₃ group, a -O-C(O)R₁₃ group, a -C(O)-(CH₂)ₙ-R₁₃ group, a -NR₁₃R₁₄ group,
a -C(O)-(CH₂)ₙ-NR₁₃R₁₄ group, a -NR₁₃C(O)-(CH₂)ₙ-Rₗ₄ group, or
a -NR₁₃C(O)-(CH₂)ₙ-NR₁₄Rₗ₅ group, wherein n is 0, 1 or 2,
or R₈ together with R₅ and the nitrogen atom to which R₅ is bonded form a 4- to 10-membered, saturated heterocyclyl group, which contains, as heteroatoms, one or two nitrogen atoms and which is substituted by a linear or branched C₁-C₆ alkyl group, a monocyclic or polycyclic C₅-C₁₄ aryl group, a 5- to 14- membered heteroaryl group containing at least one heteroatom selected from O, S and N, a 5- to 14- membered heterocyclyl group containing at least one heteroatom selected from O, S and N,
a -SOR₁₀ group, a -C(O)-(CH₂)ₙ-R_{1O} group, or a -C(O)-(CH₂)ₙ-NR₁₀R₁₁ group, wherein each n is 0, 1 or 2,
wherein the alkyl, aryl, heteroaryl and heterocyclyl groups are unsubstituted or substituted by one or more substituents selected from a halogen atom, a hydroxy group, a cyano group, a linear or branched C₁-C₆ alkyl group or a C₁-C₄ haloalkyl group, and wherein the alkyl groups are unsubstituted or substituted by one or more substituents selected from a halogen atom, a hydroxy group, a cyano group or a C₁-C₄ haloalkyl group;
provided that when m is zero, R₈ is other than a -SR₁₃ group, a -SOR₁₃ group, a -S(O)₂R₁₃ group, a -S(O)₂NR₁₃R₁₄ group, a -NR₁₃S(O)₂R₁₄ group, a -NR₁₃S(O)₂NR₁₄ group, a -OR₁₃ group, a -O-C(O)R₁₃ group, a -NR₁₃R₁₄ group, a -NR₁₃C(O)-(CH₂)ₙ-R₁₄ group, or a -NR₁₃C(O)-(CH₂)ₙ-NR₁₄R₁₅ group,
wherein
Ra is a halogen atom, a cyano group, a hydroxy group, a linear or branched C₁-C₆ alkyl group, a C₁-C₄ haloalkyl group, a C₁-C₄ hydroxyalkyl group, a C₃-C₇ cycloalkyl or a C₃-C₇ cycloalkenyl group, a monocyclic or polycyclic C₅-C₁₄ aryl group, which aryl group is unsubstituted or substituted by one or more halogen atoms, a 5- to 14- membered heteroaryl group containing at least one heteroatom selected from O, S and N, a 5- to 14- membered heterocyclyl group containing at least one heteroatom selected from O, S and N, a -SR₁₀ group, a -SOR₁₀ group, a -S(O)₂R₁₀ group, a -S(O)₂NR₁₀R₁₁ group,
a -NR₁₀S(O)₂R₁₁ group, a -NR₁₀S(O)₂NR₁₁ group, a -OR₁₀ group, a -C(O)OR₁₀ group,
a -O-C(O)R₁₀ group, a -C(O)-(CH₂)ₙ-R₁₀ group, a -NR₁₀R₁₁ group,
a -C(O)-(CH₂)ₙ-NR₁₀R₁₁ group, a -NR₁₀C(O)-(CH₂)n-R₁₁ group or
a -NR₁₀C(O)-(CH₂)ₙ-NR₁₁R₁₂ group, wherein each n is 0, 1 or 2;
Rb is a cyano group, a C₁-C₄ haloalkyl group, a C₁-C₄ hydroxyalkyl group, a C₃-C₇ cycloalkyl or a C₃-C₇ cycloalkenyl group, a monocyclic or polycyclic C₅-C₁₄ aryl group, which aryl group is unsubstituted or substituted by one or more halogen atoms, a 5- to 14- membered heteroaryl group containing at least one heteroatom selected from O, S and N, a 5- to 14- membered heterocyclyl group containing at least one heteroatom selected from O, S and N, a -SR₁₀ group, a -SOR₁₀ group, a -S(O)₂R₁₀ group,
a -S(O)₂NR₁₀R₁₁ group, a -NR₁₀S(O)₂R₁₁ group, a -NR₁₀S(O)₂NR₁₁ group, a -OR₁₀ group, a -C(O)OR₁₀ group, a -O-C(O)R₁₀ group, a -C(O)-(CH₂)ₙ-R₁₀ group, a -NR₁₀R₁₁ group, a -C(O)-(CH₂)ₙ-NR₁₀R₁₁ group, a -NR₁₀C(O)-(CH₂)ₙ-R₁₁ group or
a -NR₁₀C(O)-(CH₂)ₙ-NR₁₁R₁₂ group, wherein n is 0, 1 or 2;
R₁₀, R₁₁ and R₁₂ each independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, a bicyclyl group containing a monocyclic C₅-C₆ aryl or heteroaryl group bonded directly to a 5- to 6- membered cycloalkyl or heterocyclyl group which heteroaryl or heterocyclyl group contains 1, 2 or 3 nitrogen atoms, the haloalkyl, hydroxyalkyl, alkoxycarbonyl, cycloalkyl, phenyl, heteroaryl, heterocyclyl and bicyclyl group being unsubstituted or substituted by one or more substituents selected from substituents Rc, and the alkyl groups being unsubstituted or substituted with one or more substituents selected from substituents Rd;
Rc is a halogen atom, a hydroxy group, a cyano group, a linear or branched C₁-C₆ alkyl group, a C₁-C₄ haloalkyl group, a C₁-C₄ alkoxy group, a C₁-C₄ hydroxyalkyl group, a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃-C₇ heterocycloalkyl ketone group containing 1, 2 or 3 nitrogen atoms, said phenyl group being unsubstituted or substituted with one or more halogen atoms, and said heteroaryl, heterocyclyl and heterocycloalkyl ketone groups being unsubstituted or substituted by one or more linear or branched C₁-C₃ alkyl groups;
Rd is a cyano group, a C₁-C₄ haloalkyl group, a C₁-C₄ alkoxy group, a C₁-C₄ hydroxyalkyl group, a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃-C₇ heterocycloalkyl ketone group containing 1, 2 or 3 nitrogen atoms, said phenyl group being unsubstituted or substituted with one or more halogen atoms, and said heteroaryl, heterocyclyl and heterocycloalkyl ketone groups being unsubstituted or substituted by one or more linear or branched C₁-C₃ alkyl groups;
R₁₃, R₁₄, and R₁₅ each independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N, wherein the haloalkyl, hydroxyalkyl, alkoxycarbonyl, cycloalkyl, aryl, heteroaryl and heterocyclyl groups are unsubstituted or substituted by one or more substituents selected from the substituents Ra, and the alkyl groups are optionally substituted by one or more substitutents selected from Rb.

2. A compound according to claim 1, wherein
R₁, R₂ and R₄ are the same or different and each represent a hydrogen atom, a halogen atom, a hydroxyl group, a cyano group, a linear or branched C₁-C₆ alkyl group, a C₁-C₄ haloalkyl group or C₁-C₄ hydroxyalkyl group;
R₃ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group, a C₃-C₁₀cycloalkyl group, a 5- to 10- membered heterocyclyl group, a C₆-C₁₀ aryl group, a 5- to 10- membered heteroaryl group, a -C(O)OR' group or a -C(O)NR"R"' group, which cycloalkyl, heterocyclyl, aryl and heteroaryl groups are unsubstituted or substituted with one or more halogen atoms or a linear or branched C₁-C₆ alkyl group, a hydroxy group, a cyano group, or a C₁-C₄ alkoxy group; wherein R', R" and R'" are the same or different and each represents a hydrogen atom, a linear or branched C₁-C₆ alkyl group, a C₁-C₄ haloalkyl group or C₁-C₄ hydroxyalkyl group;
R₅ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group or a -(C₁-C₄ alkyl)-(C₃-C₇ cycloalkyl) group, or R₅ together with R₈ and the nitrogen atom to which R₅ is bonded form a 5- to 9-membered, saturated heterocyclyl group, which contains, as heteroatoms, one or two nitrogen atoms and which heterocyclyl ring is unsubstituted or substituted with a -C(O)-(CH₂)ₙ-R' group or a -C(O)-(CH₂)ₙ-NR"R"' group, wherein n is 0, 1 or 2, R' represents a hydrogen atom, or a linear or branched C₁-C₆ alkyl group, a C₁-C₄ haloalkyl group, a C₁-C₄ hydroxyalkyl group, or a cyano group and R" and R"' are the same or different and each represents a hydrogen atom, a linear or branched C₁-C₆ alkyl group, a C₁-C₄ haloalkyl group, or C₁-C₄ hydroxyalkyl group;
R₆ and R₇ are the same or different and each represent a hydrogen atom, a linear or branched C₁-C₆ alkyl group, or a C₁-C₄ haloalkyl group or a C₁-C₄ hydroxyalkyl group;
R₉ is a hydrogen atom, a halogen atom, a hydroxyl group, a cyano group, a linear or branched C₁-C₆ alkyl group, a C₁-C₄ haloalkyl group, a C₁-C₄ hydroxyalkyl group, a 5-to 10- membered heterocyclyl group, or a 5- to 10- membered heteroaryl group, which heterocyclyl and heteroaryl groups are unsubstituted or substituted with one or more halogen atoms or a linear or branched C₁-C₆ alkyl group, a cyano group, a hydroxy group or a C₁-C₄ alkoxy substituents, or in the case when two adjacent -CR₉ groups are present, the two adjacent -CR₉ groups and the carbon atoms to which they are bonded optionally form a C₆-C₁₀ aryl group which is unsubstituted or substituted by one or more substituents selected from a halogen atom, a linear or branched C₁-C₆ alkyl group, a hydroxy group or a C₁-C₄ alkoxy group;
R₈ together with R₅ and the nitrogen atom to which R₅ is bonded form a said 5- to 9-membered heterocyclyl ring, or R₈ is a hydrogen atom, a linear or branched C₁-C₆ alkyl group, a C₁-C₄ haloalkyl group, a C₁-C₄ hydroxyalkyl group, a C₃-C₁₀ cycloalkyl group, a 5- to 10- membered heterocyclyl group, a C₆-C₁₀ aryl group , a 5- to 10- membered heteroaryl group, -L-Het-R"', -L-A, -A-SO₂-R', -A-SO-R"', -A-A', -A-L-C(O)NR'R", -A-L-CN, -A-C(O)-Het'-L-CN, -A-C(O)-NR'R", -A-C(O)₂-A", -A-C(O)-R"', -A-CO₂-R', -A-C(O)_{z}-L-A"', -A-C(O)_{z}-L-CN, or -A-C(O)_{z}-L-Het-R' group, wherein z is 1 or 2, R' and R" are the same or different and each represents a hydrogen atom or linear or a branched C₁-C₆ alkyl group, a C₁-C₄ haloalkyl group or a C₁-C₄ hydroxyalkyl group group, and R"' represents a linear or branched C₁-C₆ alkyl group, a C₁-C₄ haloalkyl group or a C₁-C₄ hydroxyalkyl group, the heterocyclyl and heteroaryl groups being optionally fused to a phenyl group, and wherein the cycloalkyl, heterocyclyl, aryl and heteroaryl groups being unsubstituted or substituted with one or more halogen atoms, a hydroxyl group, a cyano, a linear or branched C₁-C₄ alkyl group, or C₁-C₄ alkoxy group, and wherein
L is a linear or branched C₁-C₆ alkylene group,
Het represents O or NR, and Het' represents NR, wherein R is a hydrogen atom, a straight or branced C₁-C₄ alkyl group, a C₁-C₄ haloalkyl group, or C₁-C₄ hydroxyalkyl group,
A, A', A" and A"' are the same or different and each represent a C₃-C₁₀ cycloalkyl group, a 5- to 10- membered heterocyclyl group, a C₆-C₁₀ aryl group, or a 5- to 10-membered heteroaryl group, the cycloalkyl, heterocyclyl, aryl and heteroaryl groups being unsubstituted or substituted with one or more halogen atoms, a hydroxyl group, a cyano group, a linear or branched C₁-C₄ alkyl group, or a C₁-C₄ alkoxy group.

3. A compound according to claim 1 or 2, wherein Z is a group NR₅, wherein R₅ is as defined in claim 1.

4. A compound according to any one of the preceding claims, wherein R₁ represents a hydrogen atom, a halogen atom, a hydroxy group, a cyano group, a linear or branched C₁-C₆ alkyl group, a C₁-C₄ haloalkyl group, a C₁-C₄ hydroxyalkyl group, a C₁-C₄ alkoxy group, a C₁-C₄ alkoxycarbonyl group, a C₃-C₇ cycloalkyl group, a phenyl group, a pyridyl group, a 6-membered, saturated N-containing heterocyclyl ring, a -C(O)OR₁₃ group, a -C(O)-(CH₂)ₙ-R₁₃ group, a -NR₁₃R₁₄ group, or a -C(O)-(CH₂)ₙ-NR₁₃R₁₄ group, wherein n, R₁₃ and R₁₄ are as defined in claim 1; and
wherein R₁ preferably represents a hydrogen atom, a halogen atom, a cyano group, a linear or branched C₁-C₆ alkyl group or a C₃-C₇ cycloalkyl group or a -NR₁₃R₁₄ group wherein R₁₃ and R₁₄ independently represent a hydrogen atom or a linear or branched C₁-C₃ alkyl group; and
wherein R₁ more preferably represents a hydrogen atom or a -NR₁₃R₁₄ group wherein R₁₃ and R₁₄ independently represent a hydrogen atom or a linear or branched C₁-C₃ alkyl group; and
wherein R₁ most preferably represents a hydrogen atom.

5. A compound according to any one of the preceding claims, wherein R₂ represents a hydrogen atom, a halogen atom, a hydroxy group, a cyano group, a linear or branched C₁-C₆ alkyl group, a C₁-C₄ haloalkyl group, a C₁-C₄ alkoxy group, a C₁-C₄ hydroxyalkyl group, a C₁-C₄ alkoxycarbonyl group, a C₃-C₇ cycloalkyl group, a phenyl group, a pyridyl group or a 6 membered, saturated N-containing heterocyclyl ring; and
wherein R₂ preferably represents a hydrogen atom, a halogen atom, a cyano group, a linear or branched C₁-C₆ alkyl group or a C₃-C₇ cycloalkyl group; and
wherein R₂ more preferably represents a hydrogen atom or a halogen atom; and wherein R₂ most preferably represents a hydrogen atom.

6. A compound according to any one of the preceding claims, wherein R₃ represents a hydrogen atom, a halogen atom, a cyano group, a linear or branched C₁-C₆ alkyl group, a C₁-C₄ haloalkyl group, a C₁-C₄ hydroxyalkyl group, a C₁-C₄ alkoxy group, a C₁-C₄ alkoxycarbonyl group, a C₃-C₇ cycloalkyl group, a phenyl group, a 5- to 6- membered heteroaryl group containing at least one heteroatom selected from O, S and N, or a 6 membered, saturated N-containing heterocyclyl ring,
the haloalkyl, hydroxyalkyl, alkoxycarbonyl, cycloalkyl, phenyl, heteroaryl and heterocyclyl groups being unsubstituted or substituted by one or more substituents selected from a halogen atom, a hydroxy group, a cyano group or a linear or branched C₁-C₆ alkyl group, and the alkyl groups being unsubstituted or substituted by one or more cyano groups; or
R₃ represents a -S(O)₂NR₁₃R₁₄ group, a -NR₁₃S(O)₂NR₁₄ group, a-OR₁₃ group,
a -C(O)OR₁₃ group, a -NR₁₃R₁₄ group, a -NC(O)-(CH₂)ₙ-R₁₃ group,
a -C(O)-(CH₂)ₙ-NR₁₃R₁₄ group, a -NR₁₃C(O)-(CH₂)ₙ-R₁₄ group or a -NR₁₃C(O)-(CH₂)ₙ-NR₁₄R₁₅ group, wherein n is 0 or 1, and R₁₃, R₁₄, and R₁₅ each independently represent a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6-membered, saturated N-containing heterocyclyl ring.

7. A compound according to claim 6, wherein R₃ represents a hydrogen atom, a halogen atom, a cyano group, a C₁-C₄ haloalkyl group, a C₃-C₄ cycloalkyl group, a phenyl group, a 5- to 6- membered monocyclic heteroaryl group containing 1, 2 or 3 heteroatoms selected from N, O and S,
the phenyl and heteroaryl groups being unsubstituted or substituted with one or more substituents selected from a halogen atom, a hydroxy group, a cyano group or a linear or branched C₁-C₆ alkyl group; or
R₃ represents a -C(O)OR₁₃ group or a -C(O)-(CH₂)ₙ-NR₁₃R₁₄ group wherein R₁₃ and R₁₄ independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring; and
wherein R₃ preferably represents a hydrogen atom, a halogen atom or a cyano group or a -C(O)-(CH₂)ₙ-NR₁₃R₁₄ group wherein n is 0 or 1 and R₁₃ and R₁₄ each independently represent a hydrogen atom or a linear or branched C₁-C₃ alkyl group; and
wherein R₃ most preferably represents a halogen atom or a cyano group.

8. A compound according to any one of the preceding claims, wherein R₄ represents a hydrogen atom, a halogen atom, a hydroxy group, a cyano group, a linear or branched C₁-C₆ alkyl group, a C₁-C₄ haloalkyl group, a C₁-C₄ hydroxyalkyl group, a C₁-C₄ alkoxy group, a C₁-C₄ alkoxycarbonyl group, a C₃-C₇ cycloalkyl group, a phenyl group, a pyridyl group or a 6 membered, saturated N-containing heterocyclyl ring; and wherein R₄ preferably represents a hydrogen atom, a halogen atom, a cyano group, a linear or branched C₁-C₆ alkyl group or a C₃-C₄ cycloalkyl group; and wherein R₄ more preferably represents a hydrogen atom.

9. A compound according to any one of the preceding claims, wherein R₅ represents a hydrogen atom, or a linear or branched C₁-C₆ alkyl group optionally substituted by one or more substituents selected from a hydroxy group, a cyano group, a linear or branched C₁-C₆ alkyl group, a C₁-C₄ haloalkyl group, a C₁-C₄ hydroxyalkyl group, a C₃-C₇ cycloalkyl group, a phenyl group or a 6 membered, saturated N-containing heterocyclyl ring; and
wherein R₅ preferably represents a hydrogen atom, a linear or branched C₁-C₆ alkyl group optionally substituted by a C₃-C₇ cycloalkyl group; and
wherein R₅ more preferably represents a hydrogen atom or a linear or branched C₁-C₃ alkyl group.

10. A compound according to any one of the preceding claims, wherein R₆ and R₇ each independently represent a hydrogen atom or a linear or branched C₁-C₆ alkyl group; and
wherein R₆ and R₇ preferably each independently represent a hydrogen atom or a linear or branched C₁-C₃ alkyl group.

11. A compound according to any one of the preceding claims, wherein R₈ represents a linear or branched C₁-C₆ alkyl group, a C₁-C₄ haloalkyl group, a C₃-C₁₀ cycloalkyl group, a phenyl group, a naphthyl group, a 5- to 6-membered monocyclic heteroaryl group containing 1, 2 or 3 heteroatoms selected from N, O and S, a 5- to 7-membered heterocyclyl group containing 1, 2 or 3 heteroatoms selected from N, O and S, or a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N,
the alkyl, haloalkyl, alkoxy, cycloalkyl, phenyl, naphthyl, heteroaryl, heterocyclyl and bicyclyl groups being unsubstituted or substituted by one or more substituents selected from a halogen atom, a hydroxy group, a cyano group, a linear or branched C₁-C₆ alkyl group, a C₁-C₄ haloalkyl group, a C₃-C₇ cycloalkyl group, a phenyl group unsubstituted or substituted by one or more halogen atoms, a 5- to 6-membered monocyclic heteroaryl group containing 1, 2 or 3 heteroatoms selected from N, O and S, a 6 membered, saturated N-containing heterocyclyl ring, a -S(O)₂R₁₀ group, -S(O)₂NR₁₀R₁₁ group, a -NR₁₀S(O)₂NR₁₁ group, a -OR₁₀group, a -C(O)OR₁₀ group, a -C(O)-(CH₂)ₙ-R₁₀ group, a -NR₁₀R₁₁ group, a -C(O)-(CH₂)ₙ-NR₁₀R₁₁ group or a -R₁₀C(O)-(CH₂)ₙ-NRₙR₁₂ group,
a -(C₁-C₄ alkyl)-CN group, or a -(C₁-C₄ alkyl)-C(O)NR'R" group wherein R' and R" are the same or different and are selected from hydrogen atoms and linear or branched C₁-C₄ alkyl groups; wherein n is 0 or 1, and
R₁₀ and R₁₁ each independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms,
the alkyl, haloalkyl, cycloalkyl, phenyl, heteroaryl, heterocyclyl and bicyclyl groups being unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms, said heteroaryl group being unsubstituted or substituted by one or more linear or branched C₁-C₃ alkyl groups, or a C₃-C₇ heterocycloalkyl ketone group containing 1, 2 or 3 nitrogen atoms, said heterocycloalkyl ketone group being unsubstituted or substituted by one or more C₁-C₃ alkyl groups; or
R₈ represents a -S(O)₂NR₁₃R₁₄ group, a -NR₁₃S(O)₂NR₁₄ group, a -OR₁₃ group,
a -C(O)OR₁₃ group or a -C(O)-(CH₂)ₙ-NR₁₃R₁₄ group, wherein n is 0 or 1, and R₁₃ and R₁₄ each independently represent a hydrogen atom, a cyano group, a linear or branched C₁-C₆ alkyl group unsubstituted or substituted by one or more phenyl groups, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring; or
R₈ together with R₅ and the nitrogen atom to which R₅ is bonded form a 5- to 9-membered, saturated heterocyclyl group, which contains, as heteroatoms, one or two nitrogen atoms and which is substituted by a -C(O)-(CH₂)ₙ-R₁₀ group or
a -C(O)-(CH₂)ₙ-NR₁₀R₁₁ group, wherein wherein n is 0 or 1, and R₁₀ and R₁₁ independently represent a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring,
provided that when m is zero, R₈ is other than a -S(O)₂NR₁₃R₁₄ group,
a -NR₁₃S(O)₂NR₁₄ group, or a -OR₁₃ group.

12. A compound according to claim 11, wherein R₈ represents a linear or branched C₁-C₆ alkyl group, a C₁-C₄ haloalkyl group, a C₁-C₄ alkoxy group, a C₃-C₇ cycloalkyl group, an adamantyl group, a phenyl group, a 5- to 6-membered monocyclic heteroaryl group containing 1, 2 or 3 heteroatoms selected from N, O and S, a 5- to 7- membered heterocyclyl group containing 1, 2 or 3 heteroatoms selected from N, O and S, or a bicyclyl group containing a phenyl group bonded directly to a 5- to 7- membered heterocyclyl group containing at least one heteroatom selected from O, S and N,
the alkyl, haloalkyl, alkoxy, cycloalkyl, adamantyl, phenyl, heteroaryl, heterocyclyl and bicyclyl groups being unsubstituted or substituted by one or more substituents selected from a halogen atom, a hydroxy group, a linear or branched C₁-C₆ alkyl group, a phenyl group, a pyridyl group, a -S(O)₂R₁₀,
a -C(O)OR₁₀, a -C(O)-(CH₂)ₙ-R₁₀, a 1-NR_{1O}R₁₁ group,
a -C(O)-(CH₂)ₙ-NR₁₀R₁₁group, a -(C₁-C₄ alkyl)-CN group, or
a -(C₁-C₄ alkyl)-C(O)NR'R" group wherein R' and R" are the same or different and are selected from hydrogen atoms and linear or branched C₁-C₄ alkyl groups; wherein n is 0 or 1, and
R₁₀ and R₁₁ each independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms,
the alkyl, haloalkyl, cycloalkyl, phenyl, heteroaryl, heterocyclyl and bicyclyl groups being unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms, said heteroaryl group being unsubstituted or substituted by one or more linear or branched C₁-C₃ alkyl groups, or a C₃-C₇ heterocycloalkyl ketone group containing 1, 2 or 3 nitrogen atoms, said heterocycloalkyl ketone group being unsubstituted or substituted by one or more linear or branched C₁-C₃ alkyl groups; or
R₈ represents a -OR₁₃ group wherein R₁₃ represents a C₁-C₃ alkyl group unsubstituted or substituted by one or more phenyl groups, or
R₈ together with R₅ and the nitrogen atom to which R₅ is bonded form a 5- to 7-membered, saturated heterocyclyl group, which contains, as heteroatoms, one or two nitrogen atoms and which is substituted by a -C(O)-(CH₂)ₙ-R₁₀ group or
a -C(O)-(CH₂)ₙ-NR₁₀R₁₁ group, wherein n is 0 or 1, and R₁₀ and R₁₁ independently represent a hydrogen atom, a cyano group, a linear or branched C₁-C₃ alkyl group or a C₁-C₄ haloalkyl group,
provided that when m is zero, R₈ is other than an -OR₁₃ group.

13. A compound according to any one of the preceding claims, wherein R₉ represents a hydrogen atom, a halogen atom, a hydroxy group, a cyano group, a linear or branched C₁-C₆ alkyl group, a C₁-C₄ haloalkyl group, a C₁-C₄ alkoxy group, a C₃-C₇ cycloalkyl group, a -C₁-C₄ alkyl-C₃-C₇ cycloalkyl group, a phenyl group, a 5- to 6-membered monocyclic heteroaryl group containing 1, 2 or 3 heteroatoms selected from N, O and S, a 5- to 7- membered heterocyclyl group containing at least one heteroatom selected from O, S and N,
the alkyl, haloalkyl, alkoxy, cycloalkyl, phenyl, heteroaryl and heterocyclyl groups being unsubstituted or substituted by one or more substituents selected from a halogen atom, a hydroxy group, a cyano group, a linear or branched C₁-C₆ alkyl group, a -C(O)-(CH₂)-R₁₀ group or a -OR₁₀ group,
wherein R₁₀ represents a hydrogen atom, a cyano group, linear or branched C₁-C₄ alkyl group, a C₁-C₄ haloalkyl group or a C₃-C₇ cycloalkyl group,
the alkyl, haloalkyl and cycloalkyl groups being unsubstituted or substituted by one or more substituents selected from a halogen atom, a hydroxy group, a cyano group, a linear or branched C₁-C₆ alkyl group or a C₁-C₄ haloalkyl group; or
R₉ represents a -S(O)₂R₁₃ group, a -C(O)OR₁₃ group, a -NR₁₃R₁₄ group or
a -C(O)-(CH₂)ₙ-NR₁₃R₁₄ group wherein n is 0 or 1 and R₁₃ and R₁₄ independently represent a hydrogen atom or a linear or branched C₁-C₆ alkyl group; or
in the case that two ―CR₉ adjacent groups are present, two adjacents ―CR₉ groups and the carbon atoms to which they are bonded optionally form a 5- to 9- membered aryl or heteroaryl group, said heteroaryl group containing at least one heteroatom selected from O, S and N, the aryl and heteroaryl groups being unsubstituted or substituted by one or more substituents selected from a halogen atom or a linear or branched C₁-C₄ alkyl group.

14. A compound according to claim 13, wherein R₉ represents a hydrogen atom, a halogen atom, a cyano group, a linear or branched C₁-C₄ alkyl group, a C₁-C₄ haloalkyl group, a C₃-C₇ cycloalkyl group, C₁-C₃ alkyl - C₃-C₇ cycloalkyl group, a 5- to 6-membered monocyclic heteroaryl group containing 1, 2 or 3 nitrogen atoms, a 5- to 7-membered heterocyclyl group containing 1, 2 or 3 nitrogen atoms, the alkyl, cycloalkyl, heteroaryl and heterocyclyl group being unsubstituted or substituted by one or more substituents selected from a cyano group or a -OR₁₀ group wherein R₁₀ represents a hydrogen atom or a linear or branched C₁-C₃ alkyl group, or
R₉ represents a -S(O)₂R₁₃ group, a -C(O)OR₁₃ group, a -NR₁₃R₁₄ group or
a -C(O)-(CH₂)ₙ-NR₁₃R₁₄ group wherein n is 0 or 1 and R₁₃ and R₁₄ independently represent a hydrogen atom or a linear or branched C₁-C₆ alkyl group.

15. A compound according to claim 1, wherein:
m is 0 or 1;
Z is NR₅;
X is a nitrogen atom and Y is a group -CR₉, or Y is a nitrogen atom and X is a group -C R₉;
R₁ represents a hydrogen atom, a halogen atom, a cyano group, a linear or branched C₁-C₆ alkyl group or a C₃-C₄ cycloalkyl group or a -NR₁₃N₁₄ group wherein R₁₃ and R₁₄ each independently represent a hydrogen atom or a linear or branched C₁-C₃ alkyl group;
R₂ represents a hydrogen atom, a halogen atom, a cyano group, a linear or branched C₁-C₆ alkyl group or a C₃-C₄ cycloalkyl group;
R₃ represents a hydrogen atom, a halogen atom, a cyano group, a C₃-C₄ cycloalkyl group, a 5- to 6- membered monocyclic aryl or heteroaryl group, the heteroaryl group containing 1, 2 or 3 heteroatoms selected from N, O and S, the aryl and heteroaryl groups being unsubstituted or substituted by one or more substituents selected from a halogen atom, a hydroxy group, a cyano group or a linear or branched C₁-C₆ alkyl group, or
R₃ represents a -C(O)OR₁₃ group, a -C(O)-NR₁₃R₁₄ group or a -NR₁₃C(O)R₁₄ group wherein R₁₃ and R₁₄ each independently represent a hydrogen atom, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring;
R₄ represents a hydrogen atom, a halogen atom, a cyano group, a linear or branched C₁-C₆ alkyl group or a C₃-C₄ cycloalkyl group;
R₅ represents a hydrogen atom, a linear or branched C₁-C₃ alkyl group or a C₁-C₃ alkyl - C₃-C₇ cycloalkyl group;
R₆ and R₇ each independently represent a hydrogen atom or a linear or branched C₁-C₃ alkyl group;
R₈ represents a linear or branched C₁-C₆ alkyl group, a C₁-C₄ haloalkyl group, a C₁-C₄ alkoxy group, a C₃-C₇ cycloalkyl group, an adamantyl group, a phenyl group, a 5- to 6-membered monocyclic heteroaryl group containing 1, 2 or 3 heteroatoms selected from N, O and S, a 5- to 7- membered heterocyclyl group containing containing 1, 2 or 3 heteroatoms selected from N, O and S, or a bicyclyl group containing a phenyl group bonded directly to a 5- to 7- membered heterocyclyl group containing at least one heteroatom selected from O, S and N, the alkyl, haloalkyl, alkoxy, cycloalkyl, adamantyl, phenyl, heteroaryl, heterocyclyl and bicyclyl groups being unsubstituted or substituted by one or more substituents selected from
a halogen atom, a hydroxy group, a linear or branched C₁-C₆ alkyl group,
a phenyl group, a pyridyl group, a -S(O)₂R₁₀, a -C(O)OR₁₀, a -C(O)-(CH₂)ₙ-R₁₀, a -NR₁₀R₁₁ group, a -C(O)-(CH₂)ₙ-NR₁₀R₁₁ group, a -(C₁-C₄ alkyl)-CN group, or a -(C₁-C₄ alkyl)-C(O)NR'R" group wherein R' and R" are the same or different and are selected from hydrogen atoms and linear or branched C₁-C₄ alkyl groups;
wherein n is 0 or 1, and
R₁₀ and R₁₁ each independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms,
the alkyl, haloalkyl, cycloalkyl, phenyl, heteroaryl, heterocyclyl and bicyclyl groups being unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms, said heteroaryl group unsubstituted or substituted by one or more linear or branched C₁-C₃ alkyl groups, or a C₃-C₇ heterocycloalkyl ketone group containing 1, 2 or 3 nitrogen atoms, said heterocycloalkyl ketone group beingunsubstituted or substituted by one or more linear or branched C₁-C₃ alkyl groups, or
R₈ represents a -OR₁₃ group wherein R₁₃ represents a C₁-C₃ alkyl group unsubstituted or substituted by one or more phenyl groups, or
R₈ together with R₅ and the nitrogen atom to which R₅ is bonded form a 5- to 7-membered, saturated heterocyclyl group, which contains, as heteroatoms, one or two nitrogen atoms and which is substituted by a -C(O)-(CH₂)ₙ-R₁₀ group or a
-C(O)-(CH₂)ₙ-NR₁₀R₁₁ group, wherein n is 0 or 1, and R₁₀ and R₁₁ independently represent a hydrogen atom, a cyano group, a linear or branched C₁-C₃ alkyl group or a C₁-C₄ haloalkyl group;
R₉ represents a hydrogen atom, a halogen atom, a cyano group, a linear or branched C₁-C₄ alkyl group, a C₁-C₄ haloalkyl group, a C₃-C₇ cycloalkyl group, a -C₁-C₃ alkyl-C₃-C₇ cycloalkyl group, a 5- to 6-membered monocyclic heteroaryl group containing 1, 2 or 3 nitrogen atoms, a 5- to 7- membered heterocyclyl group containing 1, 2 or 3 nitrogen atoms, the alkyl, cycloalkyl, heteroaryl and heterocyclyl groups being unsubstituted or substituted by one or more substituents selected from a cyano group or a -OR₁₀ group, wherein R₁₀ represents a linear or branched C₁-C₃ alkyl group, or
R₉ represents a -S(O)₂R₁₃ group, a -C(O)OR₁₃ group or a -NR₁₃R₁₄ group wherein R₁₃ and R₁₄ each independently represent a hydrogen atom or a linear or branched C₁-C₄ alkyl group,
or in the case that two ―CR₉ adjacent groups are present, both ―CR₉ groups and the carbon atoms to which they are bonded optionally form a 5- to 9-membered aryl group, the aryl group being unsubstituted or substituted by one or more substituents selected from a halogen atom or a linear or branched C₁-C₄ alkyl group,
provided that when m is zero, R₈ is other than an -OR₁₃ group.

16. A compound according to claim 15, wherein:
m is 0 or 1;
Z is NR₅;
X is a nitrogen atom and Y is a group -CR₉, or Y is a nitrogen atom and X is a group -CR₉;
R₁ represents a hydrogen atom;
R₂ represents a hydrogen atom;
R₃ represents a hydrogen atom, a halogen atom, a cyano group, a C₁-C₄ haloalkyl group, a cyclopropyl group, a phenyl group unsubstituted or substituted by one or more halogen atoms, a 5- to 6- membered monocyclic heteroaryl group containing 1, 2 or 3 heteroatoms selected from N and O, or
R₃ represents a -C(O)OR₁₃ group, a -C(O)-NR₁₃R₁₄ group or a -NR₁₃C(O)R₁₄ group wherein R₁₃ and R₁₄ independently represent a hydrogen atom or a methyl group;
R₄ represents a hydrogen atom;
R₅ represents a hydrogen atom, a methyl group, an ethyl group or a -C₁-C₃ alkyl-C₃-C₄ cycloalkyl group;
R₆ and R₇ each independently represent a hydrogen atom or a methyl group;
R₈ represents a linear or branched C₁-C₆ alkyl group, a C₁-C₄ haloalkyl group, a C₁-C₄ alkoxy group, a C₃-C₇ cycloalkyl group, an adamantyl group, a phenyl group, a 5- to 6-membered monocyclic heteroaryl group containing 1, 2 or 3 heteroatoms selected from N, O and S, a 5- to 7- membered heterocyclyl group containing 1, 2 or 3 heteroatoms selected from N, O and S, or a bicyclyl group containing a phenyl group bonded directly to a 5- to 7- membered heterocyclyl group containing at least one heteroatom selected from O, S and N,
the alkyl, haloalkyl, alkoxy, cycloalkyl, adamantyl, phenyl, heteroaryl, heterocyclyl and bicyclyl groups being unsubstituted or substituted by one or more substituents selected from a halogen atom, a hydroxy group, a linear or branched C₁-C₆ alkyl group, a phenyl group, a pyridyl group, a -S(O)₂R₁₀, a -C(O)OR₁₀, a -C(O)-(CH₂)ₙ-R₁₀, a -NR₁₀R₁₁ a -C(O)-(CH₂)ₙ-NR₁₀R₁₁group, a -(C₁C₄ alkyl)-CN group, or a -(C₁C₄ alkyl)-C(O)NR'R" group wherein R' and R" are the same or different and are selected from hydrogen atoms and linear or branched C₁-C₄ alkyl groups; wherein n is 0 or 1, and
R₁₀ and R₁₁, independently represent a hydrogen atom, a cyano group, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, or a bicyclyl group containing a monocyclic C₅-C₆ aryl or heteroaryl group bonded directly to a 5- to 6- membered cycloalkyl or heterocyclyl group, said heteroaryl or heterocyclyl group containing 1, 2 or 3 nitrogen atoms,
the alkyl, haloalkyl, cycloalkyl, phenyl, heteroaryl, heterocyclyl and bicyclyl groups being unsubstituted or substituted by one or more substituents selected from a halogen atom, a hydroxy group, a cyano group, a linear or branched C₁-C₆ alkyl group, a C₁-C₄ haloalkyl group, a phenyl group unsubstituted or substituted by one or more halogen atoms, a 5- to 6-membered monocyclic heteroaryl group containing 1, 2 or 3 nitrogen atoms said heteroaryl group being unsubstituted or substituted by one or more linear or branched C₁-C₃ alkyl groups, or a C₃-C₇ heterocycloalkyl ketone group containing 1, 2 or 3 nitrogen atoms, said heterocycloalkyl ketone group being unsubstituted or substituted by one or more linear or branched C₁-C₃ alkyl groups, or
R₈ represents a -OR₁₃ group wherein R₁₃ represents a C₁-C₃ alkyl group unsubstituted or substituted by one or more phenyl groups, or
R₈ together with the R₅ and the nitrogen atom to which R₅ is bonded form a 5- to 7-membered, saturated heterocyclyl group, which contains, as heteroatoms, one or two nitrogen atoms and which is substituted by a -C(O)-(CH₂)ₙ-R₁₀ group or a -C(O)-(CH₂)ₙ-NR₁₀R₁₁group, wherein n is 0 or 1, and R₁₀ and R₁₁ independently represent a hydrogen atom, a cyano group, a linear or branched C₁-C₃ alkyl group or a C₁-C₄ haloalkyl group;
R₉ represents a hydrogen atom, a halogen atom, a cyano group, a linear or branched C₁-C₄ alkyl group, a C₃-C₇ cycloalkyl group, a 5- to 6-membered monocyclic heteroaryl group containing 1, 2 or 3 nitrogen atoms, a 5- to 7- membered heterocyclyl group containing 1, 2 or 3 nitrogen atoms, the alkyl, cycloalkyl, heteroaryl, and heterocyclyl groups being unsubstituted or substituted by one or more substituents selected from a cyano group or a -OR₁₀ group wherein R₁₀ represents a linear or branched C₁-C₃ alkyl group, or
R₉ represents a -S(O)₂R₁₃ group, a -C(O)OR₁₃ group or a -NR₁₃R₁₄ group, wherein R₁₃ and R₁₄ each independently represent a hydrogen atom or a linear or branched C₁-C₄ alkyl group,
or in the case that two ―CR₉ adjacent groups are present, both ―CR₉ groups and the carbon atoms to which they are bonded optionally form a 5- to 6-membered aryl group,
the aryl group being unsubstituted or substituted by one or more substituents selected from a halogen atom or a linear or branched C₁-C₄ alkyl group,
provided that when m is zero, R₈ is other than an -OR₁₃ group.

17. A compound according to claim 1, which is of formula (I'), 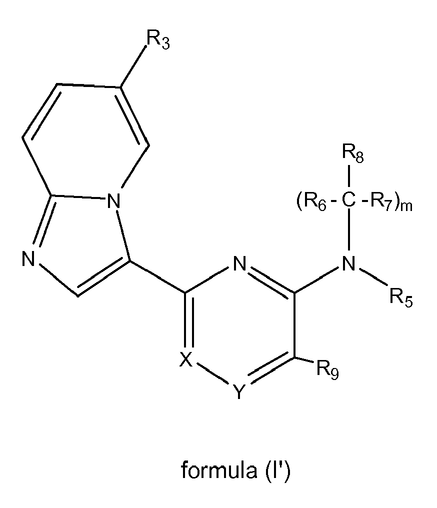 wherein X is a nitrogen atom and Y is a -CR₉ group, or Y is a nitrogen atom and X is a CR₉ group;
R₃ is a hydrogen atom, a halogen atom, a cyano group, a ―C(O)OR' group, a C₃-C₄ cycloalkyl group, a pyridine group, a pyrazole group, or a phenyl group, which phenyl group is unsubstituted or substituted with a halogen atom, wherein R' is a hydrogen atom or a linear or branched C₁-C₄ alkyl group;
R₅ is a hydrogen atom, a methyl group, an ethyl group, a -CH₂-cyclopropyl group, or R₅ together with R₈ and the nitrogen atom to which R₅ is bonded form a 1,4-diazepane-1-yl group, which 1,4-diazepane-1-yl group is unsubstituted or substituted with a -C(O)CH₂CN group;
R₆ and R₇ each independently represent a hydrogen atom or a methyl group;
R₉ is a hydrogen atom, halogen atom, or a methyl, ethyl, piperazine, pyridone or pyridine group, which pyridine group is unsubstituted or substituted with a C₁-C₂ alkoxy group, or when Y is a group R₉, the two adjacent R₉ groups, together with the carbon atoms to which they are attached, may form a benzene ring;
R₈ together with R₅ and the nitrogen atom to which R₅ is bonded form a said 1,4-diazepane-1-yl group, or R₈ is a hydrogen atom, or a linear or branched C₁-C₅ alkyl, cyclohexyl, adamantyl, pyranyl, piperidinyl, chromanyl, -(C₁-C₅ alkyl)-phenyl, -(C₁-C₅ alkyl)-cyclohexyl, -(C₁-C₅ alkyl)-(C₁-C₂ alkoxy), -A-SO₂-R', -A-A', -A-L-C(O)NR'R", -A-L-CN, -A-C(O)-N(C₁-C₂alkyl)-L-CN, -A-C(O)-NR'R", -A-C(O)-A", -A-C(O)-R"', -A-CO₂-R', -A-C(O)-L-A'", -A-C(O)-L-CN, or -A-C(O)-L-O-R' group,
wherein R' and R" are the same or different and each represents a hydrogen atom or linear or a branched C₁-C₄ alkyl group, and R'" represents a linear or branched C₁-C₅ alkyl, C₁-C₂ haloalkyl or C₁-C₄ hydroxyalkyl group, the cyclohexyl, adamantyl, pyranyl, piperidinyl and chromanyl groups being unsubstituted or substituted with a halogen atom, or a hydroxy, linear or branched C₁-C₂ alkyl, or C₁-C₂ alkoxy group, and wherein
L is a linear or branched C₁-C₅ alkylene group,
A is a piperidinyl or pyrrolidinyl group, which is unsubstituted or substituted with a C₁-C₂ alkyl group,
A' is a phenyl or pyridinyl group, which is unsubstituted or substituted with 1 or 2 halogen atoms or CN groups,
A" is a pyrrolidinyl, pyridinyl, pyrazolyl or cyclopropyl group, the pyrrolidinyl, pyridinyl, pyrazolyl and cyclopropyl groups being unsubstituted or substituted with 1 or 2 halogen atoms or cyano groups, and
A'" is an imidazolyl group.

18. A compound according to claim 1 which is one of:
3-(4-{[(1S)-1-phenylethyl]amino}pyrimidin-2-yl)imidazo[1,2-a]pyridine-6-carbonitrile;
3-(4-{[(1 R)-1-phenylethyl]amino}pyrimidin-2-yl)imidazo[1,2-a]pyridine-6-carbonitrile;
3-[4-(Benzylamino)pyrimidin-2-yl]imidazo[1,2-a]pyridine-6-carbonitrile;
3-(4-{[(1 S)-2-methoxy-1-methylethyl]amino}pyrimidin-2-yl)imidazo[1,2-a]pyridine-6-carbonitrile;
3-{4-[(Cyclohexylmethyl)amino]pyrimidin-2-yl}imidazo[1,2-a]pyridine-6-carbonitrile;
3-{4-[(2-Methoxyethyl)amino]pyrimidin-2-yl}imidazo[1,2-*a*]pyridine-6-carbonitrile;
3-{4-[(1-Adamantylmethyl)amino]pyrimidin-2-yl}imidazo[1,2-a]pyridine-6-carbonitrile;
3-4-[(2,2-Dimethylpropyl)amino]pyrimidin-2-yl}imidazo[1,2-a]pyridine-6-carbonitrile;
3-{5-Bromo-4-[(2,2-dimethylpropyl)amino]pyrimidin-2-yl}imidazo[1,2-*a*]pyridine-6-carbonitrile;
3-{4-[(2,2-Dimethylpropyl)amino]-5-piperazin-1-ylpyrimidin-2-yl}imidazo[1,2-a]pyridine-6-carbonitrile;
3-[4-[(2,2-Dimethylpropyl)amino]-5-(2-methoxypyridin-4-yl)pyrimidin-2-yl]imidazo[1,2-a]pyridine-6-carbonitrile;
3-[4-[(2,2-Dimethylpropyl)amino]-5-(2-oxo-1,2-dihydropyridin-4-yl)pyrimidin-2-yl]imidazo[1,2-a]pyridine-6-carbonitrile;
3-{4-[(2,2-Dimethylpropyl)amino]-5-pyridin-3-ylpyrimidin-2 -yl}imidazo[1,2-a]pyridine-6-carbonitrile;
3-{4-[(3-Fluorobenzyl)amino]pyrimidin-2-yl}imidazo[1,2-a]pyridine-6-carbonitrile;
3-{4-[(4-Fluorobenzyl)amino]pyrimidin-2-yl}imidazo[1,2-a]pyridine-6-carbonitrile;
3-{4-[(2-Methylbenzyl)amino]pyrimidin-2-yl}imidazo[1,2-*a*]pyridine-6-carbonitrile; *Tert*-butyl-2-({[2-(6-cyanoimidazo[1,2-*a*]pyridin-3-yl)pyrimidin-4-yl]amino}methyl) piperidine-1-carboxylate;
3-(4-{[(1-Acetylpiperidin-2-yl)methyl]amino}pyrimidin-2-yl)imidazo[1,2-*a*]pyridine-6-carbonitrile;
3-[4-(Tetrahydro-2H-pyran-4-ylamino)pyrimidin-2-yl]imidazo[1,2-a]pyridine-6-carbonitrile;
3-(4-(8-Fluorochroman-4-ylamino)pyrimidin-2-yl)imidazo[1,2-a]pyridine-6-carbonitrile;
3-[4-(Cyclohexylamino)pyrimidin-2-yl]imidazo[1,2-a]pyridine-6-carbonitrile;
3-{4-[(*Trans*-4-hydroxycyclohexyl)amino]pyrimidin-2-yl}imidazo[1,2-*a*]pyridine-6-carbonitrile;
3-{4-[(5-Hydroxy-2-adamantyl)amino]pyrimidin-2-yl}imidazo[1,2-*a*]pyridine-6-carbonitrile;
3-{4-[(5-Hydroxy-2-adamantyl)amino]pyrimidin-2-yl}imidazo[1,2-*a*]pyridine-6-carbonitrile;
2-{4-[(2,2-Dimethylpropyl)amino]quinazolin-2-yl}imidazo[1,2-*a*]pyridine-6-carbonitrile;
3-{4-[Benzyl(methyl)amino]pyrimidin-2-yl}imidazo[1,2-a]pyridine-6-carbonitrile;
3-(4-{[(1 S)-1-phenylethyl]amino}pyrimidin-2-yl)imidazo[1,2-a]pyridine-6-carboxylic acid;
2-(6-Fluoroimidazo[1,2-a]pyridin-3-yl)-N-[(1 S)-1-phenylethyl]pyrimidin-4-amine; *Trans*-4-{[2-(6-fluoroimidazo[1,2-a]pyridin-3-yl)pyrimidin-4-yl]amino}cyclohexanol;
3-(4-{[(2S,7S)-5-hydroxy-2-adamantyl]amino}pyrimidin-2-yl)imidazo[1,2-*a*]pyridine-6-carbonitrile;
N-(2,2-dimethylpropyl)-2-imidazo[1,2-a]pyridin-3-ylpyrimidin-4-amine;
N-(2,2-dimethylpropyl)-2-imidazo[1,2-a]pyridin-3-yl-6-pyridin-3-ylpyrimidin-4-amine;
3-(6-{[(1 S)-1-phenylethyl]amino}pyrazin-2-yl)imidazo[1,2-*a*]pyridine-6-carbonitrile;
3-{6-[(Cyclohexylmethyl)amino]pyrazin-2-yl}imidazo[1,2-*a*]pyridine-6-carbonitrile;
6-(6-Fluoroimidazo[1,2-*a*]pyridin-3-yl)-*N*-[(1S)-1-phenylethyl]pyrazin-2-amine;
6-(6-Chloroimidazo[1,2-a]pyridin-3-yl)-*N*-[(1 S)-1-phenylethyl]pyrazin-2-amine;
(S)-6-(imidazo[1,2-a]pyridin-3-yl)-N-(1-phenylethyl)pyrazin-2-amine;
6-(6-Cyclopropylimidazo[1,2-*a*]pyridin-3-yl)-*N*-[(1 S)-1-phenylethyl]pyrazin-2-amine;
*N*-[(1S)-1-phenylethyl]-6-(6-pyridin-3-ylimidazo[1,2-a]pyridin-3-yl)pyrazin-2-amine;
*N*-[(1S)-1-phenylethyl]-6-(6-pyridin-4-ylimidazo[1,2-a]pyridin-3-yl)pyrazin-2-amine;
*N*-[(1S)-1-phenylethyl]-6-[6-(1 H-pyrazol-4-yl)imidazo[1,2-a]pyridin-3-yl]pyrazin-2-amine;
*N*-[(1S)-1-phenylethyl]-6-(6-phenylimidazo[1,2-*a*]pyridin-3-yl)pyrazin-2-amine;
6-[6-(4-Fluorophenyl)imidazo[1,2-a]pyridin-3-yl]-*N*-[(1 S)-1-phenylethyl]pyrazin-2-amine;
3-(4-{[(3R)-1-(ethylsulfonyl)piperidin-3-yl]amino}pyrimidin-2-yl)imidazo[1,2-*a*]pyridine-6-carbonitrile;
3-(4-{[(3R)-1-(isopropylsulfonyl)piperidin-3-yl]amino}pyrimidin-2-yl)imidazo[1,2-a]pyridine-6-carbonitrile;
3-(4-{[(3*R*)-1-(cyanoacetyl)piperidin-3-yl]amino}pyrimidin-2-yl)imidazo[1,2-*a*]pyridine-6-carbonitrile;
3-(4-{[(3R)-1-propionylpiperidin-3-yl]amino}pyrimidin-2-yl)imidazo[1,2-a]pyridine-6-carbonitrile;
3-[4-({(3R)-1-[(1-cyanocyclopropyl)carbonyl]piperidin-3-yl}amino)pyrimidin-2-yl]imidazo[1,2-a]pyridine-6-carbonitrile;
3-(4-{[(3R)-1-( methoxyacetyl)piperidin-3-yl]amino}pyrimidin-2-yl)imidazo[1,2-*a*]pyridine-6-carbonitrile;
3-(4-{[(3R)-1-(3-hydroxy-3-methylbutanoyl)piperidin-3-yl]amino}pyrimidin-2-yl)imidazo[1,2-a]pyridine-6-carbonitrile;
3-(4-{[(3R)-1-(3,3-dimethylbutanoyl)piperidin-3-yl]amino}pyrimidin-2-yl)imidazo[1,2-a]pyridine-6-carbonitrile;
3-(4-([(3*R*)-1-(1*H*-imidazol-4-ylacetyl)piperidin-3-yl]aminolpyrimidin-2-yl)imidazo[1,2-a]pyridine-6-carbonitrile;
3-[4-({(3*R*)-1-[(5-cyanopyridin-2-yl)carbonyl]piperidin-3-yl}amino)pyrimidin-2-yl] imidazo[1,2-a]pyridine-6-carbonitrile;
3-(4-{[(3*R*)-1-(3,3,3-trifluoropropanoyl)piperidin-3-yl]amino}pyrimidin-2-yl)imidazo[1,2-a]pyridine-6-carbonitrile;
(3R)-3-{[2-(6-cyanoimidazo[1,2-a]pyridin-3-yl)pyrimidin-4-yl]amino}-*N*,*N-*dimethyl piperidine-1-carboxamide;
3-{4-[((3*R*)-1-{[(2*S*,4*S*)-2-cyano-4-fluoropyrrolidin-1-yl]carbonyl}piperidin-3-yl) amino]pyrimidin-2-yl}imidazo[1,2-a]pyridine-6-carbonitrile;
3-(4-{[(3*R*)-1-(5-cyanopyridin-2-yl)piperidin-3-yl]amino}pyrimidin-2-yl)imidazo[1,2-a]pyridine-6-carbonitrile;
3-(4-{[(3*R*)-1-(4-cyano-2-fluorophenyl)piperidin-3-yl]amino}pyrimidin-2-yl)imidazo [1,2-a]pyridine-6-carbonitrile;
3-{4-[[(3*R*)-1-(cyanoacetyl)piperidin-3-yl](methyl)amino]pyrimidin-2-yl}imidazo [1,2-a]pyridine-6-carbonitrile;
3-(4-{[(3*S*)-1-(cyanoacetyl)piperidin-3-yl]amino}pyrimidin-2-yl)imidazo[1,2-*a*]pyridine-6-carbonitrile;
*Cis*-3-(4-{1-(cyanoacetyl)-4-methylpiperidin-3-yl]amino}pyrimidin-2-yl)imidazo[1,2-a]pyridine-6-carbonitrile;
*Cis*-3-(4-{1-(ethylsulfonyl)-4-methylpiperidin-3-yl]amino}pyrimidin-2-yl)imidazo[1,2-a]pyridine-6-carbonitrile;
3-{4-[[1-(Ethylsulfonyl)-4-methylpiperidin-3-yl](methyl)amino]pyrimidin-2-yl} imidazo[1,2-a]pyridine-6-carbonitrile;
3-((3R)-3-{[2-(6-fluoroimidazo[1,2-*a*]pyridin-3-yl)pyrimidin-4-yl]amino}piperidin-1-yl)-3-oxopropanenitrile;
(R)-1-(3-(2-(6-fluoroimidazo[1,2-a]pyridin-3-yl)pyrimidin-4-ylamino)piperidine-1-carbonyl)cyclopropanecarbonitrile;
2-(6-Fluoroimidazo[1,2-a]pyridin-3-yl)-*N*-[(3R)-1-(3,3,3-trifluoropropanoyl)piperidin-3-yl]pyrimidin-4-amine;
2-(6-Fluoroimidazo[1,2-a]pyridin-3-yl)-*N*-[(3R)-1-(1*H*-pyrazol-4ylcarbonyl)piperidin-3-yl]pyrimidin-4-amine;
(3R)-*N*-(cyanomethyl)-3-{[2 -(6-fluoroimidazo[1,2-*a*]pyridin-3-yl)pyrimidin-4-yl]amino}-*N-*methylpiperidine-1-carboxamide;
2-((3R)-3-{[2-(6-fluoroimidazo[1,2-*a*]pyridin-3-yl)pyrimidin-4-yl]amino}piperidin-1-yl)-2-methylpropanamide;
2-(6-Fluoroimidazo[1,2-a]pyridin-3-yl)-*N*-methyl-*N*-[(3R)-1-(3,3,3-trifluoro propanoyl)piperidin-3-yl]pyrimidin-4-amine;
(R)-3-(3-((2-(6-fluoroimidazo[1,2-a]pyridin-3-yl)pyrimidin-4-yl)(methyl)amino)piperidin-1-yl)-3-oxopropanenitrile;
(R)-1-(3-((2-(6-fluoroimidazo[1,2-a]pyridin-3-yl)pyrimidin-4-yl)(methyl)amino)piperidine-1-carbonyl)cyclopropanecarbonitrile;
3-((3R)-3-{ethyl[2-(6-fluoroimidazo[1,2-*a*]pyridin-3-yl)pyrimidin-4-yl]amino}piperidin-1-yl)-3-oxopropanenitrile;
*N*-ethyl-2-(6-fluoroimidazo[1,2-a]pyridin-3-yl)-*N*-[(3R)-1-(3,3,3-trifluoropropanoyl) piperidin-3-yl]pyrimidin-4-amine;
3-((3*R*)-3-{(cyclopropylmethyl)[2-(6-fluoroimidazo[1,2-a]pyridin-3-yl)pyrimidin-4-yl]amino}piperidin-1-yl)-3-oxopropanenitrile;
*N*-(cyclopropylmethyl)-2-(6-fluoroimidazo[1,2-a]pyridin-3-yl)-*N*-[(3R)-1-(3,3,3-trifluoropropanoyl)piperidin-3-yl]pyrimidin-4-amine;
3-((3R)-3-{[5-fluoro-2-(6-fluoroimidazo[1,2-*a*]pyridin-3-yl)pyrimidin-4-yl]amino}piperidin-1-yl)-3-oxopropanenitrile;
5-Fluoro-2-(6-fluoroimidazo[1,2-a]pyridin-3-yl)-*N*-[(3R)-1-(3,3,3-trifluoropropanoyl) piperidin-3-yl]pyrimidin-4-amine;
3-((3R)-3-{[2-(6-fluoroimidazo[1,2-*a*]pyridin-3-yl)-5-methylpyrimidin-4-yl]amino} piperidin-1-yl)-3-oxopropanenitrile;
2-(6-fluoroimidazo[1,2-*a*]pyridin-3-yl)-5-methyl-*N*-[(3*R*)-1-(3,3,3-trifluoropropanoyl) piperidin-3-yl]pyrimidin-4-amine;
3-((3*R*)-3-{[2-(6-fluoroimidazo[1,2-*a*]pyridin-3-yl)pyrimidin-4-yl]amino}pyrrolidin-1-yl)-3-oxopropanenitrile;
3-{4-[4-(cyanoacetyl)-1,4-diazepan-1-yl]pyrimidin-2-yl}imidazo[1,2-*a*]pyridine-6-carbonitrile;
3-((3*R*)-3-{[5-chloro-2-(6-fluoroimidazo[1,2-*a*]pyridin-3-yl)pyrimidin-4-yl]amino} piperidin-1-yl)-3-oxopropanenitrile;
3-((3*R*)-3-{[2-(6-fluoroimidazo[1,2-*a*]pyridin-3-yl)pyrimidin-4-yl]amino}piperidin-1-yl)-2,2-dimethylpropanenitrile;
2-((3*R*)-3-{[2-(6-fluoroimidazo[1,2-*a*]pyridin-3-yl)pyrimidin-4-yl]amino}piperidin-1-yl)acetamide,
and pharmaceutically acceptable salts, solvates or N-oxides thereof.

19. A compound according to any one of claims 1 to 18, for use in the treatment of a pathological condition or disease susceptible to amelioration by inhibition of Janus Kinases.

20. A compound according to claim 19, wherein the pathological condition or disease is selected from myeloproliferative disorders, leukemia, lymphoid malignancies and solid tumors; bone marrow and organ transplant rejection; and immune-mediated diseases.

21. A compound according to claims 19 or 20, wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

22. A pharmaceutical composition comprising a compound as defined in any one of claims 1 to 18 in association with a pharmaceutically acceptable diluent or carrier.

23. Use of a compound as defined in any one of claims 1 to 18, for the manufacture of a medicament for the treatment of a pathological condition or disease as defined in any one of claims 19 to 21.

24. A method for treating a subject afflicted with a pathological condition or disease as defined in any one of claims 19 to 21, which comprises administering to said subject a therapeutically effective amount of a compound as defined in any one of claims 1 to 18, or a pharmaceutical composition as defined in claim 22.

25. A combination product comprising (i) a compound as defined in any one of claims 1 to 18; and (ii) another compound selected from:
a) Dyhydrofolate reductase inhibitors, such as Methotrexate or CH-1504,
b) DHODH inhibitors such as leflunomide, teriflunomide, or the compounds described in the International Patent Application Nos. WO2008/077639 and WO2009021696,
c) Immunomodulators such as Glatiramer acetate (Copaxone), Laquinimod or Imiquimod,
d) Inhibitors of DNA synthesis and repair, such as Mitoxantrone or Cladribine,
e) Anti-alpha 4 integrin antibodies, such as Natalizumab (Tysabri),
f) Alpha 4 integrin antagonists such as R-1295 , TBC-4746, CDP-323, ELND-002, Firategrast or TMC-2003,
*g)* Corticoids and glucocorticoids such as prednisone or methylprednisolone, fluticasone, mometasone, or beta-metasone,
*h)* Fumaric acid esters, such as *BG-12,*
i) Anti-TNF alpha antibodies, such as Infliximab, Adalimumab, or Certolizumab pegol,
j) Soluble TNF alpha receptors such as Ethanercept,
k) Anti-CD20 monoclonal antibodies such as Rituximab, Ocrelizumab Ofatumumab or TRU-015,
l) Anti-CD52 such as alemtuzumab,
m) Anti-CD25 such as daclizumab,
n) Anti-CD88, such as eculizumab or pexilizumab,
o) Anti-IL12R /IL23R, such as ustekinumab,
p) Calcineurin inhibitors such as cyclosporine A or tacrolimus,
q) IMPDH inhibitors, such as mycophenolate mophetyl,
r) Cannabinoid receptor agonists such as Sativex,
s) Chemokine CCR1 antagonists such as MLN-3897 or PS-031291,
t) Chemokine CCR2 antagonists such as INCB-8696,
u) NF-kappaB activation inhibitors such as MLN-0415,
v) S1 P receptor agonists such as fingolimod, BAF-312, or ACT128800,
w) S1P liase inhibitors such as LX2931,
x) Syk inhibitors, such as R-112,
y) PKC inhibitors, such as NVP-AEB071,
z) M3 antagonist such as tiotropium or aclidinium,
aa) Long-acting beta adrenergic agonist such as formoterol,
bb) Vitamin D derivatives like calcipotriol (Daivonex), cc) Phosphosdiesterase IV inhibitors such as roflumilast or GRC-4039, dd) p38 Inhibitors such as ARRY-797,
ee) MEK inhibitors, such as ARRY-142886 or ARRY-438162,
ff) PI3Kδγ inhibitors,
gg) Interferons comprising Interferon beta 1 a such as Avonex from
Biogen Idec, CinnoVex from CinnaGen and Rebif from EMD Serono, and Interferon beta 1b such as Betaferon from Schering and Betaseron from Berlex, and
hh) Interferon alpha such as Sumiferon MP,
for simultaneous, separate or sequential use in the treatment of the human or animal body.
